(19) Europäisches Patentamt
European Patent Office
Office européen des brevets

(11) EP 2 552 456 B1

(12) **EUROPEAN PATENT SPECIFICATION**

(45) Date of publication and mention of the grant of the patent:
**23.08.2017 Bulletin 2017/34**

(21) Application number: **11763336.2**

(22) Date of filing: **29.03.2011**

(51) Int Cl.:
***A61K 31/7088*** *(2006.01)* ***C07H 21/02*** *(2006.01)*
***A61K 48/00*** *(2006.01)* ***A61K 31/7115*** *(2006.01)*
***A61K 31/713*** *(2006.01)* ***C12N 15/113*** *(2010.01)*
***A61P 27/02*** *(2006.01)*

(86) International application number:
**PCT/US2011/030392**

(87) International publication number:
**WO 2011/123468 (06.10.2011 Gazette 2011/40)**

(54) **SIRNA THERAPY FOR TRANSTHYRETIN (TTR) RELATED OCULAR AMYLOIDOSIS**

SIRNA-THERAPIE FÜR DURCH TRANSTHYRETIN (TTR) VERMITTELTE AUGENAMYLOIDOSE

THÉRAPIE SIARN POUR AMYLOSE OCULAIRE LIÉE À LA TRANSTHYRÉTINE (TTR)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2010 US 318702 P**
**29.03.2010 US 318704 P**

(43) Date of publication of application:
**06.02.2013 Bulletin 2013/06**

(73) Proprietors:
- **Alnylam Pharmaceuticals, Inc.**
  **Cambridge, MA 02142 (US)**
- **Kumamoto University**
  **Kumamoto-shi**
  **Kumamoto 860-8555 (JP)**
- **Ando, Yukio**
  **Cambridge, MA 02142 (US)**
- **Jono, Hirofumi**
  **Cambridge, MA 02142 (US)**

(72) Inventors:
- **ANDO, Yukio**
  **Cambridge, MA 02142 (US)**
- **JONO, Hirofumi**
  **Cambridge, MA 02142 (US)**
- **ALVAREZ, Rene**
  **Cambridge, MA 02142 (US)**
- **SAH, Dinah, Wen-Yee**
  **Cambridge, MA 02142 (US)**

(74) Representative: **Vossius & Partner**
**Patentanwälte Rechtsanwälte mbB**
**Siebertstrasse 3**
**81675 München (DE)**

(56) References cited:
**EP-A1- 2 496 238**
**EP-A2- 2 344 639**
**US-A1- 2006 135 460**
**US-A1- 2009 082 300**
**US-A1- 2009 082 300**

- **SEKIJIMA Y ET AL: "Pathogenesis of and Therapeutic Strategies to Ameliorate the Transthyretin Amyloidoses", CURRENT PHARMACEUTICAL DESIGN, BENTHAM SCIENCE PUBLISHERS LTD, NL, vol. 14, no. 30, 1 January 2008 (2008-01-01), pages 3219-3230, XP008121491, ISSN: 1873-4286**
- **KUROSAWA T ET AL: "Selective silencing of a mutant transthyretin allele by small interfering RNAs", BIOCHEMICAL AND BIOPHYSICAL RESEARCH COMMUNICATIONS, ACADEMIC PRESS INC. ORLANDO, FL, US, vol. 337, no. 3, 25 November 2005 (2005-11-25), pages 1012-1018, XP027218514, ISSN: 0006-291X, DOI: 10.1016/J.BBRC.2005.09.142 [retrieved on 2005-10-18]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 552 456 B1

- TASAKI M ET AL: "siRNA therapy for TTR-related ocular amyloidosis", AMYLOID, vol. 17, no. Suppl. 1, April 2010 (2010-04), pages 52-53, XP009172275, & 12TH INTERNATIONAL SYMPOSIUM ON AMYLOIDOSIS FROM MOLECULAR MECHANISMS TOWARD THE CURE OF SYSTEMIC AM; ROME, ITALY; APRIL 18 -21, 2010 ISSN: 1350-6129
- ALMEIDA M R ET AL: "Small transthyretin (TTR) ligands as possible therapeutic agents in TTR amyloidoses", CURRENT DRUG TARGETS: CNS AND NEUROLOGICAL DISORDERS 200510 NL, vol. 4, no. 5, October 2005 (2005-10), pages 587-596, XP009172276, ISSN: 1568-007X
- UEDA ET AL.: 'A transgenic rat with the human ATTR V30M: a novel tool for analyses of ATTR metabolisms.' BIOCHEM. BIOPHYS. RES. COMMUN. vol. 352, no. 2, 12 January 2007, pages 299 - 304, XP005724505
- DATABASE GENBANK [Online] 14 February 2010 CENDRON ET AL.: 'Rattus norvegicus transthyretin (Ttr), mRNA.', XP008158084 Database accession no. NM_012681.
- HARA ET AL.: 'Impact of liver transplantation on transthyretin-related ocular amyloidosis in Japanese patients.' ARCH. OPHTHAMOL. vol. 128, no. 2, February 2010, pages 206 - 210, XP008158127

## Description

### Field of the Invention

The present invention is defined by the claims. In general trems the present invention relates to the treatment of TTR related ocular amyloidosis with siRNA.

### Background of the Invention

Transthyretin (TTR) is a secreted thyroid hormone-binding protein. TTR binds and transports retinol binding protein (RBP)/ Vitamin A, and serum thyroxine (T4) in plasma and cerebrospinal fluid.

Both normal-sequence TTR and variant-sequence TTR cause amyloidosis. Normal-sequence TTR causes cardiac amyloidosis in people who are elderly and is termed senile systemic amyloidosis (SSA) (also called senile cardiac amyloidosis (SCA)). SSA often is accompanied by microscopic deposits in many other organs. TTR mutations accelerate the process of TTR amyloid formation and are the most important risk factor for the development of clinically significant TTR amyloidosis (also called ATTR (amyloidosis-transthyretin type)). More than 85 amyloidogenic TTR variants are known to cause systemic familial amyloidosis. The liver is the major site of TTR expression. Other significant sites of expression include the choroid plexus, retina and pancreas.

TTR amyloidosis manifests in various forms. When the peripheral nervous system is affected more prominently, the disease is termed familial amyloidotic polyneuropathy (FAP). When the heart is primarily involved but the nervous system is not, the disease is called familial amyloidotic cardiomyopathy (FAC). A third major type of TTR amyloidosis is called leptomeningeal/CNS (Central Nervous System) amyloidosis.

Double-stranded RNA molecules (dsRNA) have been shown to block gene expression in a highly conserved regulatory mechanism known as RNA interference (RNAi). WO 99/32619 (Fire et al.) disclosed the use of a dsRNA of at least 25 nucleotides in length to inhibit the expression of genes in *C. elegans.* dsRNA has also been shown to degrade target RNA in other organisms, including plants (see, *e.g.*, WO 99/53050, Waterhouse et al.; and WO 99/61631, Heifetz et al.), *Drosophila* (see, *e.g.*, Yang, D., et al., Curr. Biol. (2000) 10:1191-1200), and mammals (see WO 00/44895, Limmer; and DE 101 00 586.5, Kreutzer et al.).

U.S. 20070207974 discloses functional and hyperfunctional siRNAs. U.S. 20090082300 discloses antisense molecules directed against TTR. U.S. Pat. No. 7,250,496 discloses microRNAs directed against TTR.

### Summary

The present invention is defined by the claims. In more detail, the invention provides a dsRNA for use in a method of treating of TTR-related ocular amyloidosis by reducing TTR expression in a retinal pigment epithelium of a subject, wherein the dsRNA is to be administered to the retina of the subject, and wherein the dsRNA has

(i) the sense strand GGAuuucAuGuAAccAAGAdTdT and the antisense strand UCUUGGUuAcAUGAAAUCCdTdT,

(ii) the sense strand cAGuGuucuuGcucuAuAAdTdT and the antisense strand UuAuAGAGcAAGAAcACUGdTdT, or

(iii) the sense strand cAGuGuucuuGcucuAuAAdTdTL10 and the antisense strand UuAuAGAGcAAgAAcACUGdTdT,

wherein c is 2'-O-methylcytidine-3'-phosphate, u is 2'-O-methyluridine-3'-phosphate, dT is 2'-deoxythymidine-3'-phosphate and L10 is a conjugated cholesterol. The invention furthermore provides a dsRNA for use in a method of treating or preventing TTR-related ocular amyloidosis, wherein the dsRNA is to be administered to a patient in need of such treatment or prevention and to the retina of said patient, and wherein the dsRNA is as defined in item (i) herein above. The invention also relates to a dsRNA for use in a method of treating or preventing TTR-related ocular amyloidosis in a human, wherein the human has been diagnosed as having TTR-related ocular amyloidosis or as having a risk for developing TTR-related ocular amyloidosis and the dsRNA is to be administered to the retina of said human, and wherein the dsRNA is as defined in item (i) herein above. Finally, the the invention provides an *in vitro* method for inhibiting TTR expression in a retinal epithelium cell, the method comprising: (a) introducing into the retinal epithelium cell a dsRNA, wherein the dsRNA is as defined in item (i) or item (ii) of claim 1; and (b) maintaining the cell produced in step (a) for a time sufficient to obtain degradation of the mRNA transcript of a TTR gene, thereby inhibiting expression of the TTR gene in the cell.

Also described herein is a method for reducing transthyretin (TTR) expression in the retinal pigment epithelium (RPE) of a subject by administering a sufficient amount of a double stranded ribonucleic acid (dsRNA) to the retina of the subject. The dsRNA may be AD-18324 or AD-18534. The dsRNA can be conjugated to, e.g., cholesterol.

The subject may be a human. The subject may be a human in need of treatment for TTR-related ocular amyloidosis. The dsRNA may be AD-18324. The method may result in reduced TTR expression in the RPE. The method may result in reduced TTR mRNA expression by at least 40% or by at least 60% compared to a control. The administration of the dsRNA may not result in an inflammatory response in the human as measured by IL-6 or TNF-alpha levels.

The subject may be a human possessing a ATTR (amyloidogenic transthyretin) V30M gene in need of treatment for TTR-related ocular amyloidosis. The dsRNA administered to the ATTR V30M subject may be AD-18324. The method may result in a reduction of V30M TTR expression in the retinal pigment epithelium. The method may result in reduction of V30M TTR mRNA expression by at least 60% compared to a control. The administration of the dsRNA may not result in an inflammatory response in the human possessing a ATTR V30M gene as measured by IL-6 or TNF-alpha levels.

The subject may be a Dark Agouti (DA) ratThe dsRNA that is administered to the DA rat may be AD-18534. The method may result in a reduction of TTR expression in the retinal pigment epithelium. The method may resuls in reduction of TTR mRNA expression in the DA rat by at least 60% compared to a control. The administration of the dsRNA may not result in an inflammatory response in the DA rat as measured by IL-6 or TNF-alpha levels.

The subject may be a transgenic rat possessing a human ATTR (amyloidogenic transthyretin) V30M gene. The dsRNA administered to the ATTR V30M transgenic rat may be AD-18324. The method may result in a reduction of TTR expression in the retinal pigment epithelium. The method may result in reduction of TTR mRNA expression by at least 60% compared to a control. The administration of the dsRNA may not result in an inflammatory response in the ATTR V30M Tg rat as measured by IL-6 or TNF-alpha levels.

Also described herein is a method for treating, preventing or managing TTR-related ocular amyloidosis by administering to a patient in need of such treatment, prevention or management a therapeutically or prophylactically effective amount of AD-18324 to the retina of the patient.

Also described herein is a method of treating a human, which includes identifying a human diagnosed as having TTR-related ocular amyloidosis or at risk for developing TTR-related ocular amyloidosis and administering to the human a therapeutically or prophylactically effective amount of AD-18324 to the retina of the human.

Also described herein is a method for inhibiting TTR expression in a retinal epithelium cell, wherein the method comprises (a) introducing into the retinal epithelium cell a dsRNA, wherein the dsRNA is AD-18324 or AD-18534, and (b) maintaining the cell produced in step (a) for a time sufficient to obtain degradation of the mRNA transcript of a TTR gene, thereby inhibiting expression of the TTR gene in the cell. The dsRNA administered in this method may be AD-18324. The retinal epithelium cell may be a human retinal pigment epithelium transgenic cell. The method may result in inhibition of TTR expression by at least 10%, 40%, or at least 60%. Introducing the dsRNA into the retinal epithelium cell may not result in an inflammatory response as measured by IL-6 or TNF-alpha levels.

The details of one or more embodiments of the invention are set forth in the description below. Other features, objects, and advantages of the invention will be apparent from the description and the drawings, and from the claims.

## Description of the Drawings

FIG. 1 is a graph of TNFalpha and IFNalpha levels in cultured human PBMCs following transfection with TTR siRNAs.

FIG. 2A and 2B are dose response curves for AD-18324 and AD-18328, respectively, in HepG2 cells.

FIG. 3 is a dose response curve for AD-18246 in HepG2 cells.

FIG. 4A and FIG. 4B show inhibition of liver mRNA and plasma protein levels, respectively, in transgenic H129-mTTR-KO/iNOS-KO/hTTR mice by an intravenous bolus administration of TTR-dsRNA (AD-18324, AD-18328 and AD-18246) formulated in LNP01.

FIG. 5 is a graph summarizing the measurements of TTR mRNA levels in livers of non-human primates following 15-minute intravenous infusion of TTR-dsRNA (AD-18324 and AD-18328) formulated in SNALP.

FIG. 6A and FIG. 6B show inhibition of human V30M TTR liver mRNA and serum protein levels, respectively, in transgenic mice by an intravenous bolus administration of SNALP-18328. Group means were determined, normalized to the PBS control group, and then plotted. Error bars represent standard deviations. The percentage reduction of the group mean, relative to PBS, is indicated for the SNALP-1955 and SNALP-18328 groups. (*** $p < 0.001$, One-way ANOVA, with Dunn's post-hoc test).

FIG. 7A and FIG. 7B show the durability of reduction of human V30M TTR liver mRNA and serum protein levels,

respectively, in transgenic mice over 22 days following a single intravenous bolus administration of SNALP-18328. Group means were determined. TTR/GAPDH mRNA levels were normalized to day 0 levels and plotted. The percent reduction of normalized TTR mRNA levels relative to SNALP-1955 for each time point were calculated and are indicated for the SNALP-18328 groups. (*** p< 0.001, One-way ANOVA, with Dunn's post-hoc test).

FIG. 8 shows the timecourse of TTR serum protein levels in non-human primates over 14 days following a single 15-minute intravenous infusion of SNALP-18328.

FIG. 9 shows reduction of TTR-immunoreactivity in various tissues of human V30M TTR/HSF-1 knock-out mice following intravenous bolus administration of SNALP-18328. E, esophagus; S, stomach; I1, intestine/duodenum; I4, intestine/colon; N, nerve; D, dorsal root ganglia.

FIG. 10 shows the measurements of TTR mRNA levels in livers of non-human primates following 15-minute intravenous infusion of XTC-SNALP-18328.

FIGs. 11A and 11B show the measurements of TTR mRNA and serum protein levels, respectively, in livers of non-human primates following 15-minute intravenous infusion of LNP09-18328 or LNP11-18328. FIG.11C shows the timecourse ofTTR serum protein levels over 28 days following a 15-minute intravenous infusion of 0.3mg/kg LNP09-18328, as compared to the PBS control group.

FIG. 12 shows the sequence of human TTR mRNA (Ref. Seq. N_000371.3, SEQ ID NO:1331).

FIGs. 13A and 13B are the sequences of human and rat TTR mRNA, respectively. FIG. 13A is the sequence of human TTR mRNA (Ref. Seq. NM_000371.2, SEQ ID NO:1329). FIG. 13B is the sequence of rat TTR mRNA (Ref. Seq. N_012681.1, SEQ ID NO:1330).

FIG. 14 shows the nucleotide alignment of NM_000371.3, NM_000371.2, and AD-18328.

FIG. 15 illustrates symptoms and mutations in TTR associated with familial amyloidotic neuropathy, familial amyloidotic cardiomyopathy and CNS amyloidosis.

FIG. 16 shows reduction of TTR mRNA levels in the liver with SNALP-18534 with different infusion durations. Groups of animals (n=4/group) were administered 1 mg/kg SNALP-18534 via a 15-minute, or 1, 2, or 3 hour infusion. Forty-eight hours later, rats were euthanized and livers harvested. TTR and GAPDH mRNA levels were measured from liver lysates using the Quantigene bDNA assay. The ratio of TTR to GAPDH mRNA levels was calculated for each animal. Group means were determined and normalized to a PBS control group, and then plotted. Error bars represent standard deviations. (*** p < 0.001, One-way ANOVA with Bonferroni post- hoc test, relative to PBS).

FIG 17 shows the measurements of TTR mRNA levels in livers of rats following 15-minute intravenous infusion of LNP07-18534 or LNP08-18534.

FIG. 18 shows *in vivo* inhibition of endogenous TTR mRNA levels in livers of Sprague-Dawley Rats following a 15-min IV infusion of LNP09-18534 or LNP11-18534. Groups of animals (n=4/group) were intravenously administered 0.01, 0.03, 0.1, or 0.3 mg/kg LNP09-18534, LNP-11-18534; or PBS via a 15-minute infusion. Forty-eight hours later, animals were euthanized and livers harvested. TTR and GAPDH mRNA levels were measured from liver biopsy lysates using the Quantigene bDNA assay. The ratio of TTR to GAPDH mRNA levels was calculated for each animal. Group means were determined, normalized to the PBS control group, and then plotted. Error bars represent standard deviations.

FIG. 19 shows the efficacy of LNP12 formulated siRNA targeting TTR in non-human primates. Data points represent group mean $\pm$ s.d.

FIG. 20 is a graph with results from a GLP study in NHP illustrating the durability of mRNA suppression by ALN-TTR01.

FIG. 21 is a graph illustrating regression of TTR deposits in various tissues of mature animals (hV30M TTR/HSF-1 knock-out mice) following intravenous bolus administration of SNALP-18328 (ALN-TTR01).

FIG. 22 is a graph showing the effects of human TTR siRNA AD-18324 on TTR mRNA expression in ARPE 19 cells.

AD-18324 was compared with rat TTR siRNA AD-18534. The human TTR mRNA expression was calculated relative to human GAPDH expression.

FIG. 23 is a graph showing the effect of AD-18534 on TTR mRNA expression in retinal pigment epithelium cells of Dark Agouti (DA) rats. AD-18534 was compared to a control siRNA group, a saline group, and no treatment group. Endogenous rat TTR mRNA expression was calculated relative to rat GAPDH expression.

FIG. 24 is a graph showing the effect of AD-18324 on ATTR mRNA expression in retinal pigment epithelium cells in ATTR V30M transgenic rats. AD-18324 was compared to a control siRNA group, a saline group, and no treatment group. Human TTR mRNA expression was calculated relative to rat GAPDH expression.

FIG. 25 is a Western blot showing the effect of human TTR siRNA on human TTR protein expression in retinal pigment epithelial cells in ATTR V30M transgenic rats compared to a control siRNA.

FIG. 26 is a graph showing the effect of AD-23043 (cho-TTR siRNA) on rat TTR mRNA expression in retinal pigment epithelium cells of Dark Agouti (DA) rats 14 and 21 days after administration. Endogenous rat TTR mRNA expression was calculated relative to rat GAPDH expression.

FIG. 27 is a graph showing the effect of AD-23043 (cho-TTR siRNA) on rat TTR mRNA expression in retinal pigment epithelium cells of Dark Agouti (DA) rats 21 days after administration. Endogenous rat TTR mRNA expression was calculated relative to rat GAPDH expression.

## Detailed Description

The present invention is defined by the claims. In general terms the invention provides dsRNAs and methods of using the dsRNAs for inhibiting the expression of a TTR gene in a cell or a mammal where the dsRNA targets a TTR gene. Also provided herein are compositions and methods for treating pathological conditions and diseases, such as a TTR amyloidosis, in a mammal caused by the expression of a TTR gene. dsRNA directs the sequence-specific degradation of mRNA through a process known as RNA interference (RNAi).

The dsRNAs of the compositions described herein include an RNA strand (the antisense strand) having a region which is less than 30 nucleotides in length, generally 19-24 nucleotides in length, and is substantially complementary to at least part of an mRNA transcript of a TTR gene. The use of these dsRNAs enables the targeted degradation of mRNAs of genes that are implicated in pathologies associated with TTR expression in mammals. Very low dosages of TTR dsRNAs in particular can specifically and efficiently mediate RNAi, resulting in significant inhibition of expression of a TTR gene. Using cell-based assays, the present inventors have demonstrated that dsRNAs targeting TTR can specifically and efficiently mediate RNAi, resulting in significant inhibition of expression of a TTR gene. Thus, methods and compositions including these dsRNAs are useful for treating pathological processes that can be mediated by down regulating TTR, such as in the treatment of a liver disorder or a TTR amyloidosis, *e.g.*, FAP.

The methods and compositions containing a TTR dsRNA are useful for treating pathological processes mediated by TTR expression, such as a TTR amyloidosis. A method of treating a disorder mediated by TTR expression may include administering to a human in need of such treatment a therapeutically effective amount of a dsRNA targeted to TTR. A dsRNA may be administered to the human at about 0.01, 0.1, 0.5, 1.0, 2, 2.5, 3, 4, 5, 6, 7, 8, 9, 10, 11, 12, 13, 14, 15, 16, 17, 18, 19, 20, 21, 22, 23, 24, or 25 mg/kg.

The following detailed description discloses how to make and use the compositions containing dsRNAs to inhibit the expression of a TTR gene, as well as compositions and methods for treating diseases and disorders caused by the expression of this gene. The pharmaceutical compositions described herein include a dsRNA having an antisense strand comprising a region of complementarity which is less than 30 nucleotides in length, generally 19-24 nucleotides in length, and is substantially complementary to at least part of an RNA transcript of a TTR gene, together with a pharmaceutically acceptable carrier. The compositions described herein also include a dsRNA having an antisense strand having a region of complementarity which is less than 30 nucleotides in length, generally 19-24 nucleotides in length, and is substantially complementary to at least part of an RNA transcript of a TTR gene.

The sense strand of a dsRNA can include 15, 16, 17, 18, 19, 20, 21, or more contiguous nucleotides of any of the sense strands disclosed herein. The antisense strand of a dsRNA can include 15, 16, 17, 18, 19, 20, 21, or more contiguous nucleotides of any of the antisense strands disclosed herein.

A dsRNA can include at least 1, 2, 3, 4, 5, 6, 7, 8, 9, 10, or more modified nucleotides. A modified nucleotide can include a 2'-O-methyl modified nucleotide, a nucleotide comprising a 5'-phosphorothioate group, and/or a terminal nucleotide linked to a cholesteryl derivative or dodecanoic acid bisdecylamide group. A modified nucleotide can include a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and/or a non-natural base comprising nucleotide.

The region of complementary of a dsRNA may be at least 10, 11, 12, 13, 14, 15, 16, 17, 18, 19, 20, 21, or more nucleotides in length. The region of complementary may include 10, 11, 12, 13, 14, 15, 16, 17, 18,19, 20, 21, or more contiguous nucleotides of SEQ ID NO:169.

Each strand of a dsRNA may be 15, 16, 17, 18, 19, 20, 21, 22, 23, 24, 25, 26, 27, 28, 29, 30 or more nucleotides in length. The dsRNA may include a sense strand, or 10, 11, 12, 13, 14, 15, 16, 17, 18, 19, 20, or 21 nucleotide fragment thereof, selected from Tables 3A, 3B, 4, 6A, 6B, 7, and 16, and an antisense strand, or 10, 11, 12, 13, 14, 15, 16, 17, 18, 19, 20, or 21 nucleotide fragment thereof, selected from Tables 3A, 3B, 4, 6A, 6B, 7, and 16.

Administration of a dsRNA to a cell may result in about 40%, 45%, 50%, 55%, 60%, 65%, 70%, 75%, 80%, 90%, 95% or more inhibition of TTR mRNA expression as measured by a real time PCR assay. Administration of a dsRNA to a cell may result in about 40% to 45%, 45% to 50%, 50% to 55%, 55% to 60%, 60% to 65%, 65% to 70%, 70% to 75%, 75% to 80%, 80% to 85%, 85% to 90%, 90% to 95% or more inhibition of TTR mRNA expression as measured by a real time PCR assay. Administration of a dsRNA to a cell may result in about 40%, 45%, 50%, 55%, 60%, 65%, 70%, 75%, 80%, 90%, 95% or more inhibition of TTR mRNA expression as measured by a branched DNA assay. Administration of a dsRNA to a cell may result in about 40% to 45%, 45% to 50%, 50% to 55%, 55% to 60%, 60% to 65%, 65% to 70%, 70% to 75%, 75% to 80%, 80% to 85%, 85% to 90%, 90% to 95% or more inhibition of TTR mRNA expression as measured by a branched DNA assay.

A dsRNA may have an IC50 of less than 0.01pM, 0.1pM, 1pM, 5pM, 10 pM, 100pM, or 1000pM. A dsRNA may have an ED50 of about 0.01, 0.1, 1, 5, or 10 mg/kg.

Administration of a dsRNA can reduce TTR mRNA by about 40%, 45%, 50%, 55%, 60%, 65%, 70%, 75%, 80%, 90%, 95% or more in cynomolgus monkeys. Administration of a dsRNA may reduce liver TTR mRNA levels by about 40%, 45%, 50%, 55%, 60%, 65%, 70%, 75%, 80%, 90%, 95% or more or serum TTR protein levels by about 40%, 45%, 50%, 55%, 60%, 65%, 70%, 75%, 80%, 90%, 95% or more. Administration of a dsRNA may reduce liver TTR mRNA levels and/or serum TTR protein levels up to 1, 2, 3, 4, 5, 6, 7, 8, 9, 10, 11, 12, 13, 14, 15, 16, 17, 18, 19, 20, 21, 22, 23, 24, 25, or more days.

A dsRNA may be formulated in a LNP formulation and may reduce TTR mRNA levels by about 40%, 45%, 50%, 55%, 60%, 65%, 70%, 75%, 80%, 90%, 95% or more at a dose of 0.1, 0.2, 0.3, 0.4, 0.5, 0.6, 0.7, 0.8, 0.9, or 1 mg/kg, relative to a PBC control group. A dsRNA may be formulated in a LNP formulation and may reduce TTR protein levels about 40%, 45%, 50%, 55%, 60%, 65%, 70%, 75%, 80%, 90%, 95% or more relative to a PBC control group as measured by a western blot. A dsRNA may suppresses serum TTR protein levels up to day 1, 2, 3, 4, 5, 6, 7, 8, 9, 10, 11, 12, 13, 14, 15, 16, 17, 18, 19, 20, 21, 22, 23, 24, or 25 post-treatment when administered to a subject in need thereof at 1, 2, 3, 4, 5, 6, 7, 8, 9, 10, 11, 12, 13, 14, 15, 16, 17, 18, 19, 20, 21, 22, 23, 24, or 25 mg/kg.

Accordingly, in some aspects, pharmaceutical compositions containing a TTR dsRNA and a pharmaceutically acceptable carrier, methods of using the compositions to inhibit expression of a TTR gene, and methods of using the pharmaceutical compositions to treat diseases caused by expression of a TTR gene are described herein.

## I. Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A" and "U" each generally stand for a nucleotide that contains guanine, cytosine, adenine, and uracil as a base, respectively. "T" and "dT" are used interchangeably herein and refer to a deoxyribonucleotide wherein the nucleobase is thymine, e.g., deoxyribothymine. However, it will be understood that the term "ribonucleotide" or "nucleotide" or "deoxyribonucleotide" can also refer to a modified nucleotide, as further detailed below, or a surrogate replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine may be replaced in the nucleotide sequences described herein by a nucleotide containing, for example, inosine. Sequences comprising such replacement moieties are also described herein.

As used herein, "transthyretin" ("TTR") refers to a gene in a cell. TTR is also known as ATTR, HsT2651, PALB, prealbumin, TBPA, and transthyretin (prealbumin, amyloidosis type I). The sequence of a human TTR mRNA transcript can be found at N_000371. The sequence of mouse TTR mRNA can be found at N_013697.2, and the sequence of rat TTR mRNA can be found at NM_012681.1.

As used herein, "target sequence" refers to a contiguous portion of the nucleotide sequence of an mRNA molecule formed during the transcription of a TTR gene, including mRNA that is a product of RNA processing of a primary

transcription product.

As used herein, the term "strand comprising a sequence" refers to an oligonucleotide comprising a chain of nucleotides that is described by the sequence referred to using the standard nucleotide nomenclature.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with an oligonucleotide or polynucleotide comprising the second nucleotide sequence, as will be understood by the skilled person. Such conditions can, for example, be stringent conditions, where stringent conditions may include: 400 mM NaCl, 40 mM PIPES pH 6.4, 1 mM EDTA, 50°C or 70°C for 12-16 hours followed by washing. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

This includes base-pairing of the oligonucleotide or polynucleotide comprising the first nucleotide sequence to the oligonucleotide or polynucleotide comprising the second nucleotide sequence over the entire length of the first and second nucleotide sequence. Such sequences can be referred to as "fully complementary" with respect to each other herein. However, where a first sequence is referred to as "substantially complementary" with respect to a second sequence herein, the two sequences can be fully complementary, or they may form one or more, but generally not more than 4, 3 or 2 mismatched base pairs upon hybridization, while retaining the ability to hybridize under the conditions most relevant to their ultimate application. However, where two oligonucleotides are designed to form, upon hybridization, one or more single stranded overhangs, such overhangs shall not be regarded as mismatches with regard to the determination of complementarity. For example, a dsRNA comprising one oligonucleotide 21 nucleotides in length and another oligonucleotide 23 nucleotides in length, wherein the longer oligonucleotide comprises a sequence of 21 nucleotides that is fully complementary to the shorter oligonucleotide, may yet be referred to as "fully complementary" for the purposes described herein.

"Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled. Such non-Watson-Crick base pairs includes, but not limited to, G:U Wobble or Hoogstein base pairing.

The terms "complementary," "fully complementary" and "substantially complementary" herein may be used with respect to the base matching between the sense strand and the antisense strand of a dsRNA, or between the antisense strand of a dsRNA and a target sequence, as will be understood from the context of their use.

As used herein, a polynucleotide that is "substantially complementary to at least part of" a messenger RNA (mRNA) refers to a polynucleotide that is substantially complementary to a contiguous portion of the mRNA of interest (e.g., an mRNA encoding TTR) including a 5' UTR, an open reading frame (ORF), or a 3' UTR. For example, a polynucleotide is complementary to at least a part of a TTR mRNA if the sequence is substantially complementary to a non-interrupted portion of an mRNA encoding TTR.

The term "double-stranded RNA" or "dsRNA," as used herein, refers to a complex of ribonucleic acid molecules, having a duplex structure comprising two anti-parallel and substantially complementary, as defined above, nucleic acid strands. In general, the majority of nucleotides of each strand are ribonucleotides, but as described in detail herein, each or both strands can also include at least one non-ribonucleotide, e.g., a deoxyribonucleotide and/or a modified nucleotide. In addition, as used in this specification, "dsRNA" may include chemical modifications to ribonucleotides, including substantial modifications at multiple nucleotides and including all types of modifications disclosed herein or known in the art. Any such modifications, as used in an siRNA type molecule, are encompassed by "dsRNA" for the purposes of this specification and claims.

The two strands forming the duplex structure may be different portions of one larger RNA molecule, or they may be separate RNA molecules. Where the two strands are part of one larger molecule, and therefore are connected by an uninterrupted chain of nucleotides between the 3'-end of one strand and the 5'-end of the respective other strand forming the duplex structure, the connecting RNA chain is referred to as a "hairpin loop." Where the two strands are connected covalently by means other than an uninterrupted chain of nucleotides between the 3'-end of one strand and the 5'-end of the respective other strand forming the duplex structure, the connecting structure is referred to as a "linker." The RNA strands may have the same or a different number of nucleotides. The maximum number of base pairs is the number of nucleotides in the shortest strand of the dsRNA minus any overhangs that are present in the duplex. In addition to the duplex structure, a dsRNA may comprise one or more nucleotide overhangs. The term "siRNA" is also used herein to refer to a dsRNA as described above.

As used herein, a "nucleotide overhang" refers to the unpaired nucleotide or nucleotides that protrude from the duplex structure of a dsRNA when a 3'-end of one strand of the dsRNA extends beyond the 5'-end of the other strand, or vice versa. "Blunt" or "blunt end" means that there are no unpaired nucleotides at that end of the dsRNA, *i.e.*, no nucleotide overhang. A "blunt ended" dsRNA is a dsRNA that is double-stranded over its entire length, *i.e.*, no nucleotide

overhang at either end of the molecule.

The term "antisense strand" refers to the strand of a dsRNA which includes a region that is substantially complementary to a target sequence. As used herein, the term "region of complementarity" refers to the region on the antisense strand that is substantially complementary to a sequence, for example a target sequence, as defined herein. Where the region of complementarity is not fully complementary to the target sequence, the mismatches are most tolerated in the terminal regions and, if present, are generally in a terminal region or regions, *e.g.*, within 6, 5, 4, 3, or 2 nucleotides of the 5' and/or 3' terminus.

The term "sense strand," as used herein, refers to the strand of a dsRNA that includes a region that is substantially complementary to a region of the antisense strand.

As used herein, the term "SNALP" refers to a stable nucleic acid-lipid particle. A SNALP represents a vesicle of lipids coating a reduced aqueous interior comprising a nucleic acid such as a dsRNA or a plasmid from which a dsRNA is transcribed. SNALP are described, *e.g.*, in U.S. Patent Application Publication Nos. 20060240093, 20070135372, and USSN 61/045,228 filed on April 15, 2008.

"Introducing into a cell," when referring to a dsRNA, means facilitating uptake or absorption into the cell, as is understood by those skilled in the art. Absorption or uptake of dsRNA can occur through unaided diffusive or active cellular processes, or by auxiliary agents or devices. The meaning of this term is not limited to cells *in vitro*; a dsRNA may also be "introduced into a cell," wherein the cell is part of a living organism. In such instance, introduction into the cell will include the delivery to the organism. For example, for *in vivo* delivery, dsRNA can be injected into a tissue site or administered systemically. *In vitro* introduction into a cell includes methods known in the art such as electroporation and lipofection. Further approaches are described herein or known in the art.

The terms "silence," "inhibit the expression of," "down-regulate the expression of," "suppress the expression of" and the like in as far as they refer to a TTR gene, herein refer to the at least partial suppression of the expression of a TTR gene, as manifested by a reduction of the amount of mRNA which may be isolated from a first cell or group of cells in which a TTR gene is transcribed and which has or have been treated such that the expression of a TTR gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Alternatively, the degree of inhibition may be given in terms of a reduction of a parameter that is functionally linked to TTR gene expression, *e.g.*, the amount of protein encoded by a TTR gene which is secreted by a cell, or the number of cells displaying a certain phenotype, *e.g.*, apoptosis. In principle, TTR gene silencing may be determined in any cell expressing the target, either constitutively or by genomic engineering, and by any appropriate assay. However, when a reference is needed in order to determine whether a given dsRNA inhibits the expression of a TTR gene by a certain degree and therefore is encompassed by the instant disclosure, the assays provided in the Examples below shall serve as such reference.

For example, in certain instances, expression of a TTR gene is suppressed by at least about 5%, 10%, 15%, 20%, 25%, 30%, 35%, 40%, 45%, or 50% by administration of the double-stranded oligonucleotide described herein. A TTR gene may be suppressed by at least about 60%, 70%, or 80% by administration of the double-stranded oligonucleotide described herein. A TTR gene may be suppressed by at least about 85%, 90%, or 95% by administration of the double-stranded oligonucleotide described herein.

As used herein in the context of TTR expression, the terms "treat," "treatment," and the like, refer to relief from or alleviation of pathological processes mediated by TTR expression. In the context herein insofar as it relates to any of the other conditions recited herein below (other than pathological processes mediated by TTR expression), the terms "treat," "treatment," and the like mean to relieve or alleviate at least one symptom associated with such condition, or to slow or reverse the progression of such condition, such as the slowing the progression of a TTR amyloidosis, such as FAP. Symptoms of TTR amyloidosis include sensory neuropathy (e.g. paresthesia, hypesthesia in distal limbs), autonomic neuropathy (e.g., gastrointestinal dysfunction, such as gastric ulcer, or orthostatic hypotension), motor neuropathy, seizures, dementia, myelopathy, polyneuropathy, carpal tunnel syndrome, autonomic insufficiency, cardiomyopathy, vitreous opacities, renal insufficiency, nephropathy, substantially reduced mBMI (modified Body Mass Index), cranial nerve dysfunction, and corneal lattice dystrophy.

As used herein, the phrases "therapeutically effective amount" and "prophylactically effective amount" refer to an amount that provides a therapeutic benefit in the treatment, prevention, or management of pathological processes mediated by TTR expression or an overt symptom of pathological processes mediated by TTR expression. The specific amount that is therapeutically effective can be readily determined by an ordinary medical practitioner, and may vary depending on factors known in the art, such as, for example, the type of pathological processes mediated by TTR

expression, the patient's history and age, the stage of pathological processes mediated by TTR expression, and the administration of other anti-pathological processes mediated by TTR expression agents.

As used herein, a "pharmaceutical composition" comprises a pharmacologically effective amount of a dsRNA and a pharmaceutically acceptable carrier. As used herein, "pharmacologically effective amount," "therapeutically effective amount" or simply "effective amount" refers to that amount of an RNA effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount necessary to effect at least a 25% reduction in that parameter. For example, a therapeutically effective amount of a dsRNA targeting TTR can reduce TTR serum levels by at least 25%. In another example, a therapeutically effective amount of a dsRNA targeting TTR can improve liver function or renal function by at least 25%.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

As used herein, a "transformed cell" is a cell into which a vector has been introduced from which a dsRNA molecule may be expressed.

## II. Double-stranded ribonucleic acid (dsRNA)

As described in more detail herein, provided herein are double-stranded ribonucleic acid (dsRNA) molecules for inhibiting the expression of a TTR gene in a cell or mammal, e.g., in a human having a TTR amyloidosis, where the dsRNA includes an antisense strand having a region of complementarity which is complementary to at least a part of an mRNA formed in the expression of a TTR gene, and where the region of complementarity is less than 30 nucleotides in length, generally 19-24 nucleotides in length, and where said dsRNA, upon contact with a cell expressing said TTR gene, inhibits the expression of said TTR gene by at least 30% as assayed by, for example, a PCR or branched DNA (bDNA)-based method, or by a protein-based method, such as by Western blot. Expression of a TTR gene can be reduced by at least 30% when measured by an assay as described in the Examples below. For example, expression of a TTR gene in cell culture, such as in Hep3B cells, can be assayed by measuring TTR mRNA levels, such as by bDNA or TaqMan assay, or by measuring protein levels, such as by ELISA assay. The dsRNA described herein can further include one or more single-stranded nucleotide overhangs.

The dsRNA can be synthesized by standard methods known in the art as further discussed below, *e.g.*, by use of an automated DNA synthesizer, such as are commercially available from, for example, Biosearch, Applied Biosystems, Inc. The dsRNA includes two RNA strands that are sufficiently complementary to hybridize to form a duplex structure. One strand of the dsRNA (the antisense strand) includes a region of complementarity that is substantially complementary, and generally fully complementary, to a target sequence, derived from the sequence of an mRNA formed during the expression of a TTR gene, the other strand (the sense strand) includes a region that is complementary to the antisense strand, such that the two strands hybridize and form a duplex structure when combined under suitable conditions. Generally, the duplex structure is between 15 and 30 or between 25 and 30, or between 18 and 25, or between 19 and 24, or between 19 and 21, or 19, 20, or 21 base pairs in length. The duplex may be 19 base pairs in length. Also the duplex may be 21 base pairs in length. When two different siRNAs are used in combination, the duplex lengths can be identical or can differ.

Each strand of the dsRNA described herein is generally between 15 and 30, or between 18 and 25, or 18, 19, 20, 21, 22, 23, 24, or 25 nucleotides in length. Each strand may be 25-30 nucleotides in length. Each strand of the duplex can be the same length or of different lengths. When two different siRNAs are used in combination, the lengths of each strand of each siRNA can be identical or can differ.

The dsRNA described herein can include one or more single-stranded overhang(s) of one or more nucleotides. At least one end of the dsRNA may have a single-stranded nucleotide overhang of 1 to 4, generally 1 or 2 nucleotides. The antisense strand of the dsRNA may have 1-10 nucleotides overhangs each at the 3' end and the 5' end over the sense strand. The sense strand of the dsRNA may have 1-10 nucleotides overhangs each at the 3' end and the 5' end over the antisense strand.

A dsRNAs having at least one nucleotide overhang can have unexpectedly superior inhibitory properties than the blunt-ended counterpart. The presence of only one nucleotide overhang may strengthen the interference activity of

the dsRNA, without affecting its overall stability. A dsRNA having only one overhang has proven particularly stable and effective *in vivo*, as well as in a variety of cells, cell culture mediums, blood, and serum. Generally, the single-stranded overhang is located at the 3'-terminal end of the antisense strand or, alternatively, at the 3'-terminal end of the sense strand. The dsRNA can also have a blunt end, generally located at the 5'-end of the antisense strand. Such dsRNAs can have improved stability and inhibitory activity, thus allowing administration at low dosages, *i.e.*, less than 5 mg/kg body weight of the recipient per day. Generally, the antisense strand of the dsRNA has a nucleotide overhang at the 3'-end, and the 5'-end is blunt. One or more of the nucleotides in the overhang may be replaced with a nucleoside thiophosphate.

A TTR gene may be a human TTR gene. The sense strand of the dsRNA may be one of the sense sequences from Tables 3A, 3B, 4, 6A, 6B, or 7, and the antisense strand may be one of the antisense sequences of Tables 3A, 3B, 4, 6A, 6B, or 7. Alternative antisense agents that target elsewhere in the target sequence provided in Tables 3A, 3B, 4, 6A, 6B, or 7 can readily be determined using the target sequence and the flanking TTR sequence.

The skilled person is well aware that dsRNAs having a duplex structure of between 20 and 23, but specifically 21, base pairs have been hailed as particularly effective in inducing RNA interference (Elbashir et al., EMBO 2001, 20:6877-6888). However, others have found that shorter or longer dsRNAs can be effective as well. In the example described above, by virtue of the nature of the oligonucleotide sequences provided in Tables 3A, 3B, 4, 6A, 6B, and 7, the dsRNAs described herein can include at least one strand of a length described herein. It can be reasonably expected that shorter dsRNAs having one of the sequences of Tables 3A, 3B, 4, 6A, 6B, or 7 minus only a few nucleotides on one or both ends may be similarly effective as compared to the dsRNAs described above. Hence, dsRNAs having a partial sequence of at least 15, 16, 17, 18, 19, 20, or more contiguous nucleotides from one of the sequences of Tables 3, 4, 6 or 7, and differing in their ability to inhibit the expression of a TTR gene in an assay as described herein below by not more than 5, 10, 15, 20, 25, or 30 % inhibition from a dsRNA comprising the full sequence, are also described herein. Further, dsRNAs that cleave within a desired TTR target sequence can readily be made using the corresponding TTR antisense sequence and a complementary sense sequence.

In addition, the dsRNAs provided in Tables 3A, 3B, 4, 6A, 6B, or 7 identify a site in a TTR that is susceptible to RNAi based cleavage. As such, also described herein are dsRNAs that target within the sequence targeted by one of the agents described herein. As used herein, a second dsRNA is said to target within the sequence of a first dsRNA if the second dsRNA cleaves the message anywhere within the mRNA that is complementary to the antisense strand of the first dsRNA. Such a second dsRNA will generally consist of at least 15 contiguous nucleotides from one of the sequences provided in Tables 3A, 3B, 4, 6A, 6B, or 7 coupled to additional nucleotide sequences taken from the region contiguous to the selected sequence in a TTR gene.

Cleavage of the RNA target can be routinely detected by gel electrophoresis and, if necessary, associated nucleic acid hybridization techniques known in the art. The cleavage site on the target mRNA of a dsRNA can be determined using methods generally known to one of ordinary skill in the art, e.g., the 5'-RACE method described in Soutschek et al., Nature; 2004, Vol. 432, pp. 173-178. In an example, using the 5'-RACE method described by Soutschek et al., ALN-18328 was determined to cleave a TTR mRNA between the guanine nucleotide at position 636 of SEQ ID NO:1331 (NM_000371.3) and the adenine nucleotide at position 637 of SEQ ID NO:1331. In an example, it was determined that ALN-18328 does not cleave a TTR mRNA between the adenine nucleotide at position 637 of SEQ ID NO: 1331 and the guanine nucleotide at position 638 of SEQ ID NO: 1331.

The dsRNA described herein can contain one or more mismatches to the target sequence. The dsRNA described herein may contain no more than 3 mismatches. If the antisense strand of the dsRNA contains mismatches to a target sequence, it is preferable that the area of mismatch not be located in the center of the region of complementarity. If the antisense strand of the dsRNA contains mismatches to the target sequence, it is preferable that the mismatch be restricted to 5 nucleotides from either end, for example 5, 4, 3, 2, or 1 nucleotide from either the 5' or 3' end of the region of complementarity. For example, for a 23 nucleotide dsRNA strand which is complementary to a region of a TTR gene, the dsRNA generally does not contain any mismatch within the central 13 nucleotides. The methods described herein can be used to determine whether a dsRNA containing a mismatch to a target sequence is effective in inhibiting the expression of a TTR gene. Consideration of the efficacy of dsRNAs with mismatches in inhibiting expression of a TTR gene is important, especially if the particular region of complementarity in a TTR gene is known to have polymorphic sequence variation within the population.

Modifications

The dsRNA may be chemically modified to enhance stability. The nucleic acids described herein may be synthesized and/or modified by methods well established in the art, such as those described in "Current protocols in nucleic acid chemistry," Beaucage, S.L. et al. (Eds.), John Wiley & Sons, Inc., New York, NY, USA. Specific examples of dsRNA compounds useful in the present disclosure include dsRNAs containing modified backbones or no natural internucleoside linkages. As defined in this specification, dsRNAs having modified backbones include those that retain

a phosphorus atom in the backbone and those that do not have a phosphorus atom in the backbone. For the purposes of this specification, and as sometimes referenced in the art, modified dsRNAs that do not have a phosphorus atom in their internucleoside backbone can also be considered to be oligonucleosides.

Modified dsRNA backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those) having inverted polarity wherein the adjacent pairs of nucleoside units are linked 3'-5' to 5'-3' or 2'-5' to 5'-2'. Various salts, mixed salts and free acid forms are also included.

Representative U.S. patents that teach the preparation of the above phosphorus-containing linkages include, but are not limited to, U.S. Pat. Nos. 3,687,808; 4,469,863; 4,476,301; 5,023,243; 5,177,195; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,316; 5,550,111; 5,563,253; 5,571,799; 5,587,361; and 5,625,050.

Modified dsRNA backbones that do not include a phosphorus atom therein have backbones that are formed by short chain alkyl or cycloalkyl internucleoside linkages, mixed heteroatoms and alkyl or cycloalkyl internucleoside linkages, or ore or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

Representative U.S. patents that teach the preparation of the above oligonucleosides include, but are not limited to, U.S. Pat. Nos. 5,034,506; 5,166,315; 5,185,444; 5,214,134; 5,216,141; 5,235,033; 5,64,562; 5,264,564; 5,405,938; 5,434,257; 5,466,677; 5,470,967; 5,489,677; 5,541,307; 5,561,225; 5,596,086; 5,602,240; 5,608,046; 5,610,289; 5,618,704; 5,623,070; 5,663,312; 5,633,360; 5,677,437; and, 5,677,439.

In other suitable dsRNA mimetics, both the sugar and the internucleoside linkage, *i.e.*, the backbone, of the nucleotide units are replaced with novel groups. The base units are maintained for hybridization with an appropriate nucleic acid target compound. One such oligomeric compound, a dsRNA mimetic that has been shown to have excellent hybridization properties, is referred to as a peptide nucleic acid (PNA). In PNA compounds, the sugar backbone of a dsRNA is replaced with an amide containing backbone, in particular an aminoethylglycine backbone. The nucleobases are retained and are bound directly or indirectly to aza nitrogen atoms of the amide portion of the backbone. Representative U.S. patents that teach the preparation of PNA compounds include, but are not limited to, U.S. Pat. Nos. 5,539,082; 5,714,331; and 5,719,262. Further teaching of PNA compounds can be found in Nielsen et al., Science, 1991, 254, 1497-1500.

Also described herein are dsRNAs with phosphorothioate backbones and oligonucleosides with heteroatom backbones, and in particular $--CH_2--NH--CH_2--$, $--CH_2-N(CH_3)--O--CH_2--$[known as a methylene (methylimino) or MMI backbone], $--CH_2--O-N(CH_3)--CH_2--$, $--CH_2--N(CH_3)--N(CH_3)--CH_2--$ and $--N(CH_3)--CH_2--CH_2--$ [wherein the native phosphodiester backbone is represented as $--O--P--O--CH_2--$] of the above-referenced U.S. Pat. No. 5,489,677, and the amide backbones of the above-referenced U.S. Pat. No. 5,602,240. Also preferred are dsRNAs having morpholino backbone structures of the above-referenced U.S. Pat. No. 5,034,506.

Modified dsRNAs may also contain one or more substituted sugar moieties. Preferred dsRNAs comprise one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. Particularly preferred are $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other preferred dsRNAs comprise one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an dsRNA, or a group for improving the pharmacodynamic properties of an dsRNA, and other substituents having similar properties. A preferred modification includes 2'-methoxyethoxy (2'-O--$CH_2CH_2OCH_3$, also known as 2'-O-(2-methoxyethyl) or 2'-MOE) (Martin et al., Helv. Chim. Acta, 1995, 78, 486-504) *i.e.*, an alkoxy-alkoxy group. A further preferred modification includes 2'-dimethylaminooxyethoxy, *i.e.*, a $O(CH_2)_2ON(CH_3)_2$ group, also known as 2'-DMAOE, as described in examples herein below, and 2'-dimethylaminoethoxyethoxy (also known in the art as 2'-O-dimethylaminoethoxyethyl or 2'-DMAEOE), *i.e.*, 2'-O--$CH_2$--O--$CH_2$-$N(CH_2)_2$, also described in examples herein below.

Other preferred modifications include 2'-methoxy (2'-$OCH_3$), 2'-aminopropoxy (2'-$OCH_2CH_2CH_2NH_2$) and 2'-fluoro (2'-F). Similar modifications may also be made at other positions on the dsRNA, particularly the 3' position of the sugar on the 3' terminal nucleotide or in 2'-5' linked dsRNAs and the 5' position of 5' terminal nucleotide. DsRNAs may

also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

dsRNAs may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine. Further nucleobases include those disclosed in U.S. Pat. No. 3,687,808, those disclosed in The Concise Encyclopedia Of Polymer Science And Engineering, pages 858-859, Kroschwitz, J. L, ed. John Wiley & Sons, 1990, these disclosed by Englisch et al., Angewandte Chemie, International Edition, 1991, 30, 613, and those disclosed by Sanghvi, Y S., Chapter 15, DsRNA Research and Applications, pages 289-302, Crooke, S. T. and Lebleu, B., Ed., CRC Press, 1993. Certain of these nucleobases are particularly useful for increasing the binding affinity of the oligomeric compounds described herein. These include 5-substituted pyrimidines, 6-azapyrimidines and N-2, N-6 and 0-6 substituted purines, including 2-aminopropyladenine, 5-propynyluracil and 5-propynylcytosine. 5-methylcytosine substitutions have been shown to increase nucleic acid duplex stability by 0.6-1.2°C. (Sanghvi, Y. S., Crooke, S. T. and Lebleu, B., Eds., DsRNA Research and Applications, CRC Press, Boca Raton, 1993, pp. 276-278) and are exemplary base substitutions, even more particularly when combined with 2'-O-methoxyethyl sugar modifications.

Representative U.S. patents that teach the preparation of certain of the above noted modified nucleobases as well as other modified nucleobases include, but are not limited to, the above noted U.S. Pat. No. 3,687,808, as well as U.S. Pat. Nos. 4,845,205; 5,130,30; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,681,941, , and 5,750,692.

Conjugates

Another modification of the dsRNAs described herein involves chemically linking to the dsRNA one or more moieties or conjugates which enhance the activity, cellular distribution or cellular uptake of the dsRNA. Such moieties include but are not limited to lipid moieties such as a cholesterol moiety (Letsinger et al., Proc. Natl. Acid. Sci. USA, 1989, 86: 6553-6556), cholic acid (Manoharan et al., Biorg. Med. Chem. Let., 1994, 4:1053-1060), a thioether, *e.g.*, beryl-S-tritylthiol (Manoharan et al., Ann. N.Y. Acad. Sci., 1992, 660:306-309; Manoharan et al., Biorg. Med. Chem. Let., 1993, 3:2765-2770), a thiocholesterol (Oberhauser et al., Nucl. Acids Res., 1992, 20:533-538), an aliphatic chain, *e.g.*, dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75:49-54), a phospholipid, *e.g.*, di-hexadecyl-rac-glycerol or triethyl-ammonium 1,2-di-O-hexadecyl-rac-glycero-3-Hphosphonate (Manoharan et al., Tetrahedron Lett., 1995, 36:3651-3654; Shea et al., Nucl. Acids Res., 1990, 18:3777-3783), a polyamine or a polyethylene glycol chain (Manoharan et al., Nucleosides & Nucleotides, 1995, 14:969-973), or adamantane acetic acid (Manoharan et al., Tetrahedron Lett., 1995, 36:3651-3654), a palmityl moiety (Mishra et al., Biochim. Biophys. Acta, 1995, 1264:229-237), or an octadecylamine or hexylamino-carbonyloxycholesterol moiety (Crooke et al., J. Pharmacol. Exp. Ther., 1996, 277:923-937).

Representative U.S. patents that teach the preparation of such dsRNA conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

It is not necessary for all positions in a given compound to be uniformly modified, and in fact more than one of the aforementioned modifications may be incorporated in a single compound or even at a single nucleoside within a dsRNA. Also described herein are dsRNA compounds which are chimeric compounds. "Chimeric" dsRNA compounds or "chimeras," in the context described herein, are dsRNA compounds, particularly dsRNAs, which contain two or more chemically distinct regions, each made up of at least one monomer unit, *i.e.*, a nucleotide in the case of a dsRNA compound. These dsRNAs typically contain at least one region wherein the dsRNA is modified so as to confer upon the dsRNA increased resistance to nuclease degradation, increased cellular uptake, and/or increased binding affinity for

the target nucleic acid. An additional region of the dsRNA may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of dsRNA inhibition of gene expression. Consequently, comparable results can often be obtained with shorter dsRNAs when chimeric dsRNAs are used, compared to phosphorothioate deoxydsRNAs hybridizing to the same target region.

In certain instances, the dsRNA may be modified by a non-ligand group. A number of non-ligand molecules have been conjugated to dsRNAs in order to enhance the activity, cellular distribution or cellular uptake of the dsRNA, and procedures for performing such conjugations are available in the scientific literature. Such non-ligand moieties have included lipid moieties, such as cholesterol (Letsinger et al., Proc. Natl. Acad. Sci. USA, 1989, 86:6553), cholic acid (Manoharan et al., Bioorg. Med. Chem. Lett., 1994, 4:1053), a thioether, *e.g.*, hexyl-S-tritylthiol (Manoharan et al., Ann. N.Y. Acad. Sci., 1992, 660:306; Manoharan et al., Bioorg. Med. Chem. Let., 1993, 3:2765), a thiocholesterol (Oberhauser et al., Nucl. Acids Res., 1992, 20:533), an aliphatic chain, *e.g.*, dodecandiol or undecyl residues (Saison-Behmoaras et al., EMBO J., 1991, 10:111; Kabanov et al., FEBS Lett., 1990, 259:327; Svinarchuk et al., Biochimie, 1993, 75:49), a phospholipid, *e.g.*, di-hexadecyl-rac-glycerol or triethylammonium 1,2-di-O-hexadecyl-rac-glycero-3-H-phosphonate (Manoharan et al., Tetrahedron Lett., 1995, 36:3651; Shea et al., Nucl. Acids Res., 1990, 18:3777), a polyamine or a polyethylene glycol chain (Manoharan et al., Nucleosides & Nucleotides, 1995, 14:969), or adamantane acetic acid (Manoharan et al., Tetrahedron Lett., 1995, 36:3651), a palmityl moiety (Mishra et al., Biochim. Biophys. Acta, 1995, 1264:229), or an octadecylamine or hexylamino-carbonyl-oxycholesterol moiety (Crooke et al., J. Pharmacol. Exp. Ther., 1996, 277:923). Representative United States patents that teach the preparation of such dsRNA conjugates have been listed above. Typical conjugation protocols involve the synthesis of dsRNAs bearing an aminolinker at one or more positions of the sequence. The amino group is then reacted with the molecule being conjugated using appropriate coupling or activating reagents. The conjugation reaction may be performed either with the dsRNA still bound to the solid support or following cleavage of the dsRNA in solution phase. Purification of the dsRNA conjugate by HPLC typically affords the pure conjugate.

Vector encoded dsRNAs

In another aspect, TTR dsRNA molecules are expressed from transcription units inserted into DNA or RNA vectors (see, *e.g.*, Couture, A, et al., TIG. (1996), 12:5-10; Skillern, A., et al., International PCT Publication No. WO 00/22113, Conrad, International PCT Publication No. WO 00/22114, and Conrad, U.S. Pat. No. 6,054,299). These transgenes can be introduced as a linear construct, a circular plasmid, or a viral vector, which can be incorporated and inherited as a transgene integrated into the host genome. The transgene can also be constructed to permit it to be inherited as an extrachromosomal plasmid (Gassmann, et al., Proc. Natl. Acad. Sci. USA (1995) 92:1292).

The individual strands of a dsRNA can be transcribed by promoters on two separate expression vectors and co-transfected into a target cell. Alternatively each individual strand of the dsRNA can be transcribed by promoters both of which are located on the same expression plasmid. A dsRNA may be expressed as an inverted repeat joined by a linker polynucleotide sequence such that the dsRNA has a stem and loop structure.

The recombinant dsRNA expression vectors are generally DNA plasmids or viral vectors. dsRNA expressing viral vectors can be constructed based on, but not limited to, adeno-associated virus (for a review, see Muzyczka, et al., Curr. Topics Micro. Immunol. (1992) 158:97-129)); adenovirus (see, for example, Berkner, et al., BioTechniques (1998) 6:616), Rosenfeld et al. (1991, Science 252:431-434), and Rosenfeld et al. (1992), Cell 68:143-155)); or alphavirus as well as others known in the art. Retroviruses have been used to introduce a variety of genes into many different cell types, including epithelial cells, *in vitro* and/or *in vivo* (see, *e.g.,* Eglitis, et al., Science (1985) 230:1395-1398; Danos and Mulligan, Proc. NatI. Acad. Sci. USA (1998) 85:6460-6464; Wilson et al., 1988, Proc. NatI. Acad. Sci. USA 85:3014-3018; Armentano et al., 1990, Proc. NatI. Acad. Sci. USA 87:61416145; Huber et al., 1991, Proc. NatI. Acad. Sci. USA 88:8039-8043; Ferry et al., 1991, Proc. NatI. Acad. Sci. USA 88:8377-8381; Chowdhury et al., 1991, Science 254:1802-1805; van Beusechem. et al., 1992, Proc. Natl. Acad. Sci. USA 89:7640-19 ; Kay et al., 1992, Human Gene Therapy 3:641-647; Dai et al., 1992, Proc. Natl. Acad. Sci. USA 89:10892-10895; Hwu et al., 1993, J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573). Recombinant retroviral vectors capable of transducing and expressing genes inserted into the genome of a cell can be produced by transfecting the recombinant retroviral genome into suitable packaging cell lines such as PA317 and Psi-CRIP (Comette et al., 1991, Human Gene Therapy 2:5-10; Cone et al., 1984, Proc. Natl. Acad. Sci. USA 81:6349). Recombinant adenoviral vectors can be used to infect a wide variety of cells and tissues in susceptible hosts (*e.g.*, rat, hamster, dog, and chimpanzee) (Hsu et al., 1992, J. Infectious Disease, 166:769), and also have the advantage of not requiring mitotically active cells for infection.

Any viral vector capable of accepting the coding sequences for the dsRNA molecule(s) to be expressed can

be used, for example vectors derived from adenovirus (AV); adeno-associated virus (AAV); retroviruses (*e.g*, lentiviruses (LV), Rhabdoviruses, murine leukemia virus); herpes virus, and the like. The tropism of viral vectors can be modified by pseudotyping the vectors with envelope proteins or other surface antigens from other viruses, or by substituting different viral capsid proteins, as appropriate.

For example, lentiviral vectors described herein can be pseudotyped with surface proteins from vesicular stomatitis virus (VSV), rabies, Ebola, Mokola, and the like. AAV vectors described herein can be made to target different cells by engineering the vectors to express different capsid protein serotypes. For example, an AAV vector expressing a serotype 2 capsid on a serotype 2 genome is called AAV 2/2. This serotype 2 capsid gene in the AAV 2/2 vector can be replaced by a serotype 5 capsid gene to produce an AAV 2/5 vector. Techniques for constructing AAV vectors which express different capsid protein serotypes are within the skill in the art; see, *e.g.*, Rabinowitz J E et al. (2002), J Virol 76:791-801.

Selection of recombinant viral vectors suitable for use herein, methods for inserting nucleic acid sequences for expressing the dsRNA into the vector, and methods of delivering the viral vector to the cells of interest are within the skill in the art. See, for example, Dornburg R (1995), Gene Therap. 2: 301-310; Eglitis M A (1988), Biotechniques 6: 608-614; Miller A D (1990), Hum Gene Therap. 1: 5-14; Anderson W F (1998), Nature 392: 25-30; and Rubinson D A et al., Nat. Genet. 33: 401-406.

Viral vectors can be derived from AV and AAV. The dsRNA described herein may be expressed as two separate, complementary single-stranded RNA molecules from a recombinant AAV vector having, for example, either the U6 or H1 RNA promoters, or the cytomegalovirus (CMV) promoter.

A suitable AV vector for expressing the dsRNA described herein, a method for constructing the recombinant AV vector, and a method for delivering the vector into target cells, are described in Xia H et al. (2002), Nat. Biotech. 20: 1006-1010.

Suitable AAV vectors for expressing the dsRNA described herein, methods for constructing the recombinant AV vector, and methods for delivering the vectors into target cells are described in Samulski R et al. (1987), J. Virol. 61: 3096-3101; Fisher K J et al. (1996), J. Virol, 70: 520-532; Samulski R et al. (1989), J. Virol. 63: 3822-3826; U.S. Pat. No. 5,252,479; U.S. Pat. No. 5,139,941; International Patent Application No. WO 94/13788; and International Patent Application No. WO 93/24641.

The promoter driving dsRNA expression in either a DNA plasmid or viral vector described herein may be a eukaryotic RNA polymerase I (*e.g.*, ribosomal RNA promoter), RNA polymerase II (*e.g.*, CMV early promoter or actin promoter or U1 snRNA promoter) or generally RNA polymerase III promoter (*e.g.*, U6 snRNA or 7SK RNA promoter) or a prokaryotic promoter, for example the T7 promoter, provided the expression plasmid also encodes T7 RNA polymerase required for transcription from a T7 promoter. The promoter can also direct transgene expression to the pancreas (see, *e.g.*, the insulin regulatory sequence for pancreas (Bucchini et al., 1986, Proc. Natl. Acad. Sci. USA 83:2511-2515)).

In addition, expression of the transgene can be precisely regulated, for example, by using an inducible regulatory sequence and expression systems such as a regulatory sequence that is sensitive to certain physiological regulators, *e.g.*, circulating glucose levels, or hormones (Docherty et al., 1994, FASEB J. 8:20-24). Such inducible expression systems, suitable for the control of transgene expression in cells or in mammals include regulation by ecdysone, by estrogen, progesterone, tetracycline, chemical inducers of dimerization, and isopropyl-beta-D1 - thiogalactopyranoside (EPTG). A person skilled in the art would be able to choose the appropriate regulatory/promoter sequence based on the intended use of the dsRNA transgene.

Generally, recombinant vectors capable of expressing dsRNA molecules are delivered as described below, and persist in target cells. Alternatively, viral vectors can be used that provide for transient expression of dsRNA molecules. Such vectors can be repeatedly administered as necessary. Once expressed, the dsRNAs bind to target RNA and modulate its function or expression. Delivery of dsRNA expressing vectors can be systemic, such as by intravenous or intramuscular administration, by administration to target cells ex-planted from the patient followed by reintroduction into the patient, or by any other means that allows for introduction into a desired target cell.

dsRNA expression DNA plasmids are typically transfected into target cells as a complex with cationic lipid carriers (*e.g.*, Oligofectamine) or non-cationic lipid-based carriers (*e.g.*, Transit-TKO™). Multiple lipid transfections for dsRNA-mediated knockdowns targeting different regions of a single TTR gene or multiple TTR genes over a period of a week or more are also described herein. Successful introduction of vectors into host cells can be monitored using various known methods. For example, transient transfection can be signaled with a reporter, such as a fluorescent marker, such as Green Fluorescent Protein (GFP). Stable transfection of cells *ex vivo* can be ensured using markers that provide the transfected cell with resistance to specific environmental factors (*e.g.*, antibiotics and drugs), such as hygromycin B resistance.

TTR specific dsRNA molecules can also be inserted into vectors and used as gene therapy vectors for human patients. Gene therapy vectors can be delivered to a subject by, for example, intravenous injection, local administration (see U.S. Patent 5,328,470) or by stereotactic injection (see *e.g.*, Chen et al. (1994) Proc. Natl. Acad. Sci. USA 91:3054-3057). The pharmaceutical preparation of the gene therapy vector can include the gene therapy vector in an

acceptable diluent, or can include a slow release matrix in which the gene delivery vehicle is imbedded. Alternatively, where the complete gene delivery vector can be produced intact from recombinant cells, *e.g.*, retroviral vectors, the pharmaceutical preparation can include one or more cells which produce the gene delivery system.

## III. Pharmaceutical compositions containing dsRNA

Also provided herein are pharmaceutical compositions containing a dsRNA, as described herein, and a pharmaceutically acceptable carrier. The pharmaceutical composition containing the dsRNA is useful for treating a disease or disorder associated with the expression or activity of a TTR gene, such as pathological processes mediated by TTR expression. Such pharmaceutical compositions are formulated based on the mode of delivery. One example is compositions formulated for direct delivery to the eye. Another example is compositions that are formulated for systemic administration via parenteral delivery, e.g., by intravenous (IV) delivery. Another example is compositions that are formulated for direct delivery into the brain parenchyma, e.g., by infusion into the brain, such as by continuous pump infusion.

The pharmaceutical compositions described herein are administered in dosages sufficient to inhibit expression of TTR genes.

In general, a suitable dose of dsRNA will be in the range of 0.00001 to 200.0 milligrams per kilogram body weight of the recipient per day, generally in the range of 1 to 50 mg per kilogram body weight per day.

The dosage may not scale with body weight when the siRNA is administered to the eye.

The dosage can be, e.g., 25 μg for a 75 kg person, e.g. 0.3 μg/kg. Other dosages include, 0.01, 0.03, 0.05, 0.07, 0.1, 0.3, 0.5, 0.7, 1.0, 3.0. 5.0, 7.0, 10.0, 30.0, 50.0, 70.0, or 100.0 μg/kg.

For example, the dsRNA can be administered at 0.0059 mg/kg, 0.01 mg/kg, 0.0295 mg/kg, 0.05 mg/kg, 0.0590 mg/kg, 0.163 mg/kg, 0.2 mg/kg, 0.3 mg/kg, 0.4 mg/kg, 0.5 mg/kg, 0.543 mg/kg, 0.5900 mg/kg, 0.6 mg/kg, 0.7 mg/kg, 0.8 mg/kg, 0.9 mg/kg, 1 mg/kg, 1.1 mg/kg, 1.2 mg/kg, 1.3 mg/kg, 1.4 mg/kg, 1.5 mg/kg, 1.628 mg/kg, 2 mg/kg, 3 mg/kg, 5.0 mg/kg, 10 mg/kg, 20 mg/kg, 30 mg/kg, 40 mg/kg, or 50 mg/kg per single dose.

The dosage may be between 0.01 and 0.2 mg/kg. For example, the dsRNA can be administered at a dose of 0.01 mg/kg, 0.02 mg/kg, 0.03 mg/kg, 0.04 mg/kg, 0.05 mg/kg, 0.06 mg/kg, 0.07 mg/kg 0.08 mg/kg 0.09 mg/kg, 0.10 mg/kg, 0.11 mg/kg, 0.12 mg/kg, 0.13 mg/kg, 0.14 mg/kg, 0.15 mg/kg, 0.16 mg/kg, 0.17 mg/kg, 0.18 mg/kg, 0.19 mg/kg, or 0.20 mg/kg.

The dosage may be between 0.005 mg/kg and 1.628 mg/kg. For example, the dsRNA can be administered at a dose of 0.0059 mg/kg, 0.0295 mg/kg, 0.0590 mg/kg, 0.163 mg/kg, 0.543 mg/kg, 0.5900 mg/kg, or 1.628 mg/kg.

The dosage may be between 0.2 mg/kg and 1.5 mg/kg. For example, the dsRNA can be administered at a dose of 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 1.1 mg/kg, 1.2 mg/kg, 1.3 mg/kg, 1.4 mg/kg, or 1.5 mg/kg.

The dsRNA can be administered at a dose of 0.03 mg/kg, or 0.03, 0.1, 0.2, or 0.4 mg/kg.

The pharmaceutical composition may be administered once daily, or the dsRNA may be administered as two, three, or more sub-doses at appropriate intervals throughout the day or even using continuous infusion or delivery through a controlled release formulation. In that case, the dsRNA contained in each sub-dose must be correspondingly smaller in order to achieve the total daily dosage. The dosage unit can also be compounded for delivery over several days, *e.g.*, using a conventional sustained release formulation which provides sustained release of the dsRNA over a several day period. Sustained release formulations are well known in the art and are particularly useful for delivery of agents at a particular site, such as could be used with the agents described herein. The dosage unit may contain a corresponding multiple of the daily dose.

The effect of a single dose on TTR levels is long lasting, such that subsequent doses are administered at not more than 3, 4, or 5 day intervals, or at not more than 1, 2, 3, or 4 week intervals, or at not more than 5, 6, 7, 8, 9, or 10 week intervals.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and *in vivo* half-lives for the individual dsRNAs encompassed by the disclosure can be made using conventional methodologies or on the basis of *in vivo* testing using an appropriate animal model, as described elsewhere herein.

Advances in mouse genetics have generated a number of mouse models for the study of various human diseases, such as pathological processes mediated by TTR expression. Such models are used for *in vivo* testing of dsRNA, as well as for determining a therapeutically effective dose. A suitable mouse model is, for example, a mouse containing a plasmid expressing human TTR. Another suitable mouse model is a transgenic mouse carrying a transgene that expresses human TTR.

The data obtained from cell culture assays and animal studies can be used in formulating a range of dosage for use in humans. The dosage of compositions described herein lies generally within a range of circulating concentrations

that include the ED50 with little or no toxicity. The dosage may vary within this range depending upon the dosage form employed and the route of administration utilized. For any compound used in the methods described herein, the therapeutically effective dose can be estimated initially from cell culture assays. A dose may be formulated in animal models to achieve a circulating plasma concentration range of the compound or, when appropriate, of the polypeptide product of a target sequence (*e.g.*, achieving a decreased concentration of the polypeptide) that includes the IC50 (*i.e.*, the concentration of the test compound which achieves a half-maximal inhibition of symptoms) as determined in cell culture. Such information can be used to more accurately determine useful doses in humans. Levels in plasma may be measured, for example, by high performance liquid chromatography.

The dsRNAs described herein can be administered in combination with other known agents effective in treatment of pathological processes mediated by target gene expression. In any event, the administering physician can adjust the amount and timing of dsRNA administration on the basis of results observed using standard measures of efficacy known in the art or described herein.

Administration

Also decribed herein are pharmaceutical compositions and formulations which include the dsRNA compounds described herein. The pharmaceutical compositions descrinbed herein may be administered in a number of ways depending upon whether local or systemic treatment is desired and upon the area to be treated. Administration may be topical, pulmonary, *e.g.*, by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal or intramuscular injection or infusion; or intracranial, *e.g.*, intraparenchymal, intrathecal or intraventricular, administration.

The dsRNA can be delivered in a manner to target a particular tissue, such as the liver (*e.g.*, the hepatocytes of the liver).

The dsRNA can be delivered in a manner to target a particular tissue, such as the eye. Modes of ocular delivery include retrobulbar, subcutaneous eyelid, subconjunctival, subtenon, anterior chamber or intravitreous injection (or internal injection or infusion).

Als described herein are pharmaceutical compositions that can be delivered by injection directly into the brain. The injection can be by stereotactic injection into a particular region of the brain (*e.g.*, the substantia nigra, cortex, hippocampus, striatum, or globus pallidus), or the dsRNA can be delivered into multiple regions of the central nervous system (*e.g.*, into multiple regions of the brain, and/or into the spinal cord). The dsRNA can also be delivered into diffuse regions of the brain (*e.g.*, diffuse delivery to the cortex of the brain).

A dsRNA targeting TTR can be delivered by way of a cannula or other delivery device having one end implanted in a tissue, *e.g.*, the brain, *e.g.*, the substantia nigra, cortex, hippocampus, striatum, corpus callosum or globus pallidus of the brain. The cannula can be connected to a reservoir of the dsRNA composition. The flow or delivery can be mediated by a pump, *e.g.*, an osmotic pump or minipump, such as an Alzet pump (Durect, Cupertino, CA). A pump and reservoir may be implanted in an area distant from the tissue, *e.g.*, in the abdomen, and delivery is effected by a conduit leading from the pump or reservoir to the site of release. Infusion of the dsRNA composition into the brain can be over several hours or for several days, *e.g.*, for 1, 2, 3, 5, or 7 days or more. Devices for delivery to the brain are described, for example, in U.S. Patent Nos. 6,093,180, and 5,814,014.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful. Suitable topical formulations include those in which the dsRNAs described herein are in admixture with a topical delivery agent such as lipids, liposomes, fatty acids, fatty acid esters, steroids, chelating agents and surfactants. Suitable lipids and liposomes include neutral (*e.g.*, dioleoylphosphatidyl DOPE ethanolamine, dimyristoylphosphatidyl choline DMPC, distearoylphosphatidyl choline) negative (*e.g.*, dimyristoylphosphatidyl glycerol DMPG) and cationic (*e.g.*, dioleoyltetramethylaminopropyl DOTAP and dioleoylphosphatidyl ethanolamine DOTMA). DsRNAs described herein may be encapsulated within liposomes or may form complexes thereto, in particular to cationic liposomes. Alternatively, dsRNAs may be complexed to lipids, in particular to cationic lipids. Suitable fatty acids and esters include but are not limited to arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a $C_{1-10}$ alkyl ester (*e.g.*, isopropylmyristate IPM), monoglyceride, diglyceride or pharmaceutically acceptable salt thereof. Topical formulations are described in detail in U.S. Patent No. 6,747,014.

Cholesterol conjugation

An siRNA can be conjugated to a ligand such as cholesterol and vitamin E as described herein. It is understood that other conjugates can be linked to the oligonucleotides via a similar method known to one of ordinary skill in the art, such methods can be found in US publication nos. 2005/0107325, 2005/0164235, 2005/0256069 and 2008/0108801.

Liposomal formulations

There are many organized surfactant structures besides microemulsions that have been studied and used for the formulation of drugs. These include monolayers, micelles, bilayers and vesicles. Vesicles, such as liposomes, have attracted great interest because of their specificity and the duration of action they offer from the standpoint of drug delivery. As used herein, the term "liposome" means a vesicle composed of amphiphilic lipids arranged in a spherical bilayer or bilayers.

Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall. Non-cationic liposomes, although not able to fuse as efficiently with the cell wall, are taken up by macrophages *in vivo*.

In order to cross intact mammalian skin, lipid vesicles must pass through a series of fine pores, each with a diameter less than 50 nm, under the influence of a suitable transdermal gradient. Therefore, it is desirable to use a liposome which is highly deformable and able to pass through such fine pores.

Further advantages of liposomes include; liposomes obtained from natural phospholipids are biocompatible and biodegradable; liposomes can incorporate a wide range of water and lipid soluble drugs; liposomes can protect encapsulated drugs in their internal compartments from metabolism and degradation (Rosoff, in Pharmaceutical Dosage Forms, Lieberman, Rieger and Banker (Eds.), 1988, Marcel Dekker, Inc., New York, N.Y., volume 1, p. 245). Important considerations in the preparation of liposome formulations are the lipid surface charge, vesicle size and the aqueous volume of the liposomes.

Liposomes are useful for the transfer and delivery of active ingredients to the site of action. Because the liposomal membrane is structurally similar to biological membranes, when liposomes are applied to a tissue, the liposomes start to merge with the cellular membranes and as the merging of the liposome and cell progresses, the liposomal contents are emptied into the cell where the active agent may act.

Liposomal formulations have been the focus of extensive investigation as the mode of delivery for many drugs. There is growing evidence that for topical administration, liposomes present several advantages over other formulations. Such advantages include reduced side-effects related to high systemic absorption of the administered drug, increased accumulation of the administered drug at the desired target, and the ability to administer a wide variety of drugs, both hydrophilic and hydrophobic, into the skin.

Several reports have detailed the ability of liposomes to deliver agents including high-molecular weight DNA into the skin. Compounds including analgesics, antibodies, hormones and high-molecular weight DNAs have been administered to the skin. The majority of applications resulted in the targeting of the upper epidermis

Liposomes fall into two broad classes. Cationic liposomes are positively charged liposomes which interact with the negatively charged DNA molecules to form a stable complex. The positively charged DNA/liposome complex binds to the negatively charged cell surface and is internalized in an endosome. Due to the acidic pH within the endosome, the liposomes are ruptured, releasing their contents into the cell cytoplasm (Wang et al., Biochem. Biophys. Res. Commun., 1987, 147, 980-985).

Liposomes which are pH-sensitive or negatively-charged, entrap DNA rather than complex with it. Since both the DNA and the lipid are similarly charged, repulsion rather than complex formation occurs. Nevertheless, some DNA is entrapped within the aqueous interior of these liposomes. pH-sensitive liposomes have been used to deliver DNA encoding the thymidine kinase gene to cell monolayers in culture. Expression of the exogenous gene was detected in the target cells (Zhou et al., Journal of Controlled Release, 1992, 19, 269-274).

One major type of liposomal composition includes phospholipids other than naturally-derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine (DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Several studies have assessed the topical delivery of liposomal drug formulations to the skin. Application of liposomes containing interferon to guinea pig skin resulted in a reduction of skin herpes sores while delivery of interferon via other means (*e.g.*, as a solution or as an emulsion) were ineffective (Weiner et al., Journal of Drug Targeting, 1992, 2, 405-410). Further, an additional study tested the efficacy of interferon administered as part of a liposomal formulation

to the administration of interferon using an aqueous system, and concluded that the liposomal formulation was superior to aqueous administration (du Plessis et al., Antiviral Research, 1992, 18, 259-265).

Non-ionic liposomal systems have also been examined to determine their utility in the delivery of drugs to the skin, in particular systems comprising non-ionic surfactant and cholesterol. Non-ionic liposomal formulations comprising Novasome™ I (glyceryl dilaurate/cholesterol/polyoxyethylene-10-stearyl ether) and Novasome™ II (glyceryl distearate/cholesterol/polyoxyethylene-10-stearyl ether) were used to deliver cyclosporin-A into the dermis of mouse skin. Results indicated that such non-ionic liposomal systems were effective in facilitating the deposition of cyclosporin-A into different layers of the skin (Hu et al. S.T.P.Pharma. Sci., 1994, 4, 6, 466).

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome (A) comprises one or more glycolipids, such as monosialoganglioside $G_{M1}$, or (B) is derivatized with one or more hydrophilic polymers, such as a polyethylene glycol (PEG) moiety. While not wishing to be bound by any particular theory, it is thought in the art that, at least for sterically stabilized liposomes containing gangliosides, sphingomyelin, or PEG-derivatized lipids, the enhanced circulation half-life of these sterically stabilized liposomes derives from a reduced uptake into cells of the reticuloendothelial system (RES) (Allen et al., FEBS Letters, 1987, 223, 42; Wu et al., Cancer Research, 1993, 53, 3765).

Various liposomes comprising one or more glycolipids are known in the art. Papahadjopoulos et al. (Ann. N.Y. Acad. Sci., 1987, 507, 64) reported the ability of monosialoganglioside $G_{M1}$, galactocerebroside sulfate and phosphatidylinositol to improve blood half-lives of liposomes. These findings were expounded upon by Gabizon et al. (Proc. Natl. Acad. Sci. U.S.A., 1988, 85, 6949). U.S. Pat. No. 4,837,028 and WO 88/04924, both to Allen *et al.*, disclose liposomes comprising (1) sphingomyelin and (2) the ganglioside $G_{M1}$ or a galactocerebroside sulfate ester. U.S. Pat. No. 5,543,152 (Webb et al.) discloses liposomes comprising sphingomyelin. Liposomes comprising 1,2-sn-dimyristoylphosphatidylcholine are disclosed in WO 97/13499 (Lim et al).

Many liposomes comprising lipids derivatized with one or more hydrophilic polymers, and methods of preparation thereof, are known in the art. Sunamoto et al. (Bull. Chem. Soc. Jpn., 1980, 53, 2778) described liposomes comprising a nonionic detergent, $2C_{1215G}$, that contains a PEG moiety. Illum et al. (FEBS Lett., 1984, 167, 79) noted that hydrophilic coating of polystyrene particles with polymeric glycols results in significantly enhanced blood half-lives. Synthetic phospholipids modified by the attachment of carboxylic groups of polyalkylene glycols (*e.g.*, PEG) are described by Sears (U.S. Pat. Nos. 4,426,330 and 4,534,899). Klibanov et al. (FEBS Lett., 1990, 268, 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029, 91) extended such observations to other PEG-derivatized phospholipids, *e.g.*, DSPE-PEG, formed from the combination of distearoylphosphatidylethanolamine (DSPE) and PEG. Liposomes having covalently bound PEG moieties on their external surface are described in European Patent No. EP 0 445 131 B1 and WO 90/04384 to Fisher. Liposome compositions containing 1-20 mole percent of PE derivatized with PEG, and methods of use thereof, are described by Woodle et al. (U.S. Pat. Nos. 5,013,556 and 5,356,633) and Martin et al. (U.S. Pat. No. 5,213,804 and European Patent No. EP 0 496 813 B1). Liposomes comprising a number of other lipid-polymer conjugates are disclosed in WO 91/05545 and U.S. Pat. No. 5,225,212 (both to Martin et al.) and in WO 94/20073 (Zalipsky et al.) Liposomes comprising PEG-modified ceramide lipids are described in WO 96/10391 (Choi et al). U.S. Pat. No. 5,540,935 (Miyazaki et al.) and U.S. Pat. No. 5,556,948 (Tagawa et al.) describe PEG-containing liposomes that can be further derivatized with functional moieties on their surfaces.

A number of liposomes comprising nucleic acids are known in the art. WO 96/40062 to Thierry et al. discloses methods for encapsulating high molecular weight nucleic acids in liposomes. U.S. Pat. No. 5,264,221 to Tagawa et al. discloses protein-bonded liposomes and asserts that the contents of such liposomes may include a dsRNA. U.S. Pat. No. 5,665,710 to Rahman et al. describes certain methods of encapsulating oligodeoxynucleotides in liposomes. WO 97/04787 to Love et al. discloses liposomes comprising dsRNAs targeted to the raf gene.

Transfersomes are yet another type of liposomes, and are highly deformable lipid aggregates which are attractive candidates for drug delivery vehicles. Transfersomes may be described as lipid droplets which are so highly deformable that they are easily able to penetrate through pores which are smaller than the droplet. Transfersomes are adaptable to the environment in which they are used, *e.g.*, they are self-optimizing (adaptive to the shape of pores in the skin), self-repairing, frequently reach their targets without fragmenting, and often self-loading. To make transfersomes it is possible to add surface edge-activators, usually surfactants, to a standard liposomal composition. Transfersomes have been used to deliver serum albumin to the skin. The transfersome-mediated delivery of serum albumin has been shown to be as effective as subcutaneous injection of a solution containing serum albumin.

Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature of the hydrophilic group (also known

as the "head") provides the most useful means for categorizing the different surfactants used in formulations (Rieger, in Pharmaceutical Dosage Forms, Marcel Dekker, Inc., New York, N.Y., 1988, p. 285).

If the surfactant molecule is not ionized, it is classified as a nonionic surfactant. Nonionic surfactants find wide application in pharmaceutical and cosmetic products and are usable over a wide range of pH values. In general their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in Pharmaceutical Dosage Forms, Marcel Dekker, Inc., New York, N.Y., 1988, p. 285).

Nucleic acid lipid particles

A TTR dsRNA described herein may be fully encapsulated in the lipid formulation, e.g., to form a SPLP, pSPLP, SNALP, or other nucleic acid-lipid particle. As used herein, the term "SNALP" refers to a stable nucleic acid-lipid particle, including SPLP. As used herein, the term "SPLP" refers to a nucleic acid-lipid particle comprising plasmid DNA encapsulated within a lipid vesicle. SNALPs and SPLPs typically contain a cationic lipid, a non-cationic lipid, and a lipid that prevents aggregation of the particle (*e.g.*, a PEG-lipid conjugate). SNALPs and SPLPs are extremely useful for systemic applications, as they exhibit extended circulation lifetimes following intravenous (i.v.) injection and accumulate at distal sites (*e.g.*, sites physically separated from the administration site). SPLPs include "pSPLP", which include an encapsulated condensing agent-nucleic acid complex as set forth in PCT Publication No. WO 00/03683. The particles described herein typically have a mean diameter of about 50 nm to about 150 nm, more typically about 60 nm to about 130 nm, more typically about 70 nm to about 110 nm, most typically about 70 nm to about 90 nm, and are substantially nontoxic. In addition, the nucleic acids when present in the nucleic acid- lipid particles described herein are resistant in aqueous solution to degradation with a nuclease. Nucleic acid-lipid particles and their method of preparation are disclosed in, *e.g.*, U.S. Patent Nos. 5,976,567; 5,981,501; 6,534,484; 6,586,410; 6,815,432; and PCT Publication No. WO 96/40964.

The lipid to drug ratio (mass/mass ratio) (*e.g.*, lipid to dsRNA ratio) may be in the range of from about 1:1 to about 50:1, from about 1:1 to about 25:1, from about 3:1 to about 15:1, from about 4:1 to about 10:1, from about 5:1 to about 9:1, or about 6:1 to about 9:1. The lipid to dsRNA ratio can be about 1:1, 2:1, 3:1, 4:1, 5:1, 6:1, 7:1, 8:1, 9:1, 10:1, or 11:1.

In general, the lipid-nucleic acid particle is suspended in a buffer, e.g., PBS, for administration. The pH of the lipid formulated siRNA may be between 6.8 and 7.8, e.g., 7.3 or 7.4. The osmolality can be, e.g., between 250 and 350 mOsm/kg, e.g., around 300, e.g., 298, 299, 300, 301, 302, 303, 304, or 305.

The cationic lipid may be, for example, N,N-dioleyl-N,N-dimethylammonium chloride (DODAC), N,N-distearyl-N,N-dimethylammonium bromide (DDAB), N-(I-(2,3-dioleoyloxy)propyl)-N,N,N-trimethylammonium chloride (DOTAP), N-(I-(2,3-dioleyloxy)propyl)-N,N,N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), 1,2-Dilinoleylcarbamoyloxy-3-dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3 -dimethylaminopropane (DLin-2-DMAP), 1,2-Dilinoleyloxy-3-trimethylaminopropane chloride salt (DLin-TMA.Cl), 1,2-Dilinoleoyl-3-trimethylaminopropane chloride salt (DLin-TAP.Cl), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DMA), 1,2-Dilinolenyloxy-N,N-dimethylaminopropane (DLinDMA), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1), or a mixture thereof. The cationic lipid may comprise from about 20 mol % to about 50 mol % or about 40 mol % of the total lipid present in the particle.

The non-cationic lipid may be an anionic lipid or a neutral lipid including, but not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE), dioleoyl-phosphatidylethanolamine-4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1 -trans PE, 1-stearoyl-2-oleoyl-phosphatidyethanolamine (SOPE), cholesterol, or a mixture thereof. The non-cationic lipid may be from about 5 mol % to about 90 mol %, about 10 mol %, or about 58 mol % if cholesterol is included, of the total lipid present in the particle.

The conjugated lipid that inhibits aggregation of particles may be, for example, a polyethyleneglycol (PEG)-lipid including, without limitation, a PEG-diacylglycerol (DAG), a PEG-dialkyloxypropyl (DAA), a PEG-phospholipid, a PEG-ceramide (Cer), or a mixture thereof. The PEG-DAA conjugate may be, for example, a PEG-dilauryloxypropyl ($Ci_2$), a PEG-dimyristyloxypropyl ($Ci_4$), a PEG-dipalmityloxypropyl ($Cl_6$), or a PEG- distearyloxypropyl ($C_{18}$). Other examples of PEG conjugates include PEG-cDMA (N-[(methoxy poly(ethylene glycol)2000)carbamyl]-1,2-dimyristyloxlpropyl-3-amine), mPEG2000-DMG (mPEG-dimyrystylglycerol (with an average molecular weight of 2,000) and PEG-C-DOMG (R-3-[(ω-methoxy-poly(ethylene glycol)2000)carbamoyl)]-1,2-dimyristyloxlpropyl-3-amine). The conjugated lipid that prevents aggregation of particles may be from 0 mol % to about 20 mol % or about 1.0, 1.1., 1.2, 1.3, 1.4, 1.5, 1.6,1.7, 1.8, 1.9, or 2 mol % of the total lipid present in the particle.

The nucleic acid-lipid particle may further include cholesterol at, *e.g.*, about 10 mol % to about 60 mol % or about 48 mol % of the total lipid present in the particle.

The compound 2,2-Dilinoleyl-4-dimethylaminoethyl-[1,3]-dioxolane can be used to prepare lipid-siRNA nanoparticles. Synthesis of 2,2-Dilinoleyl-4-dimethylaminoethyl-[1,3]-dioxolane is described in United States provisional patent application number 61/107,998 filed on October 23, 2008.

For example, the lipid-siRNA particle can include 40% 2, 2-Dilinoleyl-4-dimethylaminoethyl-[1,3]-dioxolane: 10% DSPC: 40% Cholesterol: 10% PEG-C-DOMG (mole percent) with a particle size of 63.0 $\pm$ 20 nm and a 0.027 siRNA/Lipid Ratio.

The compound 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1) can be used to prepare lipid-siRNA particles. For example, the dsRNA can be formulated in a lipid formulation comprising Tech-G1, distearoyl phosphatidylcholine (DSPC), cholesterol and mPEG2000-DMG at a molar ratio of 50:10:38.5:1.5 at a total lipid to siRNA ratio of 7:1 (wt:wt).

LNP01

The lipidoid ND98·4HCl (MW 1487) (Formula 1), Cholesterol (Sigma-Aldrich), and PEG-Ceramide C16 (Avanti Polar Lipids) can be used to prepare lipid-siRNA nanoparticles (*i.e.*, LNP01 particles). Stock solutions of each in ethanol can be prepared as follows: ND98, 133 mg/ml; Cholesterol, 25 mg/ml, PEG-Ceramide C16, 100 mg/ml. The ND98, Cholesterol, and PEG-Ceramide C16 stock solutions can then be combined in a, *e.g.*, 42:48:10 molar ratio. The combined lipid solution can be mixed with aqueous siRNA (*e.g.*, in sodium acetate pH 5) such that the final ethanol concentration is about 35-45% and the final sodium acetate concentration is about 100-300 mM. Lipid-siRNA nanoparticles typically form spontaneously upon mixing. Depending on the desired particle size distribution, the resultant nanoparticle mixture can be extruded through a polycarbonate membrane (*e.g.*, 100 nm cut-off) using, for example, a thermobarrel extruder, such as Lipex Extruder (Northern Lipids, Inc). In some cases, the extrusion step can be omitted. Ethanol removal and simultaneous buffer exchange can be accomplished by, for example, dialysis or tangential flow filtration. Buffer can be exchanged with, for example, phosphate buffered saline (PBS) at about pH 7, *e.g.*, about pH 6.9, about pH 7.0, about pH 7.1, about pH 7.2, about pH 7.3, or about pH 7.4.

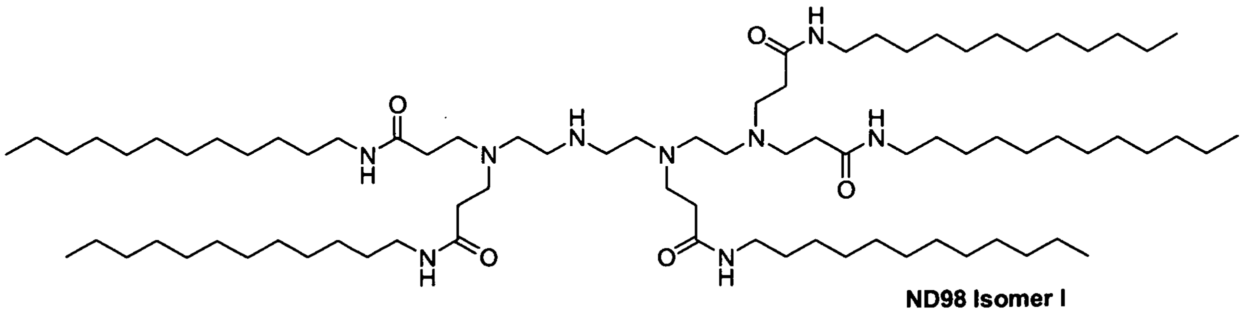

ND98 Isomer I

Formula 1

LNP01 formulations are described, *e.g.*, in International Application Publication No. WO 2008/042973.

Additional exemplary lipid-siRNA formulations are as follows:

| | Cationic Lipid | cationic lipid/non-cationic lipid/cholesterol/PEG-lipid conjugate Lipid:siRNA ratio | Process |
|---|---|---|---|
| SNALP | 1,2-Dilinolenyloxy-N,N-dimethylaminopropane (DLinDMA) | DLinDMA/DPPC/Cholesterol/PEG-cDMA<br>(57.1/7.1/34.4/1.4)<br>lipid:siRNA ~ 7:1 | |
| SNALP-XTC | 2,2-Dilinoleyl-4-dimethylaminoethyl-[1,3]-dioxolane (XTC) | XTC/DPPC/Cholesterol/PEG-cDMA<br>57.1/7.1/34.4/1.4<br>lipid:siRNA ~ 7:1 | |
| LNP05 | 2,2-Dilinoleyl-4-dimethylaminoethyl-[1,3]-dioxolane (XTC) | XTC/DSPC/Cholesterol/PEG-DMG<br>57.5/7.5/31.5/3.5<br>lipid:siRNA ~ 6:1 | Extrusion |
| LNP06 | 2,2-Dilinoleyl-4-dimethylaminoethyl-[1,3]-dioxolane (XTC) | XTC/DSPC/Cholesterol/PEG-DMG<br>57.5/7.5/31.5/3.5<br>lipid:siRNA ~ 11:1 | Extrusion |
| LNP07 | 2,2-Dilinoleyl-4-dimethylaminoethyl-[1,3]-dioxolane (XTC) | XTC/DSPC/Cholesterol/PEG-DMG<br>60/7.5/31/1.5,<br>lipid:siRNA ~ 6:1 | In-line mixing |
| LNP08 | 2,2-Dilinoleyl-4-dimethylaminoethyl-[1,3]-dioxolane (XTC) | XTC/DSPC/Cholesterol/PEG-DMG<br>60/7.5/31/1.5,<br>lipid:siRNA ~ 11:1 | In-line mixing |
| LNP09 | 2,2-Dilinoleyl-4-dimethylaminoethyl-[1,3]-dioxolane (XTC) | XTC/DSPC/Cholesterol/PEG-DMG<br>50/10/38.5/1.5<br>Lipid:siRNA 10:1 | In-line mixing |
| LNP10 | (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100) | ALN100/DSPC/Cholesterol/PEG-DMG<br>50/10/38.5/1.5<br>Lipid:siRNA 10:1 | In-line mixing |
| LNP11 | (6Z,9Z,28Z,31Z)-heptatriaconta-6,9,28,31-tetraen-19-yl 4-(dimethylamino)butanoate (MC3) | MC-3/DSPC/Cholesterol/PEG-DMG<br>50/10/38.5/1.5<br>Lipid:siRNA 10:1 | In-line mixing |
| LNP12 | 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1) | Tech G1/DSPC/Cholesterol/PEG-DMG<br>50/10/38.5/1.5<br>Lipid:siRNA 10:1 | In-line mixing |

LNP09 formulations and XTC comprising formulations are described, e.g., in U.S. Provisional Serial No. 61/239,686, filed September 3, 2009.

LNP11 formulations and MC3 comprising formulations are described, e.g., in U.S. Provisional Serial No. 61/244,834, filed September 22, 2009.

LNP12 formulations and TechG1 comprising formulations are described, e.g., in U.S. Provisional Serial No. 61/175,770, filed May 5, 2009.

Formulations prepared by either the standard or extrusion-free method can be characterized in similar manners. For example, formulations are typically characterized by visual inspection. They should be whitish translucent solutions free from aggregates or sediment. Particle size and particle size distribution of lipid-nanoparticles can be measured by light scattering using, for example, a Malvern Zetasizer Nano ZS (Malvern, USA). Particles should be about 20-300 nm, such as 40-100 nm in size. The particle size distribution should be unimodal. The total siRNA concentration in the formulation, as well as the entrapped fraction, is estimated using a dye exclusion assay. A sample of the formulated siRNA can be incubated with an RNA-binding dye, such as Ribogreen (Molecular Probes) in the presence or absence of a formulation disrupting surfactant, *e.g.*, 0.5% Triton-X100. The total siRNA in the formulation can be determined by the signal from the sample containing the surfactant, relative to a standard curve. The entrapped fraction is determined by subtracting the "free" siRNA content (as measured by the signal in the absence of surfactant) from the total siRNA content. Percent entrapped siRNA is typically >85%. For SNALP formulation, the particle size is at least 30 nm, at least 40 nm, at least 50 nm, at least 60 nm, at least 70 nm, at least 80 nm, at least 90 nm, at least 100 nm, at least 110 nm, and at least 120 nm. The suitable range is typically about at least 50 nm to about at least 110 nm, about at least 60 nm to about at least 100 nm, or about at least 80 nm to about at least 90 nm.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Oral formulations may be those in which dsRNAs described herein are administered in conjunction with one or more penetration enhancers surfactants and chelators. Suitable surfactants include fatty acids and/or esters or salts thereof, bile acids and/or salts thereof. Suitable bile acids/salts include chenodeoxycholic acid (CDCA) and ursodeoxychenodeoxycholic acid (UDCA), cholic acid, dehydrocholic acid, deoxycholic acid, glucholic acid, glycholic acid, glycodeoxycholic acid, taurocholic acid, taurodeoxycholic acid, sodium tauro-24,25-dihydro-fusidate and sodium glycodihydrofusidate. Suitable fatty acids include arachidonic acid, undecanoic acid, oleic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a monoglyceride, a diglyceride or a pharmaceutically acceptable salt thereof (e.g., sodium). Combinations of penetration enhancers may beused, for example, fatty acids/salts in combination with bile acids/salts. One exemplary combination is the sodium salt of lauric acid, capric acid and UDCA. Further penetration enhancers include polyoxyethylene-9-lauryl ether, polyoxyethylene-20-cetyl ether. DsRNAs described herein may be delivered orally, in granular form including sprayed dried particles, or complexed to form micro or nanoparticles. DsRNA complexing agents include poly-amino acids; polyimines; polyacrylates; polyalkylacrylates, polyoxethanes, polyalkylcyanoacrylates; cationized gelatins, albumins, starches, acrylates, polyethyleneglycols (PEG) and starches; polyalkylcyanoacrylates; DEAE-derivatized polyimines, pollulans, celluloses and starches. Suitable complexing agents include chitosan, N-trimethylchitosan, poly-L-lysine, polyhistidine, polyornithine, polyspermines, protamine, polyvinylpyridine, polythiodiethylaminomethylethylene P(TDAE), polyaminostyrene (*e.g.*, p-amino), poly(methylcyanoacrylate), poly(ethylcyanoacrylate), poly(butylcyanoacrylate), poly(isobutylcyanoacrylate), poly(isohexylcynaoacrylate), DEAE-methacrylate, DEAE-hexylacrylate, DEAE-acrylamide, DEAE-albumin and DEAE-dextran, polymethylacrylate, polyhexylacrylate, poly(D,L-lactic acid), poly(DL-lactic-co-glycolic acid (PLGA), alginate, and polyethyleneglycol (PEG). Oral formulations for dsRNAs and their preparation are described in detail in U.S. Patent 6,887,906, US Publn. No. 20030027780, and U.S. Patent No. 6,747,014.

Compositions and formulations for parenteral, intraparenchymal (into the brain), intrathecal, intraventricular or intrahepatic administration may include sterile aqueous solutions which may also contain buffers, diluents and other suitable additives such as, but not limited to, penetration enhancers, carrier compounds and other pharmaceutically acceptable carriers or excipients.

Pharmaceutical compositions described herein include, but are not limited to, solutions, emulsions, and liposome-containing formulations. These compositions may be generated from a variety of components that include, but are not limited to, preformed liquids, self-emulsifying solids and self-emulsifying semisolids. Particularly preferred are formulations that target the liver when treating hepatic disorders such as hepatic carcinoma.

The pharmaceutical formulations described herein, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions described herein may be formulated into any of many possible dosage forms such as, but not limited to, tablets, capsules, gel capsules, liquid syrups, soft gels, suppositories, and enemas. The compositions described herein may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

Emulsions

The compositions described herein may be prepared and formulated as emulsions. Emulsions are typically heterogeneous systems of one liquid dispersed in another in the form of droplets usually exceeding 0.1 μm in diameter (Idson, in Pharmaceutical Dosage Forms, Lieberman, Rieger and Banker (Eds.), 1988, Marcel Dekker, Inc., New York, N.Y., volume 1, p. 199; Rosoff, in Pharmaceutical Dosage Forms, Lieberman, Rieger and Banker (Eds.), 1988, Marcel Dekker, Inc., New York, N.Y., Volume 1, p. 245; Block in Pharmaceutical Dosage Forms, Lieberman, Rieger and Banker (Eds.), 1988, Marcel Dekker, Inc., New York, N.Y., volume 2, p. 335; Higuchi et al., in Remington's Pharmaceutical Sciences, Mack Publishing Co., Easton, Pa., 1985, p. 301). Emulsions are often biphasic systems comprising two immiscible liquid phases intimately mixed and dispersed with each other. In general, emulsions may be of either the water-in-oil (w/o) or the oil-in-water (o/w) variety. When an aqueous phase is finely divided into and dispersed as minute droplets into a bulk oily phase, the resulting composition is called a water-in-oil (w/o) emulsion. Alternatively, when an oily phase is finely divided into and dispersed as minute droplets into a bulk aqueous phase, the resulting composition is called an oil-in-water (o/w) emulsion. Emulsions may contain additional components in addition to the dispersed phases, and the active drug which may be present as a solution in either the aqueous phase, oily phase or itself as a separate phase. Pharmaceutical excipients such as emulsifiers, stabilizers, dyes, and anti-oxidants may also be present in emulsions as needed. Pharmaceutical emulsions may also be multiple emulsions that are comprised of more than two phases such as, for example, in the case of oil-in-water-in-oil (o/w/o) and water-in-oil-in-water (w/o/w) emulsions. Such complex formulations often provide certain advantages that simple binary emulsions do not. Multiple emulsions in which individual oil droplets of an o/w emulsion enclose small water droplets constitute a w/o/w emulsion. Likewise a system of oil droplets enclosed in globules of water stabilized in an oily continuous phase provides an o/w/o emulsion.

Emulsions are characterized by little or no thermodynamic stability. Often, the dispersed or discontinuous phase of the emulsion is well dispersed into the external or continuous phase and maintained in this form through the means of emulsifiers or the viscosity of the formulation. Either of the phases of the emulsion may be a semisolid or a solid, as is the case of emulsion-style ointment bases and creams. Other means of stabilizing emulsions entail the use of emulsifiers that may be incorporated into either phase of the emulsion. Emulsifiers may broadly be classified into four categories: synthetic surfactants, naturally occurring emulsifiers, absorption bases, and finely dispersed solids (Idson, in Pharmaceutical Dosage Forms, Lieberman, Rieger and Banker (Eds.), 1988, Marcel Dekker, Inc., New York, N.Y., volume 1, p. 199).

Synthetic surfactants, also known as surface active agents, have found wide applicability in the formulation of emulsions and have been reviewed in the literature (Rieger, in Pharmaceutical Dosage Forms, Lieberman, Rieger and Banker (Eds.), 1988, Marcel Dekker, Inc., New York, N.Y., volume 1, p. 285; Idson, in Pharmaceutical Dosage Forms, Lieberman, Rieger and Banker (Eds.), Marcel Dekker, Inc., New York, N.Y., 1988, volume 1, p. 199). Surfactants are typically amphiphilic and comprise a hydrophilic and a hydrophobic portion. The ratio of the hydrophilic to the hydrophobic nature of the surfactant has been termed the hydrophile/lipophile balance (HLB) and is a valuable tool in categorizing and selecting surfactants in the preparation of formulations. Surfactants may be classified into different classes based on the nature of the hydrophilic group: nonionic, anionic, cationic and amphoteric (Rieger, in Pharmaceutical Dosage Forms, Lieberman, Rieger and Banker (Eds.), 1988, Marcel Dekker, Inc., New York, N.Y., volume 1, p. 285).

Naturally occurring emulsifiers used in emulsion formulations include lanolin, beeswax, phosphatides, lecithin and acacia. Absorption bases possess hydrophilic properties such that they can soak up water to form w/o emulsions yet retain their semisolid consistencies, such as anhydrous lanolin and hydrophilic petrolatum. Finely divided solids have also been used as good emulsifiers especially in combination with surfactants and in viscous preparations. These include polar inorganic solids, such as heavy metal hydroxides, nonswelling clays such as bentonite, attapulgite, hectorite, kaolin, montmorillonite, colloidal aluminum silicate and colloidal magnesium aluminum silicate, pigments and nonpolar solids such as carbon or glyceryl tristearate.

A large variety of non-emulsifying materials are also included in emulsion formulations and contribute to the properties of emulsions. These include fats, oils, waxes, fatty acids, fatty alcohols, fatty esters, humectants, hydrophilic colloids, preservatives and antioxidants (Block, in Pharmaceutical Dosage Forms, Lieberman, Rieger and Banker (Eds.), 1988, Marcel Dekker, Inc., New York, N.Y., volume 1, p. 335; Idson, in Pharmaceutical Dosage Forms, Lieberman, Rieger and Banker (Eds.), 1988, Marcel Dekker, Inc., New York, N.Y., volume 1, p. 199).

Hydrophilic colloids or hydrocolloids include naturally occurring gums and synthetic polymers such as polysaccharides (for example, acacia, agar, alginic acid, carrageenan, guar gum, karaya gum, and tragacanth), cellulose derivatives (for example, carboxymethylcellulose and carboxypropylcellulose), and synthetic polymers (for example, carbomers, cellulose ethers, and carboxyvinyl polymers). These disperse or swell in water to form colloidal solutions that stabilize emulsions by forming strong interfacial films around the dispersed-phase droplets and by increasing the viscosity of the external phase.

Since emulsions often contain a number of ingredients such as carbohydrates, proteins, sterols and phosphatides that may readily support the growth of microbes, these formulations often incorporate preservatives. Commonly used preservatives included in emulsion formulations include methyl paraben, propyl paraben, quaternary ammonium salts, benzalkonium chloride, esters of p-hydroxybenzoic acid, and boric acid. Antioxidants are also commonly added to emulsion formulations to prevent deterioration of the formulation. Antioxidants used may be free radical scavengers such as tocopherols, alkyl gallates, butylated hydroxyanisole, butylated hydroxytoluene, or reducing agents such as ascorbic acid and sodium metabisulfite, and antioxidant synergists such as citric acid, tartaric acid, and lecithin.

The application of emulsion formulations via dermatological, oral and parenteral routes and methods for their manufacture have been reviewed in the literature (Idson, in Pharmaceutical Dosage Forms, Lieberman, Rieger and Banker (Eds.), 1988, Marcel Dekker, Inc., New York, N.Y., volume 1, p. 199). Emulsion formulations for oral delivery have been very widely used because of ease of formulation, as well as efficacy from an absorption and bioavailability standpoint (Rosoff, in Pharmaceutical Dosage Forms, Lieberman, Rieger and Banker (Eds.), 1988, Marcel Dekker, Inc., New York, N.Y., volume 1, p. 245; Idson, in Pharmaceutical Dosage Forms, Lieberman, Rieger and Banker (Eds.), 1988, Marcel Dekker, Inc., New York, N.Y., volume 1, p. 199). Mineral-oil base laxatives, oil-soluble vitamins and high fat nutritive preparations are among the materials that have commonly been administered orally as o/w emulsions.

The compositions of dsRNAs and nucleic acids may be formulated as microemulsions. A microemulsion may be defined as a system of water, oil and amphiphile which is a single optically isotropic and thermodynamically stable liquid solution (Rosoff, in Pharmaceutical Dosage Forms, Lieberman, Rieger and Banker (Eds.), 1988, Marcel Dekker, Inc., New York, N.Y., volume 1, p. 245). Typically microemulsions are systems that are prepared by first dispersing an oil in an aqueous surfactant solution and then adding a sufficient amount of a fourth component, generally an intermediate chain-length alcohol to form a transparent system. Therefore, microemulsions have also been described as thermodynamically stable, isotropically clear dispersions of two immiscible liquids that are stabilized by interfacial films of surface-active molecules (Leung and Shah, in: Controlled Release of Drugs: Polymers and Aggregate Systems, Rosoff, M., Ed., 1989, VCH Publishers, New York, pages 185-215). Microemulsions commonly are prepared via a combination of three to five components that include oil, water, surfactant, cosurfactant and electrolyte. Whether the microemulsion is of the water-in-oil (w/o) or an oil-in-water (o/w) type is dependent on the properties of the oil and surfactant used and on the structure and geometric packing of the polar heads and hydrocarbon tails of the surfactant molecules (Schott, in Remington's Pharmaceutical Sciences, Mack Publishing Co., Easton, Pa., 1985, p. 271).

The phenomenological approach utilizing phase diagrams has been extensively studied and has yielded a comprehensive knowledge, to one skilled in the art, of how to formulate microemulsions (Rosoff, in Pharmaceutical Dosage Forms, Lieberman, Rieger and Banker (Eds.), 1988, Marcel Dekker, Inc., New York, N.Y., volume 1, p. 245; Block, in Pharmaceutical Dosage Forms, Lieberman, Rieger and Banker (Eds.), 1988, Marcel Dekker, Inc., New York, N.Y., volume 1, p. 335). Compared to conventional emulsions, microemulsions offer the advantage of solubilizing water-insoluble drugs in a formulation of thermodynamically stable droplets that are formed spontaneously.

Surfactants used in the preparation of microemulsions include, but are not limited to, ionic surfactants, non-ionic surfactants, Brij 96, polyoxyethylene oleyl ethers, polyglycerol fatty acid esters, tetraglycerol monolaurate (ML310), tetraglycerol monooleate (MO310), hexaglycerol monooleate (PO310), hexaglycerol pentaoleate (PO500), decaglycerol monocaprate (MCA750), decaglycerol monooleate (MO750), decaglycerol sequioleate (SO750), decaglycerol decaoleate (DAO750), alone or in combination with cosurfactants. The cosurfactant, usually a short-chain alcohol such as ethanol, 1-propanol, and 1-butanol, serves to increase the interfacial fluidity by penetrating into the surfactant film and consequently creating a disordered film because of the void space generated among surfactant molecules. Microemulsions may, however, be prepared without the use of cosurfactants and alcohol-free self-emulsifying microemulsion systems are known in the art. The aqueous phase may typically be, but is not limited to, water, an aqueous solution of the drug, glycerol, PEG300, PEG400, polyglycerols, propylene glycols, and derivatives of ethylene glycol. The oil phase may include, but is not limited to, materials such as Captex 300, Captex 355, Capmul MCM, fatty acid esters, medium chain (C8-C12) mono, di, and tri-glycerides, polyoxyethylated glyceryl fatty acid esters, fatty alcohols, polyglycolized glycerides, saturated polyglycolized C8-C10 glycerides, vegetable oils and silicone oil.

Microemulsions are particularly of interest from the standpoint of drug solubilization and the enhanced absorption of drugs. Lipid based microemulsions (both o/w and w/o) have been proposed to enhance the oral bioavailability of drugs, including peptides (Constantinides et al., Pharmaceutical Research, 1994, 11, 1385-1390; Ritschel, Meth. Find. Exp. Clin. Pharmacol., 1993, 13, 205). Microemulsions afford advantages of improved drug solubilization, protection of drug from enzymatic hydrolysis, possible enhancement of drug absorption due to surfactant-induced alterations in membrane fluidity and permeability, ease of preparation, ease of oral administration over solid dosage forms, improved clinical potency, and decreased toxicity (Constantinides et al., Pharmaceutical Research, 1994, 11, 1385; Ho et al., J. Pharm.

Sci., 1996, 85, 138-143). Often microemulsions may form spontaneously when their components are brought together at ambient temperature. This may be particularly advantageous when formulating thermolabile drugs, peptides or dsRNAs. Microemulsions have also been effective in the transdermal delivery of active components in both cosmetic and pharmaceutical applications. It is expected that the microemulsion compositions and formulations described herein will facilitate the increased systemic absorption of dsRNAs and nucleic acids from the gastrointestinal tract, as well as improve the local cellular uptake of dsRNAs and nucleic acids.

Microemulsions described herein may also contain additional components and additives such as sorbitan monostearate (Grill 3), Labrasol, and penetration enhancers to improve the properties of the formulation and to enhance the absorption of the dsRNAs and nucleic acids described herein. Penetration enhancers used in the microemulsions described herein may be classified as belonging to one of five broad categories--surfactants, fatty acids, bile salts, chelating agents, and non-chelating non-surfactants (Lee et al., Critical Reviews in Therapeutic Drug Carrier Systems, 1991, p. 92). Each of these classes has been discussed above.

Penetration Enhancers

Also employed herein may be various penetration enhancers to effect the efficient delivery of nucleic acids, particularly dsRNAs, to the skin of animals. Most drugs are present in solution in both ionized and nonionized forms. However, usually only lipid soluble or lipophilic drugs readily cross cell membranes. It has been discovered that even non-lipophilic drugs may cross cell membranes if the membrane to be crossed is treated with a penetration enhancer. In addition to aiding the diffusion of non-lipophilic drugs across cell membranes, penetration enhancers also enhance the permeability of lipophilic drugs.

Penetration enhancers may be classified as belonging to one of five broad categories, *i.e.*, surfactants, fatty acids, bile salts, chelating agents, and non-chelating non-surfactants (Lee et al., Critical Reviews in Therapeutic Drug Carrier Systems, 1991, p.92). Each of the above mentioned classes of penetration enhancers are described below in greater detail.

Surfactants: In connection with the present disclosure, surfactants (or "surface-active agents") are chemical entities which, when dissolved in an aqueous solution, reduce the surface tension of the solution or the interfacial tension between the aqueous solution and another liquid, with the result that absorption of dsRNAs through the mucosa is enhanced. In addition to bile salts and fatty acids, these penetration enhancers include, for example, sodium lauryl sulfate, polyoxyethylene-9-lauryl ether and polyoxyethylene-20-cetyl ether) (Lee et al., Critical Reviews in Therapeutic Drug Carrier Systems, 1991, p.92); and perfluorochemical emulsions, such as FC-43. Takahashi et al., J. Pharm. Pharmacol., 1988, 40, 252).

Fatty acids: Various fatty acids and their derivatives which act as penetration enhancers include, for example, oleic acid, lauric acid, capric acid (n-decanoic acid), myristic acid, palmitic acid, stearic acid, linoleic acid, linolenic acid, dicaprate, tricaprate, monoolein (1-monooleoyl-rac-glycerol), dilaurin, caprylic acid, arachidonic acid, glycerol 1-monocaprate, 1-dodecylazacycloheptan-2-one, acylcarnitines, acylcholines, C.sub.1-10 alkyl esters thereof (*e.g.*, methyl, isopropyl and t-butyl), and mono- and di-glycerides thereof (*i.e.*, oleate, laurate, caprate, myristate, palmitate, stearate, linoleate, *etc*.) (Lee et al., Critical Reviews in Therapeutic Drug Carrier Systems, 1991, p.92; Muranishi, Critical Reviews in Therapeutic Drug Carrier Systems, 1990, 7, 1-33; El Hariri et al., J. Pharm. Pharmacol., 1992, 44, 651-654).

Bile salts: The physiological role of bile includes the facilitation of dispersion and absorption of lipids and fat-soluble vitamins (Brunton, Chapter 38 in: Goodman & Gilman's The Pharmacological Basis of Therapeutics, 9th Ed., Hardman et al. Eds., McGraw-Hill, New York, 1996, pp. 934-935). Various natural bile salts, and their synthetic derivatives, act as penetration enhancers. Thus the term "bile salts" includes any of the naturally occurring components of bile as well as any of their synthetic derivatives. Suitable bile salts include, for example, cholic acid (or its pharmaceutically acceptable sodium salt, sodium cholate), dehydrocholic acid (sodium dehydrocholate), deoxycholic acid (sodium deoxycholate), glucholic acid (sodium glucholate), glycholic acid (sodium glycocholate), glycodeoxycholic acid (sodium glycodeoxycholate), taurocholic acid (sodium taurocholate), taurodeoxycholic acid (sodium taurodeoxycholate), chenodeoxycholic acid (sodium chenodeoxycholate), ursodeoxycholic acid (UDCA), sodium tauro-24,25-dihydro-fusidate (STDHF), sodium glycodihydrofusidate and polyoxyethylene-9-lauryl ether (POE) (Lee et al., Critical Reviews in Therapeutic Drug Carrier Systems, 1991, page 92; Swinyard, Chapter 39 In: Remington's Pharmaceutical Sciences, 18th Ed., Gennaro, ed., Mack Publishing Co., Easton, Pa., 1990, pages 782-783; Muranishi, Critical Reviews in Therapeutic Drug Carrier Systems, 1990, 7, 1-33; Yamamoto et al., J. Pharm. Exp. Ther., 1992, 263, 25; Yamashita et al., J. Pharm. Sci., 1990, 79, 579-583).

Chelating Agents: Chelating agents, as used herein, can be defined as compounds that remove metallic ions from solution by forming complexes therewith, with the result that absorption of dsRNAs through the mucosa is enhanced. With regards to their use as penetration enhancers in the present disclosure, chelating agents have the added advantage of also serving as DNase inhibitors, as most characterized DNA nucleases require a divalent metal ion for catalysis and are thus inhibited by chelating agents (Jarrett, J. Chromatogr., 1993, 618, 315-339). Suitable chelating agents include

but are not limited to disodium ethylenediaminetetraacetate (EDTA), citric acid, salicylates (*e.g.*, sodium salicylate, 5-methoxysalicylate and homovanilate), N-acyl derivatives of collagen, laureth-9 and N-amino acyl derivatives of beta-diketones (enamines)(Lee et al., Critical Reviews in Therapeutic Drug Carrier Systems, 1991, page 92; Muranishi, Critical Reviews in Therapeutic Drug Carrier Systems, 1990, 7, 1-33; Buur et al., J. Control Rel., 1990, 14, 43-51).

Non-chelating non-surfactants: As used herein, non-chelating non-surfactant penetration enhancing compounds can be defined as compounds that demonstrate insignificant activity as chelating agents or as surfactants but that nonetheless enhance absorption of dsRNAs through the alimentary mucosa (Muranishi, Critical Reviews in Therapeutic Drug Carrier Systems, 1990, 7, 1-33). This class of penetration enhancers include, for example, unsaturated cyclic ureas, 1-alkyl- and 1-alkenylazacyclo-alkanone derivatives (Lee et al., Critical Reviews in Therapeutic Drug Carrier Systems, 1991, page 92); and non-steroidal anti-inflammatory agents such as diclofenac sodium, indomethacin and phenylbutazone (Yamashita et al., J. Pharm. Pharmacol., 1987, 39, 621-626).

Carriers

Certain compositions described herein also incorporate carrier compounds in the formulation. As used herein, "carrier compound" or "carrier" can refer to a nucleic acid, or analog thereof, which is inert (*i.e.*, does not possess biological activity per se) but is recognized as a nucleic acid by *in vivo* processes that reduce the bioavailability of a nucleic acid having biological activity by, for example, degrading the biologically active nucleic acid or promoting its removal from circulation. The coadministration of a nucleic acid and a carrier compound, typically with an excess of the latter substance, can result in a substantial reduction of the amount of nucleic acid recovered in the liver, kidney or other extracirculatory reservoirs, presumably due to competition between the carrier compound and the nucleic acid for a common receptor. For example, the recovery of a partially phosphorothioate dsRNA in hepatic tissue can be reduced when it is coadministered with polyinosinic acid, dextran sulfate, polycytidic acid or 4-acetamido-4'isothiocyano-stilbene-2,2'-disulfonic acid (Miyao et al., DsRNA Res. Dev., 1995, 5, 115-121; Takakura et al., DsRNA & Nucl. Acid Drug Dev., 1996, 6, 177-183.

Excipients

In contrast to a carrier compound, a "pharmaceutical carrier" or "excipient" is a pharmaceutically acceptable solvent, suspending agent or any other pharmacologically inert vehicle for delivering one or more nucleic acids to an animal. The excipient may be liquid or solid and is selected, with the planned manner of administration in mind, so as to provide for the desired bulk, consistency, *etc.*, when combined with a nucleic acid and the other components of a given pharmaceutical composition. Typical pharmaceutical carriers include, but are not limited to, binding agents (*e.g.*, pregelatinized maize starch, polyvinylpyrrolidone or hydroxypropyl methylcellulose, *etc.*); fillers (*e.g.*, lactose and other sugars, microcrystalline cellulose, pectin, gelatin, calcium sulfate, ethyl cellulose, polyacrylates or calcium hydrogen phosphate, *etc.*); lubricants (*e.g.*, magnesium stearate, talc, silica, colloidal silicon dioxide, stearic acid, metallic stearates, hydrogenated vegetable oils, corn starch, polyethylene glycols, sodium benzoate, sodium acetate, *etc.*); disintegrants (*e.g.*, starch, sodium starch glycolate, *etc.*); and wetting agents (*e.g.*, sodium lauryl sulphate, *etc*).

Pharmaceutically acceptable organic or inorganic excipients suitable for non-parenteral administration which do not deleteriously react with nucleic acids can also be used to formulate the compositions described herein. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

Formulations for topical administration of nucleic acids may include sterile and non-sterile aqueous solutions, non-aqueous solutions in common solvents such as alcohols, or solutions of the nucleic acids in liquid or solid oil bases. The solutions may also contain buffers, diluents and other suitable additives. Pharmaceutically acceptable organic or inorganic excipients suitable for non-parenteral administration which do not deleteriously react with nucleic acids can be used.

Suitable pharmaceutically acceptable excipients include, but are not limited to, water, salt solutions, alcohol, polyethylene glycols, gelatin, lactose, amylose, magnesium stearate, talc, silicic acid, viscous paraffin, hydroxymethylcellulose, polyvinylpyrrolidone and the like.

Other Components

The compositions described herein may additionally contain other adjunct components conventionally found in pharmaceutical compositions, at their art-established usage levels. Thus, for example, the compositions may contain additional, compatible, pharmaceutically-active materials such as, for example, antipruritics, astringents, local anesthetics or anti-inflammatory agents, or may contain additional materials useful in physically formulating various dosage forms

of the compositions described herein, such as dyes, flavoring agents, preservatives, antioxidants, opacifiers, thickening agents and stabilizers. However, such materials, when added, should not unduly interfere with the biological activities of the components of the compositions described herein. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Aqueous suspensions may contain substances which increase the viscosity of the suspension including, for example, sodium carboxymethylcellulose, sorbitol and/or dextran. The suspension may also contain stabilizers.

Pharmaceutical compositions described herein may include (a) one or more dsRNA compounds and (b) one or more anti-cytokine biologic agents which function by a non-RNAi mechanism. Examples of such biologics include, biologics that target IL1β (*e.g.*, anakinra), IL6 (tocilizumab), or TNF (etanercept, infliximab, adlimumab, or certolizumab).

Toxicity and therapeutic efficacy of such compounds can be determined by standard pharmaceutical procedures in cell cultures or experimental animals, *e.g.*, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are preferred.

The data obtained from cell culture assays and animal studies can be used in formulating a range of dosage for use in humans. The dosage of compositions described herein lies generally within a range of circulating concentrations that include the ED50 with little or no toxicity. The dosage may vary within this range depending upon the dosage form employed and the route of administration utilized. For any compound used in the methods described herein, the therapeutically effective dose can be estimated initially from cell culture assays. A dose may be formulated in animal models to achieve a circulating plasma concentration range of the compound or, when appropriate, of the polypeptide product of a target sequence (*e.g.*, achieving a decreased concentration of the polypeptide) that includes the IC50 (*i.e.*, the concentration of the test compound which achieves a half-maximal inhibition of symptoms) as determined in cell culture. Such information can be used to more accurately determine useful doses in humans. Levels in plasma may be measured, for example, by high performance liquid chromatography.

In addition to their administration, as discussed above, the dsRNAs described herein can be administered in combination with other known agents effective in treatment of pathological processes mediated by TTR expression. In any event, the administering physician can adjust the amount and timing of dsRNA administration on the basis of results observed using standard measures of efficacy known in the art or described herein.

**Methods for treating ocular disease caused by expression of a TTR gene**

The invention relates in particular to the use of a dsRNA targeting TTR for the treatment of a TTR-related ocular amyloidosis as defined by the claims. Also described herein is a method of treating, preventing or managing TTR-related ocular amyloidosis by administering to the patient in need of such treatment, prevention or management a therapeutically or prophylacticlaly effective amount of AD-18324 to the retina of the patient. The method may involve treating a human by identifying a human diagnosed as having TTR-related ocular amyloidosis or at risk for developing TTR-related ocular amyloidosis and administering to the human a therapeutically or prophylactically effective amount of AD-18324 to the retina of the human. Also described herein is the method of treating a human with TTR-related ocular amyloidosis by introducing into the retinal epithelium cell a dsRNA, wherein the dsRNA is AD-18324 or AD-18534; and maintaining the cell produced in the previous step for a time sufficient to obtain degradation of the mRNA transcript of a TTR gene, thereby inhibiting expression of the TTR gene in the cell. TTR siRNA as describe herein may be used in methods of transthyretin (TTR)-related familial amyloidotic polyneuropathy (FAP) patients and treatment of ocular manifestations, such as vitreous opacity and glaucoma. It is know to one of skill in the art that amyloidogenic transthyretin (ATTR) synthesized by retinal pigment epithelium (RPE) plays important roles in the progression of ocular amyloidosis. Previous studies have shown that panretinal laser photocoagulation, which reduced the RPE cells, prevented the progression of amyloid deposition in the vitreous, indicating that the effective suppression of ATTR expression in RPE may become a novel therapy for ocular amyloidosis (see, e.g., Kawaji, T., et al., Ophthalmology. (2010) 117: 552-555). Administration of any of the TTR siRNA disclosed herein can be used for treatment of ocular manifestations of TTR related FAP, e.g., ocular amyloidosis. The dsRNA can be delivered in a manner to target a particular tissue, such as the eye. Modes of ocular delivery include retrobulbar, subcutaneous eyelid, subconjunctival, subtenon, anterior chamber or intravitreous injection (or internal injection or infusion). Specific formulations for ocular delivery include eye drops or ointments.

The dsRNA and an additional therapeutic agent can be administered in the same combination, *e.g.*, parenterally, or the additional therapeutic agent can be administered as part of a separate composition or by another method described herein.

Also described herein is a method of administering a dsRNA targeting TTR to a patient having a disease or disorder mediated by TTR expression, such as a TTR amyloidosis, *e.g.*, FAP. Administration of the dsRNA can stabilize

and improve peripheral neurological function, for example, in a patient with FAP. Patients can be administered a therapeutic amount of dsRNA, such as 0.1 mg/kg, 0.2 mg/kg, 0.5 mg/kg, 1.0 mg/kg, 1.5 mg/kg, 2.0 mg/kg, or 2.5 mg/kg dsRNA. The dsRNA can be administered by intravenous infusion over a period of time, such as over a 5 minute, 10 minute, 15 minute, 20 minute, 25 minute, 60 minute, 120 minute or 180 minute period. The administration is repeated, for example, on a regular basis, such as biweekly (*i.e.*, every two weeks) for one month, two months, three months, four months or longer. After an initial treatment regimen, the treatments can be administered on a less frequent basis. For example, after administration biweekly for three months, administration can be repeated once per month, for six months or a year or longer. Administration of the dsRNA can reduce TTR levels in the blood or urine of the patient by at least 20%, 25%, 30%, 40%, 50%, 60%, 70%, 80 % or 90% or more.

Before administration of a full dose of the dsRNA, patients can be administered a smaller dose, such as a dose that is 5% of the full dose, and monitored for adverse effects, such as an allergic reaction or a change in liver function. For example, in patients monitored for changes in liver function, a low incidence of LFT (Liver Function Test) change (*e.g.*, a 10-20% incidence of LFT) is acceptable (*e.g.*, a reversible, 3-fold increase in ALT (alanine aminotransferase) and/or AST (aspartate aminotransferase) levels).

Many TTR-associated diseases and disorders are hereditary. Therefore, a patient in need of a TTR dsRNA can be identified by taking a family history. A healthcare provider, such as a doctor, nurse, or family member, can take a family history before prescribing or administering a TTR dsRNA. A DNA test may also be performed on the patient to identify a mutation in the TTR gene, before a TTR dsRNA is administered to the patient.

The patient may have a biopsy performed before receiving a TTR dsRNA. The biopsy can be, for example, on a tissue, such as the gastric mucosa, peripheral nerve, skin, abdominal fat, liver, or kidney, and the biopsy may reveal amyloid plaques, which are indicative of a TTR-mediated disorder. Upon the identification of amyloid plaques, the patient is administered a TTR dsRNA.

## Methods for inhibiting expression of a TTR gene

Also provided herein is a method for inhibiting the expression of a TTR gene in a mammal. The method includes administering a composition described herein to the mammal such that expression of the target TTR gene is silenced.

When the organism to be treated is a mammal such as a human, the composition may be administered by any means known in the art including, but not limited to oral or parenteral routes, including intracranial (*e.g.*, intraventricular, intraparenchymal and intrathecal), intravenous, intramuscular, subcutaneous, transdermal, airway (aerosol), nasal, rectal, and topical (including buccal and sublingual) administration. The compositions may be administered by intravenous infusion or injection.

Unless otherwise defined, all technical and scientific terms used herein have the same meaning as commonly understood by one of ordinary skill in the art to which this invention belongs. In case of conflict, the present specification, including definitions, will control. In addition, the materials, methods, and examples are illustrative only and not intended to be limiting.

## EXAMPLES

### Example 1. dsRNA synthesis

#### Source of reagents

Where the source of a reagent is not specifically given herein, such reagent may be obtained from any supplier of reagents for molecular biology at a quality/purity standard for application in molecular biology.

#### siRNA synthesis

Single-stranded RNAs were produced by solid phase synthesis on a scale of 1 μmole using an Expedite 8909 synthesizer (Applied Biosystems, Applera Deutschland GmbH, Darmstadt, Germany) and controlled pore glass (CPG, 500Å, Proligo Biochemie GmbH, Hamburg, Germany) as solid support. RNA and RNA containing 2'-*O*-methyl nucleotides were generated by solid phase synthesis employing the corresponding phosphoramidites and 2'-*O*-methyl phosphoramidites, respectively (Proligo Biochemie GmbH, Hamburg, Germany). These building blocks were incorporated at selected sites within the sequence of the oligoribonucleotide chain using standard nucleoside phosphoramidite chemistry such as described in Current protocols in nucleic acid chemistry, Beaucage, S.L. et al. (Edrs.), John Wiley & Sons, Inc., New York, NY, USA. Phosphorothioate linkages were introduced by replacement of the iodine oxidizer solution with a solution of the Beaucage reagent (Chruachem Ltd, Glasgow, UK) in acetonitrile (1%). Further ancillary reagents were obtained from Mallinckrodt Baker (Griesheim, Germany).

Deprotection and purification of the crude oligoribonucleotides by anion exchange HPLC were carried out according to established procedures. Yields and concentrations were determined by UV absorption of a solution of the respective RNA at a wavelength of 260 nm using a spectral photometer (DU 640B, Beckman Coulter GmbH, Unterschleißheim, Germany). Double stranded RNA was generated by mixing an equimolar solution of complementary strands in annealing buffer (20 mM sodium phosphate, pH 6.8; 100 mM sodium chloride), heated in a water bath at 85 - 90°C for 3 minutes and cooled to room temperature over a period of 3 - 4 hours. The annealed RNA solution was stored at -20 °C until use.

For the synthesis of 3'-cholesterol-conjugated siRNAs (herein referred to as -Chol-3'), an appropriately modified solid support was used for RNA synthesis. The modified solid support was prepared as follows:

Diethyl-2-azabutane-1,4-dicarboxylate **AA**

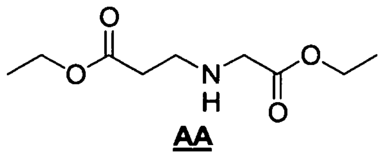

**AA**

A 4.7 M aqueous solution of sodium hydroxide (50 mL) was added into a stirred, ice-cooled solution of ethyl glycinate hydrochloride (32.19 g, 0.23 mole) in water (50 mL). Then, ethyl acrylate (23.1 g, 0.23 mole) was added and the mixture was stirred at room temperature until completion of the reaction was ascertained by TLC. After 19 h the solution was partitioned with dichloromethane (3 x 100 mL). The organic layer was dried with anhydrous sodium sulfate, filtered and evaporated. The residue was distilled to afford AA (28.8 g, 61 %).

3-{Ethoxycarbonylmethyl-[6-(9H-fluoren-9-ylmethoxycarbonyl-amino)-hexanoyl]-amino}-propionic acid ethyl ester **AB**

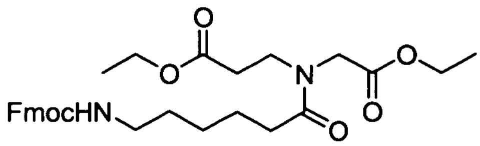

**AB**

Fmoc-6-amino-hexanoic acid (9.12 g, 25.83 mmol) was dissolved in dichloromethane (50 mL) and cooled with ice. Diisopropylcarbodiimde (3.25 g, 3.99 mL, 25.83 mmol) was added to the solution at 0°C. It was then followed by the addition of Diethyl-azabutane-1,4-dicarboxylate (5 g, 24.6 mmol) and dimethylamino pyridine (0.305 g, 2.5 mmol). The solution was brought to room temperature and stirred further for 6 h. Completion of the reaction was ascertained by TLC. The reaction mixture was concentrated under vacuum and ethyl acetate was added to precipitate diisopropyl urea. The suspension was filtered. The filtrate was washed with 5% aqueous hydrochloric acid, 5% sodium bicarbonate and water. The combined organic layer was dried over sodium sulfate and concentrated to give the crude product which was purified by column chromatography (50 % EtOAC/Hexanes) to yield 11.87 g (88%) of AB.

3-[(6-Amino-hexanoyl)-ethoxycarbonylmethyl-amino]-propionic acid ethyl ester **AC**

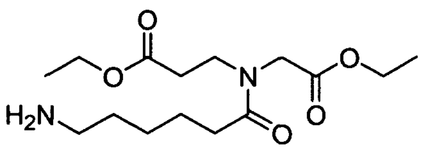

**AC**

3-{Ethoxycarbonylmethyl-[6-(9H-fluoren-9-ylmethoxycarbonylamino)-hexanoyl]-amino}-propionic acid ethyl ester AB (11.5 g, 21.3 mmol) was dissolved in 20% piperidine in dimethylformamide at 0°C. The solution was continued stirring for 1 h. The reaction mixture was concentrated under vacuum, water was added to the residue, and the product was extracted with ethyl acetate. The crude product was purified by conversion into its hydrochloride salt.

3-({6-[17-(1,5-Dimethyl-hexyl)-10,13-dimethyl-2,3,4,7,8,9,10,11,12,13,14,15,16,17-tetradecahydro-1H-cyclopenta[a]phenanthren-3-yloxycarbonylamino]-hexanoyl}ethoxycarbonylmethyl-amino)-propionic acid ethyl ester **AD**

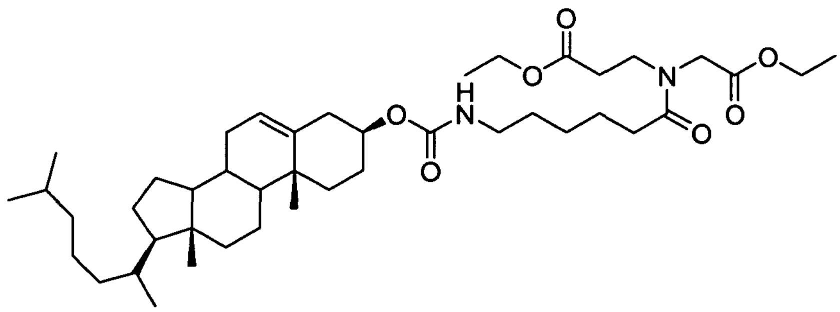

**AD**

The hydrochloride salt of 3-[(6-Amino-hexanoyl)-ethoxycarbonylmethyl-amino]-propionic acid ethyl ester AC (4.7 g, 14.8 mmol) was taken up in dichloromethane. The suspension was cooled to 0°C on ice. To the suspension diisopropylethylamine (3.87 g, 5.2 mL, 30 mmol) was added. To the resulting solution cholesteryl chloroformate (6.675 g, 14.8 mmol) was added. The reaction mixture was stirred overnight. The reaction mixture was diluted with dichloromethane and washed with 10% hydrochloric acid. The product was purified by flash chromatography (10.3 g, 92%).

1-{6-[17-(1,5-Dimethyl-hexyl)-10,13-dimethyl-2,3,4,7,8,9,10,11,12,13,14,15,16,17-tetradecahydro-1H-cyclopenta[a]phenanthren-3-yloxycarbonylamino]-hexanoyl}-4-oxo-pyrrolidine-3-carboxylic acid ethyl ester **AE**

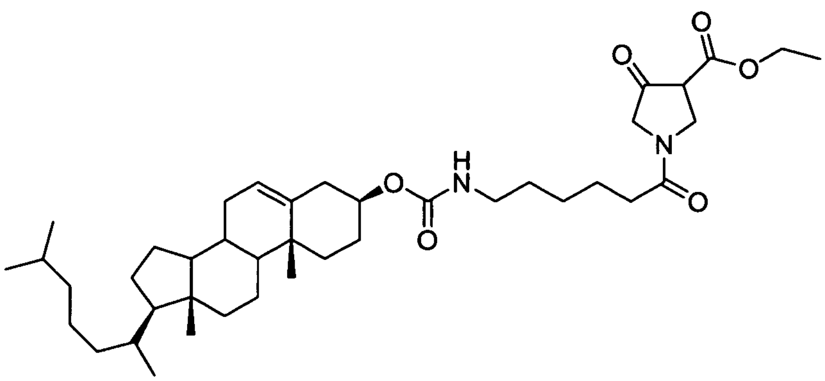

**AE**

Potassium t-butoxide (1.1 g, 9.8 mmol) was slurried in 30 mL of dry toluene. The mixture was cooled to 0°C on ice and 5 g (6.6 mmol) of diester AD was added slowly with stirring within 20 mins. The temperature was kept below 5°C during the addition. The stirring was continued for 30 mins at 0°C and 1 mL of glacial acetic acid was added, immediately followed by 4 g of $NaH_2PO_4 \cdot H_2O$ in 40 mL of water The resultant mixture was extracted twice with 100 mL of dichloromethane each and the combined organic extracts were washed twice with 10 mL of phosphate buffer each, dried, and evaporated to dryness. The residue was dissolved in 60 mL of toluene, cooled to 0°C and extracted with three 50 mL portions of cold pH 9.5 carbonate buffer. The aqueous extracts were adjusted to pH 3 with phosphoric acid, and extracted with five 40 mL portions of chloroform which were combined, dried and evaporated to dryness. The residue

was purified by column chromatography using 25% ethylacetate/hexane to afford 1.9 g of b-ketoester (39%).

[6-(3-Hydroxy-4-hydroxymethyl-pyrrolidin-1-yl)-6-oxo-hexyl]-carbamic acid 17-(1,5-dimethyl-hexyl)-10,13-dimethyl-2,3,4,7,8,9,10,11,12,13,14,15,16,17-tetradecahydro-1H-cyclopenta[a]phenanthren-3-yl ester **AF**

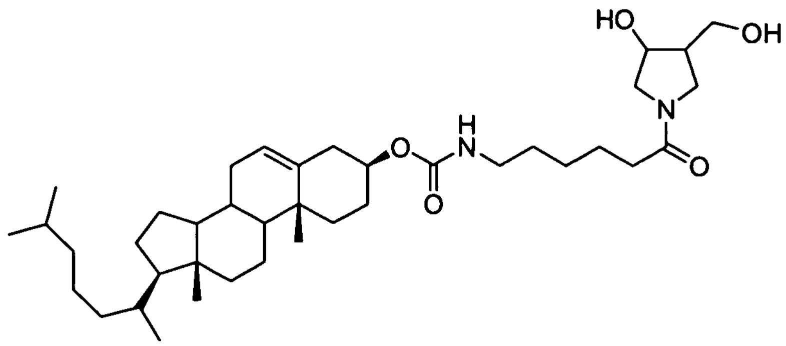

**AF**

Methanol (2 mL) was added dropwise over a period of 1 h to a refluxing mixture of b-ketoester AE (1.5 g, 2.2 mmol) and sodium borohydride (0.226 g, 6 mmol) in tetrahydrofuran (10 mL). Stirring was continued at reflux temperature for 1 h. After cooling to room temperature, 1 N HCl (12.5 mL) was added, the mixture was extracted with ethylacetate (3 x 40 mL). The combined ethylacetate layer was dried over anhydrous sodium sulfate and concentrated under vacuum to yield the product which was purified by column chromatography (10% MeOH/$CHCl_3$) (89%).

(6-{3-[Bis-(4-methoxy-phenyl)-phenyl-methoxymethyl]-4-hydroxy-pyrrolidin-1-yl}-6-oxo-hexyl)-carbamic acid 17-(1,5-dimethyl-hexyl)-10,13-dimethyl-2,3,4,7,8,9,10,11,12,13,14,15,16,17-tetradecahydro-1H-cyclopenta[a]phenanthren-3-yl ester **AG**

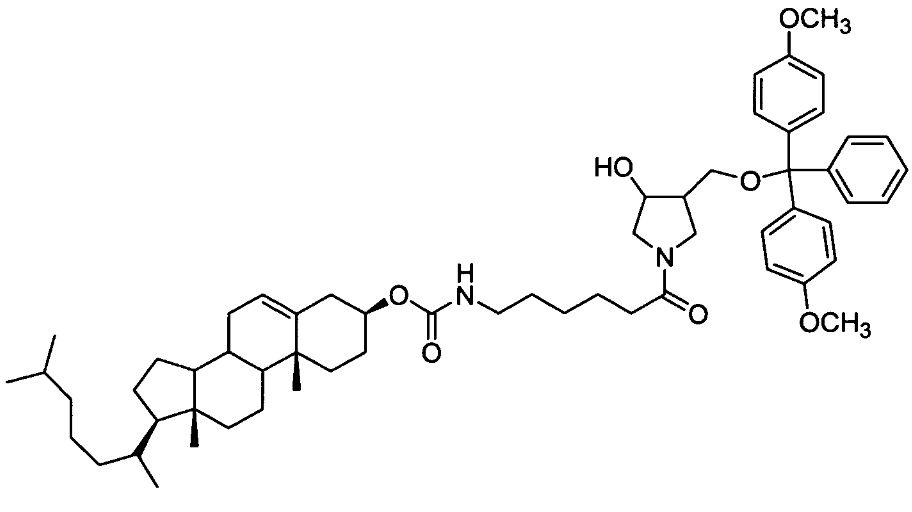

**AG**

Diol AF (1.25 gm 1.994 mmol) was dried by evaporating with pyridine (2 x 5 mL) *in vacuo*. Anhydrous pyridine (10 mL) and 4,4'-dimethoxytritylchloride (0.724 g, 2.13 mmol) were added with stirring. The reaction was carried out at room temperature overnight. The reaction was quenched by the addition of methanol. The reaction mixture was concentrated under vacuum and to the residue dichloromethane (50 mL) was added. The organic layer was washed with 1M aqueous sodium bicarbonate. The organic layer was dried over anhydrous sodium sulfate, filtered and concentrated. The residual pyridine was removed by evaporating with toluene. The crude product was purified by column chromatography (2% MeOH/Chloroform, Rf= 0.5 in 5% MeOH/CHCl3) (1.75 g, 95%).

Succinic acid mono-(4-[bis-(4-methoxy-phenyl)-phenyl-methoxymethyl]-1-{6-[17-(1,5-dimethyl-hexyl)-10,13-dimethyl 2,3,4,7,8,9,10,11,12,13,14,15,16,17-tetradecahydro-1H cyclopenta[a]phenanthren-3-yloxycarbonylamino]-hexanoyl}-pyrrolidin-3-yl) ester **AH**

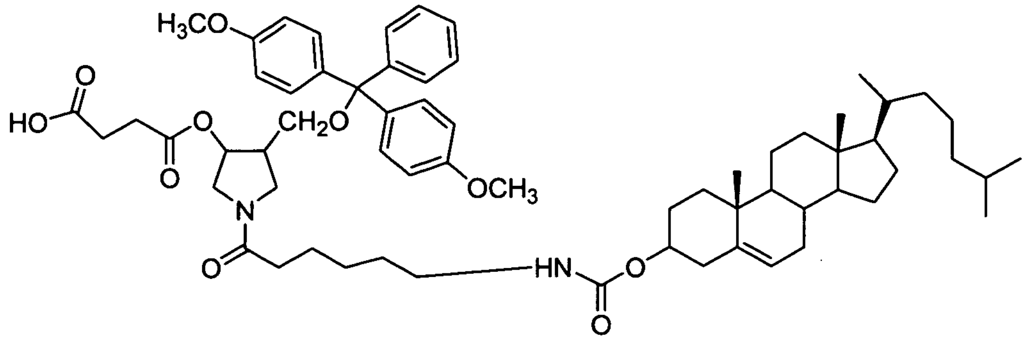

**AH**

Compound AG (1.0 g, 1.05 mmol) was mixed with succinic anhydride (0.150 g, 1.5 mmol) and DMAP (0.073 g, 0.6 mmol) and dried in a vacuum at 40°C overnight. The mixture was dissolved in anhydrous dichloroethane (3 mL), triethylamine (0.318 g, 0.440 mL, 3.15 mmol) was added and the solution was stirred at room temperature under argon atmosphere for 16 h. It was then diluted with dichloromethane (40 mL) and washed with ice cold aqueous citric acid (5 wt%, 30 mL) and water (2 X 20 mL). The organic phase was dried over anhydrous sodium sulfate and concentrated to dryness. The residue was used as such for the next step.

Cholesterol derivatised CPG **AI**

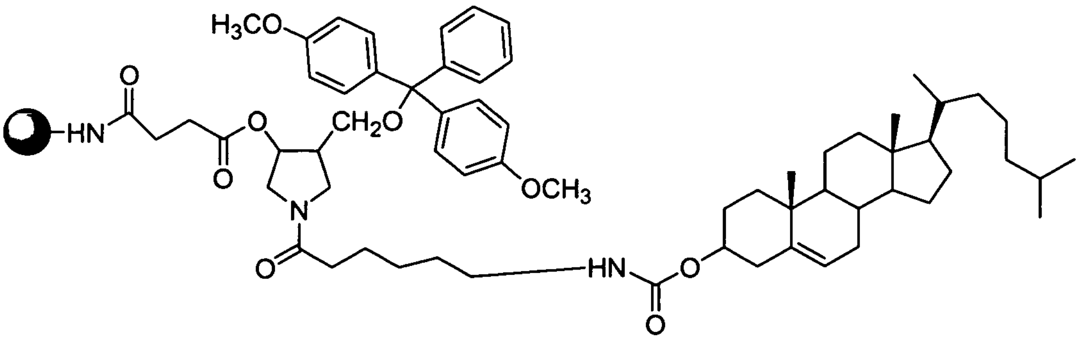

**AI**

Succinate AH (0.254 g, 0.242 mmol) was dissolved in a mixture of dichloromethane/acetonitrile (3:2, 3 mL). To that solution DMAP (0.0296 g, 0.242 mmol) in acetonitrile (1.25 mL), 2,2'-Dithio-bis(5-nitropyridine) (0.075 g, 0.242 mmol) in acetonitrile/dichloroethane (3:1, 1.25 mL) were added successively. To the resulting solution triphenylphosphine (0.064 g, 0.242 mmol) in acetonitrile (0.6 ml) was added. The reaction mixture turned bright orange in color. The solution was agitated briefly using a wrist-action shaker (5 mins). Long chain alkyl amine-CPG (LCAA-CPG) (1.5 g, 61 mM) was added. The suspension was agitated for 2 h. The CPG was filtered through a sintered funnel and washed with acetonitrile, dichloromethane and ether successively. Unreacted amino groups were masked using acetic anhydride/pyridine. The achieved loading of the CPG was measured by taking UV measurement (37 mM/g).

The synthesis of siRNAs bearing a 5'-12-dodecanoic acid bisdecylamide group (herein referred to as "5'-C32-")

or a 5'-cholesteryl derivative group (herein referred to as "5'-Chol-") was performed as described in WO 2004/065601, except that, for the cholesteryl derivative, the oxidation step was performed using the Beaucage reagent in order to introduce a phosphorothioate linkage at the 5'-end of the nucleic acid oligomer.

Nucleic acid sequences are represented below using standard nomenclature, and specifically the abbreviations of Table 1.

**Table** 1: Abbreviations

| Abbreviations of nucleotide monomers used in nucleic acid sequence representation. It will be understood that these monomers, when present in an oligonucleotide, are mutually linked by 5'-3'-phosphodiester bonds. | |
|---|---|
| **Abbreviation** | **Nucleotide(s)** |
| A | adenosine-3'-phosphate |
| C | cytidine-3'-phosphate |
| G | guanosine-3'-phosphate |
| U | uridine-3'-phosphate |
| N | any nucleotide (G, A, C, U, dT, T) |
| a | 2'-O-methyladenosine-3'-phosphate |
| c | 2'-O-methylcytidine-3'-phosphate |
| g | 2'-O-methylguanosine-3'-phosphate |
| u | 2'-O-methyluridine-3'-phosphate |
| T, dT | 2'-deoxythymidine-3'-phosphate |
| sT; sdT | 2'-deoxy-thymidine-5'phosphate-phosphorothioate |

Conjugated siRNAs

Preparation of siRNAs conjugated to a ligand such as cholesterol and vitamin E are shown in schemes 1 and 2.

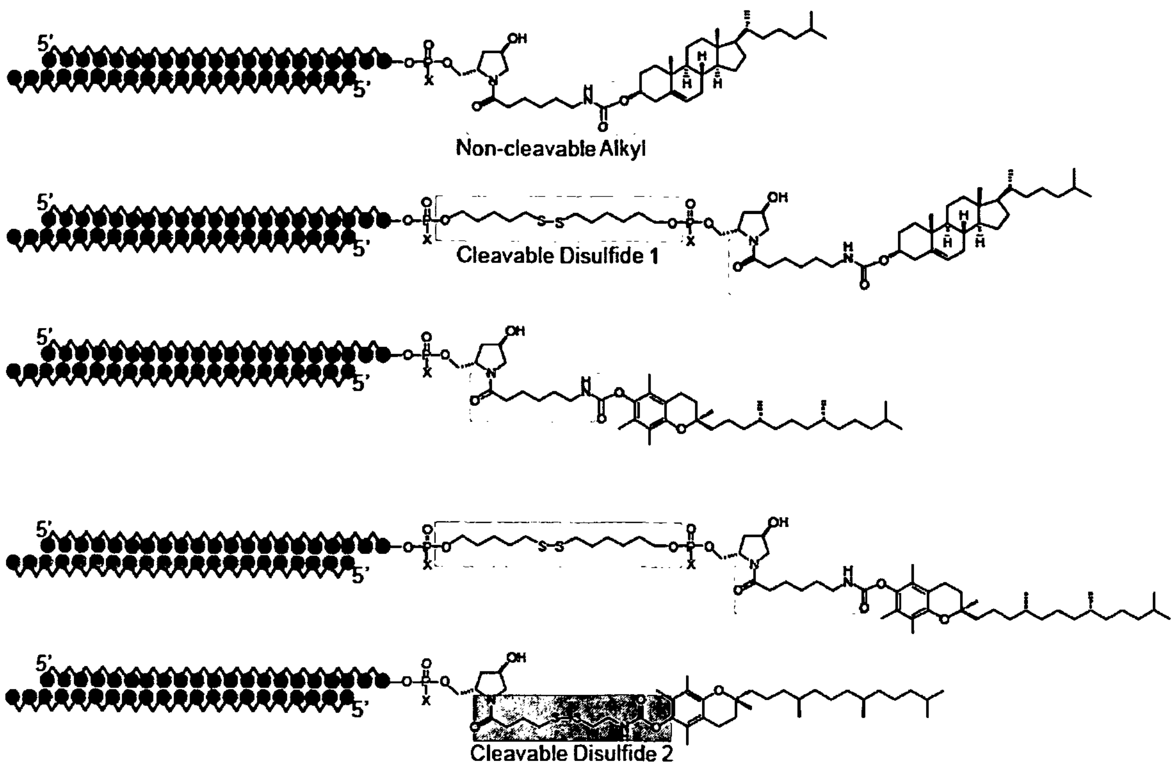


X = O or S

## Scheme 1

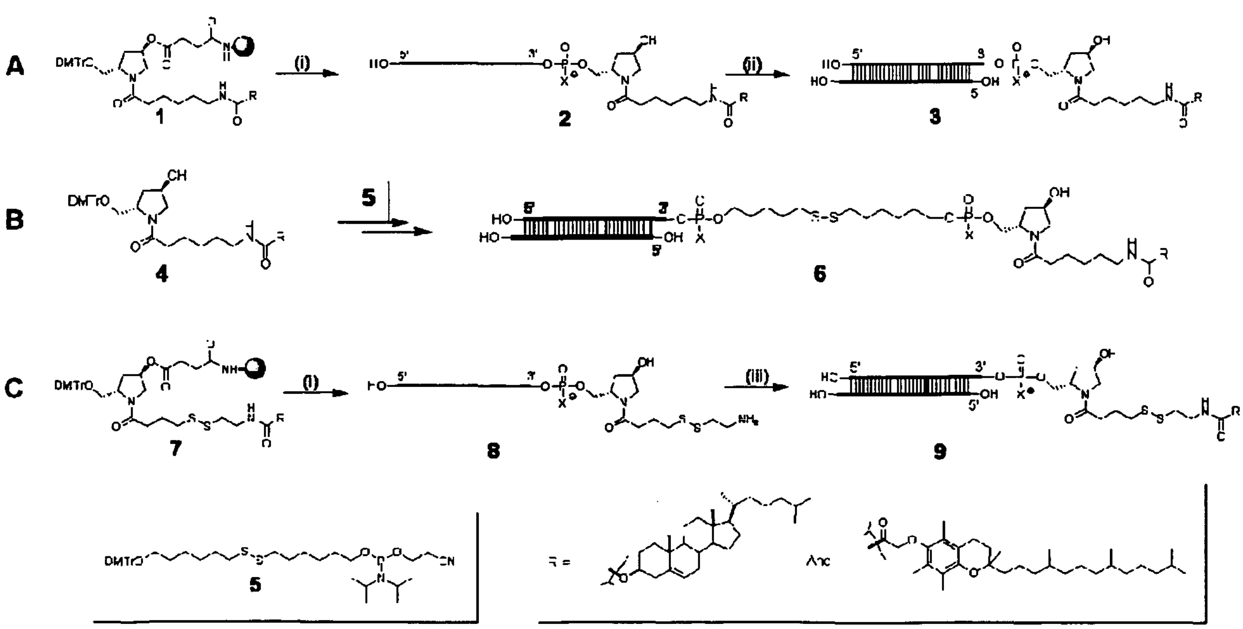


**Scheme 2.** Syntheses of siRNA-lipophilic conjugates. A , B: On column and C: Post-synthetic conjugations. (i) a. solid phase synthesis; b. deprotection and c. HPLC purification; (ii) Annealing with complementary strand; (iii) a. Post-synthetic conjugation to ligand and b. annealing with complementary strand. X = O or S.

**Example 2A. TTR siRNA Design**

Transcripts

siRNA design was carried out to identify siRNAs targeting the gene transthyretin from human (symbol TTR) and rat (symbol Ttr). The design used the TTR transcripts NM_000371.2 (SEQ ID NO:1329) (human) and NM_012681.1 (SEQ ID NO:1330) (rat) from the NCBI Refseq collection. The siRNA duplexes were designed with 100% identity to their respective TTR genes.

siRNA Design and Specificity Prediction

The predicted specificity of all possible 19mers was determined for each sequence. The TTR siRNAs were used in a comprehensive search against the human and rat transcriptomes (defined as the set of NM_ and XM_ records within the NCBI Refseq set) using the FASTA algorithm. The Python script 'offtargetFasta.py' was then used to parse the alignments and generate a score based on the position and number of mismatches between the siRNA and any potential 'off-target' transcript. The off-target score is weighted to emphasize differences in the 'seed' region of siRNAs, in positions 2-9 from the 5' end of the molecule. The off-target score is calculated as follows: mismatches between the oligo and the transcript are given penalties. A mismatch in the seed region in positions 2-9 of the oligo is given a penalty of 2.8; mismatches in the putative cleavage sites 10 and 11 are given a penalty of 1.2, and mismatches in positions 12-19 a penalty of 1. Mismatches in position 1 are not considered. The off-target score for each oligo-transcript pair is then calculated by summing the mismatch penalties. The lowest off-target score from all the oligo-transcript pairs is then determined and used in subsequent sorting of oligos. Both siRNA strands were assigned to a category of specificity according to the calculated scores: a score above 3 qualifies as highly specific, equal to 3 as specific, and between 2.2 and 2.8 as moderately specific. In picking which oligos to synthesize, off-target scores of the antisense strand were sorted from high to low, and the 144 best (lowest off-target score) oligo pairs from human, and the best 26 pairs from rat were selected.

siRNA sequence selection

A total of 140 sense and 140 antisense human TTR derived siRNA oligos were synthesized and formed into duplexes. A total of 26 sense and 26 antisense rat TTR derived siRNA oligos were synthesized and formed into duplexes. Duplexes are presented in Tables 2-4 (human TTR) and Tables 5-7 (rat TTR).

Table 2. Identification numbers for human TTR dsRNAs

| See Table 4 for sequences and modifications of oligos. | | |
|---|---|---|
| **Duplex #** | **Sense Oligo #** | **Antisense Oligo #** |
| AD-18243 | A-32153 | A-32154 |
| AD-18244 | A-32155 | A-32156 |
| AD-18245 | A-32157 | A-32158 |
| AD-18246 | A-32159 | A-32160 |
| AD-18247 | A-32163 | A-32164 |
| AD-18248 | A-32165 | A-32166 |
| AD-18249 | A-32167 | A-32168 |
| AD-18250 | A-32169 | A-32170 |
| AD-18251 | A-32171 | A-32172 |
| AD-18252 | A-32175 | A-32176 |
| AD-18253 | A-32177 | A-32178 |
| AD-18254 | A-32179 | A-32180 |
| AD-18255 | A-32181 | A-32182 |
| AD-18256 | A-32183 | A-32184 |

(continued)

| See Table 4 for sequences and modifications of oligos. | | |
|---|---|---|
| **Duplex #** | **Sense Oligo #** | **Antisense Oligo #** |
| AD-18257 | A-32187 | A-32188 |
| AD-18258 | A-32189 | A-32190 |
| AD-18259 | A-32191 | A-32192 |
| AD-18260 | A-32193 | A-32194 |
| AD-18261 | A-32195 | A-32196 |
| AD-18262 | A-32199 | A-32200 |
| AD-18263 | A-32201 | A-32202 |
| AD-18264 | A-32203 | A-32204 |
| AD-18265 | A-32205 | A-32206 |
| AD-18266 | A-32207 | A-32208 |
| AD-18267 | A-32211 | A-32212 |
| AD-18268 | A-32213 | A-32214 |
| AD-18269 | A-32215 | A-32216 |
| AD-18270 | A-32217 | A-32218 |
| AD-18271 | A-32219 | A-32220 |
| AD-18272 | A-32221 | A-32222 |
| AD-18273 | A-32223 | A-32224 |
| AD-18274 | A-32225 | A-32226 |
| AD-18275 | A-32227 | A-32228 |
| AD-18276 | A-32229 | A-32230 |
| AD-18277 | A-32231 | A-32232 |
| AD-18278 | A-32233 | A-32234 |
| AD-18279 | A-32235 | A-32236 |
| AD-18280 | A-32237 | A-32238 |
| AD-18281 | A-32239 | A-32240 |
| AD-18282 | A-32241 | A-32242 |
| AD-18283 | A-32243 | A-32244 |
| AD-18284 | A-32247 | A-32248 |
| AD-18285 | A-32249 | A-32250 |
| AD-18286 | A-32251 | A-32252 |
| AD-18287 | A-32253 | A-32254 |
| AD-18288 | A-32255 | A-32256 |
| AD-18289 | A-32259 | A-32260 |
| AD-18290 | A-32261 | A-32262 |
| AD-18291 | A-32263 | A-32264 |
| AD-18292 | A-32265 | A-32266 |
| AD-18293 | A-32267 | A-32268 |

(continued)

| See Table 4 for sequences and modifications of oligos. | | |
|---|---|---|
| **Duplex #** | **Sense Oligo #** | **Antisense Oligo #** |
| AD-18294 | A-32269 | A-32270 |
| AD-18295 | A-32271 | A-32272 |
| AD-18296 | A-32273 | A-32274 |
| AD-18297 | A-32275 | A-32276 |
| AD-18298 | A-32277 | A-32278 |
| AD-18299 | A-32279 | A-32280 |
| AD-18300 | A-32281 | A-32282 |
| AD-18301 | A-32283 | A-32284 |
| AD-18302 | A-32285 | A-32286 |
| AD-18303 | A-32287 | A-32288 |
| AD-18304 | A-32289 | A-32290 |
| AD-18305 | A-32291 | A-32292 |
| AD-18306 | A-32295 | A-32296 |
| AD-18307 | A-32297 | A-32298 |
| AD-18308 | A-32299 | A-32300 |
| AD-18309 | A-32301 | A-32302 |
| AD-18310 | A-32303 | A-32304 |
| AD-18311 | A-32307 | A-32308 |
| AD-18312 | A-32309 | A-32310 |
| AD-18313 | A-32311 | A-32312 |
| AD-18314 | A-32313 | A-32314 |
| AD-18315 | A-32315 | A-32316 |
| AD-18316 | A-32319 | A-32320 |
| AD-18317 | A-32321 | A-32322 |
| AD-18318 | A-32323 | A-32324 |
| AD-18319 | A-32325 | A-32326 |
| AD-18320 | A-32327 | A-32328 |
| AD-18321 | A-32331 | A-32332 |
| AD-18322 | A-32333 | A-32334 |
| AD-18323 | A-32335 | A-32336 |
| AD-18324 | A-32337 | A-32338 |
| AD-18325 | A-32339 | A-32340 |
| AD-18326 | A-32341 | A-32342 |
| AD-18327 | A-32343 | A-32344 |
| AD-18328 | A-32345 | A-32346 |
| AD-18329 | A-32347 | A-32348 |
| AD-18330 | A-32349 | A-32350 |

(continued)

| See Table 4 for sequences and modifications of oligos. | | |
|---|---|---|
| **Duplex #** | **Sense Oligo #** | **Antisense Oligo #** |
| AD-18331 | A-32351 | A-32352 |
| AD-18332 | A-32353 | A-32354 |
| AD-18333 | A-32355 | A-32356 |
| AD-18334 | A-32357 | A-32358 |
| AD-18335 | A-32359 | A-32360 |
| AD-18336 | A-32363 | A-32364 |
| AD-18337 | A-32367 | A-32368 |
| AD-18338 | A-32369 | A-32370 |
| AD-18339 | A-32371 | A-32372 |
| AD-18340 | A-32373 | A-32374 |
| AD-18341 | A-32375 | A-32376 |
| AD-18342 | A-32379 | A-32380 |
| AD-18343 | A-32381 | A-32382 |
| AD-18344 | A-32383 | A-32384 |
| AD-18345 | A-32385 | A-32386 |
| AD-18346 | A-32387 | A-32388 |
| AD-18347 | A-32391 | A-32392 |
| AD-18348 | A-32393 | A-32394 |
| AD-18349 | A-32395 | A-32396 |
| AD-18350 | A-32397 | A-32398 |
| AD-18351 | A-32399 | A-32400 |
| AD-18352 | A-32401 | A-32402 |
| AD-18353 | A-32403 | A-32404 |
| AD-18354 | A-32405 | A-32406 |
| AD-18355 | A-32407 | A-32408 |
| AD-18356 | A-32409 | A-32410 |
| AD-18357 | A-32411 | A-32412 |
| AD-18358 | A-32415 | A-32416 |
| AD-18359 | A-32417 | A-32418 |
| AD-18360 | A-32419 | A-32420 |
| AD-18361 | A-32421 | A-32422 |
| AD-18362 | A-32423 | A-32424 |
| AD-18363 | A-32427 | A-32428 |
| AD-18364 | A-32429 | A-32430 |
| AD-18446 | A-32161 | A-32162 |
| AD-18447 | A-32173 | A-32174 |
| AD-18448 | A-32185 | A-32186 |

(continued)

| See Table 4 for sequences and modifications of oligos. | | |
|---|---|---|
| **Duplex #** | **Sense Oligo #** | **Antisense Oligo #** |
| AD-18449 | A-32197 | A-32198 |
| AD-18450 | A-32209 | A-32210 |
| AD-18451 | A-32245 | A-32246 |
| AD-18452 | A-32257 | A-32258 |
| AD-18453 | A-32293 | A-32294 |
| AD-18454 | A-32305 | A-32306 |
| AD-18455 | A-32317 | A-32318 |
| AD-18456 | A-32329 | A-32330 |
| AD-18457 | A-32361 | A-32362 |
| AD-18458 | A-32365 | A-32366 |
| AD-18459 | A-32377 | A-32378 |
| AD-18460 | A-32389 | A-32390 |
| AD-18461 | A-32401 | A-32402 |
| AD-18462 | A-32413 | A-32414 |
| AD-18463 | A-32425 | A-32426 |

Table 3A. Sense and antisense strand sequences of human TTR dsRNAs

| Strand: s= sense; as= antisense; Position: position of 5' base on transcript (NM_000371.2, SEQ ID NO: 1329) | | | | | |
|---|---|---|---|---|---|
| **Strand** | **Position** | **Sequence (5' to 3')** | **SEQ ID NO:** | **Sequence with 3' dinucleotide overhang (5' to 3')** | **SEQ ID NO:** |
| S | 100 | CCGGUGAAUCCAAGUGUCC | 1 | CCGGUGAAUCCAAGUGUCCNN | 281 |
| as | 118 | GGACACUUGGAUUCACCGG | 2 | GGACACUUGGAUUCACCGGNN | 282 |
| S | 11 | ACUCAUUCUUGGCAGGAUG | 3 | ACUCAUUCUUGGCAGGAUGNN | 283 |
| as | 29 | CAUCCUGCCAAGAAUGAGU | 4 | CAUCCUGCCAAGAAUGAGUNN | 284 |
| S | 111 | AAGUGUCCUCUGAUGGUCA | 5 | AAGUGUCCUCUGAUGGUCANN | 285 |
| as | 129 | UGACCAUCAGAGGACACUU | 6 | UGACCAUCAGAGGACACUUNN | 286 |
| S | 13 | UCAUUCUUGGCAGGAUGGC | 7 | UCAUUCUUGGCAGGAUGGCNN | 287 |
| as | 31 | GCCAUCCUGCCAAGAAUGA | 8 | GCCAUCCUGCCAAGAAUGANN | 288 |
| s | 130 | AAGUUCUAGAUGCUGUCCG | 9 | AAGUUCUAGAUGCUGUCCGNN | 289 |
| as | 148 | CGGACAGCAUCUAGAACUU | 10 | CGGACAGCAUCUAGAACUUNN | 290 |
| s | 132 | GUUCUAGAUGCUGUCCGAG | 11 | GUUCUAGAUGCUGUCCGAGNN | 291 |
| as | 150 | CUCGGACAGCAUCUAGAAC | 12 | CUCGGACAGCAUCUAGAACNN | 292 |
| s | 135 | CUAGAUGCUGUCCGAGGCA | 13 | CUAGAUGCUGUCCGAGGCANN | 293 |
| as | 153 | UGCCUCGGACAGCAUCUAG | 14 | UGCCUCGGACAGCAUCUAGNN | 294 |
| s | 138 | GAUGCUGUCCGAGGCAGUC | 15 | GAUGCUGUCCGAGGCAGUCNN | 295 |
| as | 156 | GACUGCCUCGGACAGCAUC | 16 | GACUGCCUCGGACAGCAUCNN | 296 |

(continued)

| Strand: s= sense; as= antisense; Position: position of 5' base on transcript (NM_000371.2, SEQ ID NO: 1329) | | | | | |
|---|---|---|---|---|---|
| Strand | Position | Sequence (5' to 3') | SEQ ID NO: | Sequence with 3' dinucleotide overhang (5' to 3') | SEQ ID NO: |
| s | 14 | CAUUCUUGGCAGGAUGGCU | 17 | CAUUCUUGGCAGGAUGGCUNN | 297 |
| as | 32 | AGCCAUCCUGCCAAGAAUG | 18 | AGCCAUCCUGCCAAGAAUGNN | 298 |
| s | 140 | UGCUGUCCGAGGCAGUCCU | 19 | UGCUGUCCGAGGCAGUCCUNN | 299 |
| as | 158 | AGGACUGCCUCGGACAGCA | 20 | AGGACUGCCUCGGACAGCANN | 300 |
| s | 146 | CCGAGGCAGUCCUGCCAUC | 21 | CCGAGGCAGUCCUGCCAUCNN | 301 |
| as | 164 | GAUGGCAGGACUGCCUCGG | 22 | GAUGGCAGGACUGCCUCGGNN | 302 |
| s | 152 | CAGUCCUGCCAUCAAUGUG | 23 | CAGUCCUGCCAUCAAUGUGNN | 303 |
| as | 170 | CACAUUGAUGGCAGGACUG | 24 | CACAUUGAUGGCAGGACUGNN | 304 |
| s | 164 | CAAUGUGGCCGUGCAUGUG | 25 | CAAUGUGGCCGUGCAUGUGNN | 305 |
| as | 182 | CACAUGCACGGCCACAUUG | 26 | CACAUGCACGGCCACAUUGNN | 306 |
| s | 178 | AUGUGUUCAGAAAGGCUGC | 27 | AUGUGUUCAGAAAGGCUGCNN | 307 |
| as | 196 | GCAGCCUUUCUGAACACAU | 28 | GCAGCCUUUCUGAACACAUNN | 308 |
| s | 2 | CAGAAGUCCACUCAUUCUU | 29 | CAGAAGUCCACUCAUUCUUNN | 309 |
| as | 20 | AAGAAUGAGUGGACUUCUG | 30 | AAGAAUGAGUGGACUUCUGNN | 310 |
| s | 21 | GGCAGGAUGGCUUCUCAUC | 31 | GGCAGGAUGGCUUCUCAUCNN | 311 |
| as | 39 | GAUGAGAAGCCAUCCUGCC | 32 | GAUGAGAAGCCAUCCUGCCNN | 312 |
| s | 210 | GAGCCAUUUGCCUCUGGGA | 33 | GAGCCAUUUGCCUCUGGGANN | 313 |
| as | 228 | UCCCAGAGGCAAAUGGCUC | 34 | UCCCAGAGGCAAAUGGCUCNN | 314 |
| s | 23 | CAGGAUGGCUUCUCAUCGU | 35 | CAGGAUGGCUUCUCAUCGUNN | 315 |
| as | 41 | ACGAUGAGAAGCCAUCCUG | 36 | ACGAUGAGAAGCCAUCCUGNN | 316 |
| s | 24 | AGGAUGGCUUCUCAUCGUC | 37 | AGGAUGGCUUCUCAUCGUCNN | 317 |
| as | 42 | GACGAUGAGAAGCCAUCCU | 38 | GACGAUGAGAAGCCAUCCUNN | 318 |
| s | 245 | AGAGCUGCAUGGGCUCACA | 39 | AGAGCUGCAUGGGCUCACANN | 319 |
| as | 263 | UGUGAGCCCAUGCAGCUCU | 40 | UGUGAGCCCAUGCAGCUCUNN | 320 |
| s | 248 | GCUGCAUGGGCUCACAACU | 41 | GCUGCAUGGGCUCACAACUNN | 321 |
| as | 266 | AGUUGUGAGCCCAUGCAGC | 42 | AGUUGUGAGCCCAUGCAGCNN | 322 |
| s | 25 | GGAUGGCUUCUCAUCGUCU | 43 | GGAUGGCUUCUCAUCGUCUNN | 323 |
| as | 43 | AGACGAUGAGAAGCCAUCC | 44 | AGACGAUGAGAAGCCAUCCNN | 324 |
| s | 251 | GCAUGGGCUCACAACUGAG | 45 | GCAUGGGCUCACAACUGAGNN | 325 |
| as | 269 | CUCAGUUGUGAGCCCAUGC | 46 | CUCAGUUGUGAGCCCAUGCNN | 326 |
| s | 253 | AUGGGCUCACAACUGAGGA | 47 | AUGGGCUCACAACUGAGGANN | 327 |
| as | 271 | UCCUCAGUUGUGAGCCCAU | 48 | UCCUCAGUUGUGAGCCCAUNN | 328 |
| s | 254 | UGGGCUCACAACUGAGGAG | 49 | UGGGCUCACAACUGAGGAGNN | 329 |
| as | 272 | CUCCUCAGUUGUGAGCCCA | 50 | CUCCUCAGUUGUGAGCCCANN | 330 |
| s | 270 | GAGGAAUUUGUAGAAGGGA | 51 | GAGGAAUUUGUAGAAGGGANN | 331 |

(continued)

| Strand: s= sense; as= antisense; Position: position of 5' base on transcript (NM_000371.2, SEQ ID NO: 1329) | | | | | |
|---|---|---|---|---|---|
| Strand | Position | Sequence (5' to 3') | SEQ ID NO: | Sequence with 3' dinucleotide overhang (5' to 3') | SEQ ID NO: |
| as | 288 | UCCCUUCUACAAAUUCCUC | 52 | UCCCUUCUACAAAUUCCUCNN | 332 |
| s | 276 | UUUGUAGAAGGGAUAUACA | 53 | UUUGUAGAAGGGAUAUACANN | 333 |
| as | 294 | UGUAUAUCCCUUCUACAAA | 54 | UGUAUAUCCCUUCUACAAANN | 334 |
| s | 277 | UUGUAGAAGGGAUAUACAA | 55 | UUGUAGAAGGGAUAUACAANN | 335 |
| as | 295 | UUGUAUAUCCCUUCUACAA | 56 | UUGUAUAUCCCUUCUACAANN | 336 |
| s | 278 | UGUAGAAGGGAUAUACAAA | 57 | UGUAGAAGGGAUAUACAAANN | 337 |
| as | 296 | UUUGUAUAUCCCUUCUACA | 58 | UUUGUAUAUCCCUUCUACANN | 338 |
| s | 281 | AGAAGGGAUAUACAAAGUG | 59 | AGAAGGGAUAUACAAAGUGNN | 339 |
| as | 299 | CACUUUGUAUAUCCCUUCU | 60 | CACUUUGUAUAUCCCUUCUNN | 340 |
| s | 295 | AAGUGGAAAUAGACACCAA | 61 | AAGUGGAAAUAGACACCAANN | 341 |
| as | 313 | UUGGUGUCUAUUUCCACUU | 62 | UUGGUGUCUAUUUCCACUUNN | 342 |
| s | 299 | GGAAAUAGACACCAAAUCU | 63 | GGAAAUAGACACCAAAUCUNN | 343 |
| as | 317 | AGAUUUGGUGUCUAUUUCC | 64 | AGAUUUGGUGUCUAUUUCCNN | 344 |
| s | 300 | GAAAUAGACACCAAAUCUU | 65 | GAAAUAGACACCAAAUCUUNN | 345 |
| as | 318 | AAGAUUUGGUGUCUAUUUC | 66 | AAGAUUUGGUGUCUAUUUCNN | 346 |
| s | 303 | AUAGACACCAAAUCUUACU | 67 | AUAGACACCAAAUCUUACUNN | 347 |
| as | 321 | AGUAAGAUUUGGUGUCUAU | 68 | AGUAAGAUUUGGUGUCUAUNN | 348 |
| s | 304 | UAGACACCAAAUCUUACUG | 69 | UAGACACCAAAUCUUACUGNN | 349 |
| as | 322 | CAGUAAGAUUUGGUGUCUA | 70 | CAGUAAGAUUUGGUGUCUANN | 350 |
| s | 305 | AGACACCAAAUCUUACUGG | 71 | AGACACCAAAUCUUACUGGNN | 351 |
| as | 323 | CCAGUAAGAUUUGGUGUCU | 72 | CCAGUAAGAUUUGGUGUCUNN | 352 |
| s | 317 | UUACUGGAAGGCACUUGGC | 73 | UUACUGGAAGGCACUUGGCNN | 353 |
| as | 335 | GCCAAGUGCCUUCCAGUAA | 74 | GCCAAGUGCCUUCCAGUAANN | 354 |
| s | 32 | UUCUCAUCGUCUGCUCCUC | 75 | UUCUCAUCGUCUGCUCCUCNN | 355 |
| as | 50 | GAGGAGCAGACGAUGAGAA | 76 | GAGGAGCAGACGAUGAGAANN | 356 |
| s | 322 | GGAAGGCACUUGGCAUCUC | 77 | GGAAGGCACUUGGCAUCUCNN | 357 |
| as | 340 | GAGAUGCCAAGUGCCUUCC | 78 | GAGAUGCCAAGUGCCUUCCNN | 358 |
| s | 326 | GGCACUUGGCAUCUCCCCA | 79 | GGCACUUGGCAUCUCCCCANN | 359 |
| as | 344 | UGGGGAGAUGCCAAGUGCC | 80 | UGGGGAGAUGCCAAGUGCCNN | 360 |
| s | 333 | GGCAUCUCCCCAUUCCAUG | 81 | GGCAUCUCCCCAUUCCAUGNN | 361 |
| as | 351 | AUGGAAUGGGGAGAUGCCTT | 82 | AUGGAAUGGGGAGAUGCCTTNN | 362 |
| s | 334 | GCAUCUCCCCAUUCCAUGA | 83 | GCAUCUCCCCAUUCCAUGANN | 363 |
| as | 352 | UCAUGGAAUGGGGAGAUGC | 84 | UCAUGGAAUGGGGAGAUGCNN | 364 |
| s | 335 | CAUCUCCCCAUUCCAUGAG | 85 | CAUCUCCCCAUUCCAUGAGNN | 365 |
| as | 353 | CUCAUGGAAUGGGGAGAUG | 86 | CUCAUGGAAUGGGGAGAUGNN | 366 |

(continued)

| Strand: s= sense; as= antisense; Position: position of 5' base on transcript (NM_000371.2, SEQ ID NO: 1329) | | | | | |
|---|---|---|---|---|---|
| **Strand** | **Position** | **Sequence (5' to 3')** | **SEQ ID NO:** | **Sequence with 3' dinucleotide overhang (5' to 3')** | **SEQ ID NO:** |
| s | 336 | AUCUCCCCAUUCCAUGAGC | 87 | AUCUCCCCAUUCCAUGAGCNN | 367 |
| as | 354 | GCUCAUGGAAUGGGGAGAU | 88 | GCUCAUGGAAUGGGGAGAUNN | 368 |
| s | 338 | CUCCCCAUUCCAUGAGCAU | 89 | CUCCCCAUUCCAUGAGCAUNN | 369 |
| as | 356 | AUGCUCAUGGAAUGGGGAG | 90 | AUGCUCAUGGAAUGGGGAGNN | 370 |
| s | 341 | CCCAUUCCAUGAGCAUGCA | 91 | CCCAUUCCAUGAGCAUGCANN | 371 |
| as | 359 | UGCAUGCUCAUGGAAUGGG | 92 | UGCAUGCUCAUGGAAUGGGNN | 372 |
| s | 347 | CCAUGAGCAUGCAGAGGUG | 93 | CCAUGAGCAUGCAGAGGUGNN | 373 |
| as | 365 | CACCUCUGCAUGCUCAUGG | 94 | CACCUCUGCAUGCUCAUGGNN | 374 |
| s | 352 | AGCAUGCAGAGGUGGUAUU | 95 | AGCAUGCAGAGGUGGUAUUNN | 375 |
| as | 370 | AAUACCACCUCUGCAUGCU | 96 | AAUACCACCUCUGCAUGCUNN | 376 |
| s | 354 | CAUGCAGAGGUGGUAUUCA | 97 | CAUGCAGAGGUGGUAUUCANN | 377 |
| as | 372 | UGAAUACCACCUCUGCAUG | 98 | UGAAUACCACCUCUGCAUGNN | 378 |
| s | 355 | AUGCAGAGGUGGUAUUCAC | 99 | AUGCAGAGGUGGUAUUCACNN | 379 |
| as | 373 | GUGAAUACCACCUCUGCAU | 100 | GUGAAUACCACCUCUGCAUNN | 380 |
| s | 362 | GGUGGUAUUCACAGCCAAC | 101 | GGUGGUAUUCACAGCCAACNN | 381 |
| as | 380 | GUUGGCUGUGAAUACCACC | 102 | GUUGGCUGUGAAUACCACCNN | 382 |
| s | 363 | GUGGUAUUCACAGCCAACG | 103 | GUGGUAUUCACAGCCAACGNN | 383 |
| as | 381 | CGUUGGCUGUGAAUACCAC | 104 | CGUUGGCUGUGAAUACCACNN | 384 |
| s | 364 | UGGUAUUCACAGCCAACGA | 105 | UGGUAUUCACAGCCAACGANN | 385 |
| as | 382 | UCGUUGGCUGUGAAUACCA | 106 | UCGUUGGCUGUGAAUACCANN | 386 |
| s | 365 | GGUAUUCACAGCCAACGAC | 107 | GGUAUUCACAGCCAACGACNN | 387 |
| as | 383 | GUCGUUGGCUGUGAAUACC | 108 | GUCGUUGGCUGUGAAUACCNN | 388 |
| s | 366 | GUAUUCACAGCCAACGACU | 109 | GUAUUCACAGCCAACGACUNN | 389 |
| as | 384 | AGUCGUUGGCUGUGAAUAC | 110 | AGUCGUUGGCUGUGAAUACNN | 390 |
| s | 367 | UAUUCACAGCCAACGACUC | 111 | UAUUCACAGCCAACGACUCNN | 391 |
| as | 385 | GAGUCGUUGGCUGUGAAUA | 112 | GAGUCGUUGGCUGUGAAUANN | 392 |
| s | 370 | UCACAGCCAACGACUCCGG | 113 | UCACAGCCAACGACUCCGGNN | 393 |
| as | 388 | CCGGAGUCGUUGGCUGUGA | 114 | CCGGAGUCGUUGGCUGUGANN | 394 |
| s | 390 | CCCCGCCGCUACACCAUUG | 115 | CCCCGCCGCUACACCAUUGNN | 395 |
| as | 408 | CAAUGGUGUAGCGGCGGGG | 116 | CAAUGGUGUAGCGGCGGGGNN | 396 |
| s | 4 | GAAGUCCACUCAUUCUUGG | 117 | GAAGUCCACUCAUUCUUGGNN | 397 |
| as | 22 | CCAAGAAUGAGUGGACUUC | 118 | CCAAGAAUGAGUGGACUUCNN | 398 |
| s | 412 | CCCUGCUGAGCCCCUACUC | 119 | CCCUGCUGAGCCCCUACUCNN | 399 |
| as | 430 | GAGUAGGGGCUCAGCAGGG | 120 | GAGUAGGGGCUCAGCAGGGNN | 400 |
| s | 417 | CUGAGCCCCUACUCCUAUU | 121 | CUGAGCCCCUACUCCUAUUNN | 401 |

(continued)

| Strand: s= sense; as= antisense; Position: position of 5' base on transcript (NM_000371.2, SEQ ID NO: 1329) | | | | | |
|---|---|---|---|---|---|
| **Strand** | **Position** | **Sequence (5' to 3')** | **SEQ ID NO:** | **Sequence with 3' dinucleotide overhang (5' to 3')** | **SEQ ID NO:** |
| as | 435 | AAUAGGAGUAGGGGCUCAG | 122 | AAUAGGAGUAGGGGCUCAGNN | 402 |
| s | 418 | UGAGCCCCUACUCCUAUUC | 123 | UGAGCCCCUACUCCUAUUCNN | 403 |
| as | 436 | GAAUAGGAGUAGGGGCUCA | 124 | GAAUAGGAGUAGGGGCUCANN | 404 |
| s | 422 | CCCCUACUCCUAUUCCACC | 125 | CCCCUACUCCUAUUCCACCNN | 405 |
| as | 440 | GGUGGAAUAGGAGUAGGGG | 126 | GGUGGAAUAGGAGUAGGGGNN | 406 |
| s | 425 | CUACUCCUAUUCCACCACG | 127 | CUACUCCUAUUCCACCACGNN | 407 |
| as | 443 | CGUGGUGGAAUAGGAGUAG | 128 | CGUGGUGGAAUAGGAGUAGNN | 408 |
| s | 426 | UACUCCUAUUCCACCACGG | 129 | UACUCCUAUUCCACCACGGNN | 409 |
| as | 444 | CCGUGGUGGAAUAGGAGUA | 130 | CCGUGGUGGAAUAGGAGUANN | 410 |
| s | 427 | ACUCCUAUUCCACCACGGC | 131 | ACUCCUAUUCCACCACGGCNN | 411 |
| as | 445 | GCCGUGGUGGAAUAGGAGU | 132 | GCCGUGGUGGAAUAGGAGUNN | 412 |
| s | 429 | UCCUAUUCCACCACGGCUG | 133 | UCCUAUUCCACCACGGCUGNN | 413 |
| as | 447 | CAGCCGUGGUGGAAUAGGA | 134 | CAGCCGUGGUGGAAUAGGANN | 414 |
| s | 432 | UAUUCCACCACGGCUGUCG | 135 | UAUUCCACCACGGCUGUCGNN | 415 |
| as | 450 | CGACAGCCGUGGUGGAAUA | 136 | CGACAGCCGUGGUGGAAUANN | 416 |
| s | 433 | AUUCCACCACGGCUGUCGU | 137 | AUUCCACCACGGCUGUCGUNN | 417 |
| as | 451 | ACGACAGCCGUGGUGGAAU | 138 | ACGACAGCCGUGGUGGAAUNN | 418 |
| s | 437 | CACCACGGCUGUCGUCACC | 139 | CACCACGGCUGUCGUCACCNN | 419 |
| as | 455 | GGUGACGACAGCCGUGGUG | 140 | GGUGACGACAGCCGUGGUGNN | 420 |
| s | 438 | ACCACGGCUGUCGUCACCA | 141 | ACCACGGCUGUCGUCACCANN | 421 |
| as | 456 | UGGUGACGACAGCCGUGGU | 142 | UGGUGACGACAGCCGUGGUNN | 422 |
| s | 439 | CCACGGCUGUCGUCACCAA | 143 | CCACGGCUGUCGUCACCAANN | 423 |
| as | 457 | UUGGUGACGACAGCCGUGG | 144 | UUGGUGACGACAGCCGUGGNN | 424 |
| s | 441 | ACGGCUGUCGUCACCAAUC | 145 | ACGGCUGUCGUCACCAAUCNN | 425 |
| as | 459 | GAUUGGUGACGACAGCCGU | 146 | GAUUGGUGACGACAGCCGUNN | 426 |
| s | 442 | CGGCUGUCGUCACCAAUCC | 147 | CGGCUGUCGUCACCAAUCCNN | 427 |
| as | 460 | GGAUUGGUGACGACAGCCG | 148 | GGAUUGGUGACGACAGCCGNN | 428 |
| s | 449 | CGUCACCAAUCCCAAGGAA | 149 | CGUCACCAAUCCCAAGGAANN | 429 |
| as | 467 | UUCCUUGGGAUUGGUGACG | 150 | UUCCUUGGGAUUGGUGACGNN | 430 |
| s | 455 | CAAUCCCAAGGAAUGAGGG | 151 | CAAUCCCAAGGAAUGAGGGNN | 431 |
| as | 473 | CCCUCAUUCCUUGGGAUUG | 152 | CCCUCAUUCCUUGGGAUUGNN | 432 |
| s | 491 | CCUGAAGGACGAGGGAUGG | 153 | CCUGAAGGACGAGGGAUGGNN | 433 |
| as | 509 | CCAUCCCUCGUCCUUCAGG | 154 | CCAUCCCUCGUCCUUCAGGNN | 434 |
| s | 497 | GGACGAGGGAUGGGAUUUC | 155 | GGACGAGGGAUGGGAUUUCNN | 435 |
| as | 515 | GAAAUCCCAUCCCUCGUCC | 156 | GAAAUCCCAUCCCUCGUCCNN | 436 |

(continued)

| Strand: s= sense; as= antisense; Position: position of 5' base on transcript (NM_000371.2, SEQ ID NO: 1329) | | | | | |
|---|---|---|---|---|---|
| Strand | Position | Sequence (5' to 3') | SEQ ID NO: | Sequence with 3' dinucleotide overhang (5' to 3') | SEQ ID NO: |
| s | 5 | AAGUCCACUCAUUCUUGGC | 157 | AAGUCCACUCAUUCUUGGCNN | 437 |
| as | 23 | GCCAAGAAUGAGUGGACUU | 158 | GCCAAGAAUGAGUGGACUUNN | 438 |
| s | 508 | GGGAUUUCAUGUAACCAAG | 159 | GGGAUUUCAUGUAACCAAGNN | 439 |
| as | 526 | CUUGGUUACAUGAAAUCCC | 160 | CUUGGUUACAUGAAAUCCCNN | 440 |
| s | 509 | GGAUUUCAUGUAACCAAGA | 161 | GGAUUUCAUGUAACCAAGANN | 441 |
| as | 527 | UCUUGGUUACAUGAAAUCC | 162 | UCUUGGUUACAUGAAAUCCNN | 442 |
| s | 514 | UCAUGUAACCAAGAGUAUU | 163 | UCAUGUAACCAAGAGUAUUNN | 443 |
| as | 532 | AAUACUCUUGGUUACAUGA | 164 | AAUACUCUUGGUUACAUGANN | 444 |
| s | 516 | AUGUAACCAAGAGUAUUCC | 165 | AUGUAACCAAGAGUAUUCCNN | 445 |
| as | 534 | GGAAUACUCUUGGUUACAU | 166 | GGAAUACUCUUGGUUACAUNN | 446 |
| s | 517 | UGUAACCAAGAGUAUUCCA | 167 | UGUAACCAAGAGUAUUCCANN | 447 |
| as | 535 | UGGAAUACUCUUGGUUACA | 168 | UGGAAUACUCUUGGUUACANN | 448 |
| s | 518 | GUAACCAAGAGUAUUCCAU | 169 | GUAACCAAGAGUAUUCCAUNN | 449 |
| as | 536 | AUGGAAUACUCUUGGUUAC | 170 | AUGGAAUACUCUUGGUUACNN | 450 |
| s | 54 | UGCCUUGCUGGACUGGUAU | 171 | UGCCUUGCUGGACUGGUAUNN | 451 |
| as | 72 | AUACCAGUCCAGCAAGGCA | 172 | AUACCAGUCCAGCAAGGCANN | 452 |
| s | 543 | UAAAGCAGUGUUUUCACCU | 173 | UAAAGCAGUGUUUUCACCUNN | 453 |
| as | 561 | AGGUGAAAACACUGCUUUA | 174 | AGGUGAAAACACUGCUUUANN | 454 |
| s | 55 | GCCUUGCUGGACUGGUAUU | 175 | GCCUUGCUGGACUGGUAUUNN | 455 |
| as | 73 | AAUACCAGUCCAGCAAGGC | 176 | AAUACCAGUCCAGCAAGGCNN | 456 |
| s | 551 | UGUUUUCACCUCAUAUGCU | 177 | UGUUUUCACCUCAUAUGCUNN | 457 |
| as | 569 | AGCAUAUGAGGUGAAAACA | 178 | AGCAUAUGAGGUGAAAACANN | 458 |
| s | 552 | GUUUUCACCUCAUAUGCUA | 179 | GUUUUCACCUCAUAUGCUANN | 459 |
| as | 570 | UAGCAUAUGAGGUGAAAAC | 180 | UAGCAUAUGAGGUGAAAACNN | 460 |
| s | 553 | UUUUCACCUCAUAUGCUAU | 181 | UUUUCACCUCAUAUGCUAUNN | 461 |
| as | 571 | AUAGCAUAUGAGGUGAAAA | 182 | AUAGCAUAUGAGGUGAAAANN | 462 |
| s | 555 | UUCACCUCAUAUGCUAUGU | 183 | UUCACCUCAUAUGCUAUGUNN | 463 |
| as | 573 | ACAUAGCAUAUGAGGUGAA | 184 | ACAUAGCAUAUGAGGUGAANN | 464 |
| s | 557 | CACCUCAUAUGCUAUGUUA | 185 | CACCUCAUAUGCUAUGUUANN | 465 |
| as | 575 | UAACAUAGCAUAUGAGGUG | 186 | UAACAUAGCAUAUGAGGUGNN | 466 |
| s | 56 | CCUUGCUGGACUGGUAUUU | 187 | CCUUGCUGGACUGGUAUUUNN | 467 |
| as | 74 | AAAUACCAGUCCAGCAAGG | 188 | AAAUACCAGUCCAGCAAGGNN | 468 |
| s | 563 | AUAUGCUAUGUUAGAAGUC | 189 | AUAUGCUAUGUUAGAAGUCNN | 469 |
| as | 581 | GACUUCUAACAUAGCAUAU | 190 | GACUUCUAACAUAGCAUAUNN | 470 |
| s | 564 | UAUGCUAUGUUAGAAGUCC | 191 | UAUGCUAUGUUAGAAGUCCNN | 471 |

(continued)

| Strand: s= sense; as= antisense; Position: position of 5' base on transcript (NM_000371.2, SEQ ID NO: 1329) | | | | | |
|---|---|---|---|---|---|
| **Strand** | **Position** | **Sequence (5' to 3')** | **SEQ ID NO:** | **Sequence with 3' dinucleotide overhang (5' to 3')** | **SEQ ID NO:** |
| as | 582 | GGACUUCUAACAUAGCAUA | 192 | GGACUUCUAACAUAGCAUANN | 472 |
| s | 566 | UGCUAUGUUAGAAGUCCAG | 193 | UGCUAUGUUAGAAGUCCAGNN | 473 |
| as | 584 | CUGGACUUCUAACAUAGCA | 194 | CUGGACUUCUAACAUAGCANN | 474 |
| s | 57 | CUUGCUGGACUGGUAUUUG | 195 | CUUGCUGGACUGGUAUUUGNN | 475 |
| as | 75 | CAAAUACCAGUCCAGCAAG | 196 | CAAAUACCAGUCCAGCAAGNN | 476 |
| s | 578 | AGUCCAGGCAGAGACAAUA | 197 | AGUCCAGGCAGAGACAAUANN | 477 |
| as | 596 | AUUGUCUCUGCCUGGACUT | 198 | AUUGUCUCUGCCUGGACUTTNN | 478 |
| s | 580 | UCCAGGCAGAGACAAUAAA | 199 | UCCAGGCAGAGACAAUAAANN | 479 |
| as | 598 | UUUAUUGUCUCUGCCUGGA | 200 | UUUAUUGUCUCUGCCUGGANN | 480 |
| s | 607 | GUGAAAGGCACUUUUCAUU | 201 | GUGAAAGGCACUUUUCAUUNN | 481 |
| as | 625 | AAUGAAAAGUGCCUUUCAC | 202 | AAUGAAAAGUGCCUUUCACNN | 482 |
| s | 62 | UGGACUGGUAUUUGUGUCU | 203 | UGGACUGGUAUUUGUGUCUNN | 483 |
| as | 80 | AGACACAAAUACCAGUCCA | 204 | AGACACAAAUACCAGUCCANN | 484 |
| s | 77 | GUCUGAGGCUGGCCCUACG | 205 | GUCUGAGGCUGGCCCUACGNN | 485 |
| as | 95 | CGUAGGGCCAGCCUCAGAC | 206 | CGUAGGGCCAGCCUCAGACNN | 486 |
| s | 79 | CUGAGGCUGGCCCUACGGG | 207 | CUGAGGCUGGCCCUACGGGNN | 487 |
| as | 97 | CCCGUAGGGCCAGCCUCAG | 208 | CCCGUAGGGCCAGCCUCAGNN | 488 |
| s | 81 | GAGGCUGGCCCUACGGGCA | 209 | GAGGCUGGCCCUACGGGCANN | 489 |
| as | 99 | UGCCCGUAGGGCCAGCCUC | 210 | UGCCCGUAGGGCCAGCCUCNN | 490 |
| s | 82 | AGGCUGGCCCUACGGGCAC | 211 | AGGCUGGCCCUACGGGCACNN | 491 |
| as | 100 | GUGCCCGUAGGGCCAGCCU | 212 | GUGCCCGUAGGGCCAGCCUNN | 492 |
| s | 84 | GCUGGCCCUACGGGCACCG | 213 | GCUGGCCCUACGGGCACCGNN | 493 |
| as | 102 | CGGUGCCCGUAGGGCCAGC | 214 | CGGUGCCCGUAGGGCCAGCNN | 494 |
| s | 85 | CUGGCCCUACGGGCACCGG | 215 | CUGGCCCUACGGGCACCGGNN | 495 |
| as | 103 | CCGGUGCCCGUAGGGCCAG | 216 | CCGGUGCCCGUAGGGCCAGNN | 496 |
| s | 87 | GGCCCUACGGGCACCGGUG | 217 | GGCCCUACGGGCACCGGUGNN | 497 |
| as | 105 | CACCGGUGCCCGUAGGGCC | 218 | CACCGGUGCCCGUAGGGCCNN | 498 |
| s | 9 | CCACUCAUUCUUGGCAGGA | 219 | CCACUCAUUCUUGGCAGGANN | 499 |
| as | 27 | UCCUGCCAAGAAUGAGUGG | 220 | UCCUGCCAAGAAUGAGUGGNN | 500 |
| s | 90 | CCUACGGGCACCGGUGAAU | 221 | CCUACGGGCACCGGUGAAUNN | 501 |
| as | 108 | AUUCACCGGUGCCCGUAGG | 222 | AUUCACCGGUGCCCGUAGGNN | 502 |
| s | 91 | CUACGGGCACCGGUGAAUC | 223 | CUACGGGCACCGGUGAAUCNN | 503 |
| as | 109 | GAUUCACCGGUGCCCGUAG | 224 | GAUUCACCGGUGCCCGUAGNN | 504 |
| s | 92 | UACGGGCACCGGUGAAUCC | 225 | UACGGGCACCGGUGAAUCCNN | 505 |
| as | 110 | GGAUUCACCGGUGCCCGUA | 226 | GGAUUCACCGGUGCCCGUANN | 506 |

(continued)

| Strand: s= sense; as= antisense; Position: position of 5' base on transcript (NM_000371.2, SEQ ID NO: 1329) | | | | | |
|---|---|---|---|---|---|
| **Strand** | **Position** | **Sequence (5' to 3')** | **SEQ ID NO:** | **Sequence with 3' dinucleotide overhang (5' to 3')** | **SEQ ID NO:** |
| s | 93 | ACGGGCACCGGUGAAUCCA | 227 | ACGGGCACCGGUGAAUCCANN | 507 |
| as | 111 | UGGAUUCACCGGUGCCCGU | 228 | UGGAUUCACCGGUGCCCGUNN | 508 |
| s | 97 | GCACCGGUGAAUCCAAGUG | 229 | GCACCGGUGAAUCCAAGUGNN | 509 |
| as | 115 | CACUUGGAUUCACCGGUGC | 230 | CACUUGGAUUCACCGGUGCNN | 510 |
| s | 98 | CACCGGUGAAUCCAAGUGU | 231 | CACCGGUGAAUCCAAGUGUNN | 511 |
| as | 116 | ACACUUGGAUUCACCGGUG | 232 | ACACUUGGAUUCACCGGUGNN | 512 |
| s | 167 | UGUGGCCAUGCAUGUGUUC | 233 | UGUGGCCAUGCAUGUGUUCNN | 513 |
| as | 185 | GAACACAUGCAUGGCCACA | 234 | GAACACAUGCAUGGCCACANN | 514 |
| s | 168 | GUGGCCAUGCAUGUGUUCA | 235 | GUGGCCAUGCAUGUGUUCANN | 515 |
| as | 186 | UGAACACAUGCAUGGCCAC | 236 | UGAACACAUGCAUGGCCACNN | 516 |
| s | 171 | GCCAUGCAUGUGUUCAGAA | 237 | GCCAUGCAUGUGUUCAGAANN | 517 |
| as | 189 | UUCUGAACACAUGCAUGGC | 238 | UUCUGAACACAUGCAUGGCNN | 518 |
| s | 432 | UAUUCCACCACGGCUGUCA | 239 | UAUUCCACCACGGCUGUCANN | 519 |
| as | 449 | UGACAGCCGUGGUGGAAUA | 240 | UGACAGCCGUGGUGGAAUANN | 520 |
| s | 447 | GUCAUCACCAAUCCCAAGG | 241 | GUCAUCACCAAUCCCAAGGNN | 521 |
| as | 465 | CCUUGGGAUUGGUGAUGAC | 242 | CCUUGGGAUUGGUGAUGACNN | 522 |
| s | 115 | GUCCUCUGAUGGUCAAAGU | 243 | GUCCUCUGAUGGUCAAAGUNN | 523 |
| as | 133 | ACUUUGACCAUCAGAGGAC | 244 | ACUUUGACCAUCAGAGGACNN | 524 |
| s | 122 | GAUGGUCAAAGUUCUAGAU | 245 | GAUGGUCAAAGUUCUAGAUNN | 525 |
| as | 140 | AUCUAGAACUUUGACCAUC | 246 | AUCUAGAACUUUGACCAUCNN | 526 |
| s | 139 | AUGCUGUCCGAGGCAGUCC | 247 | AUGCUGUCCGAGGCAGUCCNN | 527 |
| as | 157 | GGACUGCCUCGGACAGCAU | 248 | GGACUGCCUCGGACAGCAUNN | 528 |
| s | 172 | CCGUGCAUGUGUUCAGAAA | 249 | CCGUGCAUGUGUUCAGAAANN | 529 |
| as | 190 | UUUCUGAACACAUGCACGG | 250 | UUUCUGAACACAUGCACGGNN | 530 |
| s | 238 | AGUCUGGAGAGCUGCAUGG | 251 | AGUCUGGAGAGCUGCAUGGNN | 531 |
| as | 256 | CCAUGCAGCUCUCCAGACU | 252 | CCAUGCAGCUCUCCAGACUNN | 532 |
| s | 252 | CAUGGGCUCACAACUGAGG | 253 | CAUGGGCUCACAACUGAGGNN | 533 |
| as | 270 | CCUCAGUUGUGAGCCCAUG | 254 | CCUCAGUUGUGAGCCCAUGNN | 534 |
| s | 33 | UCUCAUCGUCUGCUCCUCC | 255 | UCUCAUCGUCUGCUCCUCCNN | 535 |
| as | 51 | GGAGGAGCAGACGAUGAGA | 256 | GGAGGAGCAGACGAUGAGANN | 536 |
| s | 340 | CCCCAUUCCAUGAGCAUGC | 257 | CCCCAUUCCAUGAGCAUGCNN | 537 |
| as | 358 | GCAUGCUCAUGGAAUGGGG | 258 | GCAUGCUCAUGGAAUGGGGNN | 538 |
| s | 421 | GCCCCUACUCCUAUUCCAC | 259 | GCCCCUACUCCUAUUCCACNN | 539 |
| as | 439 | GUGGAAUAGGAGUAGGGGC | 260 | GUGGAAUAGGAGUAGGGGCNN | 540 |
| s | 431 | CUAUUCCACCACGGCUGUC | 261 | CUAUUCCACCACGGCUGUCNN | 541 |

(continued)

| Strand: s= sense; as= antisense; Position: position of 5' base on transcript (NM_000371.2, SEQ ID NO: 1329) | | | | | |
|---|---|---|---|---|---|
| Strand | Position | Sequence (5' to 3') | SEQ ID NO: | Sequence with 3' dinucleotide overhang (5' to 3') | SEQ ID NO: |
| as | 449 | GACAGCCGUGGUGGAAUAG | 262 | GACAGCCGUGGUGGAAUAGNN | 542 |
| s | 440 | CACGGCUGUCGUCACCAAU | 263 | CACGGCUGUCGUCACCAAUNN | 543 |
| as | 458 | AUUGGUGACGACAGCCGUG | 264 | AUUGGUGACGACAGCCGUGNN | 544 |
| s | 496 | AGGACGAGGGAUGGGAUUU | 265 | AGGACGAGGGAUGGGAUUUNN | 545 |
| as | 514 | AAAUCCCAUCCCUCGUCCU | 266 | AAAUCCCAUCCCUCGUCCUNN | 546 |
| s | 556 | UCACCUCAUAUGCUAUGUU | 267 | UCACCUCAUAUGCUAUGUUNN | 547 |
| as | 574 | AACAUAGCAUAUGAGGUGA | 268 | AACAUAGCAUAUGAGGUGANN | 548 |
| s | 559 | CCUCAUAUGCUAUGUUAGA | 269 | CCUCAUAUGCUAUGUUAGANN | 549 |
| as | 577 | UCUAACAUAGCAUAUGAGG | 270 | UCUAACAUAGCAUAUGAGGNN | 550 |
| s | 570 | AUGUUAGAAGUCCAGGCAG | 271 | AUGUUAGAAGUCCAGGCAGNN | 551 |
| as | 588 | CUGCCUGGACUUCUAACAU | 272 | CUGCCUGGACUUCUAACAUNN | 552 |
| s | 78 | UCUGAGGCUGGCCCUACGG | 273 | UCUGAGGCUGGCCCUACGGNN | 553 |
| as | 96 | CCGUAGGGCCAGCCUCAGA | 274 | CCGUAGGGCCAGCCUCAGANN | 554 |
| s | 87 | GGCCCUACGGGCACCGGUG | 275 | GGCCCUACGGGCACCGGUGNN | 555 |
| as | 105 | CACCGGUGCCCGUAGGGCC | 276 | CACCGGUGCCCGUAGGGCCNN | 556 |
| s | 95 | GGGCACCGGUGAAUCCAAG | 277 | GGGCACCGGUGAAUCCAAGNN | 557 |
| as | 113 | CUUGGAUUCACCGGUGCCC | 278 | CUUGGAUUCACCGGUGCCCNN | 558 |
| s | 167 | CCAUGCAUGUGUUCAGAAA | 279 | CCAUGCAUGUGUUCAGAAANN | 559 |
| as | 185 | UUUCUGAACACAUGCAUGG | 280 | UUUCUGAACACAUGCAUGGNN | 560 |

Table 3B. Sense and antisense strand sequences of human TTR dsRNAs

| Strand: s= sense; as= antisense; Position: position of 5' base on transcript (NM_000371.2, SEQ ID NO: 1329) | | | |
|---|---|---|---|
| Strand | Position | Sequence with 3' deoxythimidine overhang (5' to 3') | SEQ ID NO: |
| s | 100 | CCGGUGAAUCCAAGUGUCCdTdT | 561 |
| as | 118 | GGACACUUGGAUUCACCGGdTdT | 562 |
| s | 11 | ACUCAUUCUUGGCAGGAUGdTdT | 563 |
| as | 29 | CAUCCUGCCAAGAAUGAGUdTdT | 564 |
| s | 111 | AAGUGUCCUCUGAUGGUCAdTdT | 565 |
| as | 129 | UGACCAUCAGAGGACACUUdTdT | 566 |
| s | 13 | UCAUUCUUGGCAGGAUGGCdTdT | 567 |
| as | 31 | GCCAUCCUGCCAAGAAUGAdTdT | 568 |
| s | 130 | AAGUUCUAGAUGCUGUCCGdTdT | 569 |
| as | 148 | CGGACAGCAUCUAGAACUUdTdT | 570 |
| s | 132 | GUUCUAGAUGCUGUCCGAGdTdT | 571 |
| as | 150 | CUCGGACAGCAUCUAGAACdTdT | 572 |

(continued)

| Strand: s= sense; as= antisense; Position: position of 5' base on transcript (NM_000371.2, SEQ ID NO: 1329) | | | |
|---|---|---|---|
| **Strand** | **Position** | **Sequence with 3'deoxythimidine overhang (5' to 3')** | **SEQ ID NO:** |
| s | 135 | CUAGAUGCUGUCCGAGGCAdTdT | 573 |
| as | 153 | UGCCUCGGACAGCAUCUAGdTdT | 574 |
| s | 138 | GAUGCUGUCCGAGGCAGUCdTdT | 575 |
| as | 156 | GACUGCCUCGGACAGCAUCdTdT | 576 |
| s | 14 | CAUUCUUGGCAGGAUGGCUdTdT | 577 |
| as | 32 | AGCCAUCCUGCCAAGAAUGdTdT | 578 |
| s | 140 | UGCUGUCCGAGGCAGUCCUdTdT | 579 |
| as | 158 | AGGACUGCCUCGGACAGCAdTdT | 580 |
| s | 146 | CCGAGGCAGUCCUGCCAUCdTdT | 581 |
| as | 164 | GAUGGCAGGACUGCCUCGGdTdT | 582 |
| s | 152 | CAGUCCUGCCAUCAAUGUGdTdT | 583 |
| as | 170 | CACAUUGAUGGCAGGACUGdTdT | 584 |
| s | 164 | CAAUGUGGCCGUGCAUGUGdTdT | 585 |
| as | 182 | CACAUGCACGGCCACAUUGdTdT | 586 |
| s | 178 | AUGUGUUCAGAAAGGCUGCdTdT | 587 |
| as | 196 | GCAGCCUUUCUGAACACAUdTdT | 588 |
| s | 2 | CAGAAGUCCACUCAUUCUUdTdT | 589 |
| as | 20 | AAGAAUGAGUGGACUUCUGdTdT | 590 |
| s | 21 | GGCAGGAUGGCUUCUCAUCdTdT | 591 |
| as | 39 | GAUGAGAAGCCAUCCUGCCdTdT | 592 |
| s | 210 | GAGCCAUUUGCCUCUGGGAdTdT | 593 |
| as | 228 | UCCCAGAGGCAAAUGGCUCdTdT | 594 |
| s | 23 | CAGGAUGGCUUCUCAUCGUdTdT | 595 |
| as | 41 | ACGAUGAGAAGCCAUCCUGdTdT | 596 |
| s | 24 | AGGAUGGCUUCUCAUCGUCdTdT | 597 |
| as | 42 | GACGAUGAGAAGCCAUCCUdTdT | 598 |
| s | 245 | AGAGCUGCAUGGGCUCACAdTdT | 599 |
| as | 263 | UGUGAGCCCAUGCAGCUCUdTdT | 600 |
| s | 248 | GCUGCAUGGGCUCACAACUdTdT | 601 |
| as | 266 | AGUUGUGAGCCCAUGCAGCdTdT | 602 |
| s | 25 | GGAUGGCUUCUCAUCGUCUdTdT | 603 |
| as | 43 | AGACGAUGAGAAGCCAUCCdTdT | 604 |
| s | 251 | GCAUGGGCUCACAACUGAGdTdT | 605 |
| as | 269 | CUCAGUUGUGAGCCCAUGCdTdT | 606 |
| s | 253 | AUGGGCUCACAACUGAGGAdTdT | 607 |
| as | 271 | UCCUCAGUUGUGAGCCCAUdTdT | 608 |
| s | 254 | UGGGCUCACAACUGAGGAGdTdT | 609 |

(continued)

| Strand: s= sense; as= antisense; Position: position of 5' base on transcript (NM_000371.2, SEQ ID NO: 1329) | | | |
|---|---|---|---|
| **Strand** | **Position** | **Sequence with 3'deoxythimidine overhang (5' to 3')** | **SEQ ID NO:** |
| as | 272 | CUCCUCAGUUGUGAGCCCAdTdT | 610 |
| s | 270 | GAGGAAUUUGUAGAAGGGAdTdT | 611 |
| as | 288 | UCCCUUCUACAAAUUCCUCdTdT | 612 |
| s | 276 | UUUGUAGAAGGGAUAUACAdTdT | 613 |
| as | 294 | UGUAUAUCCCUUCUACAAAdTdT | 614 |
| s | 277 | UUGUAGAAGGGAUAUACAAdTdT | 615 |
| as | 295 | UUGUAUAUCCCUUCUACAAdTdT | 616 |
| s | 278 | UGUAGAAGGGAUAUACAAAdTdT | 617 |
| as | 296 | UUUGUAUAUCCCUUCUACAdTdT | 618 |
| s | 281 | AGAAGGGAUAUACAAAGUGdTdT | 619 |
| as | 299 | CACUUUGUAUAUCCCUUCUdTdT | 620 |
| s | 295 | AAGUGGAAAUAGACACCAAdTdT | 621 |
| as | 313 | UUGGUGUCUAUUUCCACUUdTdT | 622 |
| s | 299 | GGAAAUAGACACCAAAUCUdTdT | 623 |
| as | 317 | AGAUUUGGUGUCUAUUUCCdTdT | 624 |
| s | 300 | GAAAUAGACACCAAAUCUUdTdT | 625 |
| as | 318 | AAGAUUUGGUGUCUAUUUCdTdT | 626 |
| s | 303 | AUAGACACCAAAUCUUACUdTdT | 627 |
| as | 321 | AGUAAGAUUUGGUGUCUAUdTdT | 628 |
| s | 304 | UAGACACCAAAUCUUACUGdTdT | 629 |
| as | 322 | CAGUAAGAUUUGGUGUCUAdTdT | 630 |
| s | 305 | AGACACCAAAUCUUACUGGdTdT | 631 |
| as | 323 | CCAGUAAGAUUUGGUGUCUdTdT | 632 |
| s | 317 | UUACUGGAAGGCACUUGGCdTdT | 633 |
| as | 335 | GCCAAGUGCCUUCCAGUAAdTdT | 634 |
| s | 32 | UUCUCAUCGUCUGCUCCUCdTdT | 635 |
| as | 50 | GAGGAGCAGACGAUGAGAAdTdT | 636 |
| s | 322 | GGAAGGCACUUGGCAUCUCdTdT | 637 |
| as | 340 | GAGAUGCCAAGUGCCUUCCdTdT | 638 |
| s | 326 | GGCACUUGGCAUCUCCCCAdTdT | 639 |
| as | 344 | UGGGGAGAUGCCAAGUGCCdTdT | 640 |
| s | 333 | GGCAUCUCCCCAUUCCAUGdTdT | 641 |
| as | 351 | AUGGAAUGGGGAGAUGCCTTdTdT | 642 |
| s | 334 | GCAUCUCCCCAUUCCAUGAdTdT | 643 |
| as | 352 | UCAUGGAAUGGGGAGAUGCdTdT | 644 |
| s | 335 | CAUCUCCCCAUUCCAUGAGdTdT | 645 |
| as | 353 | CUCAUGGAAUGGGGAGAUGdTdT | 646 |

(continued)

| Strand: s= sense; as= antisense; Position: position of 5' base on transcript (NM_000371.2, SEQ ID NO: 1329) | | | |
|---|---|---|---|
| **Strand** | **Position** | **Sequence with 3'deoxythimidine overhang (5' to 3')** | **SEQ ID NO:** |
| s | 336 | AUCUCCCCAUUCCAUGAGCdTdT | 647 |
| as | 354 | GCUCAUGGAAUGGGGAGAUdTdT | 648 |
| s | 338 | CUCCCCAUUCCAUGAGCAUdTdT | 649 |
| as | 356 | AUGCUCAUGGAAUGGGGAGdTdT | 650 |
| s | 341 | CCCAUUCCAUGAGCAUGCAdTdT | 651 |
| as | 359 | UGCAUGCUCAUGGAAUGGGdTdT | 652 |
| s | 347 | CCAUGAGCAUGCAGAGGUGdTdT | 653 |
| as | 365 | CACCUCUGCAUGCUCAUGGdTdT | 654 |
| s | 352 | AGCAUGCAGAGGUGGUAUUdTdT | 655 |
| as | 370 | AAUACCACCUCUGCAUGCUdTdT | 656 |
| s | 354 | CAUGCAGAGGUGGUAUUCAdTdT | 657 |
| as | 372 | UGAAUACCACCUCUGCAUGdTdT | 658 |
| s | 355 | AUGCAGAGGUGGUAUUCACdTdT | 659 |
| as | 373 | GUGAAUACCACCUCUGCAUdTdT | 660 |
| s | 362 | GGUGGUAUUCACAGCCAACdTdT | 661 |
| as | 380 | GUUGGCUGUGAAUACCACCdTdT | 662 |
| s | 363 | GUGGUAUUCACAGCCAACGdTdT | 663 |
| as | 381 | CGUUGGCUGUGAAUACCACdTdT | 664 |
| s | 364 | UGGUAUUCACAGCCAACGAdTdT | 665 |
| as | 382 | UCGUUGGCUGUGAAUACCAdTdT | 666 |
| s | 365 | GGUAUUCACAGCCAACGACdTdT | 667 |
| as | 383 | GUCGUUGGCUGUGAAUACCdTdT | 668 |
| s | 366 | GUAUUCACAGCCAACGACUdTdT | 669 |
| as | 384 | AGUCGUUGGCUGUGAAUACdTdT | 670 |
| s | 367 | UAUUCACAGCCAACGACUCdTdT | 671 |
| as | 385 | GAGUCGUUGGCUGUGAAUAdTdT | 672 |
| s | 370 | UCACAGCCAACGACUCCGGdTdT | 673 |
| as | 388 | CCGGAGUCGUUGGCUGUGAdTdT | 674 |
| s | 390 | CCCCGCCGCUACACCAUUGdTdT | 675 |
| as | 408 | CAAUGGUGUAGCGGCGGGGdTdT | 676 |
| s | 4 | GAAGUCCACUCAUUCUUGGdTdT | 677 |
| as | 22 | CCAAGAAUGAGUGGACUUCdTdT | 678 |
| s | 412 | CCCUGCUGAGCCCCUACUCdTdT | 679 |
| as | 430 | GAGUAGGGGCUCAGCAGGGdTdT | 680 |
| s | 417 | CUGAGCCCCUACUCCUAUUdTdT | 681 |
| as | 435 | AAUAGGAGUAGGGGCUCAGdTdT | 682 |
| s | 418 | UGAGCCCCUACUCCUAUUCdTdT | 683 |

(continued)

| Strand: s= sense; as= antisense; Position: position of 5' base on transcript (NM_000371.2, SEQ ID NO: 1329) | | | |
|---|---|---|---|
| Strand | Position | Sequence with 3'deoxythimidine overhang (5' to 3') | SEQ ID NO: |
| as | 436 | GAAUAGGAGUAGGGGCUCAdTdT | 684 |
| s | 422 | CCCCUACUCCUAUUCCACCdTdT | 685 |
| as | 440 | GGUGGAAUAGGAGUAGGGGdTdT | 686 |
| s | 425 | CUACUCCUAUUCCACCACGdTdT | 687 |
| as | 443 | CGUGGUGGAAUAGGAGUAGdTdT | 688 |
| s | 426 | UACUCCUAUUCCACCACGGdTdT | 689 |
| as | 444 | CCGUGGUGGAAUAGGAGUAdTdT | 690 |
| s | 427 | ACUCCUAUUCCACCACGGCdTdT | 691 |
| as | 445 | GCCGUGGUGGAAUAGGAGUdTdT | 692 |
| s | 429 | UCCUAUUCCACCACGGCUGdTdT | 693 |
| as | 447 | CAGCCGUGGUGGAAUAGGAdTdT | 694 |
| s | 432 | UAUUCCACCACGGCUGUCGdTdT | 695 |
| as | 450 | CGACAGCCGUGGUGGAAUAdTdT | 696 |
| s | 433 | AUUCCACCACGGCUGUCGUdTdT | 697 |
| as | 451 | ACGACAGCCGUGGUGGAAUdTdT | 698 |
| s | 437 | CACCACGGCUGUCGUCACCdTdT | 699 |
| as | 455 | GGUGACGACAGCCGUGGUGdTdT | 700 |
| s | 438 | ACCACGGCUGUCGUCACCAdTdT | 701 |
| as | 456 | UGGUGACGACAGCCGUGGUdTdT | 702 |
| s | 439 | CCACGGCUGUCGUCACCAAdTdT | 703 |
| as | 457 | UUGGUGACGACAGCCGUGGdTdT | 704 |
| s | 441 | ACGGCUGUCGUCACCAAUCdTdT | 705 |
| as | 459 | GAUUGGUGACGACAGCCGUdTdT | 706 |
| s | 442 | CGGCUGUCGUCACCAAUCCdTdT | 707 |
| as | 460 | GGAUUGGUGACGACAGCCGdTdT | 708 |
| s | 449 | CGUCACCAAUCCCAAGGAAdTdT | 709 |
| as | 467 | UUCCUUGGGAUUGGUGACGdTdT | 710 |
| s | 455 | CAAUCCCAAGGAAUGAGGGdTdT | 711 |
| as | 473 | CCCUCAUUCCUUGGGAUUGdTdT | 712 |
| s | 491 | CCUGAAGGACGAGGGAUGGdTdT | 713 |
| as | 509 | CCAUCCCUCGUCCUUCAGGdTdT | 714 |
| s | 497 | GGACGAGGGAUGGGAUUUCdTdT | 715 |
| as | 515 | GAAAUCCCAUCCCUCGUCCdTdT | 716 |
| s | 5 | AAGUCCACUCAUUCUUGGCdTdT | 717 |
| as | 23 | GCCAAGAAUGAGUGGACUUdTdT | 718 |
| s | 508 | GGGAUUUCAUGUAACCAAGdTdT | 719 |
| as | 526 | CUUGGUUACAUGAAAUCCCdTdT | 720 |

(continued)

| Strand: s= sense; as= antisense; Position: position of 5' base on transcript (NM_000371.2, SEQ ID NO: 1329) | | | |
|---|---|---|---|
| Strand | Position | Sequence with 3'deoxythimidine overhang (5' to 3') | SEQ ID NO: |
| s | 509 | GGAUUUCAUGUAACCAAGAdTdT | 721 |
| as | 527 | UCUUGGUUACAUGAAAUCCdTdT | 722 |
| s | 514 | UCAUGUAACCAAGAGUAUUdTdT | 723 |
| as | 532 | AAUACUCUUGGUUACAUGAdTdT | 724 |
| s | 516 | AUGUAACCAAGAGUAUUCCdTdT | 725 |
| as | 534 | GGAAUACUCUUGGUUACAUdTdT | 726 |
| s | 517 | UGUAACCAAGAGUAUUCCAdTdT | 727 |
| as | 535 | UGGAAUACUCUUGGUUACAdTdT | 728 |
| s | 518 | GUAACCAAGAGUAUUCCAUdTdT | 729 |
| as | 536 | AUGGAAUACUCUUGGUUACdTdT | 730 |
| s | 54 | UGCCUUGCUGGACUGGUAUdTdT | 731 |
| as | 72 | AUACCAGUCCAGCAAGGCAdTdT | 732 |
| s | 543 | UAAAGCAGUGUUUUCACCUdTdT | 733 |
| as | 561 | AGGUGAAAACACUGCUUUAdTdT | 734 |
| s | 55 | GCCUUGCUGGACUGGUAUUdTdT | 735 |
| as | 73 | AAUACCAGUCCAGCAAGGCdTdT | 736 |
| s | 551 | UGUUUUCACCUCAUAUGCUdTdT | 737 |
| as | 569 | AGCAUAUGAGGUGAAAACAdTdT | 738 |
| s | 552 | GUUUUCACCUCAUAUGCUAdTdT | 739 |
| as | 570 | UAGCAUAUGAGGUGAAAACdTdT | 740 |
| s | 553 | UUUUCACCUCAUAUGCUAUdTdT | 741 |
| as | 571 | AUAGCAUAUGAGGUGAAAAdTdT | 742 |
| s | 555 | UUCACCUCAUAUGCUAUGUdTdT | 743 |
| as | 573 | ACAUAGCAUAUGAGGUGAAdTdT | 744 |
| s | 557 | CACCUCAUAUGCUAUGUUAdTdT | 745 |
| as | 575 | UAACAUAGCAUAUGAGGUGdTdT | 746 |
| s | 56 | CCUUGCUGGACUGGUAUUUdTdT | 747 |
| as | 74 | AAAUACCAGUCCAGCAAGGdTdT | 748 |
| s | 563 | AUAUGCUAUGUUAGAAGUCdTdT | 749 |
| as | 581 | GACUUCUAACAUAGCAUAUdTdT | 750 |
| s | 564 | UAUGCUAUGUUAGAAGUCCdTdT | 751 |
| as | 582 | GGACUUCUAACAUAGCAUAdTdT | 752 |
| s | 566 | UGCUAUGUUAGAAGUCCAGdTdT | 753 |
| as | 584 | CUGGACUUCUAACAUAGCAdTdT | 754 |
| s | 57 | CUUGCUGGACUGGUAUUUGdTdT | 755 |
| as | 75 | CAAAUACCAGUCCAGCAAGdTdT | 756 |
| s | 578 | AGUCCAGGCAGAGACAAUAdTdT | 757 |

(continued)

| Strand: s= sense; as= antisense; Position: position of 5' base on transcript (NM_000371.2, SEQ ID NO: 1329) | | | |
|---|---|---|---|
| **Strand** | **Position** | **Sequence with 3'deoxythimidine overhang (5' to 3')** | **SEQ ID NO:** |
| as | 596 | AUUGUCUCUGCCUGGACUTTdTdT | 758 |
| s | 580 | UCCAGGCAGAGACAAUAAAdTdT | 759 |
| as | 598 | UUUAUUGUCUCUGCCUGGAdTdT | 760 |
| s | 607 | GUGAAAGGCACUUUUCAUUdTdT | 761 |
| as | 625 | AAUGAAAAGUGCCUUUCACdTdT | 762 |
| s | 62 | UGGACUGGUAUUUGUGUCUdTdT | 763 |
| as | 80 | AGACACAAAUACCAGUCCAdTdT | 764 |
| s | 77 | GUCUGAGGCUGGCCCUACGdTdT | 765 |
| as | 95 | CGUAGGGCCAGCCUCAGACdTdT | 766 |
| s | 79 | CUGAGGCUGGCCCUACGGGdTdT | 767 |
| as | 97 | CCCGUAGGGCCAGCCUCAGdTdT | 768 |
| s | 81 | GAGGCUGGCCCUACGGGCAdTdT | 769 |
| as | 99 | UGCCCGUAGGGCCAGCCUCdTdT | 770 |
| s | 82 | AGGCUGGCCCUACGGGCACdTdT | 771 |
| as | 100 | GUGCCCGUAGGGCCAGCCUdTdT | 772 |
| s | 84 | GCUGGCCCUACGGGCACCGdTdT | 773 |
| as | 102 | CGGUGCCCGUAGGGCCAGCdTdT | 774 |
| s | 85 | CUGGCCCUACGGGCACCGGdTdT | 775 |
| as | 103 | CCGGUGCCCGUAGGGCCAGdTdT | 776 |
| s | 87 | GGCCCUACGGGCACCGGUGdTdT | 777 |
| as | 105 | CACCGGUGCCCGUAGGGCCdTdT | 778 |
| s | 9 | CCACUCAUUCUUGGCAGGAdTdT | 779 |
| as | 27 | UCCUGCCAAGAAUGAGUGGdTdT | 780 |
| s | 90 | CCUACGGGCACCGGUGAAUdTdT | 781 |
| as | 108 | AUUCACCGGUGCCCGUAGGdTdT | 782 |
| s | 91 | CUACGGGCACCGGUGAAUCdTdT | 783 |
| as | 109 | GAUUCACCGGUGCCCGUAGdTdT | 784 |
| s | 92 | UACGGGCACCGGUGAAUCCdTdT | 785 |
| as | 110 | GGAUUCACCGGUGCCCGUAdTdT | 786 |
| s | 93 | ACGGGCACCGGUGAAUCCAdTdT | 787 |
| as | 111 | UGGAUUCACCGGUGCCCGUdTdT | 788 |
| s | 97 | GCACCGGUGAAUCCAAGUGdTdT | 789 |
| as | 115 | CACUUGGAUUCACCGGUGCdTdT | 790 |
| s | 98 | CACCGGUGAAUCCAAGUGUdTdT | 791 |
| as | 116 | ACACUUGGAUUCACCGGUGdTdT | 792 |
| s | 167 | UGUGGCCAUGCAUGUGUUCdTdT | 793 |
| as | 185 | GAACACAUGCAUGGCCACAdTdT | 794 |

(continued)

| Strand: s= sense; as= antisense; Position: position of 5' base on transcript (NM_000371.2, SEQ ID NO: 1329) | | | |
|---|---|---|---|
| Strand | Position | Sequence with 3'deoxythimidine overhang (5' to 3') | SEQ ID NO: |
| s | 168 | GUGGCCAUGCAUGUGUUCAdTdT | 795 |
| as | 186 | UGAACACAUGCAUGGCCACdTdT | 796 |
| s | 171 | GCCAUGCAUGUGUUCAGAAdTdT | 797 |
| as | 189 | UUCUGAACACAUGCAUGGCdTdT | 798 |
| s | 432 | UAUUCCACCACGGCUGUCAdTdT | 799 |
| as | 449 | UGACAGCCGUGGUGGAAUAdTdT | 800 |
| s | 447 | GUCAUCACCAAUCCCAAGGdTdT | 801 |
| as | 465 | CCUUGGGAUUGGUGAUGACdTdT | 802 |
| s | 115 | GUCCUCUGAUGGUCAAAGUdTdT | 803 |
| as | 133 | ACUUUGACCAUCAGAGGACdTdT | 804 |
| s | 122 | GAUGGUCAAAGUUCUAGAUdTdT | 805 |
| as | 140 | AUCUAGAACUUUGACCAUCdTdT | 806 |
| s | 139 | AUGCUGUCCGAGGCAGUCCdTdT | 807 |
| as | 157 | GGACUGCCUCGGACAGCAUdTdT | 808 |
| s | 172 | CCGUGCAUGUGUUCAGAAAdTdT | 809 |
| as | 190 | UUUCUGAACACAUGCACGGdTdT | 810 |
| s | 238 | AGUCUGGAGAGCUGCAUGGdTdT | 811 |
| as | 256 | CCAUGCAGCUCUCCAGACUdTdT | 812 |
| s | 252 | CAUGGGCUCACAACUGAGGdTdT | 813 |
| as | 270 | CCUCAGUUGUGAGCCCAUGdTdT | 814 |
| s | 33 | UCUCAUCGUCUGCUCCUCCdTdT | 815 |
| as | 51 | GGAGGAGCAGACGAUGAGAdTdT | 816 |
| s | 340 | CCCCAUUCCAUGAGCAUGCdTdT | 817 |
| as | 358 | GCAUGCUCAUGGAAUGGGGdTdT | 818 |
| s | 421 | GCCCCUACUCCUAUUCCACdTdT | 819 |
| as | 439 | GUGGAAUAGGAGUAGGGGCdTdT | 820 |
| s | 431 | CUAUUCCACCACGGCUGUCdTdT | 821 |
| as | 449 | GACAGCCGUGGUGGAAUAGdTdT | 822 |
| s | 440 | CACGGCUGUCGUCACCAAUdTdT | 823 |
| as | 458 | AUUGGUGACGACAGCCGUGdTdT | 824 |
| s | 496 | AGGACGAGGGAUGGGAUUUdTdT | 825 |
| as | 514 | AAAUCCCAUCCCUCGUCCUdTdT | 826 |
| s | 556 | UCACCUCAUAUGCUAUGUUdTdT | 827 |
| as | 574 | AACAUAGCAUAUGAGGUGAdTdT | 828 |
| s | 559 | CCUCAUAUGCUAUGUUAGAdTdT | 829 |
| as | 577 | UCUAACAUAGCAUAUGAGGdTdT | 830 |
| s | 570 | AUGUUAGAAGUCCAGGCAGdTdT | 831 |

(continued)

| Strand: s= sense; as= antisense; Position: position of 5' base on transcript (NM_000371.2, SEQ ID NO: 1329) | | | |
|---|---|---|---|
| **Strand** | **Position** | **Sequence with 3'deoxythimidine overhang (5' to 3')** | **SEQ ID NO:** |
| as | 588 | CUGCCUGGACUUCUAACAUdTdT | 832 |
| s | 78 | UCUGAGGCUGGCCCUACGGdTdT | 833 |
| as | 96 | CCGUAGGGCCAGCCUCAGAdTdT | 834 |
| s | 87 | GGCCCUACGGGCACCGGUGdTdT | 835 |
| as | 105 | CACCGGUGCCCGUAGGGCCdTdT | 836 |
| s | 95 | GGGCACCGGUGAAUCCAAGdTdT | 837 |
| as | 113 | CUUGGAUUCACCGGUGCCCdTdT | 838 |
| s | 167 | CCAUGCAUGUGUUCAGAAAdTdT | 839 |
| as | 185 | UUUCUGAACACAUGCAUGGdTdT | 840 |

Table 4. Chemically modified sense and antisense strand sequences of human TTR dsRNAs

| See Table 2 for duplex #. Strand: s= sense; as= antisense; Position: position of 5' base on transcript (NM_000371.2, SEQ ID NO:1329) | | | | |
|---|---|---|---|---|
| **Strand** | **Oligo #** | **Position** | **Sequence (5' to 3')** | **SEQ ID NO:** |
| s | A-32153 | 100 | ccGGuGAAuccAAGuGuccdTdT | 841 |
| as | A-32154 | 118 | GGAcACUUGGAUUcACCGGdTdT | 842 |
| s | A-32155 | 11 | AcucAuucuuGGcAGGAuGdTdT | 843 |
| as | A-32156 | 29 | cAUCCUGCcAAGAAUGAGUdTdT | 844 |
| s | A-32157 | 111 | AAGuGuccucuGAuGGucAdTdT | 845 |
| as | A-32158 | 129 | UGACcAUcAGAGGAcACUUdTdT | 846 |
| s | A-32163 | 13 | ucAuucuuGGcAGGAuGGcdTdT | 847 |
| as | A-32164 | 31 | GCcAUCCUGCcAAGAAUGAdTdT | 848 |
| s | A-32165 | 130 | AAGuucuAGAuGcuGuccGdTdT | 849 |
| as | A-32166 | 148 | CGGAcAGcAUCuAGAACUUdTdT | 850 |
| s | A-32167 | 132 | GuucuAGAuGcuGuccGAGdTdT | 851 |
| as | A-32168 | 150 | CUCGGAcAGcAUCuAGAACdTdT | 852 |
| s | A-32169 | 135 | cuAGAuGcuGuccGAGGcAdTdT | 853 |
| as | A-32170 | 153 | UGCCUCGGAcAGcAUCuAGdTdT | 854 |
| s | A-32171 | 138 | GAuGcuGuccGAGGcAGucdTdT | 855 |
| as | A-32172 | 156 | GACUGCCUCGGAcAGcAUCdTdT | 856 |
| s | A-32175 | 14 | cAuucuuGGcAGGAuGGcudTdT | 857 |
| as | A-32176 | 32 | AGCcAUCCUGCcAAGAAUGdTdT | 858 |
| s | A-32177 | 140 | uGcuGuccGAGGcAGuccudTdT | 859 |
| as | A-32178 | 158 | AGGACUGCCUCGGAcAGcAdTdT | 860 |
| s | A-32179 | 146 | ccGAGGcAGuccuGccAucdTdT | 861 |
| as | A-32180 | 164 | GAUGGcAGGACUGCCUCGGdTdT | 862 |
| s | A-32181 | 152 | cAGuccuGccAucAAuGuGdTdT | 863 |

(continued)

| See Table 2 for duplex #. Strand: s= sense; as= antisense; Position: position of 5' base on transcript (NM_000371.2, SEQ ID NO:1329) | | | | |
|---|---|---|---|---|
| **Strand** | **Oligo #** | **Position** | **Sequence (5' to 3')** | **SEQ ID NO:** |
| as | A-32182 | 170 | cAcAUUGAUGGcAGGACUGdTdT | 864 |
| s | A-32183 | 164 | cAAuGuGGccGuGcAuGuGdTdT | 865. |
| as | A-32184 | 182 | cAcAUGcACGGCcAcAUUGdTdT | 866 |
| s | A-32187 | 178 | AuGuGuucAGAAAGGcuGcdTdT | 867 |
| as | A-32188 | 196 | GcAGCCUUUCUGAAcAcAUdTdT | 868 |
| s | A-32189 | 2 | cAGAAGuccAcucAuucuudTdT | 869 |
| as | A-32190 | 20 | AAGAAUGAGUGGACUUCUGdTdT | 870 |
| s | A-32191 | 21 | GGcAGGAuGGcuucucAucdTdT | 871 |
| as | A-32192 | 39 | GAUGAGAAGCcAUCCUGCCdTdT | 872 |
| s | A-32193 | 210 | GAGccAuuuGccucuGGGAdTdT | 873 |
| as | A-32194 | 228 | UCCcAGAGGcAAAUGGCUCdTdT | 874 |
| s | A-32195 | 23 | cAGGAuGGcuucucAucGudTdT | 875 |
| as | A-32196 | 41 | ACGAUGAGAAGCcAUCCUGdTdT | 876 |
| s | A-32199 | 24 | AGGAuGGcuucucAucGucdTdT | 877 |
| as | A-32200 | 42 | GACGAUGAGAAGCcAUCCUdTdT | 878 |
| s | A-32201 | 245 | AGAGcuGcAuGGGcucAcAdTdT | 879 |
| as | A-32202 | 263 | UGUGAGCCcAUGcAGCUCUdTdT | 880 |
| s | A-32203 | 248 | GcuGcAuGGGcucAcAAcudTdT | 881 |
| as | A-32204 | 266 | AGUUGUGAGCCcAUGcAGCdTdT | 882 |
| s | A-32205 | 25 | GGAuGGcuucucAucGucudTdT | 883 |
| as | A-32206 | 43 | AGACGAUGAGAAGCcAUCCdTdT | 884 |
| s | A-32207 | 251 | GcAuGGGcucAcAAcuGAGdTdT | 885 |
| as | A-32208 | 269 | CUcAGUUGUGAGCCcAUGCdTdT | 886 |
| s | A-32211 | 253 | AuGGGcucAcAAcuGAGGAdTdT | 887 |
| as | A-32212 | 271 | UCCUcAGUUGUGAGCCcAUdTdT | 888 |
| s | A-32213 | 254 | uGGGcucAcAAcuGAGGAGdTdT | 889 |
| as | A-32214 | 272 | CUCCUcAGUUGUGAGCCcAdTdT | 890 |
| s | A-32215 | 270 | GAGGAAuuuGuAGAAGGGAdTdT | 891 |
| as | A-32216 | 288 | UCCCUUCuAcAAAUUCCUCdTdT | 892 |
| s | A-32217 | 276 | uuuGuAGAAGGGAuAuAcAdTdT | 893 |
| as | A-32218 | 294 | UGuAuAUCCCUUCuAcAAAdTdT | 894 |
| s | A-32219 | 277 | uuGuAGAAGGGAuAuAcAAdTdT | 895 |
| as | A-32220 | 295 | UUGuAuAUCCCUUCuAcAAdTdT | 896 |
| s | A-32221 | 278 | uGuAGAAGGGAuAuAcAAAdTdT | 897 |
| as | A-32222 | 296 | UUUGuAuAUCCCUUCuAcAdTdT | 898 |
| s | A-32223 | 281 | AGAAGGGAuAuAcAAAGuGdTdT | 899 |

(continued)

| See Table 2 for duplex #. Strand: s= sense; as= antisense; Position: position of 5' base on transcript (NM_000371.2, SEQ ID NO:1329) | | | | |
|---|---|---|---|---|
| **Strand** | **Oligo #** | **Position** | **Sequence (5' to 3')** | **SEQ ID NO:** |
| as | A-32224 | 299 | cACUUUGuAuAUCCCUUCUdTdT | 900 |
| s | A-32225 | 295 | AAGuGGAAAuAGAcAccAAdTdT | 901 |
| as | A-32226 | 313 | UUGGUGUCuAUUUCcACUUdTdT | 902 |
| s | A-32227 | 299 | GGAAAuAGAcAccAAAucudTdT | 903 |
| as | A-32228 | 317 | AGAUUUGGUGUCuAUUUCCdTdT | 904 |
| s | A-32229 | 300 | GAAAuAGAcAccAAAucuudTdT | 905 |
| as | A-32230 | 318 | AAGAUUUGGUGUCuAUUUCdTdT | 906 |
| s | A-32231 | 303 | AuAGAcAccAAAucuuAcudTdT | 907 |
| as | A-32232 | 321 | AGuAAGAUUUGGUGUCuAUdTdT | 908 |
| s | A-32233 | 304 | uAGAcAccAAAucuuAcuGdTdT | 909 |
| as | A-32234 | 322 | cAGuAAGAUUUGGUGUCuAdTdT | 910 |
| s | A-32235 | 305 | AGAcAccAAAucuuAcuGGdTdT | 911 |
| as | A-32236 | 323 | CcAGuAAGAUUUGGUGUCUdTdT | 912 |
| s | A-32237 | 317 | uuAcuGGAAGGcAcuuGGcdTdT | 913 |
| as | A-32238 | 335 | GCcAAGUGCCUUCcAGuAAdTdT | 914 |
| s | A-32239 | 32 | uucucAucGucuGcuccucdTdT | 915 |
| as | A-32240 | 50 | GAGGAGcAGACGAUGAGAAdTdT | 916 |
| s | A-32241 | 322 | GGAAGGcAcuuGGcAucucdTdT | 917 |
| as | A-32242 | 340 | GAGAUGCcAAGUGCCUUCCdTdT | 918 |
| s | A-32243 | 326 | GGcAcuuGGcAucuccccAdTdT | 919 |
| as | A-32244 | 344 | UGGGGAGAUGCcAAGUGCCdTdT | 920 |
| s | A-32247 | 333 | GGcAucuccccAuuccAuGdTdT | 921 |
| as | A-32248 | 351 | cAUGGAAUGGGGAGAUGCCdTdT | 922 |
| s | A-32249 | 334 | GcAucuccccAuuccAuGAdTdT | 923 |
| as | A-32250 | 352 | UcAUGGAAUGGGGAGAUGCdTdT | 924 |
| s | A-32251 | 335 | cAucuccccAuuccAuGAGdTdT | 925 |
| as | A-32252 | 353 | CUcAUGGAAUGGGGAGAUGdTdT | 926 |
| s | A-32253 | 336 | AucuccccAuuccAuGAGcdTdT | 927 |
| as | A-32254 | 354 | GCUcAUGGAAUGGGGAGAUdTdT | 928 |
| s | A-32255 | 338 | cuccccAuuccAuGAGcAudTdT | 929 |
| as | A-32256 | 356 | AUGCUcAUGGAAUGGGGAGdTdT | 930 |
| s | A-32259 | 341 | cccAuuccAuGAGcAuGcAdTdT | 931 |
| as | A-32260 | 359 | UGcAUGCUcAUGGAAUGGGdTdT | 932 |
| s | A-32261 | 347 | ccAuGAGcAuGcAGAGGuGdTdT | 933 |
| as | A-32262 | 365 | cACCUCUGcAUGCUcAUGGdTdT | 934 |
| s | A-32263 | 352 | AGcAuGcAGAGGuGGuAuudTdT | 935 |

(continued)

| See Table 2 for duplex #. Strand: s= sense; as= antisense; Position: position of 5' base on transcript (NM_000371.2, SEQ ID NO:1329) | | | | |
|---|---|---|---|---|
| **Strand** | **Oligo #** | **Position** | **Sequence (5' to 3')** | **SEQ ID NO:** |
| as | A-32264 | 370 | AAuACcACCUCUGcAUGCUdTdT | 936 |
| s | A-32265 | 354 | cAuGcAGAGGuGGuAuucAdTdT | 937 |
| as | A-32266 | 372 | UGAAuACcACCUCUGcAUGdTdT | 938 |
| s | A-32267 | 355 | AuGcAGAGGuGGuAuucAcdTdT | 939 |
| as | A-32268 | 373 | GUGAAuACcACCUCUGcAUdTdT | 940 |
| s | A-32269 | 362 | GGuGGuAuucAcAGccAAcdTdT | 941 |
| as | A-32270 | 380 | GUUGGCUGUGAAuACcACCdTdT | 942 |
| s | A-32271 | 363 | GuGGuAuucAcAGccAAcGdTdT | 943 |
| as | A-32272 | 381 | CGUUGGCUGUGAAuACcACdTdT | 944 |
| s | A-32273 | 364 | uGGuAuucAcAGccAAcGAdTdT | 945 |
| as | A-32274 | 382 | UCGUUGGCUGUGAAuACcAdTdT | 946 |
| s | A-32275 | 365 | GGuAuucAcAGccAAcGAcdTdT | 947 |
| as | A-32276 | 383 | GUCGUUGGCUGUGAAuACCdTdT | 948 |
| s | A-32277 | 366 | GuAuucAcAGccAAcGAcudTdT | 949 |
| as | A-32278 | 384 | AGUCGUUGGCUGUGAAuACdTdT | 950 |
| s | A-32279 | 367 | uAuucAcAGccAAcGAcucdTdT | 951 |
| as | A-32280 | 385 | GAGUCGUUGGCUGUGAAuAdTdT | 952 |
| s | A-32281 | 370 | ucAcAGccAAcGAcuccGGdTdT | 953 |
| as | A-32282 | 388 | CCGGAGUCGUUGGCUGUGAdTdT | 954 |
| s | A-32283 | 390 | ccccGccGcuAcAccAuuGdTdT | 955 |
| as | A-32284 | 408 | cAAUGGUGuAGCGGCGGGGdTdT | 956 |
| s | A-32285 | 4 | GAAGuccAcucAuucuuGGdTdT | 957 |
| as | A-32286 | 22 | CcAAGAAUGAGUGGACUUCdTdT | 958 |
| s | A-32287 | 412 | cccuGcuGAGccccuAcucdTdT | 959 |
| as | A-32288 | 430 | GAGuAGGGGCUcAGcAGGGdTdT | 960 |
| s | A-32289 | 417 | cuGAGccccuAcuccuAuudTdT | 961 |
| as | A-32290 | 435 | AAuAGGAGuAGGGGCUcAGdTdT | 962 |
| s | A-32291 | 418 | uGAGccccuAcuccuAuucdTdT | 963 |
| as | A-32292 | 436 | GAAuAGGAGuAGGGGCUcAdTdT | 964 |
| s | A-32295 | 422 | ccccuAcuccuAuuccAccdTdT | 965 |
| as | A-32296 | 440 | GGUGGAAuAGGAGuAGGGGdTdT | 966 |
| s | A-32297 | 425 | cuAcuccuAuuccAccAcGdTdT | 967 |
| as | A-32298 | 443 | CGUGGUGGAAuAGGAGuAGdTdT | 968 |
| s | A-32299 | 426 | uAcuccuAuuccAccAcGGdTdT | 969 |
| as | A-32300 | 444 | CCGUGGUGGAAuAGGAGuAdTdT | 970 |
| s | A-32301 | 427 | AcuccuAuuccAccAcGGcdTdT | 971 |

(continued)

| See Table 2 for duplex #. Strand: s= sense; as= antisense; Position: position of 5' base on transcript (NM_000371.2, SEQ ID NO:1329) | | | | |
|---|---|---|---|---|
| **Strand** | **Oligo #** | **Position** | **Sequence (5' to 3')** | **SEQ ID NO:** |
| as | A-32302 | 445 | GCCGUGGUGGAAuAGGAGUdTdT | 972 |
| s | A-32303 | 429 | uccuAuuccAccAcGGcuGdTdT | 973 |
| as | A-32304 | 447 | cAGCCGUGGUGGAAuAGGAdTdT | 974 |
| s | A-32307 | 432 | uAuuccAccAcGGcuGucGdTdT | 975 |
| as | A-32308 | 450 | CGAcAGCCGUGGUGGAAuAdTdT | 976 |
| s | A-32309 | 433 | AuuccAccAcGGcuGucGudTdT | 977 |
| as | A-32310 | 451 | ACGAcAGCCGUGGUGGAAUdTdT | 978 |
| s | A-32311 | 437 | cAccAcGGcuGucGucAccdTdT | 979 |
| as | A-32312 | 455 | GGUGACGAcAGCCGUGGUGdTdT | 980 |
| s | A-32313 | 438 | AccAcGGcuGucGucAccAdTdT | 981 |
| as | A-32314 | 456 | UGGUGACGAcAGCCGUGGUdTdT | 982 |
| s | A-32315 | 439 | ccAcGGcuGucGucAccAAdTdT | 983 |
| as | A-32316 | 457 | UUGGUGACGAcAGCCGUGGdTdT | 984 |
| s | A-32319 | 441 | AcGGcuGucGucAccAAucdTdT | 985 |
| as | A-32320 | 459 | GAUUGGUGACGAcAGCCGUdTdT | 986 |
| s | A-32321 | 442 | cGGcuGucGucAccAAuccdTdT | 987 |
| as | A-32322 | 460 | GGAUUGGUGACGAcAGCCGdTdT | 988 |
| s | A-32323 | 449 | cGucAccAAucccAAGGAAdTdT | 989 |
| as | A-32324 | 467 | UUCCUUGGGAUUGGUGACGdTdT | 990 |
| s | A-32325 | 455 | cAAucccAAGGAAuGAGGGdTdT | 991 |
| as | A-32326 | 473 | CCCUcAUUCCUUGGGAUUGdTdT | 992 |
| s | A-32327 | 491 | ccuGAAGGAcGAGGGAuGGdTdT | 993 |
| as | A-32328 | 509 | CcAUCCCUCGUCCUUcAGGdTdT | 994 |
| s | A-32331 | 497 | GGAcGAGGGAuGGGAuuucdTdT | 995 |
| as | A-32332 | 515 | GAAAUCCcAUCCCUCGUCCdTdT | 996 |
| s | A-32333 | 5 | AAGuccAcucAuucuuGGcdTdT | 997 |
| as | A-32334 | 23 | GCcAAGAAUGAGUGGACUUdTdT | 998 |
| s | A-32335 | 508 | GGGAuuucAuGuAAccAAGdTdT | 999 |
| as | A-32336 | 526 | CUUGGUuAcAUGAAAUCCCdTdT | 1000 |
| s | A-32337 | 509 | GGAuuucAuGuAAccAAGAdTdT | 1001 |
| as | A-32338 | 527 | UCUUGGUuAcAUGAAAUCCdTdT | 1002 |
| s | A-32339 | 514 | ucAuGuAAccAAGAGuAuudTdT | 1003 |
| as | A-32340 | 532 | AAuACUCUUGGUuAcAUGAdTdT | 1004 |
| s | A-32341 | 516 | AuGuAAccAAGAGuAuuccdTdT | 1005 |
| as | A-32342 | 534 | GGAAuACUCUUGGUuAcAUdTdT | 1006 |
| s | A-32343 | 517 | uGuAAccAAGAGuAuuccAdTdT | 1007 |

(continued)

| See Table 2 for duplex #. Strand: s= sense; as= antisense; Position: position of 5' base on transcript (NM_000371.2, SEQ ID NO:1329) | | | | |
|---|---|---|---|---|
| Strand | Oligo # | Position | Sequence (5' to 3') | SEQ ID NO: |
| as | A-32344 | 535 | UGGAAuACUCUUGGUuAcAdTdT | 1008 |
| s | A-32345 | 518 | GuAAccAAGAGuAuuccAudTdT | 1009 |
| as | A-32346 | 536 | AUGGAAuACUCUUGGUuACdTdT | 1010 |
| s | A-32347 | 54 | uGccuuGcuGGAcuGGuAudTdT | 1011 |
| as | A-32348 | 72 | AuACcAGUCcAGcAAGGcAdTdT | 1012 |
| s | A-32349 | 543 | uAAAGcAGuGuuuucAccudTdT | 1013 |
| as | A-32350 | 561 | AGGUGAAAAcACUGCUUuAdTdT | 1014 |
| s | A-32351 | 55 | GccuuGcuGGAcuGGuAuudTdT | 1015 |
| as | A-32352 | 73 | AAuACcAGUCcAGcAAGGCdTdT | 1016 |
| s | A-32353 | 551 | uGuuuucAccucAuAuGcudTdT | 1017 |
| as | A-32354 | 569 | AGcAuAUGAGGUGAAAAcAdTdT | 1018 |
| s | A-32355 | 552 | GuuuucAccucAuAuGcuAdTdT | 1019 |
| as | A-32356 | 570 | uAGcAuAUGAGGUGAAAACdTdT | 1020 |
| s | A-32357 | 553 | uuuucAccucAuAuGcuAudTdT | 1021 |
| as | A-32358 | 571 | AuAGcAuAUGAGGUGAAAAdTdT | 1022 |
| s | A-32359 | 555 | uucAccucAuAuGcuAuGudTdT | 1023 |
| as | A-32360 | 573 | AcAuAGcAuAUGAGGUGAAdTdT | 1024 |
| s | A-32363 | 557 | cAccucAuAuGcuAuGuuAdTdT | 1025 |
| as | A-32364 | 575 | uAAcAuAGcAuAUGAGGUGdTdT | 1026 |
| s | A-32367 | 56 | ccuuGcuGGAcuGGuAuuudTdT | 1027 |
| as | A-32368 | 74 | AAAuACcAGUCcAGcAAGGdTdT | 1028 |
| s | A-32369 | 563 | AuAuGcuAuGuuAGAAGucdTdT | 1029 |
| as | A-32370 | 581 | GACUUCuAAcAuAGcAuAUdTdT | 1030 |
| s | A-32371 | 564 | uAuGcuAuGuuAGAAGuccdTdT | 1031 |
| as | A-32372 | 582 | GGACUUCuAAcAuAGcAuAdTdT | 1032 |
| s | A-32373 | 566 | uGcuAuGuuAGAAGuccAGdTdT | 1033 |
| as | A-32374 | 584 | CUGGACUUCuAAcAuAGcAdTdT | 1034 |
| s | A-32375 | 57 | cuuGcuGGAcuGGuAuuuGdTdT | 1035 |
| as | A-32376 | 75 | cAAAuACcAGUCcAGcAAGdTdT | 1036 |
| s | A-32379 | 578 | AGuccAGGcAGAGAcAAuAdTdT | 1037 |
| as | A-32380 | 596 | uAUUGUCUCUGCCUGGACUdTdT | 1038 |
| s | A-32381 | 580 | uccAGGcAGAGAcAAuAAAdTdT | 1039 |
| as | A-32382 | 598 | UUuAUUGUCUCUGCCUGGAdTdT | 1040 |
| s | A-32383 | 607 | GuGAAAGGcAcuuuucAuudTdT | 1041 |
| as | A-32384 | 625 | AAUGAAAAGUGCCUUUcACdTdT | 1042 |
| s | A-32385 | 62 | uGGAcuGGuAuuuGuGucudTdT | 1043 |

(continued)

| See Table 2 for duplex #. Strand: s= sense; as= antisense; Position: position of 5' base on transcript (NM_000371.2, SEQ ID NO:1329) | | | | |
|---|---|---|---|---|
| **Strand** | **Oligo #** | **Position** | **Sequence (5' to 3')** | **SEQ ID NO:** |
| as | A-32386 | 80 | AGAcAcAAAuACcAGUCcAdTdT | 1044 |
| s | A-32387 | 77 | GucuGAGGcuGGcccuAcGdTdT | 1045 |
| as | A-32388 | 95 | CGuAGGGCcAGCCUcAGACdTdT | 1046 |
| s | A-32391 | 79 | cuGAGGcuGGcccuAcGGGdTdT | 1047 |
| as | A-32392 | 97 | CCCGuAGGGCcAGCCUcAGdTdT | 1048 |
| s | A-32393 | 81 | GAGGcuGGcccuAcGGGcAdTdT | 1049 |
| as | A-32394 | 99 | UGCCCGuAGGGCcAGCCUCdTdT | 1050 |
| s | A-32395 | 82 | AGGcuGGcccuAcGGGcAcdTdT | 1051 |
| as | A-32396 | 100 | GUGCCCGuAGGGCcAGCCUdTdT | 1052 |
| s | A-32397 | 84 | GcuGGcccuAcGGGcAccGdTdT | 1053 |
| as | A-32398 | 102 | CGGUGCCCGuAGGGCcAGCdTdT | 1054 |
| s | A-32399 | 85 | cuGGcccuAcGGGcAccGGdTdT | 1055 |
| as | A-32400 | 103 | CCGGUGCCCGuAGGGCcAGdTdT | 1056 |
| s | A-32401 | 87 | GGcccuAcGGGcAccGGuGdTdT | 1057 |
| as | A-32402 | 105 | cACCGGUGCCCGuAGGGCCdTdT | 1058 |
| s | A-32403 | 9 | ccAcucAuucuuGGcAGGAdTdT | 1059 |
| as | A-32404 | 27 | UCCUGCcAAGAAUGAGUGGdTdT | 1060 |
| s | A-32405 | 90 | ccuAcGGGcAccGGuGAAudTdT | 1061 |
| as | A-32406 | 108 | AUUcACCGGUGCCCGuAGGdTdT | 1062 |
| s | A-32407 | 91 | cuAcGGGcAccGGuGAAucdTdT | 1063 |
| as | A-32408 | 109 | GAUUcACCGGUGCCCGuAGdTdT | 1064 |
| s | A-32409 | 92 | uAcGGGcAccGGuGAAuccdTdT | 1065 |
| as | A-32410 | 110 | GGAUUcACCGGUGCCCGuAdTdT | 1066 |
| s | A-32411 | 93 | AcGGGcAccGGuGAAuccAdTdT | 1067 |
| as | A-32412 | 111 | UGGAUUcACCGGUGCCCGUdTdT | 1068 |
| s | A-32415 | 97 | GcAccGGuGAAuccAAGuGdTdT | 1069 |
| as | A-32416 | 115 | cACUUGGAUUcACCGGUGCdTdT | 1070 |
| s | A-32417 | 98 | cAccGGuGAAuccAAGuGudTdT | 1071 |
| as | A-32418 | 116 | AcACUUGGAUUcACCGGUGdTdT | 1072 |
| s | A-32419 | 167 | uGuGGccAuGcAuGuGuucdTdT | 1073 |
| as | A-32420 | 185 | GAAcAcAUGcAUGGCcAcAdTdT | 1074 |
| s | A-32421 | 168 | GuGGccAuGcAuGuGuucAdTdT | 1075 |
| as | A-32422 | 186 | UGAAcAcAUGcAUGGCcACdTdT | 1076 |
| s | A-32423 | 171 | GccAuGcAuGuGuucAGAAdTdT | 1077 |
| as | A-32424 | 189 | UUCUGAAcAcAUGcAUGGCdTdT | 1078 |
| s | A-32427 | 432 | uAuuccAccAcGGcuGucAdTdT | 1079 |

(continued)

| See Table 2 for duplex #. Strand: s= sense; as= antisense; Position: position of 5' base on transcript (NM_000371.2, SEQ ID NO:1329) | | | | |
|---|---|---|---|---|
| Strand | Oligo # | Position | Sequence (5' to 3') | SEQ ID NO: |
| as | A-32428 | 449 | UGAcAGCCGUGGUGGAAuAdTdT | 1080 |
| s | A-32429 | 447 | GucAucAccAAucccAAGGdTdT | 1081 |
| as | A-32430 | 465 | CCUUGGGAUUGGUGAUGACdTdT | 1082 |
| s | A-32159 | 115 | GuccucuGAuGGucAAAGudTdT | 1083 |
| as | A-32160 | 133 | ACUUUGACcAUcAGAGGACdTdT | 1084 |
| s | A-32161 | 122 | GAuGGucAAAGuucuAGAudTdT | 1085 |
| as | A-32162 | 140 | AUCuAGAACUUUGACcAUCdTdT | 1086 |
| s | A-32173 | 139 | AuGcuGuccGAGGcAGuccdTdT | 1087 |
| as | A-32174 | 157 | GGACUGCCUCGGAcAGcAUdTdT | 1088 |
| s | A-32185 | 172 | ccGuGcAuGuGuucAGAAAdTdT | 1089 |
| as | A-32186 | 190 | UUUCUGAAcAcAUGcACGGdTdT | 1090 |
| s | A-32197 | 238 | AGucuGGAGAGcuGcAuGGdTdT | 1091 |
| as | A-32198 | 256 | CcAUGcAGCUCUCcAGACUdTdT | 1092 |
| s | A-32209 | 252 | cAuGGGcucAcAAcuGAGGdTdT | 1093 |
| as | A-32210 | 270 | CCUcAGUUGUGAGCCcAUGdTdT | 1094 |
| s | A-32245 | 33 | ucucAucGucuGcuccuccdTdT | 1095 |
| as | A-32246 | 51 | GGAGGAGcAGACGAUGAGAdTdT | 1096 |
| s | A-32257 | 340 | ccccAuuccAuGAGcAuGcdTdT | 1097 |
| as | A-32258 | 358 | GcAUGCUcAUGGAAUGGGGdTdT | 1098 |
| s | A-32293 | 421 | GccccuAcuccuAuuccAcdTdT | 1099 |
| as | A-32294 | 439 | GUGGAAuAGGAGuAGGGGCdTdT | 1100 |
| s | A-32305 | 431 | cuAuuccAccAcGGcuGucdTdT | 1101 |
| as | A-32306 | 449 | GAcAGCCGUGGUGGAAuAGdTdT | 1102 |
| s | A-32317 | 440 | cAcGGcuGucGucAccAAudTdT | 1103 |
| as | A-32318 | 458 | AUUGGUGACGAcAGCCGUGdTdT | 1104 |
| s | A-32329 | 496 | AGGAcGAGGGAuGGGAuuudTdT | 1105 |
| as | A-32330 | 514 | AAAUCCcAUCCCUCGUCCUdTdT | 1106 |
| s | A-32361 | 556 | ucAccucAuAuGcuAuGuudTdT | 1107 |
| as | A-32362 | 574 | AAcAuAGcAuAUGAGGUGAdTdT | 1108 |
| s | A-32365 | 559 | ccucAuAuGcuAuGuuAGAdTdT | 1109 |
| as | A-32366 | 577 | UCuAAcAuAGcAuAUGAGGdTdT | 1110 |
| s | A-32377 | 570 | AuGuuAGAAGuccAGGcAGdTdT | 1111 |
| as | A-32378 | 588 | CUGCCUGGACUUCuAAcAUdTdT | 1112 |
| s | A-32389 | 78 | ucuGAGGcuGGcccuAcGGdTdT | 1113 |
| as | A-32390 | 96 | CCGuAGGGCcAGCCUcAGAdTdT | 1114 |
| s | A-32401 | 87 | GGcccuAcGGGcAccGGuGdTdT | 1115 |

(continued)

| See Table 2 for duplex #. Strand: s= sense; as= antisense; Position: position of 5' base on transcript (NM_000371.2, SEQ ID NO:1329) | | | | |
|---|---|---|---|---|
| **Strand** | **Oligo #** | **Position** | **Sequence (5' to 3')** | **SEQ ID NO:** |
| as | A-32402 | 105 | cACCGGUGCCCGuAGGGCCdTdT | 1116 |
| s | A-32413 | 95 | GGGcAccGGuGAAuccAAGdTdT | 1117 |
| as | A-32414 | 113 | CUUGGAUUcACCGGUGCCCdTdT | 1118 |
| s | A-32425 | 167 | ccAuGcAuGuucAGAAAdTdT | 1119 |
| as | A-32426 | 185 | UUUCUGAAcAcAUGcAUGGdTdT | 1120 |

Table 5: Identification numbers for rat TTR dsRNAs

| See Table 7 for sequences. | | |
|---|---|---|
| **Duplex #** | **Sense Oligo #** | **Antisense Oligo #** |
| AD-18529 | A-32745 | A-32746 |
| AD-18530 | A-32747 | A-32748 |
| AD-18531 | A-32749 | A-32750 |
| AD-18532 | A-32751 | A-32752 |
| AD-18533 | A-32753 | A-32754 |
| AD-18534 | A-32755 | A-32756 |
| AD-18535 | A-32757 | A-32758 |
| AD-18536 | A-32759 | A-32760 |
| AD-18537 | A-32761 | A-32762 |
| AD-18538 | A-32763 | A-32764 |
| AD-18539 | A-32159 | A-32160 |
| AD-18540 | A-32765 | A-32766 |
| AD-18541 | A-32767 | A-32768 |
| AD-18542 | A-32769 | A-32770 |
| AD-18543 | A-32771 | A-32772 |
| AD-18544 | A-32773 | A-32774 |
| AD-18545 | A-32775 | A-32776 |
| AD-18546 | A-32777 | A-32778 |
| AD-18547 | A-32779 | A-32780 |
| AD-18548 | A-32781 | A-32782 |
| AD-18549 | A-32783 | A-32784 |
| AD-18550 | A-32785 | A-32786 |
| AD-18551 | A-32787 | A-32788 |
| AD-18552 | A-32791 | A-32792 |
| AD-18553 | A-32793 | A-32794 |
| AD-18554 | A-32795 | A-32796 |

Table 6A. Sense and antisense strand sequences for rat TTR dsRNAs

| Strand: s= sense; as= antisense; Position: position of 5' base on transcript (NM_012681.1, SEQ ID NO:1330) | | | | | |
|---|---|---|---|---|---|
| **Strand** | **Position** | **Sequence (5' to 3')** | **SEQ ID NO:** | **Sequence with 3' dinucleotide overhang (5' to 3')** | **SEQ ID NO:** |
| s | 115 | GUCCUCUGAUGGUCAAAGU | 1121 | GUCCUCUGAUGGUCAAAGUNN | 1173 |
| as | 133 | ACUUUGACCAUCAGAGGAC | 1122 | ACUUUGACCAUCAGAGGACNN | 1174 |
| s | 537 | UUCUUGCUCUAUAAACCGU | 1123 | UUCUUGCUCUAUAAACCGUNN | 1175 |
| as | 555 | ACGGUUUAUAGAGCAAGAA | 1124 | ACGGUUUAUAGAGCAAGAANN | 1176 |
| s | 543 | CUCUAUAAACCGUGUUAGC | 1125 | CUCUAUAAACCGUGUUAGCNN | 1177 |
| as | 561 | GCUAACACGGUUUAUAGAG | 1126 | GCUAACACGGUUUAUAGAGNN | 1178 |
| s | 392 | UCGCCACUACACCAUCGCA | 1127 | UCGCCACUACACCAUCGCANN | 1179 |
| as | 410 | UGCGAUGGUGUAGUGGCGA | 1128 | UGCGAUGGUGUAGUGGCGANN | 1180 |
| s | 538 | UCUUGCUCUAUAAACCGUG | 1129 | UCUUGCUCUAUAAACCGUGNN | 1181 |
| as | 556 | CACGGUUUAUAGAGCAAGA | 1130 | CACGGUUUAUAGAGCAAGANN | 1182 |
| s | 541 | UGCUCUAUAAACCGUGUUA | 1131 | UGCUCUAUAAACCGUGUUANN | 1183 |
| as | 559 | UAACACGGUUUAUAGAGCA | 1132 | UAACACGGUUUAUAGAGCANN | 1184 |
| s | 532 | CAGUGUUCUUGCUCUAUAA | 1133 | CAGUGUUCUUGCUCUAUAANN | 1185 |
| as | 550 | UUAUAGAGCAAGAACACUG | 1134 | UUAUAGAGCAAGAACACUGNN | 1186 |
| s | 542 | GCUCUAUAAACCGUGUUAG | 1135 | GCUCUAUAAACCGUGUUAGNN | 1187 |
| as | 560 | CUAACACGGUUUAUAGAGC | 1136 | CUAACACGGUUUAUAGAGCNN | 1188 |
| s | 134 | CCUGGAUGCUGUCCGAGGC | 1137 | CCUGGAUGCUGUCCGAGGCNN | 1189 |
| as | 152 | GCCUCGGACAGCAUCCAGG | 1138 | GCCUCGGACAGCAUCCAGGNN | 1190 |
| s | 119 | UCUGAUGGUCAAAGUCCUG | 1139 | UCUGAUGGUCAAAGUCCUGNN | 1191 |
| as | 137 | CAGGACUUUGACCAUCAGA | 1140 | CAGGACUUUGACCAUCAGANN | 1192 |
| s | 241 | CUGGAGAGCUGCACGGGCU | 1141 | CUGGAGAGCUGCACGGGCUNN | 1193 |
| as | 259 | AGCCCGUGCAGCUCUCCAG | 1142 | AGCCCGUGCAGCUCUCCAGNN | 1194 |
| s | 544 | UCUAUAAACCGUGUUAGCA | 1143 | UCUAUAAACCGUGUUAGCANN | 1195 |
| as | 562 | UGCUAACACGGUUUAUAGA | 1144 | UGCUAACACGGUUUAUAGANN | 1196 |
| s | 530 | AACAGUGUUCUUGCUCUAU | 1145 | AACAGUGUUCUUGCUCUAUNN | 1197 |
| as | 548 | AUAGAGCAAGAACACUGUU | 1146 | AUAGAGCAAGAACACUGUUNN | 1198 |
| s | 118 | CUCUGAUGGUCAAAGUCCU | 1147 | CUCUGAUGGUCAAAGUCCUNN | 1199 |
| as | 136 | AGGACUUUGACCAUCAGAG | 1148 | AGGACUUUGACCAUCAGAGNN | 1200 |
| s | 140 | UGCUGUCCGAGGCAGCCCU | 1149 | UGCUGUCCGAGGCAGCCCUNN | 1201 |
| as | 158 | AGGGCUGCCUCGGACAGCA | 1150 | AGGGCUGCCUCGGACAGCANN | 1202 |
| s | 239 | GUCUGGAGAGCUGCACGGG | 1151 | GUCUGGAGAGCUGCACGGGNN | 1203 |
| as | 257 | CCCGUGCAGCUCUCCAGAC | 1152 | CCCGUGCAGCUCUCCAGACNN | 1204 |
| s | 531 | ACAGUGUUCUUGCUCUAUA | 1153 | ACAGUGUUCUUGCUCUAUANN | 1205 |
| as | 549 | UAUAGAGCAAGAACACUGU | 1154 | UAUAGAGCAAGAACACUGUNN | 1206 |
| s | 117 | CCUCUGAUGGUCAAAGUCC | 1155 | CCUCUGAUGGUCAAAGUCCNN | 1207 |

(continued)

| Strand: s= sense; as= antisense; Position: position of 5' base on transcript (NM_012681.1, SEQ ID NO:1330) | | | | | |
|---|---|---|---|---|---|
| **Strand** | **Position** | **Sequence (5' to 3')** | **SEQ ID NO:** | **Sequence with 3' dinucleotide overhang (5' to 3')** | **SEQ ID NO:** |
| as | 135 | GGACUUUGACCAUCAGAGG | 1156 | GGACUUUGACCAUCAGAGGNN | 1208 |
| s | 131 | AGUCCUGGAUGCUGUCCGA | 1157 | AGUCCUGGAUGCUGUCCGANN | 1209 |
| as | 149 | UCGGACAGCAUCCAGGACU | 1158 | UCGGACAGCAUCCAGGACUNN | 1210 |
| s | 217 | UUGCCUCUGGGAAGACCGC | 1159 | UUGCCUCUGGGAAGACCGCNN | 1211 |
| as | 235 | GCGGUCUUCCCAGAGGCAA | 1160 | GCGGUCUUCCCAGAGGCAANN | 1212 |
| s | 242 | UGGAGAGCUGCACGGGCUC | 1161 | UGGAGAGCUGCACGGGCUCNN | 1213 |
| as | 260 | GAGCCCGUGCAGCUCUCCA | 1162 | GAGCCCGUGCAGCUCUCCANN | 1214 |
| s | 244 | GAGAGCUGCACGGGCUCAC | 1163 | GAGAGCUGCACGGGCUCACNN | 1215 |
| as | 262 | GUGAGCCCGUGCAGCUCUC | 1164 | GUGAGCCCGUGCAGCUCUCNN | 1216 |
| s | 246 | GAGCUGCACGGGCUCACCA | 1165 | GAGCUGCACGGGCUCACCANN | 1217 |
| as | 264 | UGGUGAGCCCGUGCAGCUC | 1166 | UGGUGAGCCCGUGCAGCUCNN | 1218 |
| s | 399 | UACACCAUCGCAGCCCUGC | 1167 | UACACCAUCGCAGCCCUGCNN | 1219 |
| as | 417 | GCAGGGCUGCGAUGGUGUA | 1168 | GCAGGGCUGCGAUGGUGUANN | 1220 |
| s | 132 | GUCCUGGAUGCUGUCCGAG | 1169 | GUCCUGGAUGCUGUCCGAGNN | 1221 |
| as | 150 | CUCGGACAGCAUCCAGGAC | 1170 | CUCGGACAGCAUCCAGGACNN | 1222 |
| s | 245 | AGAGCUGCACGGGCUCACC | 1171 | AGAGCUGCACGGGCUCACCNN | 1223 |
| as | 263 | GGUGAGCCCGUGCAGCUCU | 1172 | GGUGAGCCCGUGCAGCUCUNN | 1224 |

Table 6B. Sense and antisense strand sequences for rat TTR dsRNAs

| Strand: s= sense; as= antisense; Position: position of 5' base on transcript (NM_012681.1, SEQ ID NO:1330) | | | |
|---|---|---|---|
| **Strand** | **Position** | **Sequence with 3' deoxythimidine overhang (5' to 3')** | **SEQ ID NO:** |
| s | 115 | GUCCUCUGAUGGUCAAAGUdTdT | 1225 |
| as | 133 | ACUUUGACCAUCAGAGGACdTdT | 1226 |
| s | 537 | UUCUUGCUCUAUAAACCGUdTdT | 1227 |
| as | 555 | ACGGUUUAUAGAGCAAGAAdTdT | 1228 |
| s | 543 | CUCUAUAAACCGUGUUAGCdTdT | 1229 |
| as | 561 | GCUAACACGGUUUAUAGAGdTdT | 1230 |
| s | 392 | UCGCCACUACACCAUCGCAdTdT | 1231 |
| as | 410 | UGCGAUGGUGUAGUGGCGAdTdT | 1232 |
| s | 538 | UCUUGCUCUAUAAACCGUGdTdT | 1233 |
| as | 556 | CACGGUUUAUAGAGCAAGAdTdT | 1234 |
| s | 541 | UGCUCUAUAAACCGUGUUAdTdT | 1235 |
| as | 559 | UAACACGGUUUAUAGAGCAdTdT | 1236 |
| s | 532 | CAGUGUUCUUGCUCUAUAAdTdT | 1237 |
| as | 550 | UUAUAGAGCAAGAACACUGdTdT | 1238 |

(continued)

| Strand: s= sense; as= antisense; Position: position of 5' base on transcript (NM_012681.1, SEQ ID NO:1330) | | | |
|---|---|---|---|
| Strand | Position | Sequence with 3' deoxythimidine overhang (5' to 3') | SEQ ID NO: |
| s | 542 | GCUCUAUAAACCGUGUUAGdTdT | 1239 |
| as | 560 | CUAACACGGUUUAUAGAGCdTdT | 1240 |
| s | 134 | CCUGGAUGCUGUCCGAGGCdTdT | 1241 |
| as | 152 | GCCUCGGACAGCAUCCAGGdTdT | 1242 |
| s | 119 | UCUGAUGGUCAAAGUCCUGdTdT | 1243 |
| as | 137 | CAGGACUUUGACCAUCAGAdTdT | 1244 |
| s | 241 | CUGGAGAGCUGCACGGGCUdTdT | 1245 |
| as | 259 | AGCCCGUGCAGCUCUCCAGdTdT | 1246 |
| s | 544 | UCUAUAAACCGUGUUAGCAdTdT | 1247 |
| as | 562 | UGCUAACACGGUUUAUAGAdTdT | 1248 |
| s | 530 | AACAGUGUUCUUGCUCUAUdTdT | 1249 |
| as | 548 | AUAGAGCAAGAACACUGUUdTdT | 1250 |
| s | 118 | CUCUGAUGGUCAAAGUCCUdTdT | 1251 |
| as | 136 | AGGACUUUGACCAUCAGAGdTdT | 1252 |
| s | 140 | UGCUGUCCGAGGCAGCCCUdTdT | 1253 |
| as | 158 | AGGGCUGCCUCGGACAGCAdTdT | 1254 |
| s | 239 | GUCUGGAGAGCUGCACGGGdTdT | 1255 |
| as | 257 | CCCGUGCAGCUCUCCAGACdTdT | 1256 |
| s | 531 | ACAGUGUUCUUGCUCUAUAdTdT | 1257 |
| as | 549 | UAUAGAGCAAGAACACUGUdTdT | 1258 |
| s | 117 | CCUCUGAUGGUCAAAGUCCdTdT | 1259 |
| as | 135 | GGACUUUGACCAUCAGAGGdTdT | 1260 |
| s | 131 | AGUCCUGGAUGCUGUCCGAdTdT | 1261 |
| as | 149 | UCGGACAGCAUCCAGGACUdTdT | 1262 |
| s | 217 | UUGCCUCUGGGAAGACCGCdTdT | 1263 |
| as | 235 | GCGGUCUUCCCAGAGGCAAdTdT | 1264 |
| s | 242 | UGGAGAGCUGCACGGGCUCdTdT | 1265 |
| as | 260 | GAGCCCGUGCAGCUCUCCAdTdT | 1266 |
| s | 244 | GAGAGCUGCACGGGCUCACdTdT | 1267 |
| as | 262 | GUGAGCCCGUGCAGCUCUCdTdT | 1268 |
| s | 246 | GAGCUGCACGGGCUCACCAdTdT | 1269 |
| as | 264 | UGGUGAGCCCGUGCAGCUCdTdT | 1270 |
| s | 399 | UACACCAUCGCAGCCCUGCdTdT | 1271 |
| as | 417 | GCAGGGCUGCGAUGGUGUAdTdT | 1272 |
| s | 132 | GUCCUGGAUGCUGUCCGAGdTdT | 1273 |
| as | 150 | CUCGGACAGCAUCCAGGACdTdT | 1274 |
| s | 245 | AGAGCUGCACGGGCUCACCdTdT | 1275 |

(continued)

| Strand: s= sense; as= antisense; Position: position of 5' base on transcript (NM_012681.1, SEQ ID NO:1330) | | | |
|---|---|---|---|
| **Strand** | **Position** | **Sequence with 3' deoxythimidine overhang (5' to 3')** | **SEQ ID NO:** |
| as | 263 | GGUGAGCCCGUGCAGCUCUdTdT | 1276 |

Table 7. Chemically modified sense and antisense strand sequences for rat TTR dsRNAs

| See Table 5 for duplex # (dsRNA name). Strand: s= sense; as= antisense; Position: position of 5' base on transcript (NM_012681.1, SEQ ID NO:1330) | | | | |
|---|---|---|---|---|
| **Strand** | **Oligo #** | **Position** | **Sequence (5' to 3')** | **SEQ ID NO:** |
| s | A-32159 | 115 | GuccucuGAuGGucAAAGudTdT | 1277 |
| as | A-32160 | 133 | ACUUUGACcAUcAGAGGACdTdT | 1278 |
| s | A-32745 | 537 | uucuuGcucuAuAAAccGudTdT | 1279 |
| as | A-32746 | 555 | ACGGUUuAuAGAGcAAGAAdTdT | 1280 |
| s | A-32747 | 543 | cucuAuAAAccGuGuuAGcdTdT | 1281 |
| as | A-32748 | 561 | GCuAAcACGGUUuAuAGAGdTdT | 1282 |
| s | A-32749 | 392 | ucGccAcuAcAccAucGcAdTdT | 1283 |
| as | A-32750 | 410 | UGCGAUGGUGuAGUGGCGAdTdT | 1284 |
| s | A-32751 | 538 | ucuuGcucuAuAAAccGuGdTdT | 1285 |
| as | A-32752 | 556 | cACGGUUuAuAGAGcAAGAdTdT | 1286 |
| s | A-32753 | 541 | uGcucuAuAAAccGuGuuAdTdT | 1287 |
| as | A-32754 | 559 | uAAcACGGUUuAuAGAGcAdTdT | 1288 |
| s | A-32755 | 532 | cAGuGuucuuGcucuAuAAdTdT | 1289 |
| as | A-32756 | 550 | UuAuAGAGcAAGAAcACUGdTdT | 1290 |
| s | A-32757 | 542 | GcucuAuAAAccGuGuuAGdTdT | 1291 |
| as | A-32758 | 560 | CuAAcACGGUUuAuAGAGCdTdT | 1292 |
| s | A-32759 | 134 | ccuGGAuGcuGuccGAGGcdTdT | 1293 |
| as | A-32760 | 152 | GCCUCGGAcAGcAUCcAGGdTdT | 1294 |
| s | A-32761 | 119 | ucuGAuGGucAAAGuccuGdTdT | 1295 |
| as | A-32762 | 137 | cAGGACUUUGACcAUcAGAdTdT | 1296 |
| s | A-32763 | 241 | cuGGAGAGcuGcAcGGGcudTdT | 1297 |
| as | A-32764 | 259 | AGCCCGUGcAGCUCUCcAGdTdT | 1298 |
| s | A-32765 | 544 | ucuAuAAAccGuGuuAGcAdTdT | 1299 |
| as | A-32766 | 562 | UGCuAAcACGGUUuAuAGAdTdT | 1300 |
| s | A-32767 | 530 | AAcAGuGuucuuGcucuAudTdT | 1301 |
| as | A-32768 | 548 | AuAGAGcAAGAAcACUGUUdTdT | 1302 |
| s | A-32769 | 118 | cucuGAuGGucAAAGuccudTdT | 1303 |
| as | A-32770 | 136 | AGGACUUUGACcAUcAGAGdTdT | 1304 |
| s | A-32771 | 140 | uGcuGuccGAGGcAGcccudTdT | 1305 |
| as | A-32772 | 158 | AGGGCUGCCUCGGAcAGcAdTdT | 1306 |

(continued)

| See Table 5 for duplex # (dsRNA name). Strand: s= sense; as= antisense; Position: position of 5' base on transcript (NM_012681.1, SEQ ID NO:1330) | | | | |
|---|---|---|---|---|
| **Strand** | **Oligo #** | **Position** | **Sequence (5' to 3')** | **SEQ ID NO:** |
| s | A-32773 | 239 | GucuGGAGAGcuGcAcGGGdTdT | 1307 |
| as | A-32774 | 257 | CCCGUGcAGCUCUCcAGACdTdT | 1308 |
| s | A-32775 | 531 | AcAGuGuucuuGcucuAuAdTdT | 1309 |
| as | A-32776 | 549 | uAuAGAGcAAGAAcACUGUdTdT | 1310 |
| s | A-32777 | 117 | ccucuGAuGGucAAAGuccdTdT | 1311 |
| as | A-32778 | 135 | GGACUUUGACcAUcAGAGGdTdT | 1312 |
| s | A-32779 | 131 | AGuccuGGAuGcuGuccGAdTdT | 1313 |
| as | A-32780 | 149 | UCGGAcAGcAUCcAGGACUdTdT | 1314 |
| s | A-32781 | 217 | uuGccucuGGGAAGAccGcdTdT | 1315 |
| as | A-32782 | 235 | GCGGUCUUCCcAGAGGcAAdTdT | 1316 |
| s | A-32783 | 242 | uGGAGAGcuGcAcGGGcucdTdT | 1317 |
| as | A-32784 | 260 | GAGCCCGUGcAGCUCUCcAdTdT | 1318 |
| s | A-32785 | 244 | GAGAGcuGcAcGGGcucAcdTdT | 1319 |
| as | A-32786 | 262 | GUGAGCCCGUGcAGCUCUCdTdT | 1320 |
| s | A-32787 | 246 | GAGcuGcAcGGGcucAccAdTdT | 1321 |
| as | A-32788 | 264 | UGGUGAGCCCGUGcAGCUCdTdT | 1322 |
| s | A-32791 | 399 | uAcAccAucGcAGcccuGcdTdT | 1323 |
| as | A-32792 | 417 | GcAGGGCUGCGAUGGUGuAdTdT | 1324 |
| s | A-32793 | 132 | GuccuGGAuGcuGuccGAGdTdT | 1325 |
| as | A-32794 | 150 | CUCGGAcAGcAUCcAGGACdTdT | 1326 |
| s | A-32795 | 245 | AGAGcuGcAcGGGcucAccdTdT | 1327 |
| as | A-32796 | 263 | GGUGAGCCCGUGcAGCUCUdTdT | 1328 |

Synthesis of TTR Sequences

TTR sequences were synthesized on MerMade 192 synthesizer at 1μmol scale. For all the sequences in the list, 'endolight' chemistry was applied as detailed below.

- All pyrimidines (cytosine and uridine) in the sense strand were replaced with corresponding 2'-O-Methyl bases (2' O-Methyl C and 2'-O-Methyl U)
- In the antisense strand, pyrimidines adjacent to (towards 5' position) ribo A nucleoside were replaced with their corresponding 2-O-Methyl nucleosides
- A two base dTdT extension at 3' end of both sense and antisense sequences was introduced
- The sequence file was converted to a text file to make it compatible for loading in the MerMade 192 synthesis software

The synthesis of TTR sequences used solid supported oligonucleotide synthesis using phosphoramidite chemistry. The synthesis of the above sequences was performed at lum scale in 96 well plates. The amidite solutions were prepared at 0.1 M concentration and ethyl thio tetrazole (0.6M in Acetonitrile) was used as activator.

The synthesized sequences were cleaved and deprotected in 96 well plates, using methylamine in the first step and triethylamine.3HF in the second step. The crude sequences thus obtained were precipitated using acetone: ethanol mix and the pellet were re-suspended in 0.5M sodium acetate buffer. Samples from each sequence were

analyzed by LC-MS and the resulting mass data confirmed the identity of the sequences. A selected set of samples were also analyzed by IEX chromatography.

The next step in the process was purification. All sequences were purified on an AKTA explorer purification system using Source 15Q column. A single peak corresponding to the full length sequence was collected in the eluent and was subsequently analyzed for purity by ion exchange chromatography.

The purified sequences were desalted on a Sephadex G25 column using AKTA purifier. The desalted TTR sequences were analyzed for concentration and purity. The single strands were then annealed to form TTR-dsRNA.

**Example 2B: *In vitro* screening of TTR siRNAs for mRNA suppression**

Human TTR targeting dsRNAs (Table 2) were assayed for inhibition of endogenous TTR expression in HepG2 and Hep3B cells, using qPCR (real time PCR) and bDNA (branched DNA) assays to quantify TTR mRNA. Rodent TTR targeting dsRNA (Table 5) were synthesized and assayed for inhibition of endogenous TTR expression using bDNA assays in H.4.II.E cells. Results from single dose assays were used to select a subset of TTR dsRNA duplexes for dose response experiments to calculate IC50's. IC50 results were used to select TTR dsRNAs for further testing.

Cell culture and transfections:

The hepatocyte cell lines HepG2, Hep3B and H.4.II.E cells (ATCC, Manassas, VA) were grown to near confluence at 37°C in an atmosphere of 5% $CO_2$ in Dulbecco's modified Eagle's medium (ATCC) supplemented with 10% FBS, streptomycin, and glutamine (ATCC) before being released from the plate by trypsinization. H.4.II.E cells were also grown in Earle's minimal essential medium. Reverse transfection was carried out by adding 5 μl of Opti-MEM to 5 μl of siRNA duplexes per well into a 96-well plate along with 10 μl of Opti-MEM plus 0.2 μl of Lipofectamine RNAiMax per well (Invitrogen, Carlsbad CA. cat # 13778-150) and incubated at room temperature for 15 minutes. 80μl of complete growth media without antibiotics containing $4x10^4$ (HepG2), $2x10^4$ (Hep3B) or $2x10^4$ (H.4.II.E) cells were then added. Cells were incubated for 24 hours prior to RNA purification. Single dose experiments were performed at 10 nM final duplex concentration and dose response experiments were done with 10, 1, 0.5, 0.1,0.05, 0.01, 0.005, 0.001, 0.0005, 0.0001, 0.00005, 0.00001 nM.

Total RNA isolation using MagMAX-96 Total RNA Isolation Kit (Applied Biosystems, Foster City CA, part #: AM1830):

Cells were harvested and lysed in 140 μl of Lysis/Binding Solution then mixed for 1 minute at 850rpm using and Eppendorf Thermomixer (the mixing speed was the same throughout the process). Twenty micro liters of magnetic beads were added into cell-lysate and mixed for 5 minutes. Magnetic beads were captured using magnetic stand and the supernatant was removed without disturbing the beads. After removing supernatant, magnetic beads were washed with Wash Solution 1 (isopropanol added) and mixed for 1 minute. Beads were captured again and supernatant removed. Beads were then washed with 150μl Wash Solution 2 (Ethanol added), captured and supernatant was removed. 50μl of DNase mixture (MagMax turbo DNase Buffer and Turbo DNase) was then added to the beads and they were mixed for 10 to 15 minutes. After mixing, 100μl of RNA Rebinding Solution was added and mixed for 3 minutes. Supernatant was removed and magnetic beads were washed again with 150μl Wash Solution 2 and mixed for 1 minute and supernatant was removed completely. The magnetic beads were mixed for 2 minutes to dry before RNA it was eluted with 50μl of water.

cDNA synthesis using ABI High capacity cDNA reverse transcription kit (Applied Biosystems, Foster City, CA, Cat #4368813):

A master mix of 2μl 10X Buffer, 0.8μl 25X dNTPs, 2μl Random primers, 1μl Reverse Transcriptase, 1μl RNase inhibitor and 3.2μl of H2O per reaction were added into 10μl total RNA. cDNA was generated using a Bio-Rad C-1000 or S-1000 thermal cycler (Hercules, CA) through the following steps: 25°C 10 min, 37°C 120 min, 85°C 5 sec, 4°C hold.

Real time PCR:

2μl of cDNA was added to a master mix of 1μl 18S TaqMan Probe (Applied Biosystems Cat # 4319413E), 1μl TTR TaqMan probe (Applied Biosystems cat # HS00174914 M1) and 10μl TaqMan Universal PCR Master Mix (Applied Biosystems Cat #4324018) per well in a MicroAmp Optical 96 well plate (Applied Biosystems cat # 4326659). Real time PCR was done in an ABI 7000 Prism or an ABI 7900HT Real Time PCR system (Applied Biosystems) using the ΔΔ Ct(RQ) assay. All reactions were done in triplicate.

Real time data were analyzed using the ΔΔ Ct method and normalized to assays performed from cells transfected with 10nM BlockIT fluorescent Oligo (Invitrogen Cat # 2013) or 10nM AD-1955 (a control duplex that targets the non-mammalian luciferase gene) to calculate fold change.

Branched DNA assays- QuantiGene 1.0 (Panomics, Fremont, CA, cat #: QG0004)- Used to screen rodent specific duplexes

H.4.II.E cells (ATCC) were transfected with 10 nM siRNA. After removing media, H.4.II.E were lysed in 100ul of Diluted Lysis Mixture (a mixture of 1 volume of Lysis mixture, 2 volume of nuclease-free water and 10ul of Proteinase-K per ml for the final concentration of 20mg/ml) then incubated at 65 °C for 35 minutes. Then, 80μl of Working Probe Set (a mixture of TTR or GAPDH probe) and 20ul of cell-lysate were added into the Capture Plate. Capture Plates were incubated at 53 °C ±1 °C overnight (approximately 16-20hrs). Capture Plates were washed 3 times with 1X Wash Buffer (a mixture of nuclease-free water, Buffer Component 1 and Wash Buffer Component 2), then dried by centrifuging for 1 minute at 1000rpm. 100μl of Amplifier Working Reagent was added into the Capture Plate, which was then sealed and incubated for 1 hour at 46°C ± °C. Wash and dry steps were repeated after 1 hour of incubation and 100μl of Label Solution Reagent was added. The plate was then washed, dried and 100μl Substrate (a mixture of Lithium Lauryl Sulfate and Substrate solution) was added. Capture Plates were placed in the incubator for 30 minutes at 46 46°C ±1°C. Capture Plates were then removed from the incubator and incubated at room temperature for 30 minutes. Finally, the Capture Plates were read using the Victor Luminometer (Perkin Elmer, Waltham, MA).

Branched DNA assays- QuantiGene 2.0 (Panomics cat #: QS0011): Used to screen all other duplexes

After a 24 hour incubation at the dose or doses stated, media was removed and cells were lysed in 100ul Lysis Mixture (1 volume lysis mixture, 2 volumes nuclease-free water and 10μl of Proteinase-K/ml for a final concentration of 20mg/ml) then incubated at 65°C for 35 minutes. 20μl Working Probe Set (TTR probe for gene target and GAPDH for endogenous control) and 80μl of cell-lysate were then added to the Capture Plates. Capture Plates were incubated at 55°C ±1°C (approx. 16-20hrs). The next day, the Capture Plates were washed 3 times with 1X Wash Buffer (nuclease-free water, Buffer Component 1 and Wash Buffer Component 2), then dried by centrifuging for 1 minute at 240g. 100μl of pre-Amplifier Working Reagent was added to the Capture Plates, which were sealed with aluminum foil and incubated for 1 hour at 55°C ±1°C. Following a 1 hour incubation, the wash step was repeated, then 100μl Amplifier Working Reagent was added. After 1 hour, the wash and dry steps were repeated, and 100μl Label Probe was added. Capture plates were incubated 50°C ±1°C for 1 hour. The plates were then washed with 1X Wash Buffer and dried, and then 100μl Substrate was added to the Capture Plates. Capture Plates were read using the SpectraMax Luminometer (Molecular Devices, Sunnyvale, CA) following 5 to 15 minutes incubation.

bDNA data analysis:

bDNA data were analyzed by (i) subtracting the average background from each triplicate sample, (ii) averaging the resultant triplicate GAPDH (control probe) and TTR (experimental probe) values, and then (iii) taking the ratio: (experimental probe-background)/(control probe-background).

Results

A summary of the single dose and IC50 results for TTR-dsRNAs (TTR siRNAs) are presented below in Table 8. Single dose results are expressed as % TTR mRNA relative to control, assayed in HepG2 cells. IC50s were determined in HepG2 and/or Hep3B cells, as indicated.

Table 8. Single dose and IC50 results of *in vitro* screens of TTR siRNAs

| ND: no data; * indicates result that represents average of two experiments. | | | | | | |
|---|---|---|---|---|---|---|
| | **Single Dose at 10nM % relative to control** | | **IC50 (nM)** | | | |
| | **HepG2** | | **HepG2** | | **Hep3B** | |
| **Duplex #** | **qPCR** | **bDNA** | **gPCR** | **bDNA** | **qPCR** | **bDNA** |
| AD-18243 | 50.35 | 141.53 | ND | ND | ND | ND |
| AD-18244 | 64.26 | 158.55 | ND | ND | ND | ND |
| AD-18245 | 56.89 | 107.22 | ND | ND | ND | ND |
| AD-18246 | 10.53 | 32.51* | 0.265 | 0.086 | ND | ND |

(continued)

| ND: no data; * indicates result that represents average of two experiments. | | | | | | |
|---|---|---|---|---|---|---|
| | Single Dose at 10nM % relative to control | | IC50 (nM) | | | |
| | HepG2 | | HepG2 | | Hep3B | |
| **Duplex #** | **qPCR** | **bDNA** | **gPCR** | **bDNA** | **qPCR** | **bDNA** |
| AD-18247 | 125.56 | 69.57 | ND | ND | ND | ND |
| AD-18248 | 127.78 | 66.97 | ND | ND | ND | ND |
| AD-18249 | 48.77 | 48.76 | ND | ND | ND | ND |
| AD-18250 | 96.94 | 86.42 | ND | ND | ND | ND |
| AD-18251 | 170.41 | 129.15 | ND | ND | ND | ND |
| AD-18252 | 73.52 | 81.90 | ND | ND | ND | ND |
| AD-18253 | 25.25 | 61.25 | ND | ND | ND | ND |
| AD-18254 | 95.13 | 103.96 | ND | ND | ND | ND |
| AD-18255 | 119.46 | ND | ND | ND | ND | ND |
| AD-18256 | 42.64 | 95.67 | ND | ND | ND | ND |
| AD-18257 | 146.25 | 141.75 | ND | ND | ND | ND |
| AD-18258 | 10.20 | 13.41* | 0.007 | 0.005 | 0.004 | 0.005 |
| AD-18259 | 9.30 | 20.91* | 0.102 | 0.005 | ND | ND |
| AD-18260 | 125.37 | 81.36 | ND | ND | ND | ND |
| AD-18261 | 14.27 | 19.40* | 0.210 | ND | ND | ND |
| AD-18262 | 84.95 | 104.05 | ND | ND | ND | ND |
| AD-18263 | 16.32 | 23.25* | 0.110 | ND | ND | ND |
| AD-18264 | 104.18 | 83.69 | ND | ND | ND | ND |
| AD-18265 | 41.62 | 64.87 | ND | ND | ND | ND |
| AD-18266 | 39.98 | 110.53 | ND | ND | ND | ND |
| AD-18267 | 149.64 | ND | ND | ND | ND | ND |
| AD-18268 | 152.93 | 174.04 | ND | ND | ND | ND |
| AD-18269 | 37.27 | 92.28 | ND | ND | ND | ND |
| AD-18270 | 99.44 | 164.75 | ND | ND | ND | ND |
| AD-18271 | 18.89 | 28.33* | 0.503 | 0.004 | ND | ND |
| AD-18272 | 128.32 | 132.58 | ND | ND | ND | ND |
| AD-18273 | 115.78 | 201.95 | ND | ND | ND | ND |
| AD-18274 | 8.97 | 20.04* | 0.009 | 0.176 | 0.036 | 0.012 |
| AD-18275 | 4.09 | 22.25* | 0.026 | 0.118 | ND | ND |
| AD-18276 | 19.73 | 45.22* | 0.198 | 0.677 | ND | ND |
| AD-18277 | 10.55 | 26.31* | 0.121 | 0.426 | ND | ND |
| AD-18278 | 108.86 | 116.26 | ND | ND | ND | ND |
| AD-18279 | 66.59 | ND | ND | ND | ND | ND |
| AD-18280 | 103.26 | 170.52 | ND | ND | ND | ND |
| AD-18281 | 87.98 | 123.88 | ND | ND | ND | ND |

(continued)

| ND: no data; * indicates result that represents average of two experiments. | | | | | | |
|---|---|---|---|---|---|---|
| | Single Dose at 10nM % relative to control | | IC50 (nM) | | | |
| | HepG2 | | HepG2 | | Hep3B | |
| Duplex # | qPCR | bDNA | gPCR | bDNA | qPCR | bDNA |
| AD-18282 | 82.47 | 140.32 | ND | ND | ND | ND |
| AD-18283 | 106.54 | 182.78 | ND | ND | ND | ND |
| AD-18284 | 106.93 | 151.78 | ND | ND | ND | ND |
| AD-18285 | 26.58 | 60.05* | ND | 0.089 | ND | ND |
| AD-18286 | 109.95 | 173.66 | ND | ND | ND | ND |
| AD-18287 | 54.23 | 155.45 | ND | ND | ND | ND |
| AD-18288 | 73.52 | 174.09 | ND | ND | ND | ND |
| AD-18289 | 103.36 | 174.76 | ND | ND | ND | ND |
| AD-18290 | 17.06 | 52.04* | 1.253 | 0.181 | ND | ND |
| AD-18291 | 7.71 | 169.29* | 1.304 | 0.019 | ND | ND |
| AD-18292 | 7.51 | 210.03* | 0.604 | 0.005 | ND | ND |
| AD-18293 | 3.61 | 62.53* | 0.078 | 0.003 | ND | ND |
| AD-18294 | 111.53 | 107.56 | ND | ND | ND | ND |
| AD-18295 | 115.88 | 105.37 | ND | ND | ND | ND |
| AD-18296 | 57.03 | 38.03 | ND | ND | ND | ND |
| AD-18297 | 87.69 | 73.87 | ND | ND | ND | ND |
| AD-18298 | 10.39 | 7.25* | 0.455 | 0.008 | ND | ND |
| AD-18299 | 18.79 | 18.06* | 0.895 | 0.014 | ND | ND |
| AD-18300 | 108.70 | ND | ND | ND | ND | ND |
| AD-18301 | 114.22 | 70.50 | ND | ND | ND | ND |
| AD-18302 | 116.19 | 122.40 | ND | ND | ND | ND |
| AD-18303 | 124.89 | ND | ND | ND | ND | ND |
| AD-18304 | 132.99 | 89.54 | ND | ND | ND | ND |
| AD-18305 | 153.10 | ND | ND | ND | ND | ND |
| AD-18306 | 159.22 | ND | ND | ND | ND | ND |
| AD-18307 | 116.83 | 84.57 | ND | ND | ND | ND |
| AD-18308 | 156.72 | 87.80 | ND | ND | ND | ND |
| AD-18309 | 113.22 | 101.97 | ND | ND | ND | ND |
| AD-18310 | 132.33 | ND | ND | ND | ND | ND |
| AD-18311 | 161.68 | 92.92 | ND | ND | ND | ND |
| AD-18312 | 103.01 | 71.17 | ND | ND | ND | ND |
| AD-18313 | 120.65 | 53.26 | ND | ND | ND | ND |
| AD-18314 | 116.33 | ND | ND | ND | ND | ND |
| AD-18315 | 115.13 | ND | ND | ND | ND | ND |
| AD-18316 | 118.73 | 122.34 | ND | ND | ND | ND |

(continued)

| ND: no data; * indicates result that represents average of two experiments. | | | | | | |
|---|---|---|---|---|---|---|
| | Single Dose at 10nM % relative to control | | IC50 (nM) | | | |
| | HepG2 | | HepG2 | | Hep3B | |
| Duplex # | qPCR | bDNA | gPCR | bDNA | qPCR | bDNA |
| AD-18317 | 114.03 | 121.10 | ND | ND | ND | ND |
| AD-18318 | 80.85 | 122.57 | ND | ND | ND | ND |
| AD-18319 | 119.14 | 148.87 | ND | ND | ND | ND |
| AD-18320 | 22.86 | 55.43* | ND | 0.023 | 0.403 | ND |
| AD-18321 | 6.44 | 31.56* | 0.001 | 0.033 | ND | ND |
| AD-18322 | 54.21 | 100.46 | ND | ND | ND | ND |
| AD-18323 | 6.37 | 28.71* | 0.005 | 0.023 | ND | ND |
| AD-18324 | 2.53 | 15.98* | 0.002 | 0.006 | 0.005 | 0.014 |
| AD-18325 | 2.52 | 11.96* | 0.001 | 0.016 | ND | ND |
| AD-18326 | 18.34 | 43.16* | 0.025 | 0.186 | ND | ND |
| AD-18327 | 18.28 | 13.90* | 0.044 | 0.215 | ND | ND |
| AD-18328 | 4.53 | 26.04* | 0.003 | 0.004 | 0.006 | 0.006 |
| AD-18329 | 96.93 | 131.54 | ND | ND | ND | ND |
| AD-18330 | 11.80 | 45.18* | 0.0004 | 0.010 | 0.020 | ND |
| AD-18331 | 117.77 | 163.07 | ND | ND | ND | ND |
| AD-18332 | 11.53 | 35.09* | 0.001 | 0.076 | 0.065 | ND |
| AD-18333 | 12.24 | 46.94* | 0.001 | 0.115 | 0.075 | ND |
| AD-18334 | 16.27 | 55.28* | 0.0004 | 0.181 | 1.071 | ND |
| AD-18335 | 53.52 | 112.80 | ND | ND | ND | ND |
| AD-18336 | 6.39 | 33.00* | 0.001 | 0.112 | 0.081 | ND |
| AD-18337 | 51.77 | 105.33 | ND | ND | ND | ND |
| AD-18338 | 48.21 | 102.86 | ND | ND | ND | ND |
| AD-18339 | 6.48 | 26.56* | 0.004 | 0.002 | 0.018 | 0.029 |
| AD-18340 | 4.53 | 30.76* | 0.002 | 0.002 | ND | ND |
| AD-18341 | 31.27 | 100.41 | ND | ND | ND | ND |
| AD-18342 | 7.60 | 42.89* | ND | 0.016 | 0.076 | ND |
| AD-18343 | 3.42 | 17.45* | ND | 0.001 | ND | ND |
| AD-18344 | 75.08 | 134.31 | ND | ND | ND | ND |
| AD-18345 | 13.62 | 42.75* | 0.002 | 0.013 | ND | ND |
| AD-18346 | 59.25 | 121.10 | ND | ND | ND | ND |
| AD-18347 | 91.23 | 139.54 | ND | ND | ND | ND |
| AD-18348 | 89.95 | 159.29 | ND | ND | ND | ND |
| AD-18349 | 108.01 | 144.96 | ND | ND | ND | ND |
| AD-18350 | 123.65 | 125.87 | ND | ND | ND | ND |
| AD-18351 | 108.36 | 104.02 | ND | ND | ND | ND |

(continued)

| ND: no data; * indicates result that represents average of two experiments. | | | | | | |
|---|---|---|---|---|---|---|
| | **Single Dose at 10nM % relative to control** | | **IC50 (nM)** | | | |
| | **HepG2** | | **HepG2** | | **Hep3B** | |
| **Duplex #** | **qPCR** | **bDNA** | **gPCR** | **bDNA** | **qPCR** | **bDNA** |
| AD-18352 | 87.82 | 128.72 | ND | ND | ND | ND |
| AD-18353 | 14.40 | 65.77 | 0.012 | 0.027 | ND | ND |
| AD-18354 | 99.27 | 123.53 | ND | ND | ND | ND |
| AD-18355 | 135.04 | 150.88 | ND | ND | ND | ND |
| AD-18356 | 100.76 | 178.96 | ND | ND | ND | ND |
| AD-18357 | 125.30 | 162.85 | ND | ND | ND | ND |
| AD-18358 | 103.15 | 136.01 | ND | ND | ND | ND |
| AD-18359 | 34.74 | 140.48 | ND | ND | ND | ND |
| AD-18360 | 103.86 | 146.86 | ND | ND | ND | ND |
| AD-18361 | 105.74 | 152.74 | ND | ND | ND | ND |
| AD-18362 | 106.96 | 188.22 | ND | ND | ND | ND |
| AD-18363 | 124.22 | 58.46 | ND | ND | ND | ND |
| AD-18364 | 113.75 | 66.87 | ND | ND | ND | ND |
| AD-18446 | 29.73 | 13.30 | ND | ND | ND | ND |
| AD-18447 | 109.74 | 53.63 | ND | ND | ND | ND |
| AD-18448 | 22.96 | 8.81 | ND | ND | ND | ND |
| AD-18449 | 112.59 | 50.11 | ND | ND | ND | ND |
| AD-18450 | 89.41 | 34.89 | ND | ND | ND | ND |
| AD-18451 | 74.35 | 23.88 | ND | ND | ND | ND |
| AD-18452 | 125.25 | 54.86 | ND | ND | ND | ND |
| AD-18453 | 126.98 | 56.31 | ND | ND | ND | ND |
| AD-18454 | 113.88 | 52.48 | ND | ND | ND | ND |
| AD-18455 | 163.00 | 48.89 | ND | ND | ND | ND |
| AD-18456 | 15.70 | 10.52 | ND | ND | ND | ND |
| AD-18457 | 12.86 | 8.22 | ND | ND | ND | ND |
| AD-18458 | 13.00 | 7.00 | ND | ND | ND | ND |
| AD-18459 | 14.41 | 10.72 | ND | ND | ND | ND |
| AD-18460 | 121.16 | 74.87 | ND | ND | ND | ND |
| AD-18461 | 100.53 | 71.87 | ND | ND | ND | ND |
| AD-18462 | 47.75 | 29.35 | ND | ND | ND | ND |
| AD-18463 | 58.98 | 44.79 | ND | ND | ND | ND |

The dose response data used to identify the IC50 for 5 TTR-dsRNAs (AD-18258, AD-18274, AD-18324, AD-18328, and AD-18339), are presented in detail below in Table 9. All 5 siRNAs were determined to have pM IC50s. The IC50 data for dsRNAs in Table 8 is a summary of the data presented in Table 9 below.

Table 9. Dose response data for 5 TTR-dsRNAs

| | | % inhibition relative to control AD-1955 | | | | | | | | | | | | |
|---|---|---|---|---|---|---|---|---|---|---|---|---|---|---|
| **Duplex AD-18258** | | **Dose of duplex (nM)** | | | | | | | | | | | | |
| Cell type | Detection method | **10** | **1** | **0.5** | **0.1** | **0.05** | **0.01** | **0.005** | **0.001** | **0.0005** | **0.0001** | **0.00005** | **0.00001** | IC50 (nM) |
| HepG2 | qPCR | 14.4 | 14.1 | 16.2 | 23.9 | 27.26 | 40.19 | 68.46 | 78.1 | 74.48 | 104.37 | 98.28 | 113.68 | 0.007 |
| HepG2 | bDNA | 14.3 | 14.5 | 11.1 | 12.8 | 18.82 | 19.77 | 51.21 | 56.03 | 63.63 | 58.35 | 43.64 | 51.05 | 0.005 |
| Hep3B | qPCR | 11.9 | 8.62 | 12.4 | 16.4 | 28.35 | 30.49 | 58.36 | 54.57 | 81.26 | 89.43 | 81.85 | 101.87 | 0.004 |
| Hep3B | bDNA | 7.65 | 7.5 | 11.3 | 12.6 | 28.85 | 27.89 | 64.57 | 73.48 | 72.03 | 91.44 | 86.71 | 89.31 | 0.005 |
| **Duplex AD-18274** | | **Dose of duplex (nM)** | | | | | | | | | | | | |
| Cell type | Detection method | **10** | **1** | **0.5** | **0.1** | **0.05** | **0.01** | **0.005** | **0.001** | **0.0005** | **0.0001** | **0.00005** | **0.00001** | IC50 (nM) |
| HepG2 | qPCR | 6.68 | 8.45 | 11.7 | 24.2 | 42.08 | 49.89 | 56.95 | 62.99 | 64.47 | 54.92 | 67.39 | 72.67 | 0.009 |
| HepG2 | bDNA | 27.5 | 69 | 25.2 | 34.2 | 73.03 | 103.4 | 121.57 | 97.31 | 154.93 | 156.7 | Nd | 152.25 | 0.176 |
| Hep3B | qPCR | 7.58 | 17 | 15.6 | 43.9 | 42.22 | 60.55 | 78.8 | 77.81 | 79.97 | 85.84 | 86.13 | 83.99 | 0.036 |
| Hep3B | bDNA | 3.77 | 4.92 | 7.51 | 15 | 35.21 | 51.66 | 72.45 | 70.12 | 78.31 | 77.52 | 90.72 | 83.01 | 0.012 |
| **Duplex AD-18324** | | **Dose of duplex (nM)** | | | | | | | | | | | | |
| Cell type | Detection method | **10** | **1** | **0.5** | **0.1** | **0.05** | **0.01** | **0.005** | **0.001** | **0.0005** | **0.0001** | **0.00005** | **0.00001** | IC50 (nM) |
| HepG2 | qPCR | 2.07 | 2.27 | 2.74 | 6.36 | 8.18 | 15.23 | 28.82 | 52.79 | 90.86 | 94.72 | 116.07 | 98.97 | 0.002 |
| HepG2 | bDNA | 14.5 | 7.88 | 11.8 | 15.9 | 17.2 | 46.44 | 40.4 | 91.86 | 0 | 95.57 | 0 | 52.15 | 0.006 |
| Hep3B | qPCR | 2.07 | 3.48 | 5.76 | 16.2 | 18.73 | 44.54 | 49.77 | 68.88 | 63.48 | 76.61 | 74.7 | 77.83 | 0.005 |
| Hep3B | bDNA | 3.48 | 3.8 | 5.15 | 15.2 | 30.84 | 55.36 | 74.75 | 99.39 | 88.89 | 110.83 | 96.55 | 110.26 | 0.014 |
| **Duplex AD-18328** | | **Dose of duplex (nM)** | | | | | | | | | | | | |
| Cell type | Detection method | **10** | **1** | **0.5** | **0.1** | **0.05** | **0.01** | **0.005** | **0.001** | **0.0005** | **0.0001** | **0.00005** | **0.00001** | IC50 (nM) |
| HepG2 | qPCR | 5.85 | 3.97 | 3.32 | 5.62 | 8 | 16.75 | 55.01 | 39.76 | 122.41 | 102.37 | 114.02 | 124.09 | 0.003 |
| HepG2 | bDNA | 12.3 | 10.7 | 10.7 | 11.9 | 20.06 | 25 | 69.52 | 57.29 | 112.28 | 98.14 | 142.26 | 148.92 | 0.004 |
| Hep3B | qPCR | 3.17 | 5.52 | 11.7 | 13.8 | 27.68 | 39.58 | 61.21 | 61.87 | 90.51 | 87.56 | 106.03 | 108.72 | 0.006 |
| Hep3B | bDNA | 3.08 | 3.66 | 4.19 | 7.25 | 21.05 | 22.1 | 73.74 | 63.19 | 105.55 | 96.27 | 105.97 | 96.46 | 0.006 |

(continued)

| Duplex AD-18339 | | Dose of duplex (nM) | | | | | | | | | | | | |
|---|---|---|---|---|---|---|---|---|---|---|---|---|---|---|
| Cell type | Detection method | **10** | **1** | **0.5** | **0.1** | **0.05** | **0.01** | **0.005** | **0.001** | **0.0005** | **0.0001** | **0.00005** | **0.00001** | IC50 (nM) |
| HepG2 | qPCR | 6.27 | 7.28 | Nd | 11 | 15.25 | 38.69 | 38.78 | 71.7 | 84.09 | 62.2 | 75.61 | 85.46 | 0.004 |
| HepG2 | bDNA | 15.1 | 8.14 | 5.13 | 6.89 | 12.17 | 32.14 | 42.98 | 64.01 | 60.76 | 79.95 | 81.97 | 95.43 | 0.002 |
| Hep3B | qPCR | 8.3 | 9.47 | 13.2 | 34.5 | 44.54 | 77.38 | 81.04 | 81.41 | 93.95 | 81.04 | 75.61 | 78.28 | 0.018 |
| Hep3B | bDNA | 10.5 | 9.43 | 11.7 | 27.1 | 44.88 | 72.32 | 79.88 | 79.6 | 87.46 | 96.53 | 95.13 | 89.88 | 0.029 |

A summary of the single dose results for rodent specific TTR-dsRNAs (TTR siRNAs) are presented below in Table 10. Single dose results are expressed as % TTR mRNA relative to control, assayed in rat H.4.II.E cells, after transfection of rodent specific TTR siRNAs at 10 nM. These results show that some rodent specific TTR siRNAs are effective in suppressing endogenous rat TTR mRNA *in vitro.*

Table 10. Single dose results of *in vitro* screen of rodent specific TTR-dsRNAs (TTR siRNAs)

| Duplex # | % Relative to control at 10 nM | Duplex # | % Relative to control at 10 nM |
|---|---|---|---|
| AD-18529 | 19.83 | AD-18542 | 6.3 |
| AD-18530 | 44.49 | AD-18543 | 16.46 |
| AD-18531 | 6.01 | AD-18544 | 17.55 |
| AD-18532 | 24.06 | AD-18545 | 3.53 |
| AD-18533 | 37.78 | AD-18546 | 2.75 |
| AD-18534 | 8.19 | AD-18547 | 7.01 |
| AD-18535 | 10.18 | AD-18548 | 5.02 |
| AD-18536 | 16.13 | AD-18549 | 1.61 |
| AD-18537 | 15.88 | AD-18550 | 9.58 |
| AD-18538 | 19.93 | AD-18551 | 7.74 |
| AD-18539 | 49.24 | AD-18552 | 3.74 |
| AD-18540 | 2.99 | AD-18553 | 50.39 |
| AD-18541 | 1.32 | AD-18554 | 111.06 |

**Example 3. *In vitro* assay of TTR siRNAs for induction of TNF-α and IFN-α secretion**

To evaluate potential for immunostimulation, TTR siRNAs were assayed in vitro for induction of TNF-α and IFN-α secretion.

Human PBMC were isolated from freshly collected buffy coats obtained from healthy donors (Research Blood Components, Inc., Boston, MA) by a standard Ficoll-Hypaque density centrifugation. Freshly isolated cells ($1x10^5$/well/100μl) were seeded in 96-well plates and cultured in RPMI 1640 GlutaMax medium (Invitrogen) supplemented with 10% heat - inactivated fetal bovine serum and 1% antibiotic/antimycotic (Invitrogen).

siRNAs were transfected into PBMC using DOTAP transfection reagent (Roche Applied Science). The DOTAP was first diluted in Opti-MEM (Invitrogen) for 5 minutes before mixing with an equal volume of Opti-MEM containing the siRNA. siRNA/DOTAP complexes were incubated as specified by the manufacturer's instructions and subsequently added to PBMC (50μl/well) which were then cultured for 24 hours. Positive and negative control siRNAs were included in all assays. AD-5048 was used as a positive control siRNA. AD-5048 corresponds to a sequence that targets human Apolipoprotein B (Soutschek *et al.,* 2004) and elicits secretion of both IFN-α and TNF-α in this assay. AD-1955, which does not elicit IFN-α and TNF-α secretion in this assay, was used as a negative control siRNA. All siRNAs were used at a final concentration of 133 nM. The ratio of RNA to transfection reagent was16.5 pmoles per μg of DOTAP.

Cytokines were detected and quantified in culture supernatants with a commercially available ELISA kit for IFN-α (BMS216INST) and TNF-α (BMS223INST), both from Bender MedSystems (Vienna, Austria). TTR siRNA cytokine induction is expressed as percent IFN-α or TNF-α produced relative to the positive control siRNA AD-5048.

IFN-α and TNF-α stimulation results for a number of TTR siRNAs are presented in FIG. 1 (mean of quadruplicate wells ± SD) and below in Table 11 (percentage compared with AD-5048). None of the TTR siRNAs evaluated induced significant TNF-α or IFN-α secretion by cultured human PBMCs.

Table 11. IFN-α and TNF-α stimulation results for TTR siRNAs

| Duplex # | IFN-α (% of AD-5048) | TNF-αC (% of AD-5048) |
|---|---|---|
| AD-18246 | 0 | 4 |
| AD-18258 | 0 | 0 |
| AD-18259 | 0 | 0 |

(continued)

| Duplex # | IFN-α (% of AD-5048) | TNF-α C (% of AD-5048) |
|---|---|---|
| AD-18261 | 0 | 0 |
| AD-18263 | 0 | 0 |
| AD-18271 | 0 | 0 |
| AD-18274 | 2 | 1 |
| AD-18275 | 0 | 0 |
| AD-18276 | 0 | 0 |
| AD-18277 | 0 | 0 |
| AD-18285 | 0 | 0 |
| AD-18290 | 0 | 0 |
| AD-18291 | 0 | 0 |
| AD-18292 | 0 | 0 |
| AD-18293 | 0 | 0 |
| AD-18298 | 0 | 0 |
| AD-18299 | 0 | 0 |
| AD-18320 | 0 | 0 |
| AD-18321 | 0 | 0 |
| AD-18323 | 0 | 0 |
| AD-18324 | 0 | 0 |
| AD-18325 | 0 | 0 |
| AD-18326 | 0 | 0 |
| AD-18327 | 0 | 0 |
| AD-18328 | 0 | 0 |
| AD-18330 | 0 | 0 |
| AD-18332 | 1 | 0 |
| AD-18333 | 0 | 1 |
| AD-18334 | 0 | 1 |
| AD-18336 | 1 | 0 |
| AD-18339 | 0 | 0 |
| AD-18340 | 0 | 0 |
| AD-18342 | 0 | 0 |
| AD-18343 | 0 | 0 |
| AD-18345 | 0 | 0 |
| AD-18353 | 0 | 0 |
| AD-18448 | 0 | 0 |
| AD-18456 | 0 | 0 |
| AD-18457 | 0 | 0 |
| AD-18458 | 0 | 0 |
| AD-18459 | 0 | 0 |

The five lead TTR targeting dsRNAs (TTR siRNAs) were selected based on IC50s in the pM range in the human hepatocyte cell lines HepG2 and Hep3B, and the absence of immunostimulatory activity. Duplexes without any mismatches are more likely to achieve significant knockdown of the target transcript than duplexes with mismatches between the oligo and the mRNA. To better enable interpretation of cross-species toxicology data and to have the broadest applicability to human patients, duplexes that have 100% identity in orthologous genes from rat, cynomolgus monkey and human, and that do not target regions with known polymorphisms are generally preferred. The five lead compounds were selected based on IC50 in hepatocyte cell lines in the pM range, the absence of immunostimulatory activity, specificity to the human TTR transcripts, and absence of known polymorphisms (mutations) in the region of the mRNA targeted by the duplex. In the case of TTR, no 19 base oligos were found with complete identity in human, rat and cynomolgus monkey. A summary of these data are presented in Table 12, which also includes information on known TTR mutations in the region targeted by the duplex and cross-species reactivity.

Table 12. Summary of data for five most potent TTR dsRNAs.

| **Duplex #** | **IC50 (qPCR): nM HepG2** | **IC50 (bDNA): nM HepG2** | **IFNa/TNFa** | **Mutations not covered** | **Cross-species reactivity** |
|---|---|---|---|---|---|
| AD-18258 | 0.007 | 0.005 | Negative | None (non-coding region) | Cyno: 1 mismatch @ position 14 A to G<br>Rat: no homology at any position |
| AD-18274 | 0.009 | 0.176 | Negative | Lys70Asn; Val71Ala; Ile73Val; Asp74His | Cyno: no mismatch<br>Rat: no homology at any position |
| AD-18324 | 0.002 | 0.006 | Negative | None (non-coding region) | Cyno: no mismatch<br>Rat: no homology at any position |
| AD-18328 | 0.003 | 0.004 | Negative | None (non-coding region) | Cyno: no mismatch<br>Rat: 7 mismatches |
| AD-18339 | 0.004 | 0.002 | Negative | None (non-coding region) | None |

**Example 4. *In vivo* reduction of liver TTR mRNA and plasma TTR protein by LNP01-18324, LNP01-18328 and LNP01-18246 in Transgenic mice**

Two TTR siRNAs, AD-18324 and AD-18328, were chosen for *in vivo* evaluation. These duplexes exhibited potent dose-dependent silencing *in vitro* in hepatocyte cell lines (*e.g.* HepG2). FIG. 2A and FIG. 2B show the dose responses in HepG2 cells after transfection with AD-18324 (FIG. 2A) or AD-18328 (FIG. 2B) where the doses are expressed in nM on the x-axis and the responses are expressed as fraction TTR mRNA remaining relative to control, on the y-axis. In HepG2 cells, the IC50s of AD-18324 and AD-18328 were determined to be 2 pM and 3 pM, respectively. The TTR target sites for both lead dsRNA candidates are in the 3' untranslated region of the TTR mRNA, in a region where there are no reported mutations in the literature.

The sequences of each strand of the two lead candidates are reproduced below from the Tables. Strand: s= sense; as= antisense; Position: position of 5' base on transcript NM_000371.2.

| Duplex # | Strand | Oligo # | Position* | Sequence 5' to 3' | SEQ ID NO: |
|---|---|---|---|---|---|
| AD-18324 | s | A-32337 | 509 | GGAuuucAuGuAAccAAGAdTdT | 1001 |
| AD-18324 | as | A-32338 | 527 | UCUUGGUuAcAUGAAAUCCdTdT | 1002 |
| AD-18328 | s | A-32345 | 518 | GuAAccAAGAGuAuuccAudTdT | 1009 |
| AD-18328 | as | A-32346 | 536 | AUGGAAuACUCUUGGUuACdTdT | 1010 |

In addition, a rodent cross-reactive TTR dsRNA, AD-18246, was chosen for further evaluation *in vivo.* AD-18246 targets a sequence beginning at position 88 of the open reading frame, where there are three mutations reported

in the literature. A dose response curve for AD-18246 in HepG2 cells is shown in FIG. 3. AD-18246 is substantially less potent than AD-18324 and AD-18328; the IC50 of AD-18246 was determined to be 265 pM.

AD-18324, AD-18328, and AD-18246 were administered to transgenic mice after formulation in LNP01. 3-5 month old H129-mTTR-KO/iNOS-KO/hTTR transgenic mice (mouse transthyretin knock-out/ inducible nitric oxide synthase knock-out/human transthyretin transgenic) were intravenously (IV) administered 200 μl of LNP01-formulated transthyretin-specific siRNA (AD-18324, AD-18328, or AD-18246), LNP01-formulated control siRNA targeting the non-mammalian luciferase gene (AD-1955) or PBS via the tail vein at concentrations of 1.0 mg/kg, 3.0 mg/kg, or 6.0 mg/kg for siRNAs AD-18324 and AD-18328, 3.0 mg/kg for siRNA AD-18246, and 6.0 mg/kg for siRNA AD-1955. LNP01 is a lipidoid formulation comprised of ND98, Cholesterol, and PEG-Ceramide C16.

After approximately forty-hours, mice were anesthetized with 200 μl of ketamine, and then exsanguinated by severing the right caudal artery. Whole blood was isolated and plasma was isolated and stored at -80°C until assaying. Liver tissue was collected, flash-frozen and stored at -80°C until processing.

Efficacy of treatment was evaluated by (i) measurement of TTR mRNA in liver at 48 hours post-dose, and (ii) measurement of TTR protein in plasma at prebleed and at 48 hours post-dose. TTR liver mRNA levels were assayed utilizing the Branched DNA assays-QuantiGene 2.0 (Panomics cat #: QS0011). Briefly, mouse liver samples were ground and tissue lysates were prepared. Liver lysis mixture (a mixture of 1 volume of lysis mixture, 2 volume of nuclease-free water and 10ul of Proteinase-K/ml for a final concentration of 20mg/ml) was incubated at 65 °C for 35 minutes. 20μl of Working Probe Set (TTR probe for gene target and GAPDH for endogenous control) and 80ul of tissue-lysate were then added into the Capture Plate. Capture Plates were incubated at 55 °C ±1 °C (aprx. 16-20hrs). The next day, the Capture Plate were washed 3 times with 1X Wash Buffer (nuclease-free water, Buffer Component 1 and Wash Buffer Component 2), then dried by centrifuging for 1 minute at 240g. 1 OOul of pre-Amplifier Working Reagent was added into the Capture Plate, which was sealed with aluminum foil and incubated for 1 hour at 55°C ±1C. Following 1 hour incubation, the wash step was repeated, then 100μl of Amplifier Working Reagent was added. After 1 hour, the wash and dry steps were repeated, and 100μl of Label Probe was added. Capture plates were incubated 50 °C ±1 °C for 1 hour. The plate was then washed with 1X Wash Buffer, dried and 100μl Substrate was added into the Capture Plate. Capture Plates were read using the SpectraMax Luminometer following a 5 to 15 minute incubation. bDNA data were analyzed by subtracting the average background from each triplicate sample, averaging the resultant triplicate GAPDH (control probe) and TTR (experimental probe) values, and then computing the ratio: (experimental probe-background)/(control probe-background).

TTR plasma levels were assayed utilizing the commercially available kit "AssayMax Human Prealbumin ELISA Kit" (AssayPro, St. Charles, MO, Catalog # EP3010-1) according to manufacturer's guidelines. Briefly, mouse plasma was diluted 1:10,000 in 1X mix diluents and added to pre-coated plates along with kit standards, and incubated for 2 hours at room temperature followed by 5X washes with kit wash buffer. Fifty microliters of biotinylated prealbumin antibody was added to each well and incubated for 1 hr at room temperature, followed by 5X washes with wash buffer. Fifty microliters of streptavidin-peroxidase conjugate was added to each well and plates were incubated for 30 minutes at room temperature followed by washing as previously described. The reaction was developed by the addition of 50 μl/well of chromogen substrate and incubation for 10 minutes at room temperature with stopping of reaction by the addition of 50 μl/well of stop solution. Absorbance at 450 nm was read on a Versamax microplate reader (Molecular Devices, Sunnyvale, CA) and data were analyzed utilizing the Softmax 4.6 software package (Molecular Devices).

LNP01-18324 and LNP01-18328 were found to reduce liver TTR mRNA (FIG. 4A) and plasma TTR protein (FIG. 4B) levels in a dose-dependent manner with IV bolus administration. The mRNA ED50 of LNP01-18328 was determined to be ~1 mg/kg whereas the ED50 of LNP01-18324 was determined to be ~ 2 mg/kg. The effects of LNP01-18324 and LNP01-18328 were specific, because the control, LNP01-1955 at 6 mg/kg, did not significantly affect liver TTR mRNA levels, as compared with the PBS group. LNP01-18324 and LNP01-18328 reduced plasma TTR protein levels relative to the PBS group, with potencies that were similar to those on TTR mRNA levels. At 3 mg/kg, LNP01-18246 reduced liver TTR mRNA levels to a lessor extent than 3 mg/kg LNP01-18324 or LNP01-18328.

These results demonstrate that LNP01-18324 and LNP01-18328, administered by IV bolus, substantially reduce human TTR mRNA expressed by the transgenic mouse liver, which results in reduction of human TTR protein in the circulation.

## Example 5. *In vivo* reduction of wild-type TTR mRNA in the non-human primate liver by SNALP-18324 and SNALP-18328

To evaluate the efficacy of TTR siRNAs AD-18324 and AD-18328 in non-human primates on liver TTR mRNA levels, the siRNAs were formulated in SNALP and administered by 15-minute IV infusion. Cynomolgus monkeys (*Macaca fascicularis*) (2 to 5 kg, 3 animals per group) were administered 15-minute IV infusions of SNALP-18324 (0.3, 1.0 or 3.0 mg/kg), SNALP-18328 (0.3, 1 or 3 mg/kg), or SNALP-1955 (3 mg/kg, with negative control siRNA AD-1955 which targets the non-mammalian gene luciferase). At forty-eight hours post-dosing, monkeys were anesthetized with sodium pentobarbital and exsanguinated. Liver tissue for TTR mRNA determination was collected, flash-frozen, and stored at -80°C until processing.

TTR mRNA levels in the liver were assayed utilizing a custom designed Branched DNA assay, utilizing the QuantiGene1.0 technology. Briefly, monkey liver samples were ground and tissue lysates were prepared. Liver lysis mixture (1 volume lysis mixture, 2 volume nuclease-free water, and 10μl of Proteinase-K/ml for a final concentration of 20mg/ml) was incubated at 65°C for 35 minutes. 20μl Working Probe Set (TTR probe for gene target and GAPDH for endogenous control) and 80μl tissue-lysate were then added into the Capture Plate. Capture Plates were incubated at 55°C ±14°C (approx. 16-20hrs). The next day, the Capture Plates were washed three times with 1X Wash Buffer (nuclease-free water, Buffer Component 1 and Wash Buffer Component 2), then dried by centrifuging for 1 minute at 240g. 100μl of pre-Amplifier Working Reagent was added into the Capture Plate, which was sealed with aluminum foil and incubated for 1 hour at 55°C ± °C. Following a 1-hour incubation, the wash step was repeated, and then 100μl Amplifier Working Reagent was added. After 1 hour, the wash and dry steps were repeated, and 100μl Label Probe was added. Capture plates were incubated 50°C ± °C for 1 hour. The plates were then washed with 1X Wash Buffer and dried, and then 100μl Substrate was added into the Capture Plate. Capture Plates were read using the SpectraMax Luminometer following a 5 to 15 minute incubation. bDNA data were analyzed by (i) subtracting the average background from each triplicate sample, (ii) averaging the resultant GAPDH (control probe) and TTR (experimental probe) values, and then (iii) taking the ratio: (experimental probe-background)/(control probe-background).

The results are shown in FIG. 5. SNALP -18324 and SNALP -18328 reduced TTR mRNA levels in the liver in a dose-dependent manner, compared to the negative control SNALP-1955. The mRNA ED50s of SNALP-18328 and SNALP-18324 were determined to be ~0.3 and ~ 1 mg/kg, respectively.

These results demonstrate that SNALP-18324 and SNALP-18328 are effective in suppressing wild-type TTR mRNA in non-human primate liver when administered by IV infusion.

**<u>Example 6. *In vivo* reduction of mutant (V30M) TTR mRNA and protein by SNALP-18328 in the transgenic mouse</u>**

To evaluate the efficacy of TTR siRNA AD-18328 on mutant (V30M) TTR mRNA in the liver and mutant (V30M) TTR protein in the serum, AD-18328 was formulated in SNALP and administered by IV bolus to V30M hTTR transgenic mice. 8 to 12-week old V30M hTTR transgenic mice (5 animals/ group) were intravenously (IV) administered 200 μl SNALP-18328 (0.03, 0.3 or 3 mg/kg), SNALP-1955 (3 mg/kg, with negative control siRNA AD-1955 which targets the non-mammalian gene luciferase), or PBS. Mice used were the Mus musculus strain H129-hTTR KO from Institute of Molecular and Cellular Biology, Porto, Portugal. Briefly, hTTR H129 transgenic mice were crossed with a H129 endogenous TTR KO mice (null mice to generate the H129-hTTR transgenic mice, in a null mouse TTR background (Maeda, S., (2003), Use of genetically altered mice to study the role of serum amyloid P component in amyloid deposition. Amyloid Suppl. 1, 17-20.).

At 48 hrs post-injection, animals in all five treatment groups were given a lethal dose of ketamine/xylazine. Serum samples were collected and stored at -80°C until analysis. Liver tissue was collected, flash-frozen and stored at -80oC until processing.

For TTR mRNA quantitation, frozen liver tissue was ground into powder, and lysates were prepared. TTR mRNA levels relative to those of GAPDH mRNA were determined in the lysates by using a branched DNA assay (QuantiGene Reagent System, Panomics, Fremont, CA). Briefly, the QuantiGene assay (Genospectra) was used to quantify mRNA levels in tissue sample lysates according to the manufacturer's instructions. The mean level of TTR mRNA was normalized to the mean level of GAPDH mRNA for each sample. Group means of the normalized values were then further normalized to the mean value for the PBS treated group, to obtain the relative level of TTR mRNA expression.

For TTR protein quantitation, serum was assayed using the AssayPro (St. Charles, MO) Assaymax PreAlbumin ELISA Kit according to the manufacturer's protocol.

The results are shown in FIG. 6A and FIG. 6B for liver mRNA and serum protein, respectively. SNALP-18328 treated V30M hTTR transgenic mice had a dose-dependent and significant decrease in liver TTR mRNA levels relative to the PBS control group, reaching a maximum reduction of 97% ($p < 0.001$) at 3 mg/kg SNALP-18328, and a 50% reduction (ED50) at ~ 0.15 mg/kg SNALP-18328. Serum TTR protein was also suppressed in a dose-dependent manner, with a maximum reduction of serum TTR protein of 99% ($p < 0.01$) (relative to pre-dose levels) at 3 mg/kg SNALP-18328, consistent with the reduction in TTR mRNA levels. SNALP-1955 at 3 mg/kg did not have a statistically significant effect on either TTR mRNA or protein levels, compared to PBS.

These results demonstrate that SNALP-18328, when administered IV, is active in suppressing mutant V30M TTR mRNA in the transgenic mouse liver, which results in reduction of mutant V30M TTR protein in the circulation.

**Example 7. Durability of TTR mRNA and protein suppression by SNALP-18328 in the transgenic mouse**

To evaluate the durability of TTR mRNA and protein suppression by SNALP-18328, AD-18328 was formulated in SNALP and administered by IV bolus to V30M hTTR transgenic mice. At various timepoints post-dose, liver TTR mRNA levels and serum TTR protein levels were quantified. 8- to 12-week old V30M hTTR transgenic mice (4 animals/group) were intravenously (IV) administered 200 μl SNALP-18328 (1 mg/kg) or SNALP-1955 (1 mg/kg, with negative control siRNA AD-1955 which targets the non-mammalian gene luciferase). Mice used were Mus musculus strain H129-hTTR KO from Institute of Molecular and Cellular Biology, Porto, Portugal. Briefly, hTTR H129 transgenic mice were crossed with a H129 endogenous TTR KO mice (null mice to generate the H 129-hTTR transgenic mice, in a null mouse TTR background (Maeda, S., (2003), Use of genetically altered mice to study the role of serum amyloid P component in amyloid deposition. Amyloid Suppl. 1, 17-20). Days 3, 8, 15, or 22 post-dose, animals in both treatment groups were given a lethal dose of ketamine/xylazine. Serum samples were collected and stored at -80°C until analysis. Liver tissue was collected, flash-frozen and stored at -80°C until processing.

For TTR mRNA quantitation, frozen liver tissue was ground into powder, and lysates were prepared. TTR mRNA levels relative to those of GAPDH mRNA were determined in the lysates by using a branched DNA assay (QuantiGene Reagent System, Panomics, Fremont, CA). Briefly, the QuantiGene assay (Genospectra) was used to quantify mRNA levels in tissue sample lysates according to the manufacturer's instructions. The mean level of TTR mRNA was normalized to the mean level of GAPDH mRNA for each sample. Group means of the normalized values were then further normalized to the mean value for the PBS treated group, to obtain the relative level of TTR mRNA expression.

For TTR protein quantitation, serum was assayed using the AssayPro (St. Charles, MO) Assaymax PreAlbumin ELISA Kit according to the manufacturer's protocol.

The results are shown in FIG. 7A and FIG. 7B for liver mRNA and serum protein, respectively. A single IV bolus administration of SNALP-18328 in the hTTR V30M transgenic mouse resulted in durable inhibition of TTR mRNA levels in the liver and TTR protein levels in the serum. Compared to the control group (1 mg/ml SNALP-1955), a single IV administration of SNALP-18328 at 1 mg/kg significantly reduced relative TTR mRNA levels on Days 3, 8, 15 and 22 post-dose by 96% ($p < 0.001$), 90% ($p < 0.001$), 82% ($p < 0.001$) and 73% ($p < 0.001$), respectively, and did not return to baseline levels at termination of the study (Day 22 post-dose). Protein levels also decreased with a maximum reduction of serum TTR of 97% ($p < 0.001$) (relative to SNALP-1955) at Day 3 post-dose. At Days 8, 15, and 22 post-dose, TTR protein levels were suppressed by 72% ($p < 0.05$), 32% ($p < 0.05$), and 40% ($p < 0.001$), respectively, relative to SNALP-1955.

These results demonstrate that a single IV administration of SNALP-18328 produces durable suppression of target liver mRNA and serum protein levels in the V30M hTTR transgenic mouse, with significant reductions of both liver TTR mRNA and serum TTR protein at 22 days post-dose.

**Example 8. Durability of serum TTR protein and liver mRNA suppression by SNALP-18328 in the non-human primate**

To evaluate the durability of serum TTR protein suppression by SNALP-18328, AD-18328 was formulated in SNALP and administered by IV infusion to non-human primates. At various timepoints post-dose, serum TTR protein levels were quantified.

Cynomolgus monkeys (*Macaca fascicularis*) (n= 5 animals/group for SNALP-18328 groups and n = 3 animals/group for SNALP-1955 and PBS groups) were administered a 15-minute IV infusion of SNALP-18328 (0.3, 1 or 3 mg/kg), SNALP-1955 (3 mg/kg) with negative control siRNA AD-1955 which targets the non-mammalian gene luciferase), or PBS. At Days 0, 1, 2, 3, 4, 5, 7, 10, and 14 of the dosing phase, serum samples were collected and stored at -80°C until analysis.

Western blot analysis was used to evaluate TTR protein levels in serum samples. Serum samples from each group were pooled and diluted 1:1 with Laemmli sample buffer (β-mercaptoethanol was added at a 1:20 dilution). The samples were heated at 95°C for 10 minutes. 12.5 μl of each sample was loaded in each lane of a 10-20% Criterion (Biorad, Hercules, CA) prep gel and separated by SDS-PAGE at 120V for 1.5 hrs, then transferred to a nitrocellulose membrane using a semi-dry system at 15V for 1 hour. The membrane was blocked overnight at 4°C in LiCOR (Lincoln, NE) blocking buffer diluted 1:1 with 1X PBS. The blot was probed first with primary antibodies (goat anti-TTR from Santa Cruz (Santa Cruz, CA) at a dilution of 1:1000 diluted in LiCOR blocking buffer/PBS on a rocker for 1 hr at room temperature. Blots were washed 4X with PBS + 0.2% Tween 20 (10 minutes per wash). The fluorescent labeled secondary antibodies (anti-goat 680nm from Invitrogen (Carlsbad, CA) were added at a dilution of 1:10,000 in LiCOR blocking buffer/PBS and the blot was incubated for 1 hour at room temperature. After incubation, blots were washed 4X with PBS + 0.2% Tween 20 followed by one wash with 1X PBS. The Li-COR's Odyssey Infrared Imaging System was used to detect the protein bands. TTR monomer migrates at 15 kDa.

The results are shown in FIG. 8. Serum TTR protein levels showed a dose-dependent reduction with 1 or 3 mg/kg SNALP-18328, as compared to pre-dose (Day 0) levels. The duration of suppression, following a single IV administration of SNALP-18328 is at least 14 days after 1 or 3 mg/kg SNALP-18328 treatment.

These results demonstrate that a single IV administration of SNALP-18328 produces durable suppression of TTR protein in the circulation in the non-human primate (*Macaca fascicularis*), with significant reduction of TTR protein at 14 days post-dose.

To evaluate the durability of liver TTR mRNA suppression by SNALP-18328, AD-18328 was formulated in SNALP (ALN-TTR01) and administered by single IV infusion to non-human primates. Liver mRNA levels wer measured as described herein at day 3 or day 30 post-administration.

The results are shown in FIG. 20 and demonstrate that ALN-TTR01 suppression of wild type TTR mRNA is durable in non-human primates after 3 days for dosages 1.0 and 3.0 mg/kg and for 30 days at a dose of 10 mg/kg.

**Example 9: *In vivo* reduction of mutant (V30M) TTR in peripheral tissues by SNALP-18328 in the transgenic mouse**

**Propylactic efficacy**

To evaluate the efficacy of SNALP-18328 (ALN-TTR01) in reducing TTR in peripheral tissues, hTTR V30M/HSF-1 knock-out mice were evaluated with immunohistochemical staining for TTR. Two-month old hTTR V30M/HSF-1 knock-out mice (Maeda, S., (2003), Use of genetically altered mice to study the role of serum amyloid P component in amyloid deposition. Amyloid Suppl. 1, 17-20) were administered an IV bolus of 3 mg/kg SNALP-18328 (12 animals), 3 mg/kg SNALP-1955 (with negative control siRNA AD-1955 which targets the non-mammalian gene luciferase, 4 animals), or PBS (4 animals) once every two weeks for a total of four doses on days 0, 14, 28, and 42. TTR liver mRNA levels and TTR-immunoreactivity in multiple peripheral tissues were evaluated at 8 weeks post-first dose on day 56.

Mice were anesthetised with 1 mg/kg medetomidine, and given a lethal dose of ketamine. Tissues and organs of interest were collected. For immunohistochemistry, esophagus (E), stomach (S), intestine (duodenum (I1) and colon (I4)), nerve (N) and dorsal root ganglia (D) were fixed in neutral buffered formalin and embedded in paraffin. For TTR detection, rabbit anti-human TTR primary antibody (1:1000, DAKO, Denmark), and anti-rabbit biotin-conjugated secondary antibody (1:20 Sigma, USA) were followed by extravidin labelling (1:20, Sigma, USA) in order to stain for the TTR protein. The reaction was developed with 3-amino-9-ethyl carbaxole, AEC (Sigma, USA). Semi-quantitative analysis of immunohistochemical slides was performed using Scion image quant program that measures the area occupied by the substrate reaction color and normalizes this value to the total image area. Mean values of % occupied area are displayed with the corresponding standard deviation. Each animal tissue was evaluated in four different areas. The presence of human TTR in parasympathetic ganglia of the stomach and intestine was studied by double immunofluorescent staining with rabbit anti-human TTR (1:1000, DAKO, Denmark) and mouse anti-PGP9.5 (1:40, Serotec, USA) as the primary antibodies; secondary antibodies were, respectively: anti-rabbit Alexa Fluor 488 (Molecular probes, UK)and goat anti-mouse Alexa Fluor 568 (Molecular probes, UK). Slides were mounted with vectashield (Vector) and visualized in a Zeiss Cell Observer System microscope (Carl Zeiss, Germany) equipped with filters for FITC and rhodamine.

The results are graphed in FIG. 9. In contrast with PBS and SNALP-1955 treated animals, SNALP-18328 treated animals had a significant reduction of TTR-immunoreactivity in all tissues examined (esophagus (E), stomach (S), intestine (duodenum (I1) and colon (I4)), nerve (N) and dorsal root ganglia (D).

These results demonstrate that SNALP-18328 administration to hTTR V30M/HSF-1 knock-out mice causes a significant reduction of TTR protein deposition in peripheral tissues and organs, including esophagus, stomach, intestine (duodenum and colon), nerve, and dorsal root ganglion.

**Therapeutic efficacy**

ALN-TTR01 was administered to mature hTTR V30M/HSF-1 knock-out mice to determine the effects of TTR siRNA treatment on regression of mutant human TTR deposits.

Groups of 21 month old animals (hTTR V30M/HSF-1 knock-out mice) were intravenously administered an IV bolus of ALN-TTR01 or control siRNA at a dose of 3 mg/kg on days 0, 14, 28, 14, 56, and 70. On day 77, the mice were euthanized, tissue was harvested, and TTR deposition was assayed via semi-quantitative analysis of immunohistochemical stained slides using Scion image quant program as described herein. Esophagus, colon; stomach, sciatic nerve; and dorsal root ganglia tissue were examined and results were compared to historical data in this animal model demonstrating both TTR deposition and TTR fibrils present in tissues at this age.

The results are shown in the graph in FIG. 21. The results demonstrate that treatment with TTR siRNA resulted in >90% regression of existing V30M hTTR tissue deposits.

## Example 10. *In vivo* reduction of wild-type TTR mRNA in the non-human primate liver by XTC-SNALP-18328

To evaluate the efficacy of the novel lipid nanoparticle formulation XTC-SNALP for delivery of siRNA in non-human primate, TTR siRNA AD-18328 was formulated in XTC-SNALP (XTC-SNALP-18328) and administered by 15-minute IV infusion, and liver TTR mRNA was quantified. Cynomolgus monkeys (*Macaca fascicularis*) were administered 15-minute IV infusions of XTC-SNALP-18328 (0.03, 0.1, 0.3 or 1 mg/kg) or XTC-SNALP-1955 (1 mg/kg, with negative control siRNA AD-1955 which targets the non-mammalian gene luciferase). At forty-eight hours post-dosing, monkeys were anesthetized with sodium pentobarbital and exsanguinated. Liver tissue for TTR mRNA determination was collected, flash-frozen, and stored at -80°C until processing. Methods used for TTR mRNA quantitation in liver tissue were similar to those described in Example 5 above.

The results are shown in FIG. 10. XTC-SNALP -18328 reduced TTR mRNA levels in the liver in a dose-dependent manner, compared to the negative control XTC-SNALP -1955. The mRNA ED50 was determined to be ~ 0.1 mg/kg XTC-SNALP -18328.

These results demonstrate that XTC-SNALP-18328 is effective in suppressing wild-type TTR mRNA in non-human primate liver when administered by IV infusion.

## Example 11: *In vivo* reduction of wild-type TTR mRNA in the non-human primate liver by LNP09-18328 and LNP11-18328

To evaluate the efficacy of two novel lipid nanoparticle formulations, LNP09 and LNP11, for delivery of siRNA in non-human primate, TTR siRNA AD-18328 was formulated in LNP09 (LNP09-18328) or LNP11 (LNP11-18328), and administered by 15-minute IV infusion, and liver TTR mRNA and serum TTR protein levels were assayed. Cynomolgus monkeys (*Macaca fascicularis*) were administered 15-minute IV infusions of LNP09-18328 (0.03, 0.1, or 0.3 mg/kg), LNP11-18328 (0.03, 0.1, or 0.3 mg/kg), or PBS. Liver biopsy samples were collected at 48 hrs post-dosing, flash-frozen, and stored at -80°C until processing. Serum was collected before dosing (pre-bleed), and on Days 1, 2, 4, 7, 14, 21 and 28 post-dosing and stored at -80°C until processing. Methods used for TTR mRNA quantitation in liver tissue and serum TTR protein evaluation were similar to those described in Examples 5 and 8 above.

The results are shown in FIG. 11A for mRNA, and in FIG. 11B and FIG. 11C for protein. LNP09-18328 and LNP11-18328 treated animals showed a dose-dependent decrease in TTR mRNA levels in the liver, reaching a maximum reduction at 0.3 mg/kg of ~ 85% (LNP09-18328) and ~ 90% (LNP11-18328) mRNA relative to the PBS control. The mRNA ED50 was determined to be ~ 0.02 mg/kg for both LNP09-18328 and LNP11-18328. At Day 7 post-dosing, serum samples also exhibited a dose-dependent reduction of TTR protein for 0.1 and 0.3 mg/kg LNP09-18328 and LNP11-18328, compared to PBS control levels. FIG 11C shows a decrease in TTR protein levels with a 0.3mg/kg dose of LNP09-18328 that persisted over at least 28 days post-dosing, as compared to the PBS control group and as compared with the pre-bleed samples.

These results demonstrate that LNP09-18328 and LNP11-18328 are effective in suppressing wild-type TTR mRNA in non-human primate liver and wild-type TTR protein in the circulation, when administered by IV infusion. Furthermore, the suppression with LN09-18328 is durable, persisting for at least 28 days following the IV infusion.

## Example 12: *In vivo* reduction of wild-type TTR mRNA in the non-human primate liver by LNP12-18328

LNP12 formulated AD-18328 was administered to non-human primates to evaluate the efficacy of this formulation.

LNP12-18328 formulations were prepared using a method adapted from Jeffs et al. (Jeffs LB, et al. (2004) A Scalable, Extrusion-Free Method for Efficient Liposomal Encapsulation of Plasmid DNA. Pharm Res 22:362-372) Briefly, Tech-GI (described above), distearoyl phosphatidylcholine (DSPC), cholesterol and mPEG2000-DMG were solubilized in 90% ethanol at a molar ratio of 50:10:38.5:1.5. The siRNA was solubilized in 10 mM citrate, pH 3 buffer at a concentration of 0.4 mg/mL. The ethanolic lipid solution and the aqueous siRNA solution were pumped by means of a peristaltic pump fitted with dual pump heads at equivalent volumetric flow rates and mixed in a "T"-junction. Lipids were combined with siRNA at a total lipid to siRNA ratio of 7:1 (wt:wt). The spontaneously formed LNP12-18328 formulations were dialyzed against PBS (155mM NaCl, 3mM Na2HPO4, 1mM KH2PO4, pH 7.5) to remove ethanol and exchange buffer. This formulation yields a mean particle diameter of 80nm with approximately 90 percent siRNA entrapment efficiency.

Cynomolgus monkeys (n = 3 per group) received either PBS or 0.03, 0.1, or 0.3 mg/kg LNP12-18328 as 15 minute intravenous infusions (5 mL/kg) via the cephalic vein. Liver biopsies were collected from animals at 48 hours post-administration. TTR mRNA levels relative to GAPDH mRNA levels were determined in liver samples as described herein.

As shown in FIG. 19, high levels of specific knockdown of the wild -type transthyretin (TTR) gene was observed at doses as low as 0.03 mg/kg. This demonstrated that the LNP12 formulation facilitates gene silencing at orders-of-magnitude lower doses than required by any previously-described siRNA liver delivery system.

**Example 13. Synthesis of TTR Tiled Sequences**

A set of TTR duplexes ("tiled duplexes") were designed that targeted the TTR gene near the target region of AD-18328, which targets the human TTR gene starting at nucleotide 628 of NM_000371.3.

In the examples below, the numbering representing the position of the 5' base of an siRNA on the transcript is based on NM_000371.3 (FIG. 12; SEQ ID NO:1331). In the examples shown above, the numbering for siRNA targeting human siRNA was based on NM_000371.2 (FIG. 13A). NM_000371.3 extends the sequence of the 5' UTR by 110 bases compared to NM_000371.2, as shown in FIG. 14. Thus, as an example, the starting position of AD-18328 is 628 on NM_000371.3 and 518 on NM_000371.2 (FIG. 14).

TTR tiled sequences were synthesized on MerMade 192 synthesizer at 1umol scale. For all the sequences in the list, 'endolight' chemistry was applied as detailed below.

- All pyrimidines (cytosine and uridine) in the sense strand contained 2'-0-Methyl bases (2' O-Methyl C and 2'-O-Methyl U)
- In the antisense strand, pyrimidines adjacent to(towards 5' position) ribo A nucleoside were replaced with their corresponding 2-O-Methyl nucleosides
- A two base dTdT extension at 3' end of both sense and anti sense sequences was introduced
- The sequence file was converted to a text file to make it compatible for loading in the MerMade 192 synthesis software

Synthesis, Cleavage and deprotection:

The synthesis of TTR sequences used solid supported oligonucleotide synthesis using phosphoramidite chemistry. The synthesis of the sequences was performed at 1 um scale in 96 well plates. The amidite solutions were prepared at 0.1 M concentration and ethyl thio tetrazole (0.6M in Acetonitrile) was used as activator. The synthesized sequences were cleaved and deprotected in 96 well plates, using methylamine in the first step and fluoride reagent in the second step. The crude sequences were precipitated using acetone: ethanol (80:20) mix and the pellet were re-suspended in 0.2M sodium acetate buffer. Samples from each sequence were analyzed by LC-MS to confirm the identity, UV for quantification and a selected set of samples by IEX chromatography to determine purity.

Purification and desalting:

TTR tiled sequences were purified on AKTA explorer purification system using Source 15Q column. A column temperature of 65C was maintained during purification. Sample injection and collection was performed in 96 well (1.8mL -deep well) plates. A single peak corresponding to the full length sequence was collected in the eluent. The purified sequences were desalted on a Sephadex G25 column using AKTA purifier. The desalted TTR sequences were analyzed for concentration (by UV measurement at A260) and purity (by ion exchange HPLC). The single strands were then submitted for annealing.

TTR Single strands and duplexes:

A detailed list of TTR tiled duplexes and corresponding single strands (sense and antisense) are shown in the table below (Table 13).

Table 13: TTR tiled duplexes and corresponding single strands

| Strand: s= sense; as= antisense; Position: position of 5' base on transcript (NM_000371.3, SEQ ID NO:1331). | | | | | |
|---|---|---|---|---|---|
| **Duplex #** | **Position** | **Oligo #** | **Strand** | **Sequence (5' to 3")** | **SEQ ID NO:** |
| AD-18323 | 618 | A-32335 | S | GGGAuuucAuGuAAccAAGdTdT | 1332 |
| | | A-32336 | AS | CUUGGUuAcAUGAAAUCCCdTdT | 1333 |
| AD-18324 | 619 | A-32337 | S | GGAuuucAuGuAAccAAGAdTdT | 1334 |
| | | A-32338 | AS | UCUUGGUuAcAUGAAAUCCdTdT | 1335 |
| AD-23000 | 620 | A-42927 | S | GAuuucAuGuAAccAAGAGdTdT | 1336 |
| | | A-42928 | AS | CUCUUGGUuAcAUGAAAUCdTdT | 1337 |

(continued)

| Strand: s= sense; as= antisense; Position: position of 5' base on transcript (NM_000371.3, SEQ ID NO:1331). | | | | | |
|---|---|---|---|---|---|
| **Duplex #** | **Position** | **Oligo #** | **Strand** | **Sequence (5' to 3")** | **SEQ ID NO:** |
| AD-23001 | 621 | A-42929 | S | AuuucAuGuAAccAAGAGudTdT | 1338 |
| | | A-42930 | AS | ACUCUUGGUuAcAUGAAAUdTdT | 1339 |
| AD-23002 | 622 | A-42931 | S | uuucAuGuAAccAAGAGuAdTdT | 1340 |
| | | A-42932 | AS | uACUCUUGGUuAcAUGAAAdTdT | 1341 |
| AD-23003 | 623 | A-42933 | S | uucAuGuAAccAAGAGuAudTdT | 1342 |
| | | A-42934 | AS | AuACUCUUGGUuAcAUGAAdTdT | 1343 |
| AD-18325 | 624 | A-32339 | S | ucAuGuAAccAAGAGuAuudTdT | 1344 |
| | | A-32340 | AS | AAuACUCUUGGUuAcAUGAdTdT | 1345 |
| AD-23004 | 625 | A-42935 | S | cAuGuAAccAAGAGuAuucdTdT | 1346 |
| | | A-42936 | AS | GAAuACUCUUGGUuAcAUGdTdT | 1347 |
| AD-18326 | 626 | A-32341 | S | AuGuAAccAAGAGuAuuccdTdT | 1348 |
| | | A-32342 | AS | GGAAuACUCUUGGUuAcAUdTdT | 1349 |
| AD-18327 | 627 | A-32343 | S | uGuAAccAAGAGuAuuccAdTdT | 1350 |
| | | A-32344 | AS | UGGAAuACUCUUGGUuAcAdTdT | 1351 |
| AD-23005 | 628 | A-42937 | S | uAAccAAGAGuAuuccAuudTdT | 1352 |
| | | A-42938 | AS | AAUGGAAuACUCUUGGUuAdTdT | 1353 |
| AD-23006 | 629 | A-42939 | S | AAccAAGAGuAuuccAuuudTdT | 1354 |
| | | A-42940 | AS | AAAUGGAAuACUCUUGGUUdTdT | 1355 |
| AD-23007 | 631 | A-42941 | S | AccAAGAGuAuuccAuuuudTdT | 1356 |
| | | A-42942 | AS | AAAAUGGAAuACUCUUGGUdTdT | 1357 |
| AD-23008 | 632 | A-42943 | S | ccAAGAGuAuuccAuuuuudTdT | 1358 |
| | | A-42944 | AS | AAAAAUGGAAuACUCUUGGdTdT | 1359 |
| AD-23009 | 633 | A-42945 | S | cAAGAGuAuuccAuuuuuAdTdT | 1360 |
| | | A-42946 | AS | uAAAAAUGGAAuACUCUUGdTdT | 1361 |
| AD-23010 | 634 | A-42947 | S | AAGAGuAuuccAuuuuuAcdTdT | 1362 |
| | | A-42948 | AS | GuAAAAAUGGAAuACUCUUdTdT | 1363 |
| AD-23011 | 635 | A-42949 | S | AGAGuAuuccAuuuuuAcudTdT | 1364 |
| | | A-42950 | AS | AGuAAAAAUGGAAuACUCUdTdT | 1365 |
| AD-23012 | 636 | A-42951 | S | GAGuAuuccAuuuuuAcuAdTdT | 1366 |
| | | A-42952 | AS | uAGuAAAAAUGGAAuACUCdTdT | 1367 |
| AD-23013 | 637 | A-42953 | S | AGuAuuccAuuuuuAcuAAdTdT | 1368 |
| | | A-42954 | AS | UuAGuAAAAAUGGAAuACUdTdT | 1369 |
| AD-23014 | 638 | A-42955 | S | GuAuuccAuuuuuAcuAAAdTdT | 1370 |
| | | A-42956 | AS | UUuAGuAAAAAUGGAAuACdTdT | 1371 |
| AD-23015 | 639 | A-42957 | S | uAuuccAuuuuuAcuAAAGdTdT | 1372 |
| | | A-42958 | AS | CUUuAGuAAAAAUGGAAuAdTdT | 1373 |
| AD-23016 | 640 | A-42959 | S | AuuccAuuuuuAcuAAAGcdTdT | 1374 |

(continued)

| Strand: s= sense; as= antisense; Position: position of 5' base on transcript (NM_000371.3, SEQ ID NO:1331). | | | | | |
|---|---|---|---|---|---|
| **Duplex #** | **Position** | **Oligo #** | **Strand** | **Sequence (5' to 3")** | **SEQ ID NO:** |
| | | A-42960 | AS | GCUUuAGuAAAAAUGGAAUdTdT | 1375 |
| AD-23017 | 641 | A-42961 | S | uuccAuuuuuAcuAAAGcAdTdT | 1376 |
| | | A-42962 | AS | UGCUUuAGuAAAAAUGGAAdTdT | 1377 |
| AD-23018 | 642 | A-42963 | S | uccAuuuuuAcuAAAGcAGdTdT | 1378 |
| | | A-42964 | AS | CUGCUUuAGuAAAAAUGGAdTdT | 1379 |
| AD-23019 | 643 | A-42965 | S | ccAuuuuuAcuAAAGcAGudTdT | 1380 |
| | | A-42966 | AS | ACUGCUUuAGuAAAAAUGGdTdT | 1381 |
| AD-23020 | 644 | A-42967 | S | cAuuuuuAcuAAAGcAGuGdTdT | 1382 |
| | | A-42968 | AS | cACUGCUUuAGuAAAAAUGdTdT | 1383 |
| AD-23021 | 645 | A-42969 | S | AuuuuuAcuAAAGcAGuGudTdT | 1384 |
| | | A-42970 | AS | AcACUGCUUuAGuAAAAAUdTdT | 1385 |
| AD-23022 | 646 | A-42971 | S | uuuuuAcuAAAGcAGuGuudTdT | 1386 |
| | | A-42972 | AS | AAcACUGCUUuAGuAAAAAdTdT | 1387 |
| AD-23023 | 647 | A-42973 | S | uuuuAcuAAAGcAGuGuuudTdT | 1388 |
| | | A-42974 | AS | AAAcACUGCUUuAGuAAAAdTdT | 1389 |
| AD-23024 | 648 | A-42975 | S | uuuAcuAAAGcAGuGuuuudTdT | 1390 |
| | | A-42976 | AS | AAAAcACUGCUUuAGuAAAdTdT | 1391 |
| AD-23025 | 649 | A-42977 | S | uuAcuAAAGcAGuGuuuucdTdT | 1392 |
| | | A-42978 | AS | GAAAAcACUGCUUuAGuAAdTdT | 1393 |
| AD-23026 | 650 | A-42979 | S | uAcuAAAGcAGuGuuuucAdTdT | 1394 |
| | | A-42980 | AS | UGAAAAcACUGCUUuAGuAdTdT | 1395 |
| AD-23027 | 651 | A-42981 | S | AcuAAAGcAGuGuuuucAcdTdT | 1396 |
| | | A-42982 | AS | GUGAAAAcACUGCUUuAGUdTdT | 1397 |
| D-23028 | 652 | A-42983 | S | cuAAAGcAGuGuuuucAccdTdT | 1398 |
| | | A-42984 | AS | GGUGAAAAcACUGCUUuAGdTdT | 1399 |
| AD-18330 | 653 | A-32349 | S | uAAAGcAGuGuuuucAccudTdT | 1400 |
| | | A-32350 | AS | AGGUGAAAAcACUGCUUuAdTdT | 1401 |
| AD-23029 | 654 | A-42985 | S | AAAGcAGuGuuuucAccucdTdT | 1402 |
| | | A-42986 | AS | GAGGUGAAAAcACUGCUUUdTdT | 1403 |
| AD-23030 | 655 | A-42987 | S | AAGcAGuGuuuucAccucAdTdT | 1404 |
| | | A-42988 | AS | UGAGGUGAAAAcACUGCUUdTdT | 1405 |
| AD-23031 | 656 | A-42989 | S | AGcAGuGuuuucAccucAudTdT | 1406 |
| | | A-42990 | AS | AUGAGGUGAAAAcACUGCUdTdT | 1407 |
| AD-18328 | 628 | A-32345 | S | GuAAccAAGAGuAuuccAudTdT | 1408 |
| | | A-32346 | AS | AUGGAAuACUCUUGGUuACdTdT | 1409 |

**Example 14. *In vitro* Screening of TTR Tiled siRNAs**

Tiled TTR duplexes were assayed in Hep3B cells for inhibition of endogenous TTR expression using real time PCR assays.

Cell culture and transfection: Hep3B cells (ATCC, Manassas, VA) were grown to near confluence at 37°C in an atmosphere of 5% $CO_2$ in Eagle's Minimum Essential Medium (EMEM, ATCC) supplemented with 10% FBS, streptomycin, and glutamine (ATCC) before being released from the plate by trypsinization. Reverse transfection was carried out by adding 5μl of Opti-MEM to 5μl of each siRNA in individual wells of a 96-well plate. To this 10μl of Opti-MEM plus 0.2μl of Lipofectamine RNAiMax was added per well (Invitrogen, Carlsbad CA. cat # 13778-150) and the mixture was incubated at room temperature for 15 minutes. 80μl of complete growth media described above, but without antibiotic containing $2.0 \times 10^4$ Hep3B cells were then added. Cells were incubated for 24 hours prior to RNA purification. Experiments were performed at 0.1 or 10nM final duplex concentration.

Total RNA isolation using MagMAX-96 Total RNA Isolation Kit (Applied Biosystems, Foster City CA, part #: AM1830): Cells were harvested and lysed in 140μl of Lysis/Binding Solution then mixed for 1 minute at 850rpm using and Eppendorf Thermomixer (the mixing speed was the same throughout the process). Twenty micro liters of magnetic beads and Lysis/Binding Enhancer mixture were added into cell-lysate and mixed for 5 minutes. Magnetic beads were captured using magnetic stand and the supernatant was removed without disturbing the beads. After removing supernatant, magnetic beads were washed with Wash Solution 1 (isopropanol added) and mixed for 1 minute. Beads were capture again and supernatant removed. Beads were then washed with 150μl Wash Solution 2 (Ethanol added), captured and supernatant was removed. 50μl of DNase mixture (MagMax turbo DNase Buffer and Turbo DNase) was then added to the beads and they were mixed for 10 to 15 minutes. After mixing, 100μl of RNA Rebinding Solution was added and mixed for 3 minutes. Supernatant was removed and magnetic beads were washed again with 150μl Wash Solution 2 and mixed for 1 minute and supernatant was removed completely. The magnetic beads were mixed for 2 minutes to dry before RNA was eluted with 50μl of water.

cDNA synthesis using ABI High capacity cDNA reverse transcription kit (Applied Biosystems, Foster City, CA, Cat #4368813): A master mix of 2μl 10X Buffer, 0.8μl 25X dNTPs, 2μl Random primers, 1μl Reverse Transcriptase, 1μl RNase inhibitor and 3.2μl of H2O per reaction were added into 10μl total RNA. cDNA was generated using a Bio-Rad C-1000 or S-1000 thermal cycler (Hercules, CA) through the following steps: 25°C 10 min, 37°C 120 min, 85°C 5 sec, 4°C hold.

Real time PCR: 2 μl of cDNA were added to a master mix containing 0.5μl GAPDH TaqMan Probe (Applied Biosystems Cat # 4326317E), 0.5μl TTR TaqMan probe (Applied Biosystems cat #HS00174914 M1) and 10μl Roche Probes Master Mix (Roche Cat # 04887301001) per well in a LightCycler 480 384 well plate (Roche cat # 0472974001). Real time PCR was done in a LightCycler 480 Real Time PCR machine (Roche). Each duplex was tested in two independent transfections and each transfection was assayed in duplicate.

Real time data were analyzed using the ΔΔCt method. Each sample was normalized to GAPDH expression and knockdown was assessed relative to cells transfected with the non-targeting duplex AD-1955. Table 14 shows the knockdown of TTR using the siRNAs. Data are expressed as the percent of message remaining relative to cells targeted with AD-1955.

Many but not all tiled TTR-dsRNAs, targeting TTR near the target of AD-18328, reduced TTR mRNA by at least 70% when transfected into Hep3B cells at 0.1 nM.

Table 14: Inhibition of TTR by tiled dsRNA targeting TTR near target of AD-18328.

| Duplex # | % message remaining 0.1nM | % SD 0.1nM | % message remaining 10nM | % SD 10nM |
|---|---|---|---|---|
| AD-18323 | 6.7 | 1.90 | 1.7 | 0.02 |
| AD-18324 | 1.8 | 0.58 | 0.9 | 0.10 |
| AD-23000 | 5.5 | 0.93 | 2.1 | 0.87 |
| AD-23001 | 15.2 | 4.89 | 4.9 | 1.74 |
| AD-23002 | 3.1 | 1.12 | 1.4 | 0.55 |
| AD-23003 | 17.3 | 3.13 | 1.7 | 0.06 |
| AD-18325 | 1.5 | 0.27 | 1.4 | 0.66 |
| AD-23004 | 9.0 | 0.15 | 10.5 | 0.96 |
| AD-18326 | 22.0 | 1.85 | 7.6 | 0.78 |
| AD-18327 | 11.6 | 2.64 | 9.6 | 1.67 |

(continued)

| Duplex # | % message remaining 0.1nM | % SD 0.1nM | % message remaining 10nM | % SD 10nM |
|---|---|---|---|---|
| AD-18328 | 1.1 | 0.70 | 0.6 | 0.16 |
| AD-23005 | 0.8 | 0.31 | 0.6 | 0.21 |
| AD-23006 | 1.5 | 0.46 | 1.2 | 0.43 |
| AD-23007 | 2.4 | 0.91 | 1.9 | 0.46 |
| AD-23008 | 0.6 | 0.10 | 0.8 | 0.26 |
| AD-23009 | 1.0 | 0.13 | 0.9 | 0.22 |
| AD-23010 | 60.1 | 15.66 | 66.2 | 22.71 |
| AD-23011 | 56.5 | 16.99 | 53.6 | 4.70 |
| AD-23012 | 7.7 | 2.36 | 7.7 | 3.25 |
| AD-23013 | 7.0 | 0.64 | 8.0 | 1.06 |
| AD-23014 | 0.7 | 0.01 | 0.6 | 0.10 |
| AD-23015 | 15.4 | 0.25 | 16.5 | 7.07 |
| AD-23016 | 27.1 | 0.37 | 6.7 | 1.80 |
| AD-23017 | 4.5 | 1.26 | 1.4 | 0.40 |
| AD-23018 | 44.6 | 9.45 | 7.5 | 1.09 |
| AD-23019 | 2.2 | 0.68 | 0.8 | 0.10 |
| AD-23020 | 52.7 | 6.45 | 29.7 | 1.17 |
| AD-23021 | 95.4 | 16.16 | 45.0 | 3.00 |
| AD-23022 | 70.1 | 3.01 | 60.8 | 12.11 |
| AD-23023 | 2.7 | 1.12 | 1.8 | 0.07 |
| AD-23024 | 1.7 | 0.30 | 1.8 | 0.33 |
| AD-23025 | 64.2 | 13.21 | 10.5 | 1.34 |
| AD-23026 | 1.9 | 0.15 | 1.9 | 0.78 |
| AD-23027 | 2.5 | 0.21 | 1.6 | 0.49 |
| AD-23028 | 6.7 | 4.41 | 1.2 | 0.50 |
| AD-18330 | 6.0 | 0.56 | 5.7 | 1.15 |
| AD-23029 | 4.5 | 0.47 | 1.6 | 0.10 |
| AD-23030 | 3.9 | 0.25 | 3.3 | 0.84 |
| AD-23031 | 3.4 | 0.78 | 1.7 | 0.02 |

**Example 15. Evaluation of Infusion Duration on Efficacy of a Single Intravenous Administration of SNALP-18534 in Sprague-Dawley Rats**

Objectives

To determine the effect of infusion duration on efficacy of a single IV infusion of SNALP-18534 on liver TTR mRNA levels in Sprague-Dawley rats.

Table 15: Abbreviations and definitions used

| | |
|---|---|
| SNALP-18534 | Rodent transthyretin specific siRNA formulated in SNALP |
| SNALP-1955 | Non-mammalian luciferase specific siRNA formulated in SNALP |

The sequences of the sense and antisense strands of AD-18534 are reproduced below from the tables above:

| Strand | Oligo # | Position | Sequence 5' to 3' | SEQ ID NO: |
|---|---|---|---|---|
| s | A-32755 | 532 | cAGuGuucuuGcucuAuAAdTdT | 1289 |
| as | A-32756 | 550 | UuAuAGAGcAAGAAcACUGdTdT | 1290 |

Study Materials

Test Article(s)

SNALP-18534 is comprised of an siRNA targeting rodent TTR mRNA (AD-18534), formulated in stable nucleic acid lipid particles (SNALP) for delivery to target tissues. The SNALP formulation (lipid particle) consists of a novel aminolipid (DLinDMA), a PEGylated lipid (mPEG2000-C-DMA), a neutral lipid (DPPC) and cholesterol. The ratio of lipid:nucleic acid in the SNALP formulation is approximately 5.8:1 (w:w). SNALP-1955 contains an siRNA targeting the non-mammalian luciferase mRNA, is formulated with the identical lipid particle as SNALP-18534, and serves as a non-pharmacologically active control. Dose levels are expressed as mg/kg based on the weight of siRNA content.

Study Design & Procedures

Animals and test article administration:

The study was comprised of 9 groups of Sprague-Dawley rats (4 males/ group). The animals were allowed to have at least a 2 day acclimation period before the study and all animals were 7 weeks old at the initiation of dosing. The dose administered was calculated based upon body weight data collected prior to dosing on Day 1. The test and control articles were administered as a single 15-minute, 1-hour, 2-hour, or 3-hour IV infusion via the tail vein using a 24G ¾" cannula sealed with a Baxter Injection Site septum connected via 27G Terumo butterfly needle to a Baxter AS40A Syringe Pump. The dose volume was 3 ml/kg, the infusion rate was 12 ml/kg/hr, and animals were freely moving in the cages during dosing. Rats were divided into nine treatment groups and administered a single IV infusion of SNALP-18534, SNALP-1955, or PBS as shown in Table 16:

Table 16: Test Animal Dosage Groups

| Group | N | Test Article | Infusion Duration | Dose |
|---|---|---|---|---|
| A | 4 | PBS | 15 minute | --- |
| B | 4 | PBS | 3 hour | --- |
| C | 4 | SNALP -1955 | 1 hour | 1 mg/kg |
| D | 4 | SNALP -1955 | 2 hour | 1 mg/kg |
| E | 4 | SNALP -1955 | 3 hour | 1 mg/kg |
| F | 4 | SNALP-18534 | 15 minute | 1 mg/kg |
| G | 4 | SNALP-18534 | 1 hour | 1 mg/kg |
| H | 4 | SNALP-18534 | 2 hour | 1 mg/kg |
| I | 4 | SNALP-18534 | 3 hour | 1 mg/kg |

Tissue collection and RNA isolation:

On Day 0, animals were anesthetized by isofluorane inhalation and pre-dosing blood samples were collected into serum separator tubes by retro-orbital bleed. The blood samples were allowed to clot at room temperature for approximately 30 minutes prior to centrifugation at 4°C. Serum samples were then stored at -80°C until analysis was performed. On Day 3, animals in all nine treatment groups were given a lethal dose of ketamine/xylazine. Blood was collected via caudal vena cava into serum separation tubes, and then allowed to clot at room temperature for approximately 30 minutes prior to centrifugation at 4°C. Serum samples were stored at - 80°C until analysis was performed. Liver tissue was harvested and snap frozen on dry ice. Frozen liver tissue was ground and tissue lysates were prepared for liver

mRNA quantitation.

TTR mRNA Quantitation:

TTR mRNA levels relative to those of GAPDH mRNA were determined in the lysates by using a branched DNA assay (QuantiGene Reagent System, Panomics, Fremont, CA). Briefly, the QuantiGene assay (Genospectra) was used to quantify mRNA levels in tissue sample lysates according to the manufacturer's instructions. The mean level of TTR mRNA was normalized to the mean level of GAPDH mRNA for each sample.

To obtain the relative level of TTR mRNA expression, group mean values for SNALP-1955 and SNALP-18534 treated groups with 15-minute, 1 hour and 2 hour infusion durations were then normalized to the mean value for the PBS treated group with 15-minute infusion whereas group mean values for SNALP-1955 and SNALP-18534 treated groups with 3 hour infusion duration were then normalized to the mean value for the PBS treated group with 3 hour infusion duration.

Results

As shown in FIG. 16, a single IV infusion of 1 mg/kg SNALP-18534 with different infusion durations of 15 minutes to 3 hours results in comparable inhibition of liver TTR mRNA levels measured two days after dosing. A single IV infusion of 1 mg/kg SNALP-18534 also showed durable TTR downregulation over 29 days following a single 15 minute IV infusion, as compared to SNALP-1955 control (data not shown). Compared to the PBS-treated group, a single 15-minute, 1-hour, 2-hour, or 3-hour IV infusion of SNALP-18534 at 1 mg/kg significantly reduced relative TTR mRNA expression levels by 94% ($p<0.001$), 94% ($p < 0.001$), 92% ($p < 0.001$) and 93% ($p < 0.001$), respectively. Specificity of SNALP-18534 activity is demonstrated by lack of significant target inhibition by SNALP-1955 administration via 1-hour, 2-hour, or 3-hour IV infusion at the same dose level.

Conclusions

This study demonstrates that varying the infusion duration from 15 minutes to up to 3 hours does not affect the efficacy of a single IV administration of 1 mg/kg SNALP-18534 in rats, as assessed by reduction of TTR mRNA levels in the liver.

**Example 16. *In vivo* reduction of wild-type TTR mRNA in the rat liver by LNP07-18534 and LNP08-18534**

To evaluate the efficacy of 2 novel lipid nanoparticle formulations, LNP07 and LNP08, for delivery of siRNA in the rat, the rodent-specific TTR siRNA, AD-18534, was formulated in LNP07 (LNP07-18534) or LNP08 (LNP08-18534), and administered by 15-minute IV infusion, and liver TTR mRNA was quantified. Sprague-Dawley rats (4 animals per group) were administered 15-minute IV infusions of LNP07-18534 (0.03, 0.1, 0.3 or 1 mg/kg), LNP08-18534 (0.01, 0.03 or 0.1 mg/kg), or LNP07-1955 (1 mg/kg) or LNP08-1955 (0.1 mg/kg) containing the negative control siRNA AD-1955 which targets the non-mammalian gene luciferase. Forty-eight hours later, animals were euthanized and liver tissue was collected, flash-frozen and stored at -80°C until processing.

For TTR mRNA quantitation, frozen liver tissue was ground into powder, and lysates were prepared. TTR mRNA levels relative to those of GAPDH mRNA were determined in the lysates by using a branched DNA assay (QuantiGene Reagent System, Panomics, Fremont, CA). Briefly, the QuantiGene assay (Genospectra) was used to quantify mRNA levels in tissue sample lysates according to the manufacturer's instructions. The mean level of TTR mRNA was normalized to the mean level of GAPDH mRNA for each sample. Group means of the normalized values were then further normalized to the mean value for the PBS treated group, to obtain the relative level of TTR mRNA expression.

The results are shown in FIG. 17. LNP07-18534 reduced TTR mRNA levels in the liver in a dose-dependent manner, with 94% suppression of TTR mRNA at 1 mg/kg. The effect was specific, since the negative control LNP07-1955 at 1 mg/kg did not significantly affect TTR mRNA levels compared to the PBS control. The mRNA ED50 was determined to be ~ 0.05 mg/kg LNP07-18534. LNP08-18534 reduced TTR mRNA levels in the liver in a dose-dependent manner, with 86% suppression of TTR mRNA at 0.1 mg/kg. The effect was specific, since the negative control LNP08-1955 at 0.1 mg/kg did not significantly affect TTR mRNA levels compared to the PBS control. The mRNA ED50 was determined to be ~ 0.02 mg/kg LNP08-18534.

These results demonstrate that LNP07-18534 and LNP08-18534 are effective in suppressing wild-type TTR mRNA in the rat liver when administered by IV infusion, and that LNP07 and LNP08 are effective formulations for delivering siRNA to the liver.

**Example 17: Reduction of TTR liver mRNA by a single intravenous administration of LNP09-18534 or LNP11-18534 in Sprague-Dawley Rats**

Objective:

To evaluate the efficacy of two novel lipid nanoparticle (LNP) formulations for delivery of the rodent TTR-specific siRNA, AD-18534 in the Sprague-Dawley rat for reducing endogenous (wild type) liver TTR mRNA levels. Rats were intravenously dosed via a 15 minute infusion with either 0.01, 0.03, 0.1, or 0.3 mg/kg LNP09-18534, LNP11-18534, or phosphate buffered saline (PBS) and TTR liver mRNA levels were assayed at 48 hrs post-treatment.

Material and Methods:

LNP09 formulation: (XTC/DSPC/Chol/$PEG_{2000}$-C14) = 50/10/38.5/1.5 mol%; Lipid:siRNA ~ 11:1. LNP11 formulation: (MC3/DSPC/Chol/$PEG_{2000}$-C14) = 50/10/38.5/1.5 mol%; Lipid:siRNA ~ 11.1

Tissue collection and RNA isolation: On Day 3, animals in all treatment groups were given a lethal dose of ketamine/xylazine. Blood was collected via caudal vena cava into serum separation tubes, and then allowed to clot at room temperature for approximately 30 minutes prior to centrifugation at 4°C. Serum samples were stored at -80°C until for future analysis. Liver tissues were harvested and snap frozen on dry ice. Frozen liver tissue was ground and tissue lysates were prepared for liver mRNA quantitation.

TTR mRNA Quantitation: TTR mRNA levels relative to those of GAPDH mRNA were determined in the lysates by using a branched DNA assay (QuantiGene Reagent System, Panomics, Fremont, CA). Briefly, the QuantiGene assay (Genospectra) was used to quantify mRNA levels in tissue sample lysates according to the manufacturer's instructions. The mean level of TTR mRNA was normalized to the mean level of GAPDH mRNA for each sample. Group mean values were then normalized to the mean value for the PBS treated group, to obtain the relative level of TTR mRNA expression.

Results:

As shown in FIG. 18, in contrast with PBS treated animals, LNP09-18534 and LNP11-18534 treated animals had a significant dose-dependent decrease in TTR mRNA levels in the liver, reaching maximum reduction of ~ 90% mRNA reduction for both LNP09 and LNP11 formulated groups, relative to PBC control group at 0.3 mg/kg, and a dose achieving 50% reduction ($ED_{50}$) of < 0.03 mg/kg for LNP11-18534 and < 0.1 mg/kg for LNP09-18534.

Conclusions

This study demonstrates that a single 15 minute IV infusion of LNP09-18534 or LNP11-18534 in Sprague-Dawley rats results in a dose-dependent reduction of liver TTR mRNA. These data demonstrate the efficacy of LNP09-18534 and LNP11-18534 in reducing endogenously expressed (wild type) TTR mRNA with ED50 levels of <0.03 and <0.1 mg/kg for LNP11-18534 and LNP09-18534, respectively.

**Example 18: Assaying for toxicity in animals**

ALN-TTR01 was assayed for safety and toxiclogoy under non-GLP and GLP conditions. ALN-TTR01 is the siRNA AD-18328 in a SNALP formulation (DLinDMA / DPPC/ Cholesterol/ PEG2000-cDMA (57.1/7.1/34.4/1.4) lipid:siRNA ~ 7). Assays were performed in Cynomolgus monkey (1, 3, and 10 mg/kg) and Sprague- Dawley Rat (0.3, 1, 3, and 6 mg/kg). No toxicity of ALN-TTR01 was found at ≤ 1mg/kg in rats and ≤ 3 mg/kg in NHP. (data not shown).

**Example 19: Drug product ALN-TTR01**

The drug product, ALN TTR01 Injection, is a white to off-white, homogeneous sterile liquid suspension of the siRNA ALN-18328 with lipid excipients (referred to as stable nucleic acid lipid particles [SNALP]) in isotonic phosphate buffered saline. The composition of ALN TTR01 is shown in the table below.

Table 17: Composition of drug product ALN-TTR01

| Component, grade | Concentration (mg/mL) | Per vial (mg) | Function |
|---|---|---|---|
| ALN-18328, cGMP | 2.0 | 11.0 | Active ingredient |

(continued)

| Component, grade | Concentration (mg/mL) | Per vial (mg) | Function |
|---|---|---|---|
| DLinDMA (1,2-Dilinoleyloxy-N,N-dimethyl-3-aminopropane), cGMP | 7.3 | 40.2 | Novel excipient; titratable aminolipid for interaction with the active ingredient |
| $PEG_{2000}$-C-DMA (3-N-[(ω-Methoxy poly(ethylene glycol) 2000) carbamoyl]-1,2-dimyristyloxy-propylamine), cGMP | 0.8 | 4.4 | Novel excipient; stability of drug product and desired biodistribution |
| DPPC (R-1,2-Dipalmitoyl-sn-glycero-3-phosphocholine), cGMP | 1.1 | 6.1 | Structural integrity of SNALP particles |
| Cholesterol, synthetic, cGMP | 2.8 | 15.4 | Structural integrity of SNALP particles |
| Phosphate buffered saline, cGMP | q.s. | to 5.5 mL | Buffer |

The lipid excipients have the molecular weights and structures shown in the table below.

Table 18: Lipid excipients

| Lipid | Molecular Weight | Chemical Name and Structure |
|---|---|---|
| DLinDMA | 616 | 1,2-Dilinoleyloxy-*N,N*-dimethyl-3-aminopropane 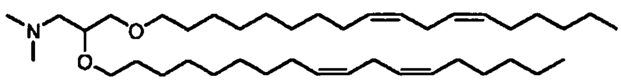 |
| $PEG_{2000}$-CDMA[a] | 2824<br>Polydispersity index 1.01 | 3-N-[(ω-Methoxy poly(ethylene glycol)2000)carbamoyl]-1,2-dimyristyloxy-propylamine 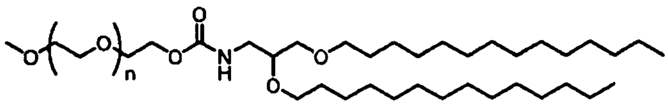 |
| DPPC | 734 | 1,2-Dipalmitoyl-sn-glycero-3-phosphocholine 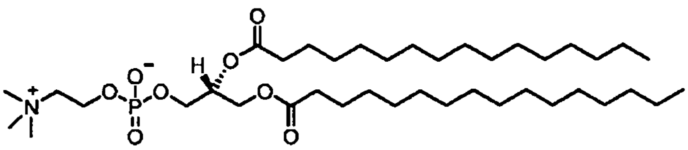 |
| Cholesterol | 387 | Cholest-5-en-3β-ol 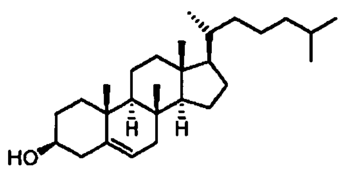 |
| • a alternate name: $mPEG_{2000}$-C-DMA | | |

The ALN TTR01 drug product is packaged in 10 mL glass vials with a fill volume of 5.5 mL (11 mg ALN-18328 per vial). The container closure system consists of a USP/EP Type I borosilicate glass vial, a teflon faced butyl rubber stopper and an aluminum flip off cap. The drug product will be stored at 5 ± 3°C.

Stability of the drug product is assayed for up to 24 months and determined using the following criteria:

Appearance: White to off-white, homogeneous opalescent liquid, no foreign particles

pH: 6.8 - 7.8

Osmolality: 250 - 350 mOsm/kg

Lipid: siRNA Ratio:5.6 - 8.4 mg/mg

Particle Size (Z-Average):60 - 120 nm ≤0.15.

**Example 20: In vitro reduction of human TTR mRNA expression by AD-18324 in ARPE 19 cells**

To determine the effect of TTR siRNA on TTR mRNA expression *in vitro*, the siRNA AD-18324 and AD-18534 were tested in human retinal pigment epithelium (ARPE-19) cells.
AD-18324 is a human TTR siRNA duplex and AD-18534 is a rat TTR siRNA duplex. The sequences of the sense and antisense strands of AD-18534 and AD-18324 are reproduced below:

| **Duplex #** | Strand | Oligo # | Position | Sequence 5' to 3' | SEQ ID NO: |
|---|---|---|---|---|---|
| AD-18534 | s | A-32755 | 532 | cAGuGuucuuGcucuAuAAdTdT | 1411 |
| | as | A-32756 | 550 | UuAuAGAGcAAGAAcACUGdTdT | 1412 |
| AD-18324 | s | A-32337 | 509 | GGAuuucAuGuAAccAAGAdTdT | 1413 |
| | as | A-32338 | 527 | UCUUGGUuAcAUGAAAUCCdTdT | 1414 |

A control siRNA was AD-1955 targeting a LUC gene.
ARPE-19 cells were transfected with siRNA using Lipofectamin 2000 (Invitrogen). In some examples of this method, other transfection agents may be used, including cholesterol or aterocollagen. After incubation for 24 hrs, 50~60% confluent ARPE-19 cells were transiently transfected with AD-18534 or AD-18324 following manufacturer's instruction. Total RNA was isolated for real-time quantitative PCR 48 hrs after the start of transfection. ARPE-19 cells were dosed with 1nM, 10nM or 50nM of AD-18534 or with 1 nM, 10 nM or 50 nM of AD-18324.
TTR mRNA expression was measured by real-time quantitative PCR. Total RNA was isolated from transfected cells by using RNeasy Mini Kit (Qiagen). Total RNA (0.5 μg) was reverse-transcribed to cDNA by using ExScript RT reagent (Takara Bio Inc.) according to the manufacturer's protocol. Each PCR was performed with 2 μL of the cDNA and 0.2 μmol/L of each primer in a LightCycler System with SYBR Premix DimerEraser (Takara Bio Inc.). The following primers were used: human TTR (forward: 5'- CATTCTTGGCAGGATGGCTTC -3' (SEQ ID NO:1415); reverse: 5'-CTCCCAGGTGTCATCAGCAG -3' (SEQ ID NO:1416). Human TTR mRNA expression was calculated relative to human GAPDH expression levels in the ARPE-19 cells.
Human TTR mRNA expression was markedly reduced by AD-18324 in a dose dependent manner. The results are shown in FIG. 22. A 1 nM dose of AD-18324 resulted in at least 10% reduction in human TTR mRNA relative expression compared to a control siRNA group. A 10 nM dose of AD-18324 resulted in at least 40% reduction in human TTR mRNA relative expression compared to the control siRNA group. A 50nM dose of AD-18324 resulted in at least 60% reduction in human TTR mRNA relative expression compared to the control siRNA group. AD-18534 did not cause a marked reduction in human TTR relative expression at each dose. There was no effect on Il-6 or TNF-alpha levels compared to controls.
These results demonstrate that AD-18324 is effective in inhibiting human TTR mRNA expression in a human retinal pigment epithelium (ARPE-19) cell in a dose dependent manner and does not produce an inflammatory response.

**Example 21: In vivo reduction of Endogenous rat TTR mRNA expression by AD-18534 in Dark Agouti (DA) rats**

To determine the effect of rat TTR siRNA on endogenous rat TTR mRNA expression, the duplex AD-18534 was tested *in vivo* in Dark Agouti (DA) rats.
The sequences of the sense and antisense strands of AD-18534 are described above.
DA rats were injected with AD-18534 in their vitreous cavities. Adult rats were anesthetized by diethyl ether inhalation. To dilate the pupils, 1-2 drops of 1% tropicamide were applied to the rat's eyes. Intravitreal injections of

siRNAs were made using Hamilton syringes and a 33 gauge needle. Injected volume was 5 μl so that vitreal volume is kept as close to normal as possible. After 24 hrs, the rat was sacrificed by diethyl ether inhalation and the eyes were harvested for subsequent dissection. The eyes were separated the cornea and lens to get the posterior cups. The RPE-choroid-sclera complexes were isolated by removing the retina from the posterior cups for analysis. Other methods may be used to optimize the siRNA delivery, including varying the amount of dose or timing of the dose. The injection method may occur in another part of the eye, including the subconjunctival space or the subretina. The amount of injected saline or siRNA may be increased.

Rat TTR mRNA expression was measured by real-time quantitative PCR (qPCR). Total RNA was isolated from each RPE-choroid-sclera complexes by using RNeasy Mini Kit (Qiagen). Total RNA was reverse-transcribed to cDNA by using ExScript RT reagent (Takara Bio Inc.). Each PCR was done in a LightCycler System with SYBR Premix DimerEraser (Takara Bio Inc.). The following primers were used: rat TTR (forward: 5'-TGCCTCGCTGGACTGATATTTG -3' (SEQ ID NO:1417); reverse: 5'-TTGAACACTTTCACGGCCACA -3' (SEQ ID NO:1418)). Rat TTR mRNA expression was calculated relative to rat GAPDH expression levels.

FIG. 23 shows the inhibition of endogenous rat TTR mRNA expression in DA rats after injection with AD-18534, compared to DA rats that were administered a control siRNA, saline, or no treatment ($p<0.01$). DA rats treated with AD-18354 exhibited a reduction in endogenous rat TTR mRNA expression by at least 60% relative to the control siRNA group and the saline control group ($p<0.01$).

These results demonstrate that AD-18354 is active in suppressing endogenous rat TTR mRNA expression in the retinal pigment epithelium cells of DA rats.

## Example 22: In vivo reduction of ATTR mRNA expression by AD-18324 in ATTR V30M Transgenic (Tg) rats

To evaluate the effect of TTR siRNA AD-18324 on human mutant (V30M) ATTR mRNA expression, AD-18324 was tested *in vivo* in retinal pigment epithelium cells of ATTR V30M Transgenic (Tg) rats.

Transgenic rats possessing a human ATTR V30M gene were injected with AD-18324 in their vitreous cavities. Intravitreal injections of AD-18324 siRNA were made using Hamilton syringes and 33 gauge needle. After 24 hrs, the ATTR V30M Tg rats were sacrificed by diethyl ether inhalation and the eyes were harvested for subsequent dissection. The eyes were separated the cornea and lens to get the posterior cups. The RPE-choroid-sclera complexes were isolated by removing the retina from the posterior cups to evaluate the effect of AD-18324 on ATTR mRNA expression. AD-18324 siRNA was injected into the vitreous cavity using a 33 gauge needle. After 24 hours, the retinal pigment epithelium was isolated to evaluate the effect of AD-18324 on ATTR mRNA expression.

ATTR mRNA expression was measured by real-time PCR. Total RNA was reverse-transcribed to cDNA by using ExScript RT reagent (Takara Bio Inc.). Each PCR was done in a LightCycler System with Premix Ex Taq (Takara Bio Inc.). The following primers were used: human TTR (forward: 5'- GCCGTGCATGTGTTCAGA -3' (SEQ ID NO:1419); reverse: 5'-GCTCTCCAGACTCACTGGTTTT -3 (SEQ ID NO: 1420)'). The probe was provided by Universal Probe Library (probe #66, Roche Diagnostics). ATTR mRNA expression was calculated relative to rat GAPDH expression in the ATTR V30M Tg rat.

FIG. 24 shows a significant reduction of ATTR mRNA expression in RPE cells of ATTR V30M Tg rats, compared to the control siRNA group, the saline group, and the no treatment group. ATTR mRNA expression was reduced by at least 60% compared to the no treatment group.

Western blot analysis was used to assess ATTR protein expression. Equal amounts of aqueous humor protein from rats were fractionated via 12% SDS-PAGE and transferred to nitrocellulose membranes (Bio-Rad Laboratories). Membranes were blocked with 2.5% non-fat milk and incubated overnight at 4°C with a primary antibody which was a rabbit polyclonal anti-TTR (dilution 1:1000, Dako), followed by a horseradish peroxidase-conjugated goat anti-rabbit immunoglobulin antibody (dilution 1:1000, Dako) as a secondary reaction for 1 hour at room temperature. The immunocomplex was visualized using the $ECL^+$ western blot detection system (GE Healthcare Bio-Science).

The results are shown in FIG. 25. ATTR protein expression was significantly reduced by AD-18324 compared to ATTR protein expression after injection of a control siRNA.

These results demonstrate that intravitreal injection of AD-18324 in ATTR V30M Tg rats significantly reduces human TTR mRNA and protein expression in the RPE.

## Example 23: In vivo reduction of endogenous rat TTR mRNA expression in Dark Agouti (DA) rats using cholesterol conjugated AD-18534

To determine the effect of cholesterol-conjugated rat TTR siRNA on endogenous rat TTR mRNA expression, the duplex AD-23043 was tested in vivo in Dark Agouti (DA) rats.

AD-23043 is a cholesterol conjugated siRNA with the following sequences.

| Duplex # | Strand | Sequence 5' to 3' | SEQ ID NO: |
|---|---|---|---|
| AD-23043 | sense | cAGuGuucuuGcucuAuAAdTdTsL10 | 1421 |
| | antisense | UuAuAGAGcAAGAAcACUGdTdT | 1422 |

DA rats were injected with AD-23043 in their vitreous cavities. Adult rats were anesthetized by diethyl ether inhalation. To dilate the pupils, 1-2 drops of 1 % tropicamide were applied to the rat's eyes. Intravitreal injections of siRNAs (5 μg) were made using Hamilton syringes and a 33 gauge needle. Injected volume was 5 μl so that vitreal volume is kept as close to normal as possible. After 14 or 21 days, the rat was sacrificed by diethyl ether inhalation and the eyes were harvested for subsequent dissection. The eyes were separated the cornea and lens to get the posterior cups. The RPE-choroid-sclera complexes were isolated by removing the retina from the posterior cups for analysis.

Rat TTR mRNA expression was measured by real-time quantitative PCR (qPCR). Total RNA was isolated from each RPE-choroid-sclera complexes by using RNeasy Mini Kit (Qiagen). Total RNA was reverse-transcribed to cDNA by using ExScript RT reagent (Takara Bio Inc.). Each PCR was done in a LightCycler System with SYBR Premix DimerEraser (Takara Bio Inc.). The following primers were used: rat TTR (forward: 5'-TGCCTCGCTGGACTGATATTTG -3' (SEQ ID NO:1423); reverse: 5'-TTGAACACTTTCACGGCCACA -3' (SEQ ID NO:1424)). Rat TTR mRNA expression was calculated relative to rat GAPDH expression levels.

The results are shown in FIG. 26 and FIG. 27. Cholesterol conjugated siRNA targeting rat TTR reduced endogenous rat TTR expression by about 40% compared to a control siRNA.

**Example 24: Treatment of ocular amyloidosis in human**

For treatment of ocular amyloidosis in humans, the pharmaceutical compositions used herein may be administered in a number of ways depending upon the invasiveness of treatment and based on whether local or systemic treatment is desired. The preferred initial treatment may be performed by ocular instillation, ointment, peroral administration or infusion. Parenteral administration includes subcutaneous eyelid, subconjunctival injection, subtenon injection, retrobulbar injection, anterior chamber injection, intravitreous injection or ophthalmovascular injection.

## Claims

1. A dsRNA for use in a method of treating of TTR-related ocular amyloidosis by reducing TTR expression in a retinal pigment epithelium of a subject, wherein the dsRNA is to be administered to the retina of the subject, and wherein the dsRNA has

   (i) the sense strand GGAuuucAuGuAAccAAGAdTdT and the antisense strand UCUUGGUuAcAUGAAAUCCdTdT,
   (ii) the sense strand cAGuGuucuuGcucuAuAAdTdT and the antisense strand UuAuAGAGcAAGAAcACUGdTdT, or
   (iii) the sense strand cAGuGuucuuGcucuAuAAdTdTL10 and the antisense strand UuAuAGAGcAAgAAcACUGdTdT,

   wherein c is 2'-O-methylcytidine-3'-phosphate, u is 2'-O-methyluridine-3'-phosphate, dT is 2'-deoxythymidine-3'-phosphate and L10 is a conjugated cholesterol.

2. The dsRNA for use of claim 1, wherein the dsRNA according to (i) or (ii) is conjugated to a cholesterol molecule.

3. The dsRNA for use of claim 1, wherein the subject is a human.

4. The dsRNA for use of claim 3, wherein the subject is a human comprising a V30M TTR gene.

5. The dsRNA for use of claim 1, wherein the subject is a Dark Agouti (DA) rat.

6. The dsRNA for use of claim 5, wherein the dsRNA is as defined in item (ii) of claim 1.

7. The dsRNA for use of claim 1, wherein the subject is a transgenic rat possessing a human ATTR V30M gene.

8. The dsRNA for use of claim 3, 5 or 7, wherein TTR expression is reduced in the retinal pigment epithelium (RPE).

9. A dsRNA for use in a method of treating or preventing TTR-related ocular amyloidosis, wherein the dsRNA is to be administered to a patient in need of such treatment or prevention and to the retina of said patient, and wherein the dsRNA is as defined in item (i) of claim 1.

10. A dsRNA for use in a method of treating or preventing TTR-related ocular amyloidosis in a human, wherein the human has been diagnosed as having TTR-related ocular amyloidosis or as having a risk for developing TTR-related ocular amyloidosis and the dsRNA is to be administered to the retina of said human, and wherein the dsRNA is as defined in item (i) of claim 1.

11. An in vitro method for inhibiting TTR expression in a retinal epithelium cell, the method comprising:

(a) introducing into the retinal epithelium cell a dsRNA, wherein the dsRNA is as defined in item (i) or item (ii) of claim 1; and
(b) maintaining the cell produced in step (a) for a time sufficient to obtain degradation of the mRNA transcript of a TTR gene, thereby inhibiting expression of the TTR gene in the cell.

**12.** The dsRNA for use of claim according to claim 3 or 7 or the method of claim 11, wherein the dsRNA is as defined in item (i) of claim 1.

**13.** The method of claim 11, wherein the retinal epithelium cell is a human retinal pigment epithelium transgenic cell.

**14.** The dsRNA for use of claim 3, 5 or 7, or the method of claim 11, wherein TTR expression is inhibited by at least 60%.

**15.** The dsRNA for use of claim 3, 5 or 7, or the method of claim 11, wherein introducing the dsRNA does not result in an inflammatory response as measured by IL-6 or TNF-alpha levels.

## Patentansprüche

**1.** dsRNA zur Verwendung in einem Verfahren zur Behandlung einer mit TTR in Beziehung stehenden okularen Amyloidose durch Vermindern der TTR-Expression im retinalen Pigmentepithel eines Individuums, wobei die dsRNA an die Retina des Individuums zu verabreichen ist und wobei die dsRNA

(i) den Sinnstrang GGAuuucAuGuAAccAAGAdTdT und den Antisinnstrang UCUUGGUuAcAUGAAAUCCdTdT,
(ii) den Sinnstrang cAGuGuucuuGcucuAuAAdTdT und den Antisinnstrang UuAuAGAGcAAGAAcACUGdTdT, oder
(iii) den Sinnstrang cAGuGuucuuGcucuAuAAdTdTL10 und den Antisinnstrang UuAuAGAGcAAgAAcACUGdTdT

hat, wobei c 2'-O-Methylcytidin-3'-phosphat ist, u 2'-O-Methyluridin-3'-phosphat ist, dT 2'-Deoxythymidin-3'-phosphat ist und L10 ein konjugiertes Cholesterin ist.

**2.** dsRNA zur Verwendung nach Anspruch 1, wobei die dsRNA nach (i) oder (ii) an ein Cholesterinmolekül konjugiert ist.

**3.** dsRNA zur Verwendung nach Anspruch 1, wobei das Individuum ein Mensch ist.

**4.** dsRNA zur Verwendung nach Anspruch 3, wobei das Individuum ein Mensch ist, der ein V30M TTR-Gen hat.

**5.** dsRNA zur Verwendung nach Anspruch 1, wobei das Individuum eine Dark Agouti(DA)-Ratte ist.

**6.** dsRNA zur Verwendung nach Anspruch 5, wobei die dsRNA so wie im Merkmal (ii) des Anspruchs 1 definiert ist.

**7.** dsRNA zur Verwendung nach Anspruch 1, wobei das Individuum eine transgene Ratte ist, die ein menschliches ATTR V30M-Gen trägt.

**8.** dsRNA zur Verwendung nach Anspruch 3, 5 oder 7, wobei die TTR-Expression im retinalen Pigmentepithel (RPE) vermindert ist.

**9.** dsRNA zur Verwendung in einem Verfahren zur Behandlung oder Prävention einer mit TTR in Beziehung stehenden okularen Amyloidose, wobei die dsRNA an einen Patienten und an die Retina des Patienten zu verabreichen ist, der eine solche Behandlung oder Verhinderung benötigt und wobei die dsRNA so wie im Merkmal (i) des Anspruchs 1 definiert ist.

**10.** dsRNA zur Verwendung in einem Verfahren zur Behandlung oder Prävention einer mit TTR in Beziehung stehenden okularen Amyloidose in einem Menschen, wobei bei dem Menschen eine mit TTR in Beziehung stehende okulare Amyloidose oder ein Risiko eine mit TTR in Beziehung stehende okulare Amyloidose zu entwickeln diagnostiziert wurde und die dsRNA an die Retina des Menschen zu verabreichen ist und wobei die dsRNA so wie im Merkmal (i) des Anspruchs 1 definiert ist.

11. *In vitro* Verfahren zur Inhibition der TTR-Expression in einer retinalen Epithelzelle, wobei das Verfahren umfasst:

   (a) Einfügen einer dsRNA in die retinale Epithelzelle, wobei die dsRNA so wie im Merkmal (i) oder (ii) des Anspruchs 1 definiert ist, und
   (b) Aufrechterhalten der im Schritt (a) hergestellten Zelle für eine Zeit, die ausreicht, um eine Degradation des mRNA-Transkripts des TTR-Gens zu erhalten, wodurch die Expression des TTR-Gens in der Zelle inhibiert wird.

12. dsRNA zur Verwendung nach Anspruch 3 oder 7 oder Verfahren nach Anspruch 11, wobei die dsRNA so wie im Merkmal (i) des Anspruchs 1 definiert ist.

13. Verfahren nach Anspruch 11, wobei die retinale Epithelzelle eine transgene menschliche retinale Pigment-Epithelzelle ist.

14. dsRNA zur Verwendung nach Anspruch 3, 5 oder 7 oder Verfahren nach Anspruch 11, wobei die TTR-Expression zu mindestens 60% inhibiert ist.

15. dsRNA zur Verwendung nach Anspruch 3, 5 oder 7 oder Verfahren nach Anspruch 11, wobei das Einfügen der dsRNA keine Entzündungsreaktion, wie sie mittels IL-6- oder TNF-alpha-Leveln gemessen wird, zur Folge hat.

## Revendications

1. ARNdb pour une utilisation dans une méthode de traitement de l'amylose oculaire associée à la TTR par réduction de l'expression de la TTR dans un épithélium pigmentaire rétinien d'un sujet, dans lequel l'ARNdb est à administrer à la rétine du sujet, et dans lequel l'ARNdb a

   (i) le brin sens GGAuuucAuGuAAccAAGAdTdT et le brin antisens UCUUGGUuAcAUGAAAUCCdTdT,
   (ii) le brin sens cAGuGuucuuGcucuAuAAdTdT et le brin antisens UuAuAGAGcAAGAAcACUGdTdT, ou
   (iii) le brin sens cAGuGuucuuGcucuAuAAdTdTL10 et le brin antisens UuAuAGAGcAAgAAcACUGdTdT,

   dans lequel c représente un 2'-O-méthylcytidine-3'-phosphate, u représente un 2'-O-méthyluridine-3'-phosphate, dT représente un 2'-désoxythymidine-3'-phosphate et L10 représente un cholestérol conjugué.

2. ARNdb pour une utilisation selon la revendication 1, dans lequel l'ARNdb selon (i) ou (ii) est conjugué à une molécule de cholestérol.

3. ARNdb pour une utilisation selon la revendication 1, dans lequel le sujet est un être humain.

4. ARNdb pour une utilisation selon la revendication 3, dans lequel le sujet est un être humain comprenant un gène de la TTR V30M.

5. ARNdb pour une utilisation selon la revendication 1, dans lequel le sujet est un rat Dark Agouti (DA).

6. ARNdb pour une utilisation selon la revendication 5, dans lequel l'ARNdb est tel que défini au point (ii) de la revendication 1.

7. ARNdb pour une utilisation selon la revendication 1, dans lequel le sujet est un rat transgénique possédant un gène humain de l'ATTR V30M.

8. ARNdb pour une utilisation selon la revendication 3, 5 ou 7, dans lequel l'expression de la TTR est réduite dans l'épithélium pigmentaire rétinien (EPR).

9. ARNdb pour une utilisation dans une méthode de traitement ou de prévention de l'amylose oculaire associée à la TTR, dans lequel l'ARNdb est à administrer à un patient ayant besoin d'un tel traitement ou d'une telle prévention et à la rétine dudit patient, et dans lequel l'ARNdb est tel que défini au point (i) de la revendication 1.

10. ARNdb pour une utilisation dans une méthode de traitement ou de prévention de l'amylose oculaire associée à la TTR chez un être humain, dans lequel l'être humain a été diagnostiqué comme souffrant d'amylose oculaire associée

à la TTR ou comme présentant un risque de développer une amylose oculaire associée à la TTR et l'ARNdb est à administrer à la rétine dudit être humain, et dans lequel l'ARNdb est tel que défini au point (i) de la revendication 1.

**11.** *Méthode in vitro* d'inhibition de l'expression de la TTR dans une cellule de l'épithélium rétinien, la méthode comprenant :

(a) l'introduction dans la cellule de l'épithélium rétinien d'un ARNdb, dans laquelle l'ARNdb est tel que défini au point (i) ou au point (ii) de la revendication 1 ; et
(b) le maintien de la cellule produite dans l'étape (a) pendant un temps suffisant pour obtenir la dégradation du transcrit d'ARNm d'un gène de la TTR, inhibant de cette façon l'expression du gène de la TTR dans la cellule.

**12.** ARNdb pour une utilisation selon la revendication 3 ou 7 ou méthode selon la revendication 11, dans lequel l'ARNdb est tel que défini au point (i) de la revendication 1.

**13.** Méthode selon la revendication 11, dans laquelle la cellule de l'épithélium rétinien est une cellule transgénique de l'épithélium pigmentaire rétinien humain.

**14.** ARNdb pour une utilisation selon la revendication 3, 5 ou 7, ou méthode selon la revendication 11, dans lequel l'expression de la TTR est inhibée d'au moins 60 %.

**15.** ARNdb pour une utilisation selon la revendication 3, 5 ou 7, ou méthode selon la revendication 11, dans lequel l'introduction de l'ARNdb n'entraîne pas de réponse inflammatoire mesurée par des taux d'IL-6 ou de TNF-alpha.

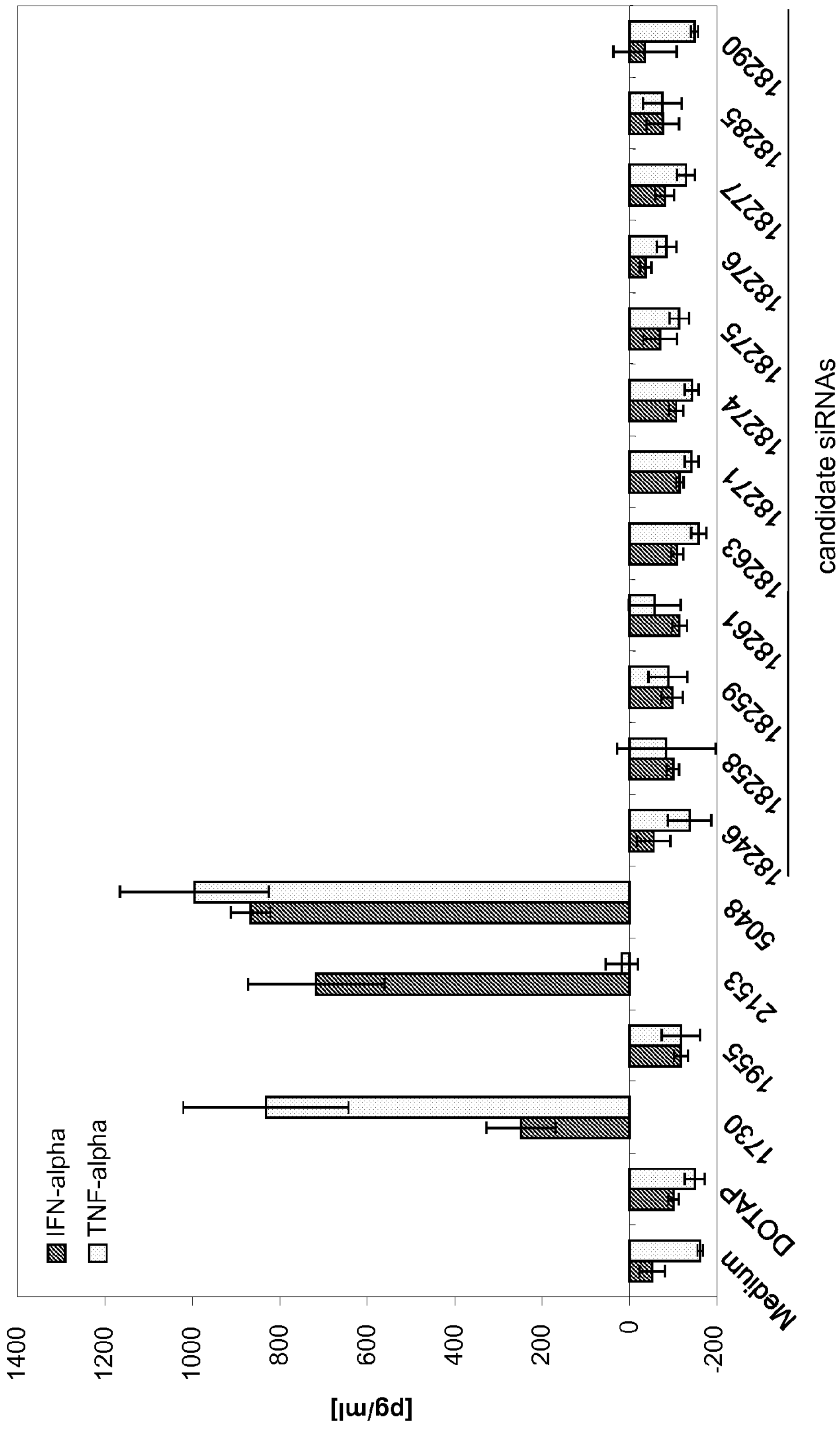
IFN-alpha
TNF-alpha
[pg/ml]
1400
1200
1000
800
600
400
200
0
-200
Medium
DOTAP
1730
1955
2153
5048
18246
18258
18259
18261
18263
18271
18274
18275
18276
18277
18285
18290
candidate siRNAs

FIG. 1

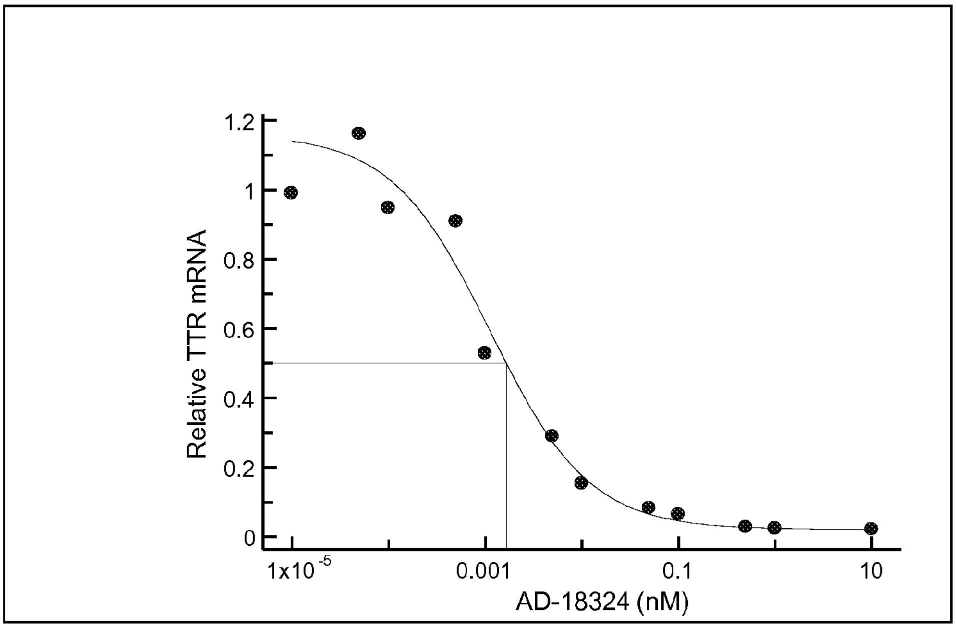
1.2
1
0.8
0.6
0.4
0.2
0
Relative TTR mRNA
1x10$^{-5}$
0.001
0.1
10
AD-18324 (nM)

FIG. 2A

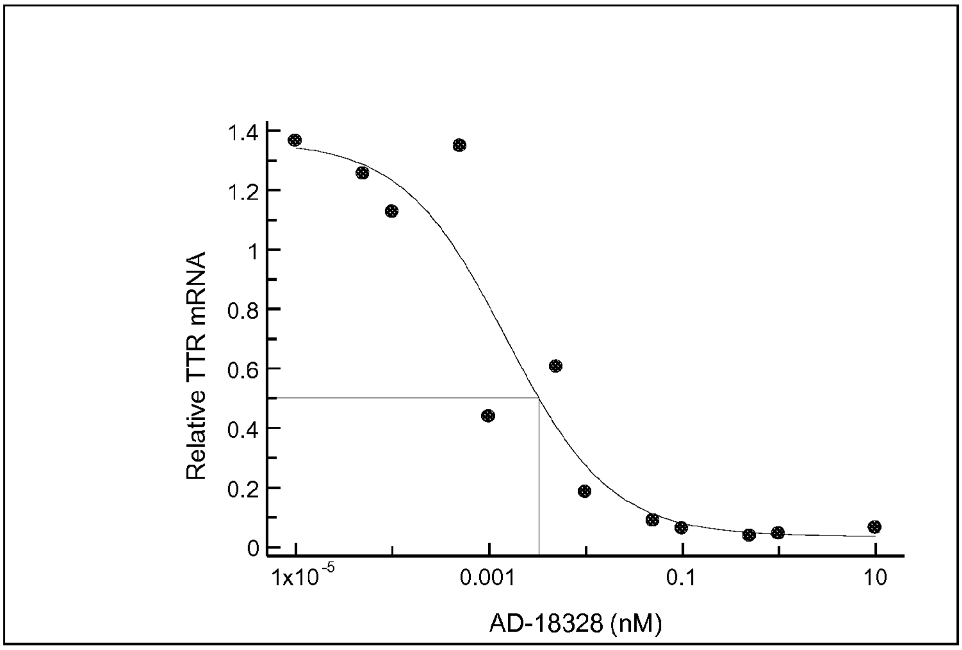
1.4
1.2
1
0.8
0.6
0.4
0.2
0
Relative TTR mRNA
1x10$^{-5}$
0.001
0.1
10
AD-18328 (nM)

FIG. 2B

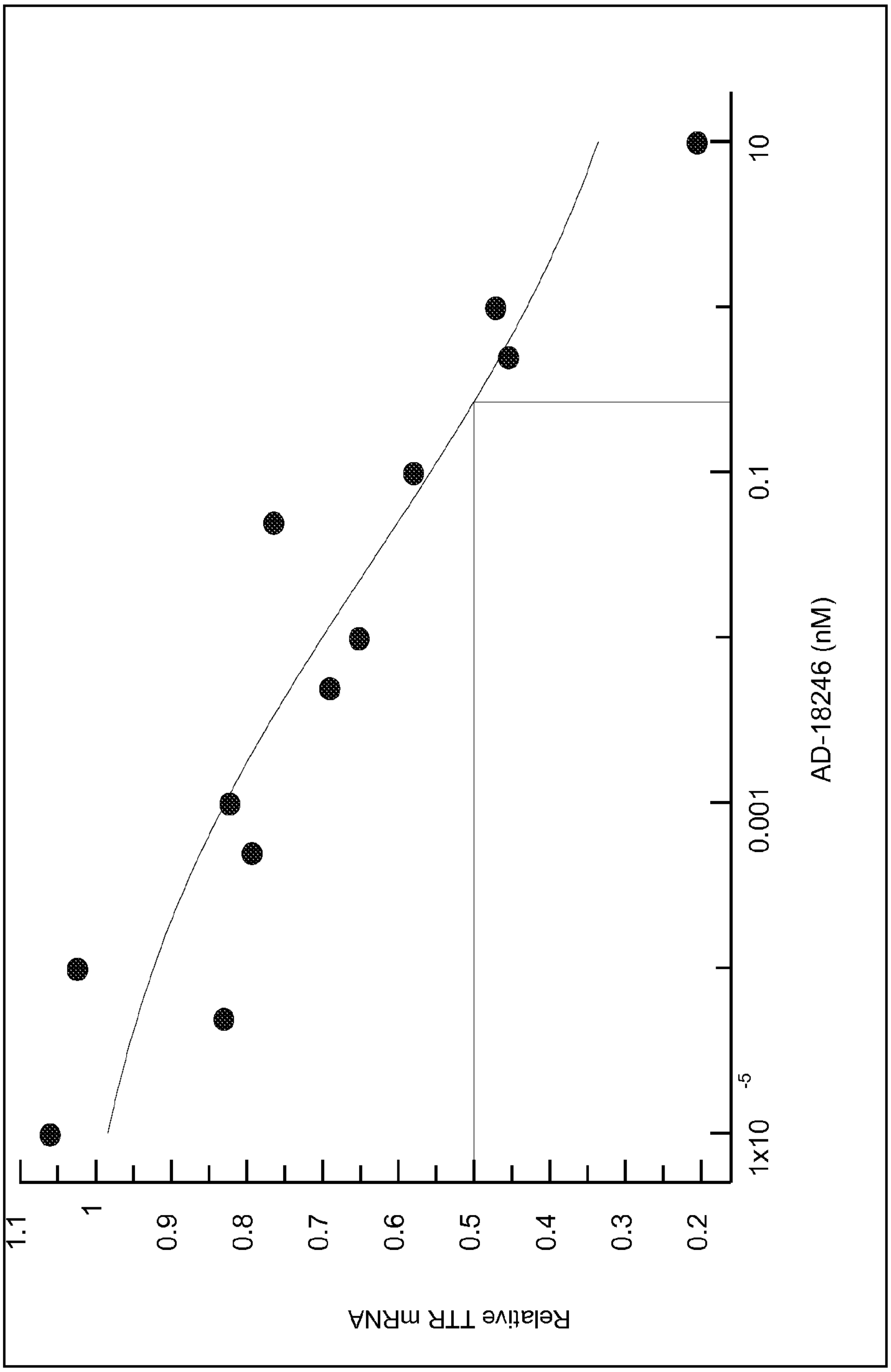
Relative TTR mRNA
1.1
1
0.9
0.8
0.7
0.6
0.5
0.4
0.3
0.2
1x10-5
0.001
0.1
10
AD-18246 (nM)

FIG. 3

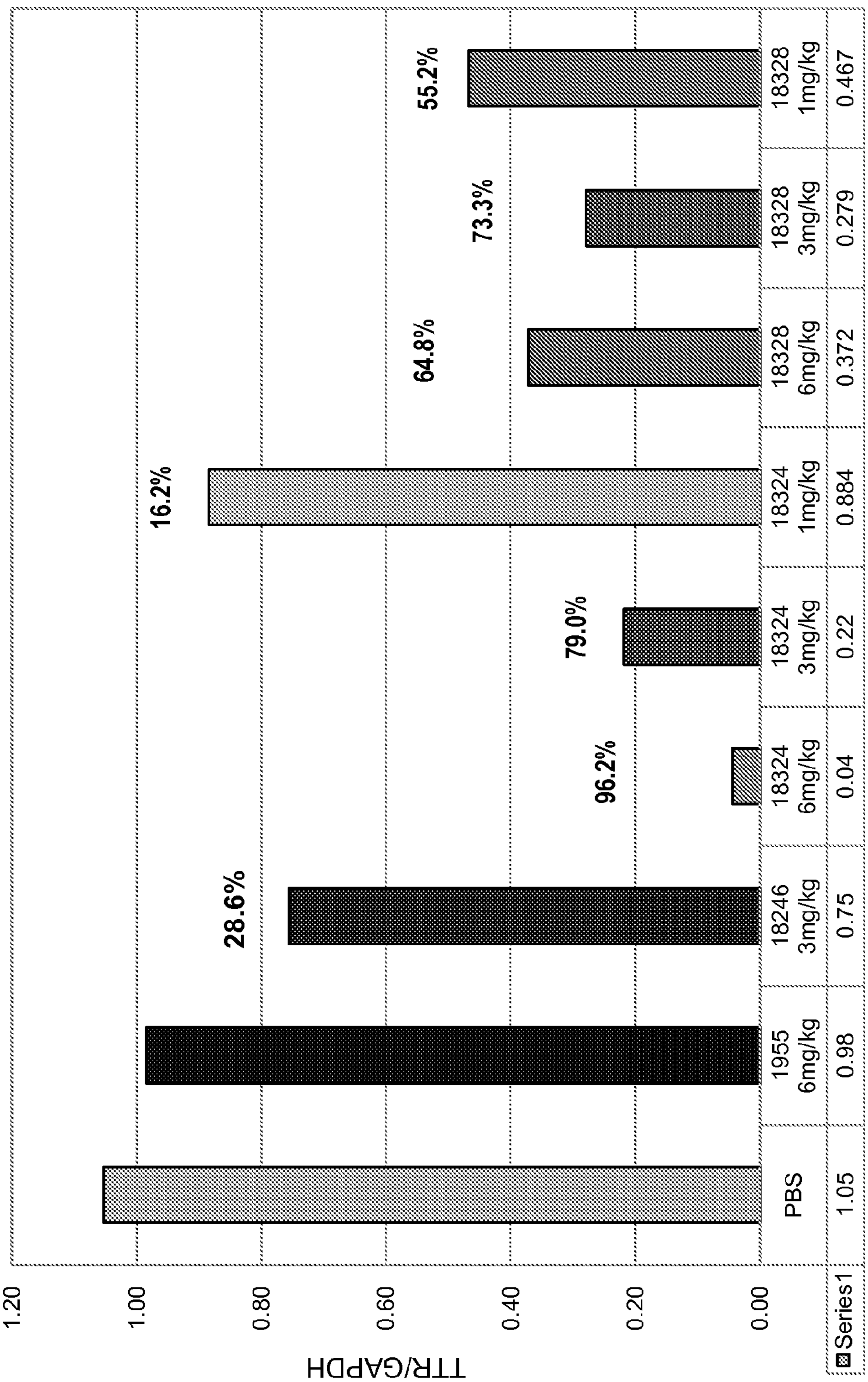
TTR/GAPDH
1.20
1.00
0.80
0.60
0.40
0.20
0.00
Series1
PBS
1.05
1955 6mg/kg
0.98
18246 3mg/kg
0.75
28.6%
18324 6mg/kg
0.04
96.2%
18324 3mg/kg
0.22
79.0%
18324 1mg/kg
0.884
16.2%
18328 6mg/kg
0.372
64.8%
18328 3mg/kg
0.279
73.3%
18328 1mg/kg
0.467
55.2%

FIG. 4A

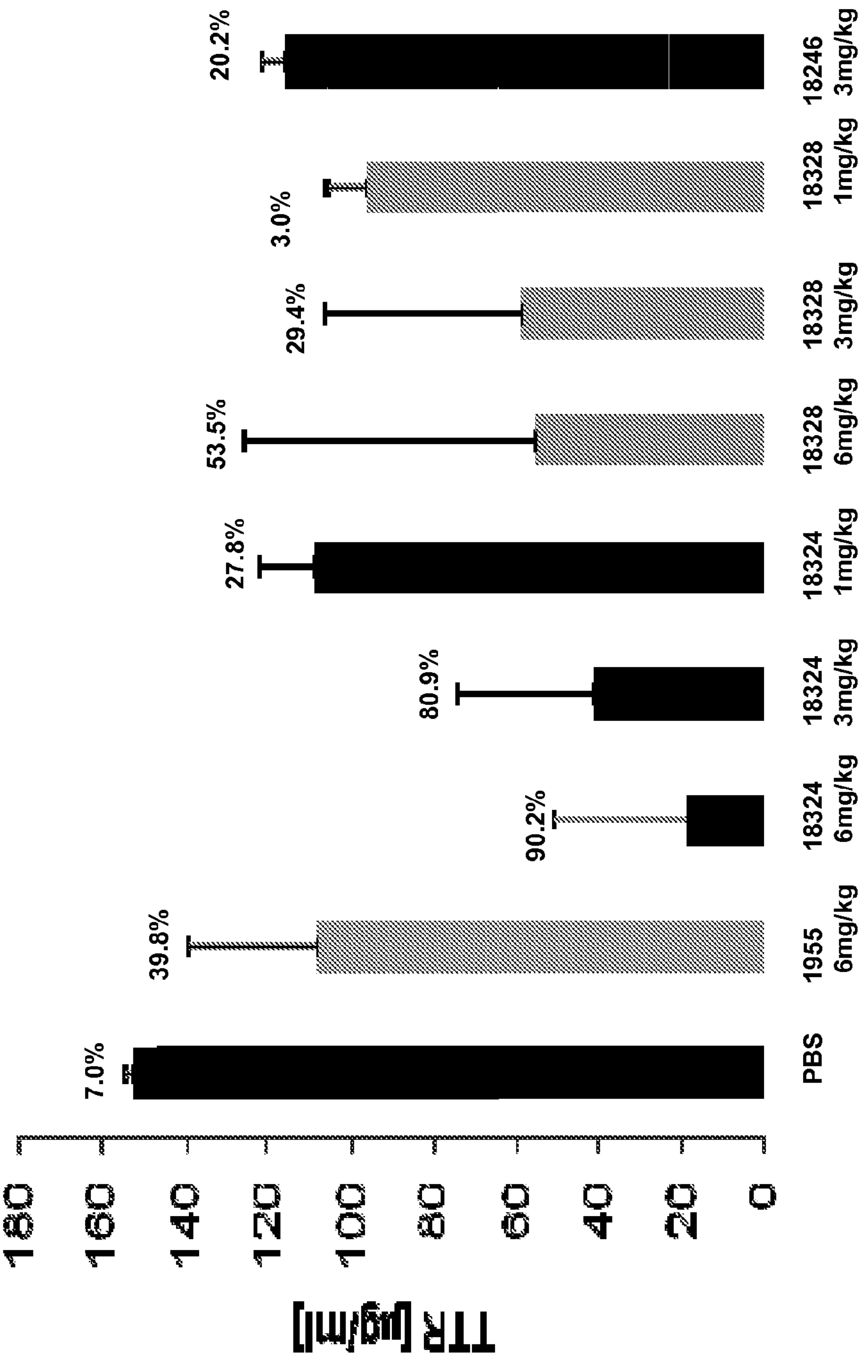
TTR [µg/ml]
180
160
140
120
100
80
60
40
20
0
7.0%
39.8%
90.2%
80.9%
27.8%
53.5%
29.4%
3.0%
20.2%
PBS
1955 6mg/kg
18324 6mg/kg
18324 3mg/kg
18324 1mg/kg
18328 6mg/kg
18328 3mg/kg
18328 1mg/kg
18246 3mg/kg

FIG. 4B

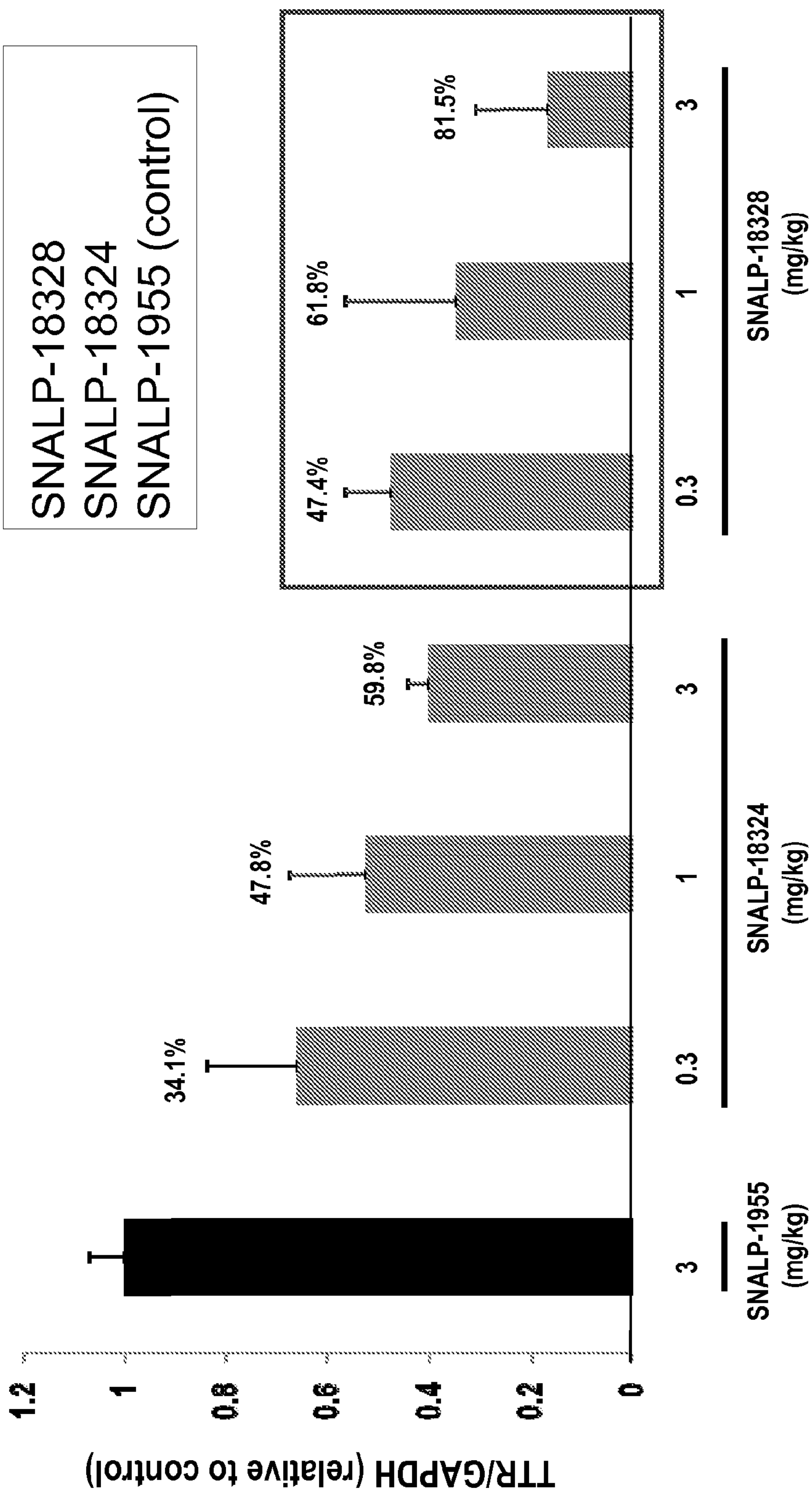
SNALP-18328
SNALP-18324
SNALP-1955 (control)
TTR/GAPDH (relative to control)
1.2
1
0.8
0.6
0.4
0.2
0
34.1%
47.8%
59.8%
47.4%
61.8%
81.5%
3
0.3
1
3
0.3
1
3
SNALP-1955
(mg/kg)
SNALP-18324
(mg/kg)
SNALP-18328
(mg/kg)

FIG. 5

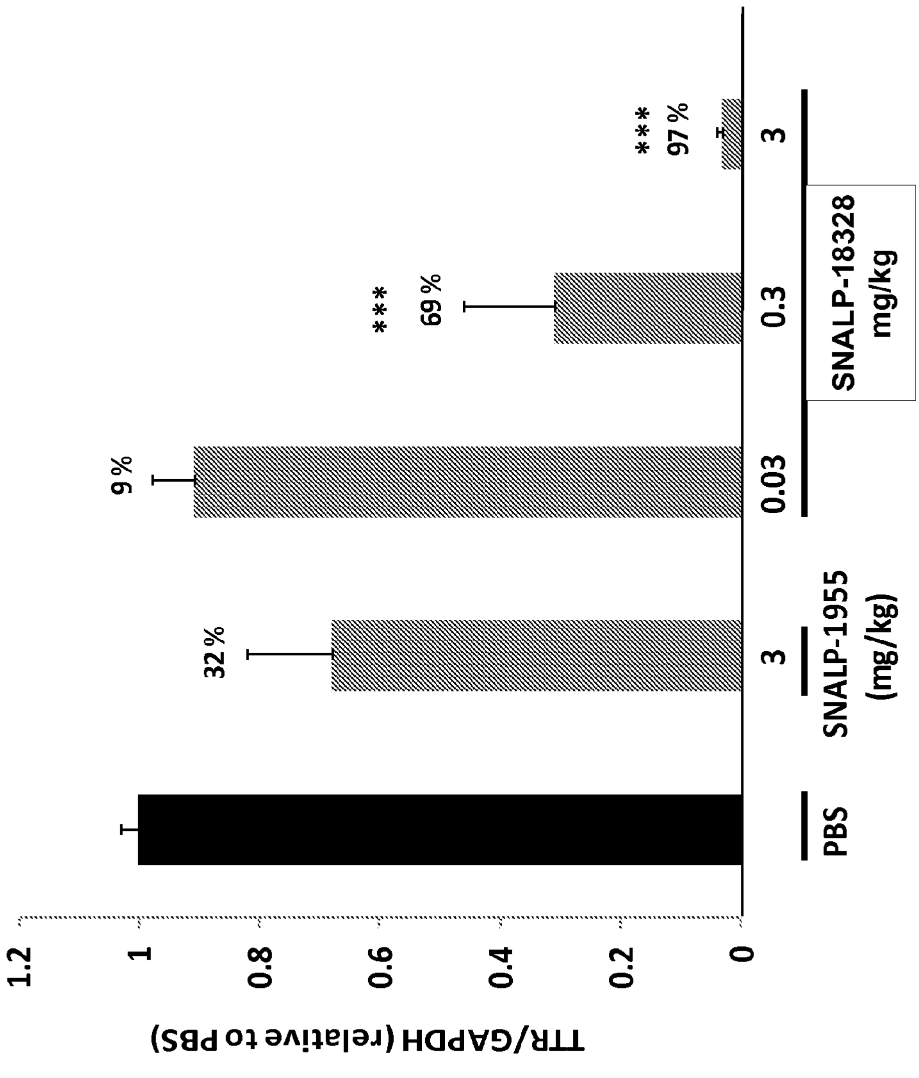
TTR/GAPDH (relative to PBS)
1.2
1
0.8
0.6
0.4
0.2
0
PBS
3
SNALP-1955
(mg/kg)
0.03
0.3
3
SNALP-18328
mg/kg
32 %
9 %
***
69 %
***
97 %

FIG. 6A

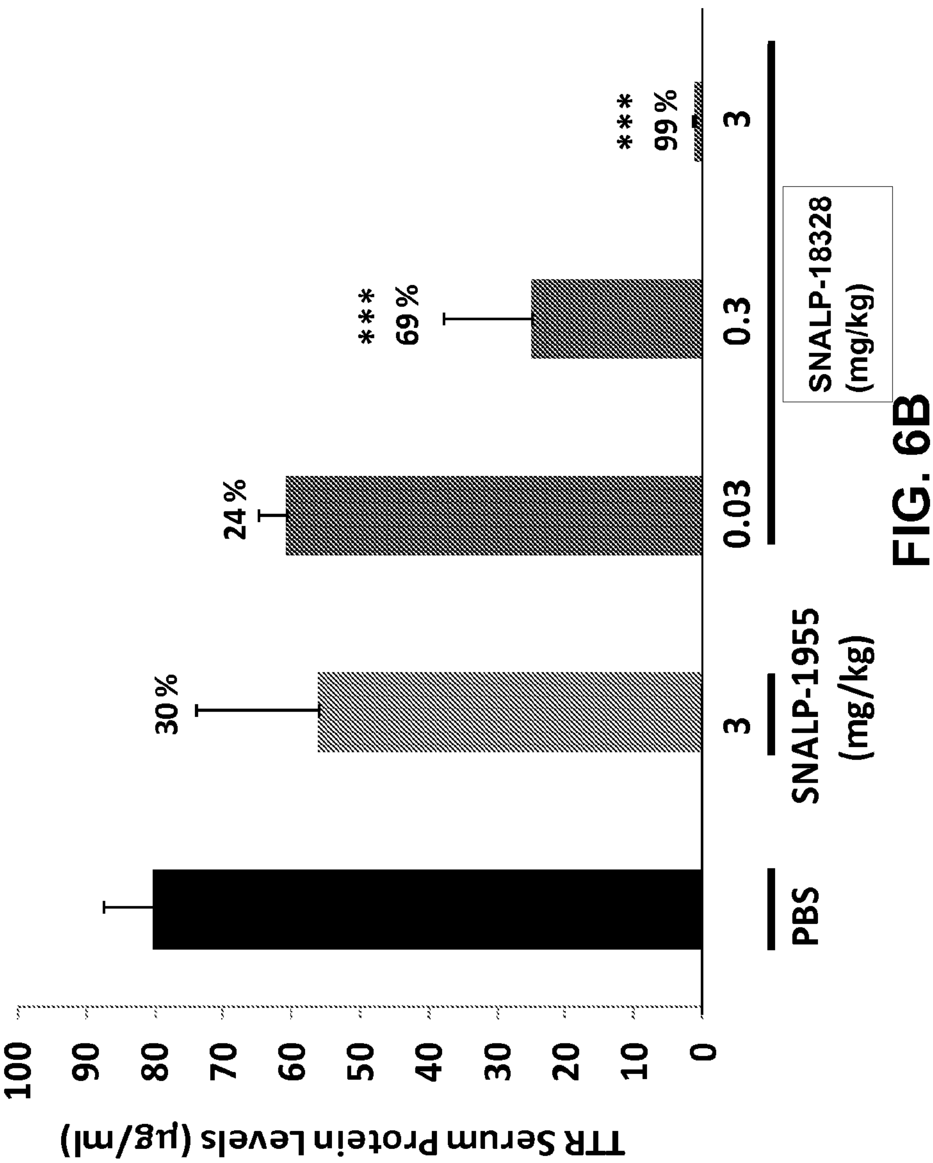
TTR Serum Protein Levels (µg/ml)
100
90
80
70
60
50
40
30
20
10
0
PBS
30 %
3
SNALP-1955
(mg/kg)
24 %
0.03
***
69 %
0.3
***
99 %
3
SNALP-18328
(mg/kg)

FIG. 6B

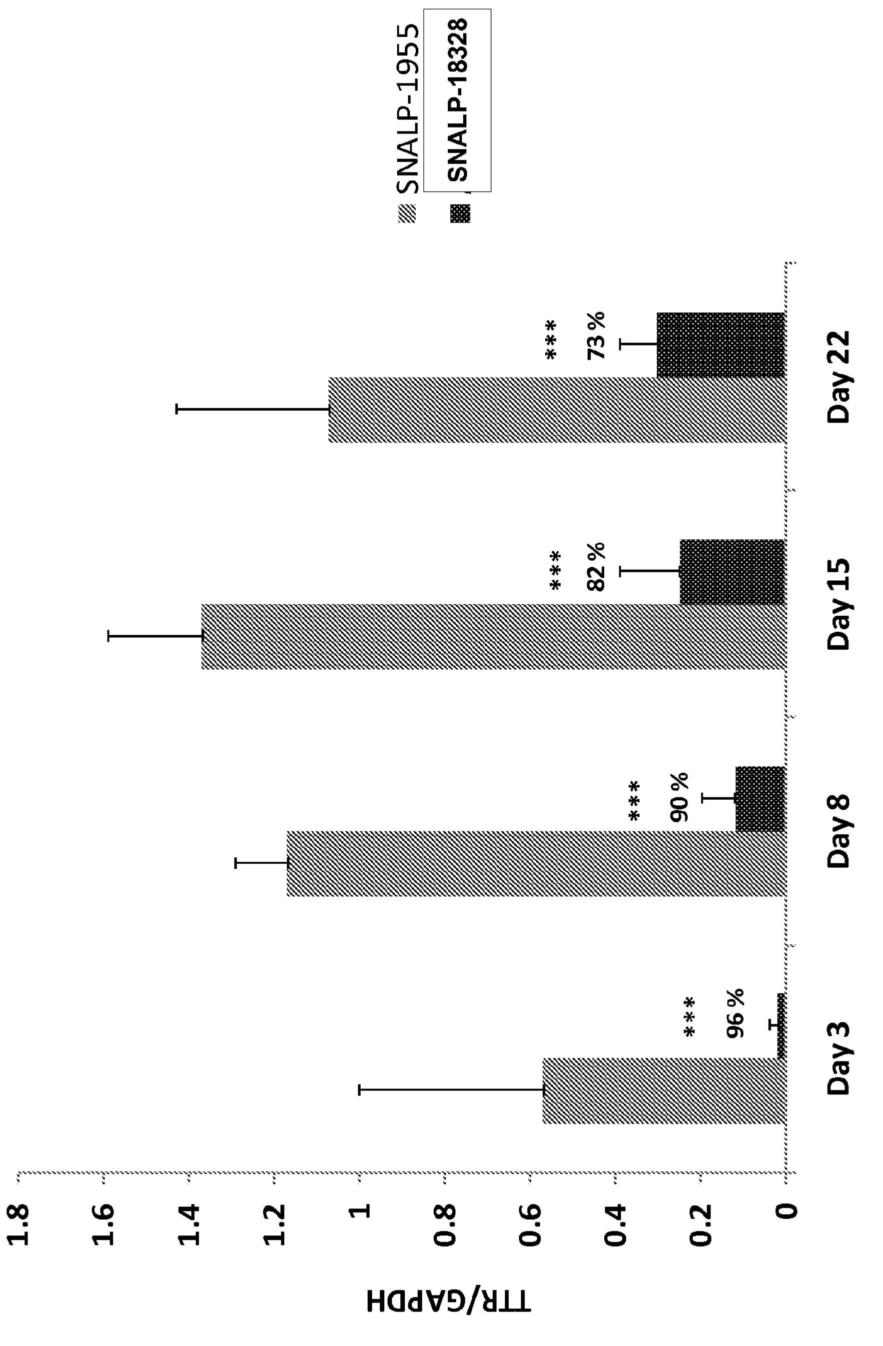
SNALP-1955
SNALP-18328
TTR/GAPDH
1.8
1.6
1.4
1.2
1
0.8
0.6
0.4
0.2
0
***
96 %
***
90 %
***
82 %
***
73 %
Day 3
Day 8
Day 15
Day 22

FIG. 7A

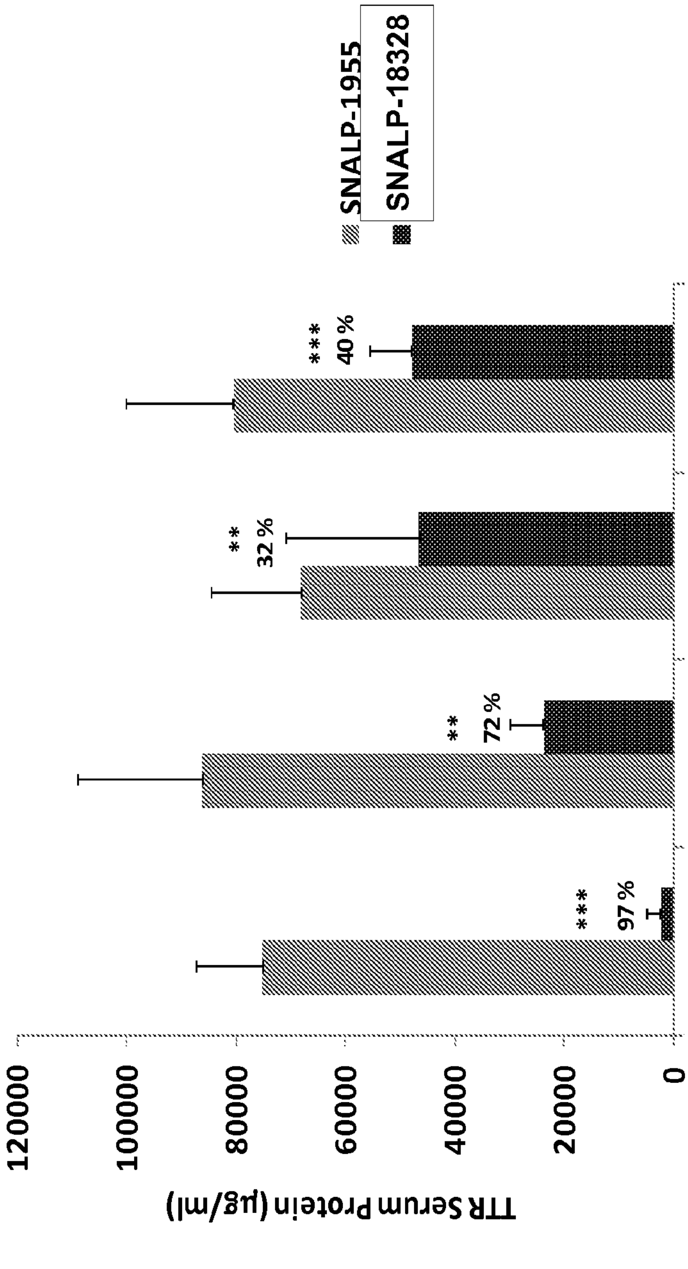
TTR Serum Protein (µg/ml)
120000
100000
80000
60000
40000
20000
0
Day 3
Day 8
Day 15
Day 22
***
97 %
**
72 %
**
32 %
***
40 %
SNALP-1955
SNALP-18328

FIG. 7B

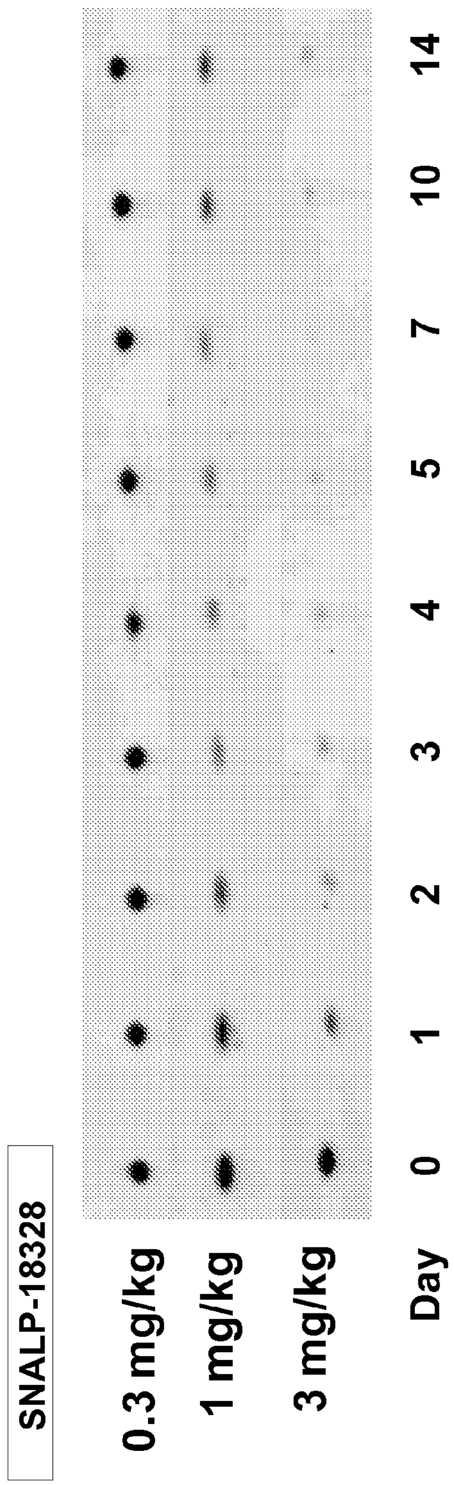


FIG. 8

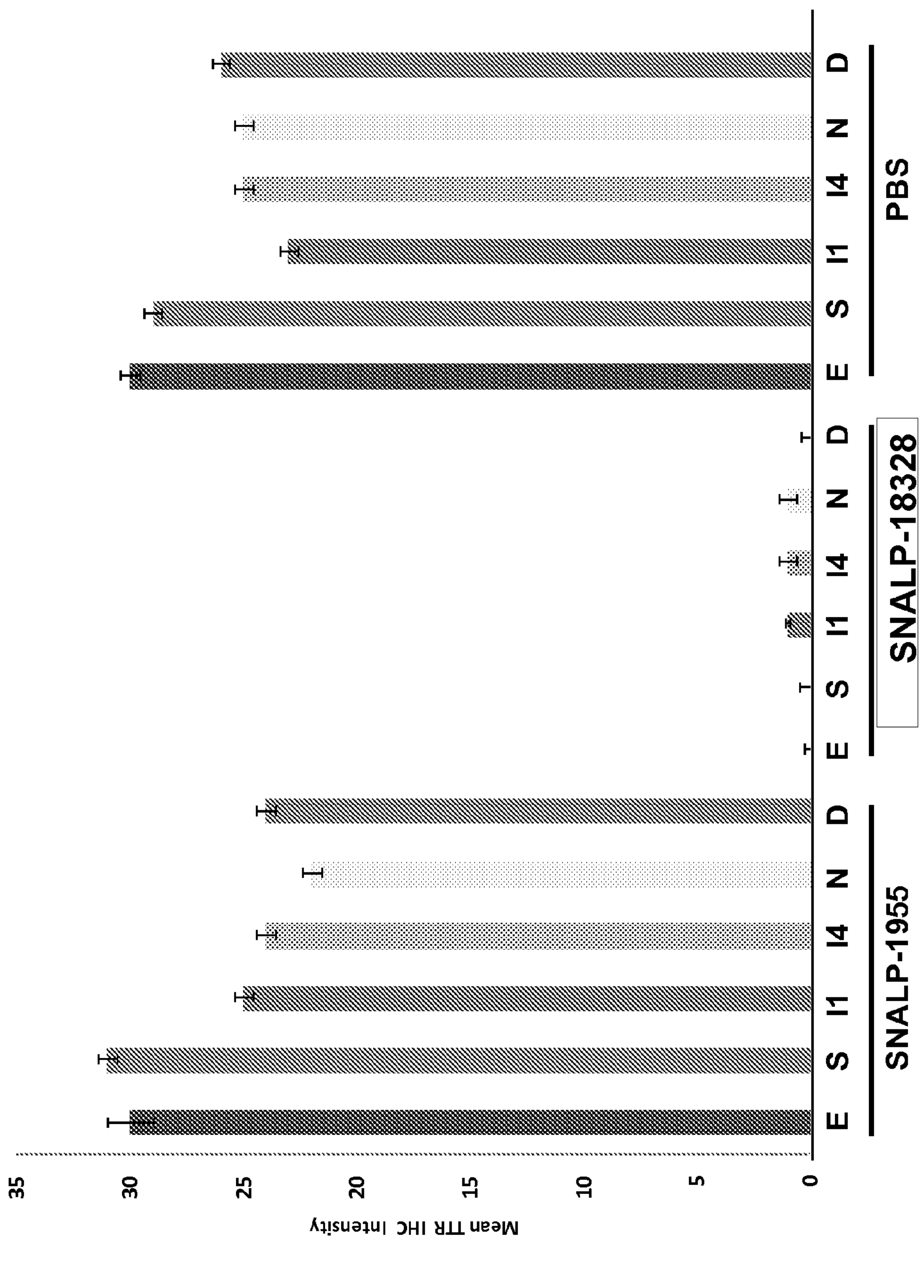
Mean TTR IHC Intensity
0
5
10
15
20
25
30
35
E S I1 I4 N D
SNALP-1955
E S I1 I4 N D
SNALP-18328
E S I1 I4 N D
PBS

FIG. 9

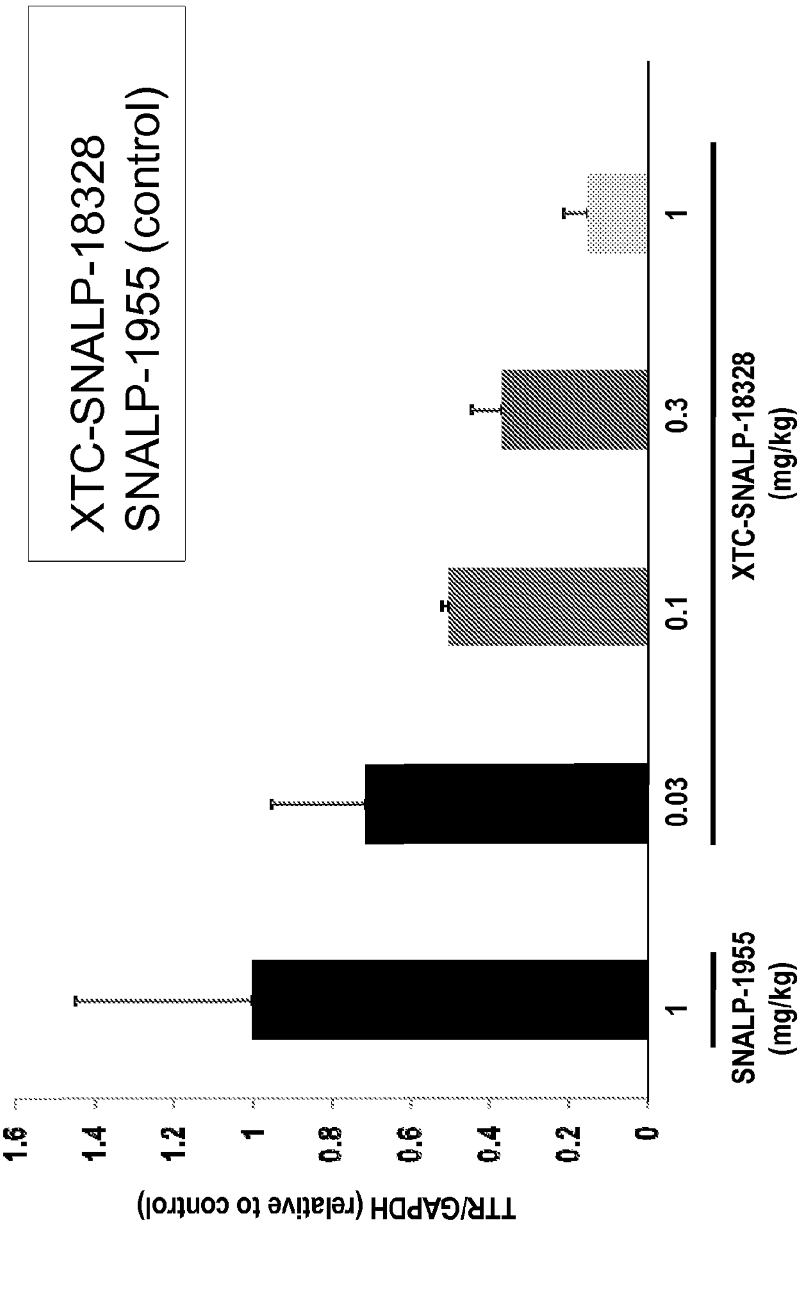
XTC-SNALP-18328
SNALP-1955 (control)
TTR/GAPDH (relative to control)
1.6
1.4
1.2
1
0.8
0.6
0.4
0.2
0
1
0.03
0.1
0.3
1
SNALP-1955
(mg/kg)
XTC-SNALP-18328
(mg/kg)

FIG. 10

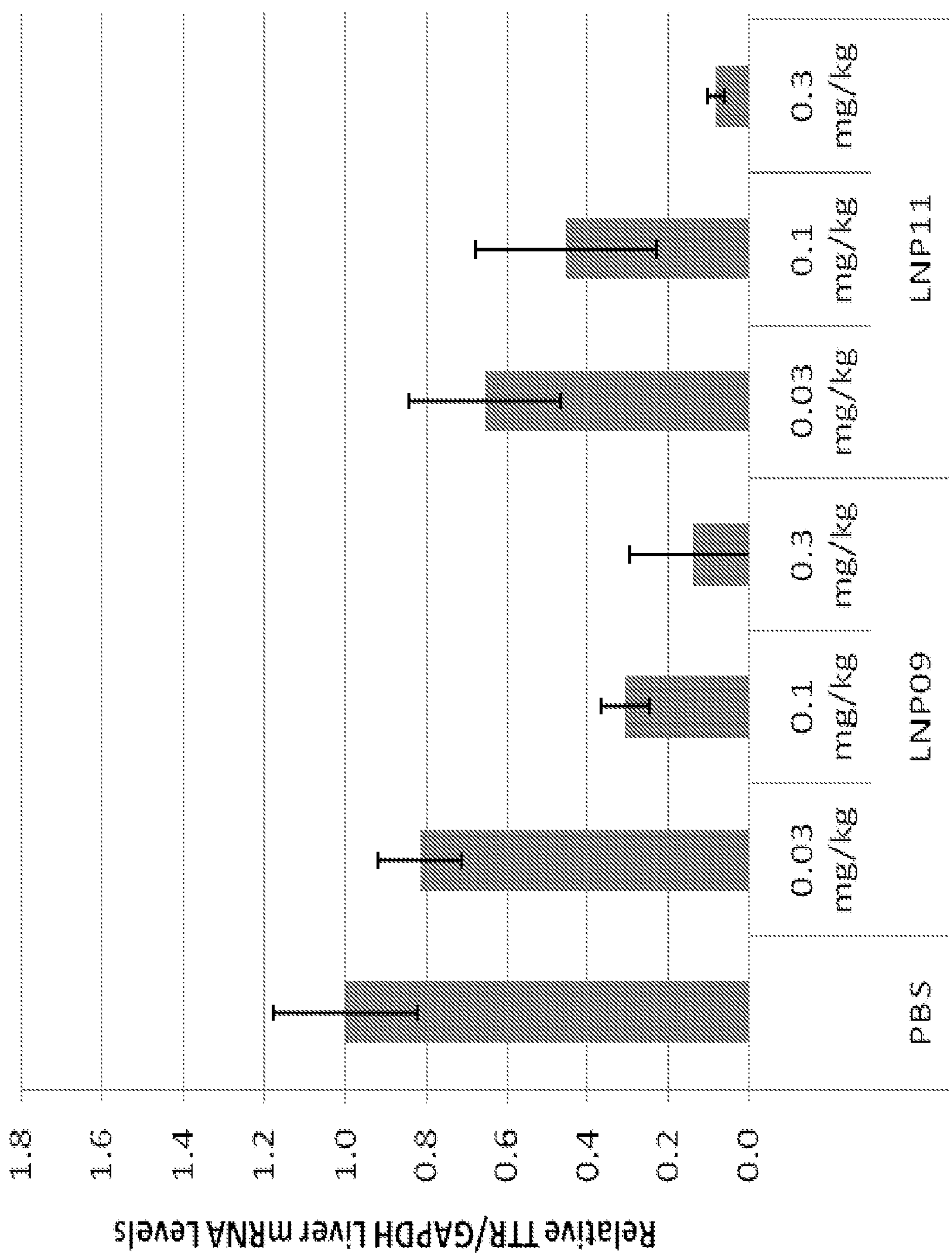
Relative TTR/GAPDH Liver mRNA Levels
1.8
1.6
1.4
1.2
1.0
0.8
0.6
0.4
0.2
0.0
PBS
0.03 mg/kg
0.1 mg/kg
0.3 mg/kg
LNP09
0.03 mg/kg
0.1 mg/kg
0.3 mg/kg
LNP11

FIG. 11A

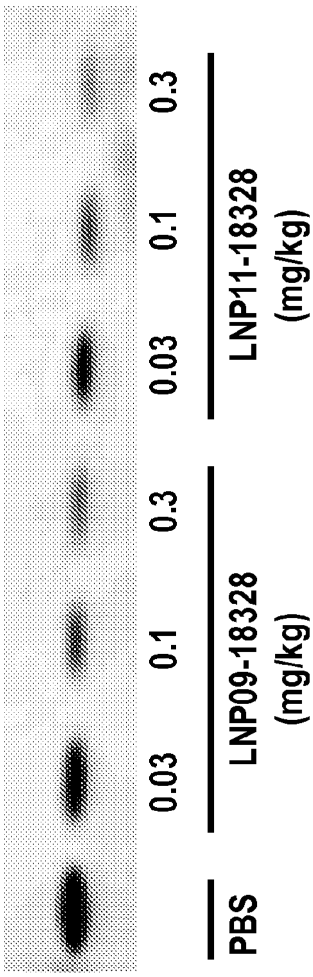


FIG. 11B

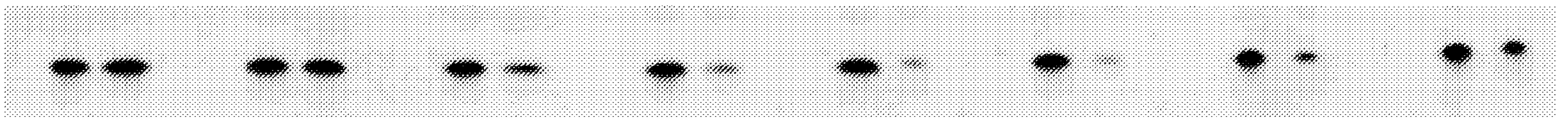


Lanes 1, 3, 5, 7, 9, 11, 13, and 15: PBS animals
Lanes 2, 4, 6, 8, 10, 14, and 16: 0.3 mg/kg LNP09-18328

FIG. 11C

gttgactaag tcaataatca gaatcagcag
gtttgcagtc agattggcag ggataagcag
cctagctcag gagaagtgag tataaaagcc
ccaggctggg agcagccatc acagaagtcc
actcattctt ggcaggatgg cttctcatcg
tctgctcctc ctctgccttg ctggactggt
atttgtgtct gaggctggcc ctacgggcac
cggtgaatcc aagtgtcctc tgatggtcaa
agttctagat gctgtccgag gcagtcctgc
catcaatgtg gccgtgcatg tgttcagaaa
ggctgctgat gacacctggg agccatttgc
ctctgggaaa accagtgagt ctggagagct
gcatgggctc acaactgagg aggaatttgt
agaagggata tacaaagtgg aaatagacac
caaatcttac tggaaggcac ttggcatctc
cccattccat gagcatgcag aggtggtatt
cacagccaac gactccggcc cccgccgcta
caccattgcc gccctgctga gcccctactc
ctattccacc acggctgtcg tcaccaatcc
caaggaatga gggacttctc ctccagtgga
cctgaaggac gagggatggg atttcatgta
accaagagta ttccattttt actaaagcag
tgttttcacc tcatatgcta tgttagaagt
ccaggcagag acaataaaac attcctgtga
aaggcacttt tcattccact ttaacttgat
tttttaaatt cccttattgt cccttccaaa
aaaaagagaa tcaaaatttt acaaagaatc
aaaggaattc tagaaagtat ctgggcagaa
cgctaggaga gatccaaatt tccattgtct
tgcaagcaaa gcacgtatta aatatgatct
gcagccatta aaaagacaca ttctgtaaaa
aaaaaaaa (SEQ ID NO: 1331)

**FIG. 12**

# FIG. 13A

ACAGAAGTCCACTCATTCTTGGCAGGATGGCTTCTCATCGTCTGCTCCTCCT
CTGCCTTGCTGGACTGGTATTTGTGTCTGAGGCTGGCCCTACGGGCACCGGT
GAATCCAAGTGTCCTCTGATGGTCAAAGTTCTAGATGCTGTCCGAGGCAGTC
CTGCCATCAATGTGGCCGTGCATGTGTTCAGAAAGGCTGCTGATGACACCTG
GGAGCCATTTGCCTCTGGGAAAACCAGTGAGTCTGGAGAGCTGCATGGGCTC
ACAACTGAGGAGGAATTTGTAGAAGGGATATACAAAGTGGAAATAGACACCA
AATCTTACTGGAAGGCACTTGGCATCTCCCCATTCCATGAGCATGCAGAGGT
GGTATTCACAGCCAACGACTCCGGCCCCCGCCGCTACACCATTGCCGCCCTG
CTGAGCCCCTACTCCTATTCCACCACGGCTGTCGTCACCAATCCCAAGGAAT
GAGGGACTTCTCCTCCAGTGGACCTGAAGGACGAGGGATGGGATTTCATGTA
ACCAAGAGTATTCCATTTTTACTAAAGCAGTGTTTTCACCTCATATGCTATG
TTAGAAGTCCAGGCAGAGACAATAAAACATTCCTGTGAAAGGCACTTTTCAT
TCCAAAAAAAAAAAAAAAAAAAAAAA (SEQ ID NO:1329)

# FIG. 13B

CCTGACAGGATGGCTTCCCTTCGCCTGTTCCTCCTCTGCCTCGCTGGACTGA
TATTTGCGTCTGAAGCTGGCCCTGGGGGTGCTGGAGAATCCAAGTGTCCTCT
GATGGTCAAAGTCCTGGATGCTGTCCGAGGCAGCCCTGCTGTCGATGTGGCC
GTGAAAGTGTTCAAAAGGACTGCAGACGGAAGCTGGGAGCCGTTTGCCTCTG
GGAAGACCGCCGAGTCTGGAGAGCTGCACGGGCTCACCACAGATGAGAAGTT
CACGGAAGGGGTGTACAGGGTAGAACTGGACACCAAATCATACTGGAAGGCT
CTTGGCATTTCCCCATTCCATGAATACGCAGAGGTGGTTTTCACAGCCAATG
ACTCTGGTCATCGCCACTACACCATCGCAGCCCTGCTCAGCCCGTACTCCTA
CAGCACCACTGCTGTCGTCAGTAACCCCCAGAACTGAGGGACCCAGCCCACG
AGGACCAAGATCTTGCCAAAGCAGTAGCTCCCATTTGTACTGAAACAGTGTT
CTTGCTCTATAAACCGTGTTAGCAACTCGGGAAGATGCCGTGAAACGTTCTT
ATTAAACCACCTTTATTTCATTC
(SEQ ID NO:1330)

```
                                  20                  40                  60
NM_000371.3    GTTGACTAAGTCAATAATCAGAATCAGCAGGTTTGCAGTCAGATTGGCAGGGATAAGCAGCCTAGCTC 68
NM_000371.2    -------------------------------------------------------------------- -
AD-18328_sense -------------------------------------------------------------------- -
                          80                  100                 120
NM_000371.3    AGGAGAAGTGAGTATAAAAGCCCCAGGCTGGGAGCAGCCATCACAGAAGTCCACTCATTCTTGGCAGG 136
NM_000371.2    ------------------------------------------ACAGAAGTCCACTCATTCTTGGCAGG 26
AD-18328_sense -------------------------------------------------------------------- -
                  140                 160                 180                 200
NM_000371.3    ATGGCTTCTCATCGTCTGCTCCTCCTCTGCCTTGCTGGACTGGTATTTGTGTCTGAGGCTGGCCCTAC 204
NM_000371.2    ATGGCTTCTCATCGTCTGCTCCTCCTCTGCCTTGCTGGACTGGTATTTGTGTCTGAGGCTGGCCCTAC 94
AD-18328_sense -------------------------------------------------------------------- -
                              220                 240                 260
NM_000371.3    GGGCACCGGTGAATCCAAGTGTCCTCTGATGGTCAAAGTTCTAGATGCTGTCCGAGGCAGTCCTGCCA 272
NM_000371.2    GGGCACCGGTGAATCCAAGTGTCCTCTGATGGTCAAAGTTCTAGATGCTGTCCGAGGCAGTCCTGCCA 162
AD-18328_sense -------------------------------------------------------------------- -
                      280                 300                 320                 340
NM_000371.3    TCAATGTGGCCGTGCATGTGTTCAGAAAGGCTGCTGATGACACCTGGGAGCCATTTGCCTCTGGGAAA 340
NM_000371.2    TCAATGTGGCCGTGCATGTGTTCAGAAAGGCTGCTGATGACACCTGGGAGCCATTTGCCTCTGGGAAA 230
AD-18328_sense -------------------------------------------------------------------- -
                                  360                 380                 400
NM_000371.3    ACCAGTGAGTCTGGAGAGCTGCATGGGCTCACAACTGAGGAGGAATTTGTAGAAGGGATATACAAAGT 408
NM_000371.2    ACCAGTGAGTCTGGAGAGCTGCATGGGCTCACAACTGAGGAGGAATTTGTAGAAGGGATATACAAAGT 298
AD-18328_sense -------------------------------------------------------------------- -
                          420                 440                 460
NM_000371.3    GGAAATAGACACCAAATCTTACTGGAAGGCACTTGGCATCTCCCCATTCCATGAGCATGCAGAGGTGG 476
NM_000371.2    GGAAATAGACACCAAATCTTACTGGAAGGCACTTGGCATCTCCCCATTCCATGAGCATGCAGAGGTGG 366
AD-18328_sense -------------------------------------------------------------------- -
                  480                 500                 520                 540
NM_000371.3    TATTCACAGCCAACGACTCCGGCCCCCGCCGCTACACCATTGCCGCCCTGCTGAGCCCCTACTCCTAT 544
NM_000371.2    TATTCACAGCCAACGACTCCGGCCCCCGCCGCTACACCATTGCCGCCCTGCTGAGCCCCTACTCCTAT 434
AD-18328_sense -------------------------------------------------------------------- -
                              560                 580                 600
NM_000371.3    TCCACCACGGCTGTCGTCACCAATCCCAAGGAATGAGGGACTTCTCCTCCAGTGGACCTGAAGGACGA 612
NM_000371.2    TCCACCACGGCTGTCGTCACCAATCCCAAGGAATGAGGGACTTCTCCTCCAGTGGACCTGAAGGACGA 502
AD-18328_sense -------------------------------------------------------------------- -
                      620                 640                 660                 680
NM_000371.3    GGGATGGGATTTCATGTAACCAAGAGTATTCCATTTTTACTAAAGCAGTGTTTTCACCTCATATGCTA 680
NM_000371.2    GGGATGGGATTTCATGTAACCAAGAGTATTCCATTTTTACTAAAGCAGTGTTTTCACCTCATATGCTA 570
AD-18328_sense ---------------GTAACCAAGAGTATTCCAT---------------------------------- 19
                                  700                 720                 740
NM_000371.3    TGTTAGAAGTCCAGGCAGAGACAATAAAACATTCCTGTGAAAGGCACTTTTCATTCCACTTTAACTTG 748
NM_000371.2    TGTTAGAAGTCCAGGCAGAGACAATAAAACATTCCTGTGAAAGGCACTTTTCATTCCA---------- 628
AD-18328_sense -------------------------------------------------------------------- 19
                          760                 780                 800
NM_000371.3    ATTTTTTAAATTCCCTTATTGTCCCTTCCAAAAAAAAGAGAATCAAAATTTTACAAAGAATCAAAGGA 816
NM_000371.2    ----------------------------AAAAAAAAAA------------------------------ 638
AD-18328_sense -------------------------------------------------------------------- 19
                  820                 840                 860                 880
NM_000371.3    ATTCTAGAAAGTATCTGGGCAGAACGCTAGGAGAGATCCAAATTTCCATTGTCTTGCAAGCAAACCAC 884
NM_000371.2    -------------------------------------------------------------------- 638
AD-18328_sense -------------------------------------------------------------------- 19
                              900                 920
NM_000371.3    GTATTAAATATGATCTGCAGCCATTAAAAAGACACATTCTGTAAAAAAAAAAAA 938
NM_000371.2    ------------------------------------------AAAAAAAAAAAA 650
AD-18328_sense ------------------------------------------------------ 19
```

FIG. 14

| Phenotype | Features | Genotypes (associated mutation in TTR) |
|---|---|---|
| Familial amyloidotic neuropathy (FAP) | Early: Impotence<br>Sensorimotor polyneuropathy of the legs<br>Carpal tunnel syndrome<br>Autonomic dysfunction<br>Constipation/diarrhea<br><br>Later: Cardiomyopathy<br>Vitreous opacities<br>Nephropathy | V28M<br>L58H V30M<br>L58R<br>K70N<br>Y78F<br>I84S<br>Y114H<br>V30A<br>K35N<br>G47V<br>S50R<br>T60A<br>Y114C |
| Familial amyloidotic cardiomyopathy (FAC) | Cardiomegaly<br>Congestive heart failure<br>Conduction abnormalities, arrhythmias<br>Angina<br>Sudden death | D18N D18E V20I<br>P24S<br>E42D A45T T49P<br>S50I<br>H56R I68L A81T<br>Q92K<br>R103S L111M V122I<br>T60A |
| CNS amyloidosis (CNSA) | Dementia, ataxia, spasticity, seizures, hemorrhage (intracerebellar and/or subarachnoid), psychosis, hydrocephalus | L12P D18G A25T<br>V30G A36P G53E<br>F64S 769H Y114C |

FIG. 15

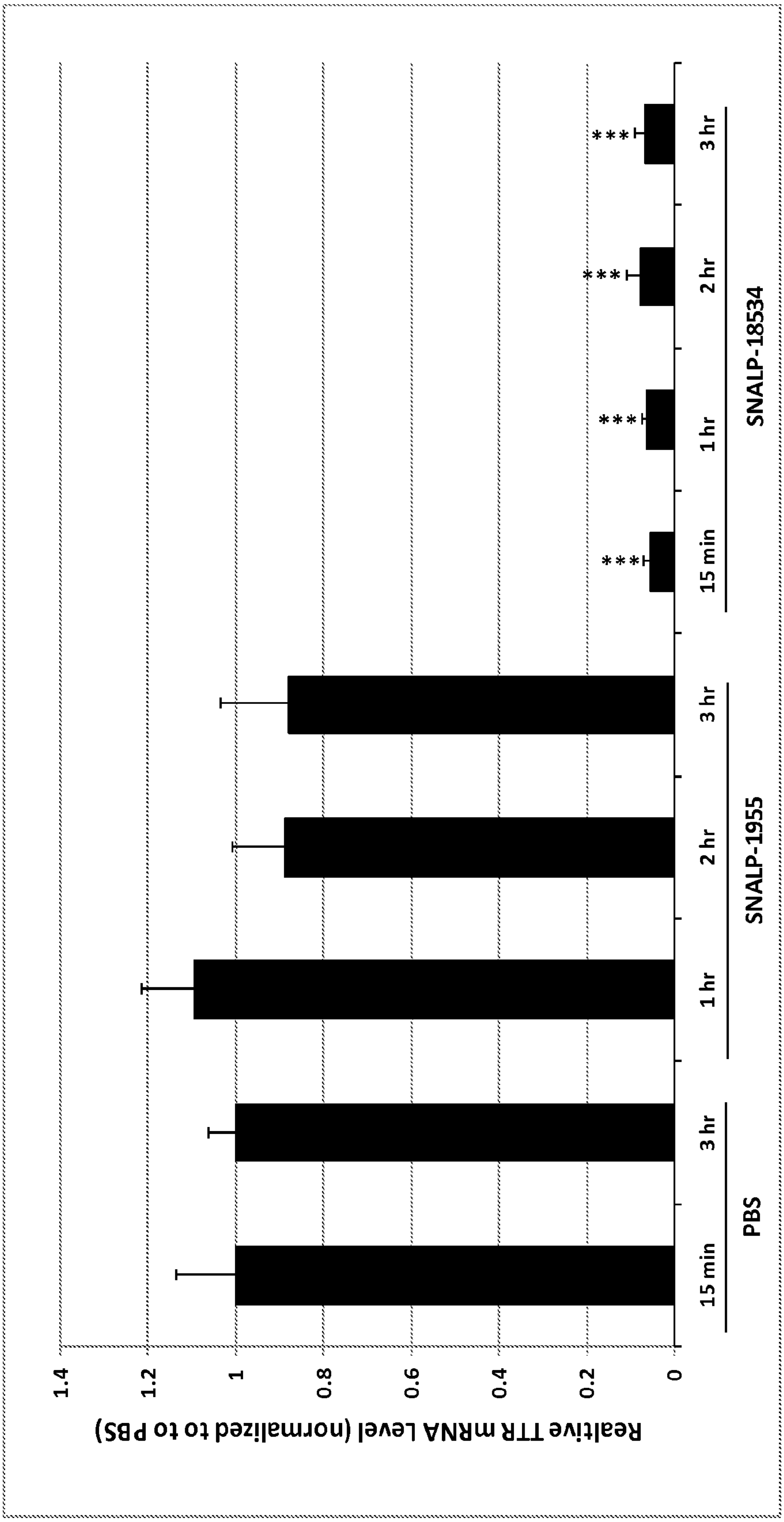

Realtive TTR mRNA Level (normalized to to PBS)
0
0.2
0.4
0.6
0.8
1
1.2
1.4
15 min
3 hr
PBS
1 hr
2 hr
3 hr
SNALP-1955
15 min
1 hr
2 hr
3 hr
SNALP-18534
***
***
***
***


FIG. 16

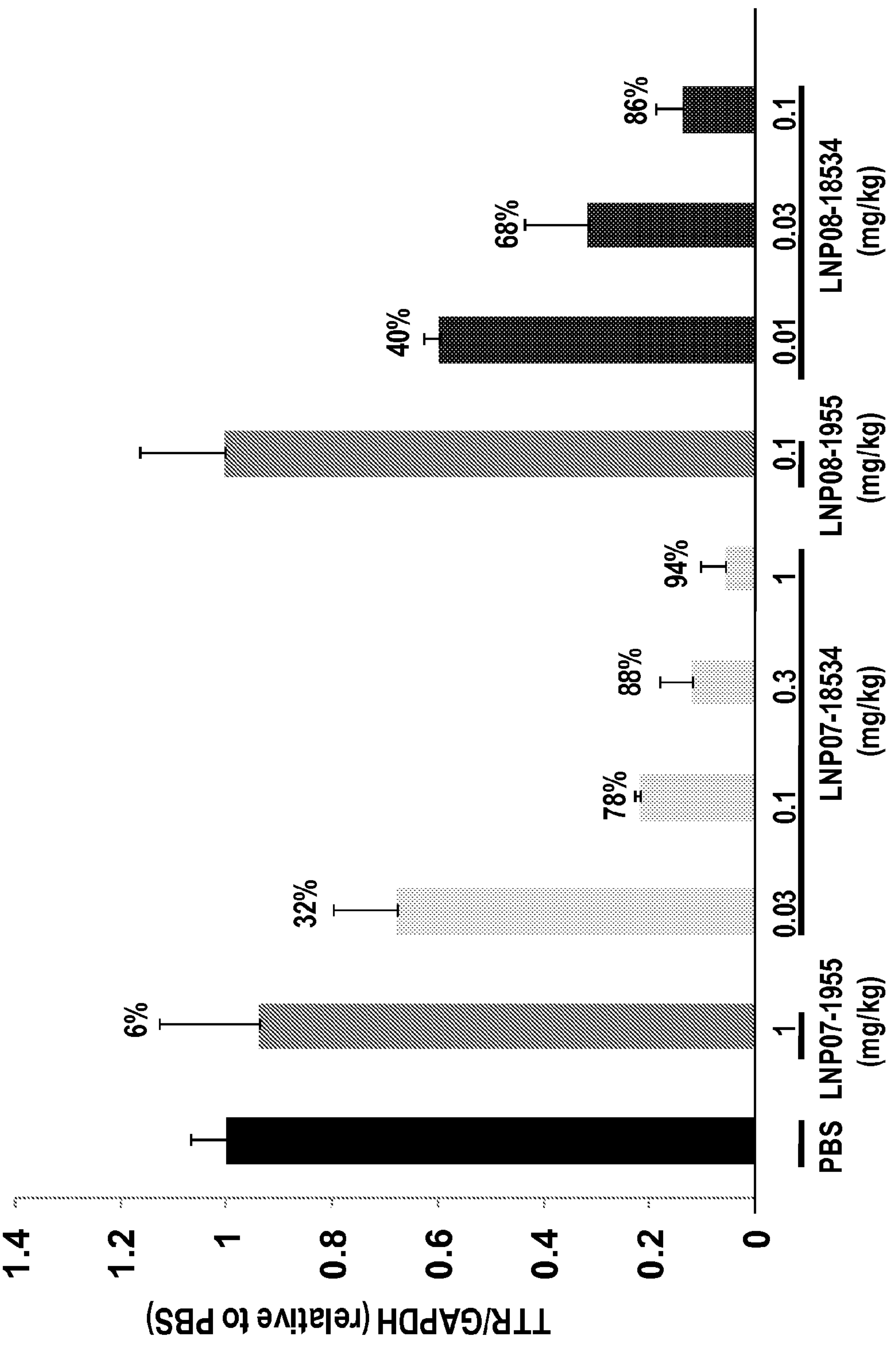
TTR/GAPDH (relative to PBS)
1.4
1.2
1
0.8
0.6
0.4
0.2
0
6%
32%
78%
88%
94%
40%
68%
86%
PBS
1
LNP07-1955
(mg/kg)
0.03
0.1
0.3
1
LNP07-18534
(mg/kg)
0.1
LNP08-1955
(mg/kg)
0.01
0.03
0.1
LNP08-18534
(mg/kg)

FIG. 17

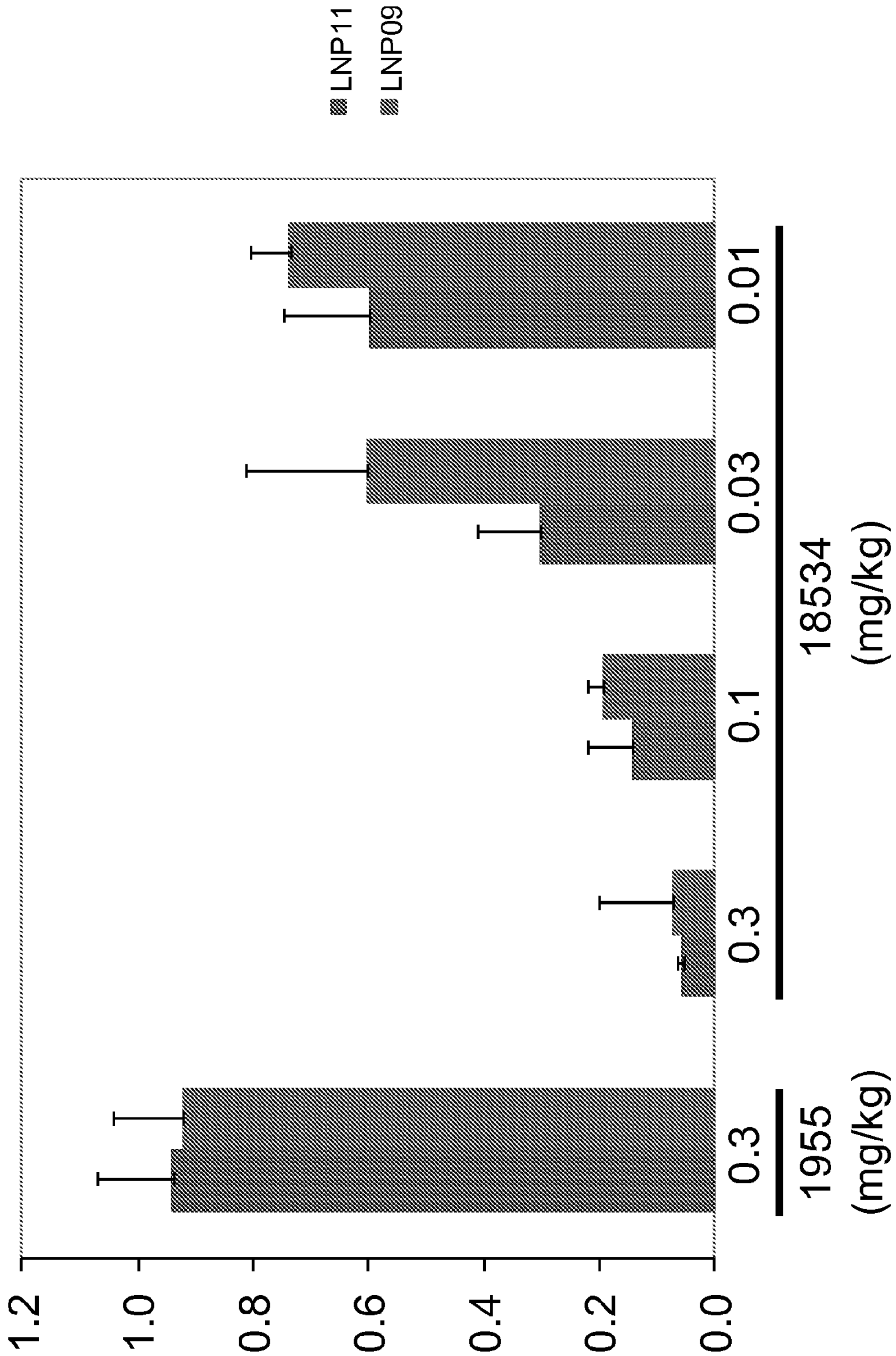
LNP11
LNP09
TTR/GAPDH (Relative to PBS)
1.2
1.0
0.8
0.6
0.4
0.2
0.0
0.3
1955
(mg/kg)
0.3
0.1
0.03
0.01
18534
(mg/kg)

**FIG. 18**

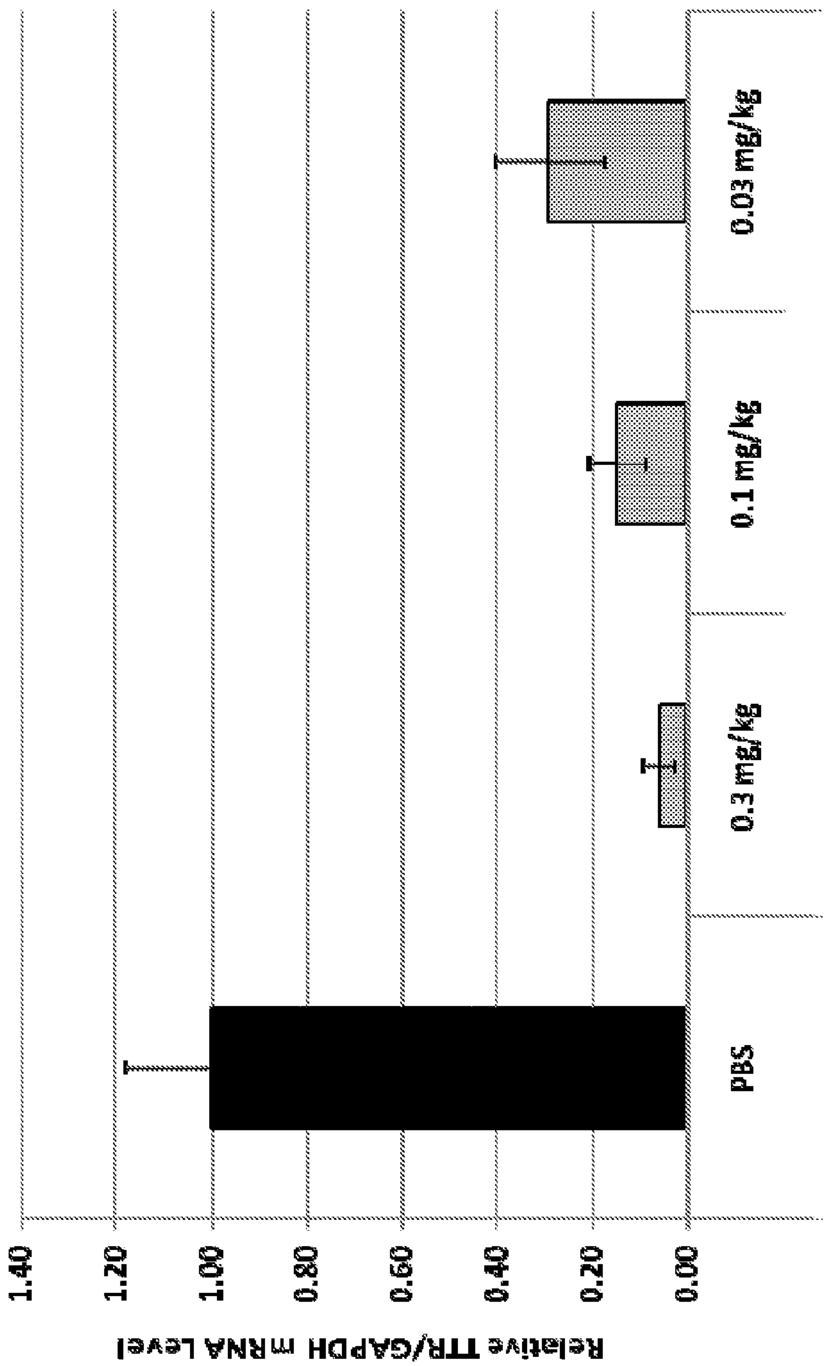

Relative TTR/GAPDH mRNA Level
1.40
1.20
1.00
0.80
0.60
0.40
0.20
0.00
PBS
0.3 mg/kg
0.1 mg/kg
0.03 mg/kg


FIG. 19

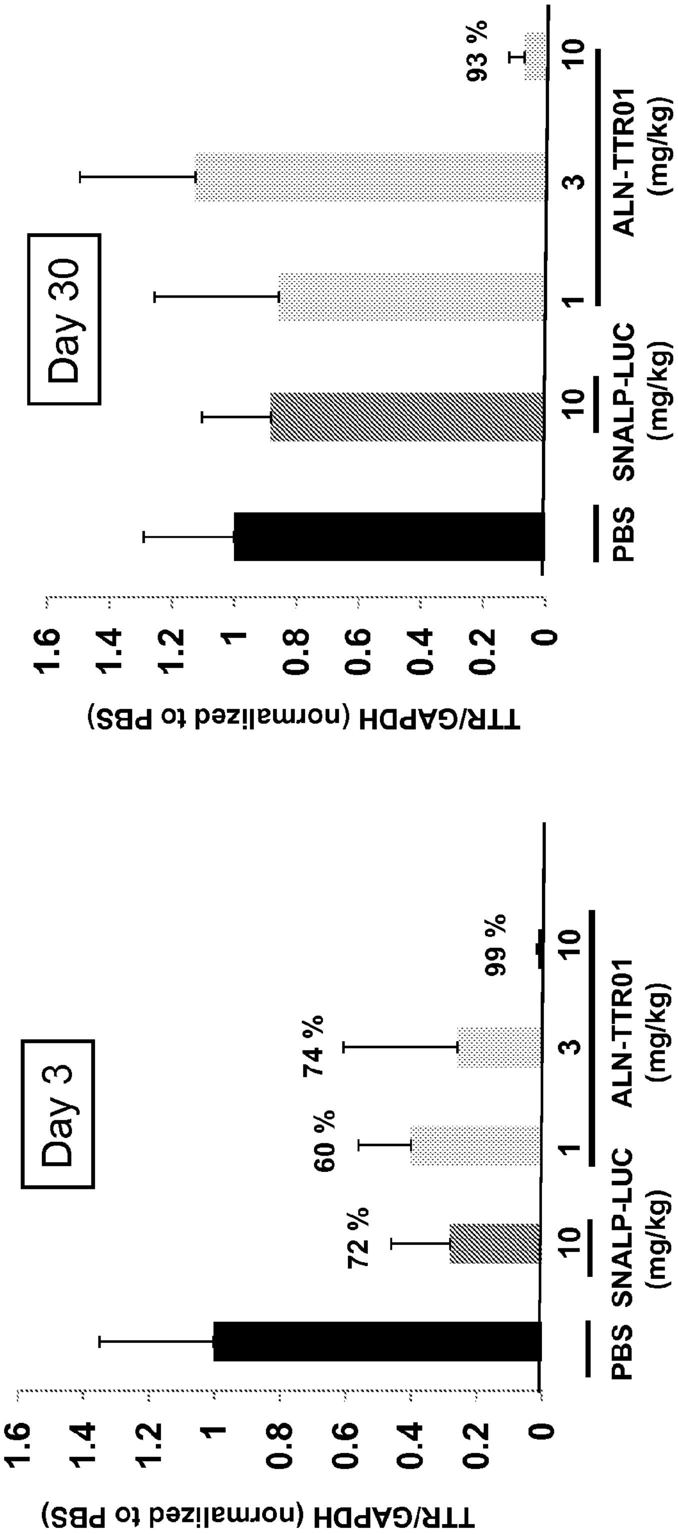
Day 3
Day 30
TTR/GAPDH (normalized to PBS)
0
0.2
0.4
0.6
0.8
1
1.2
1.4
1.6
72 %
60 %
74 %
99 %
93 %
PBS
10
1
3
10
SNALP-LUC
(mg/kg)
ALN-TTR01
(mg/kg)

FIG. 20

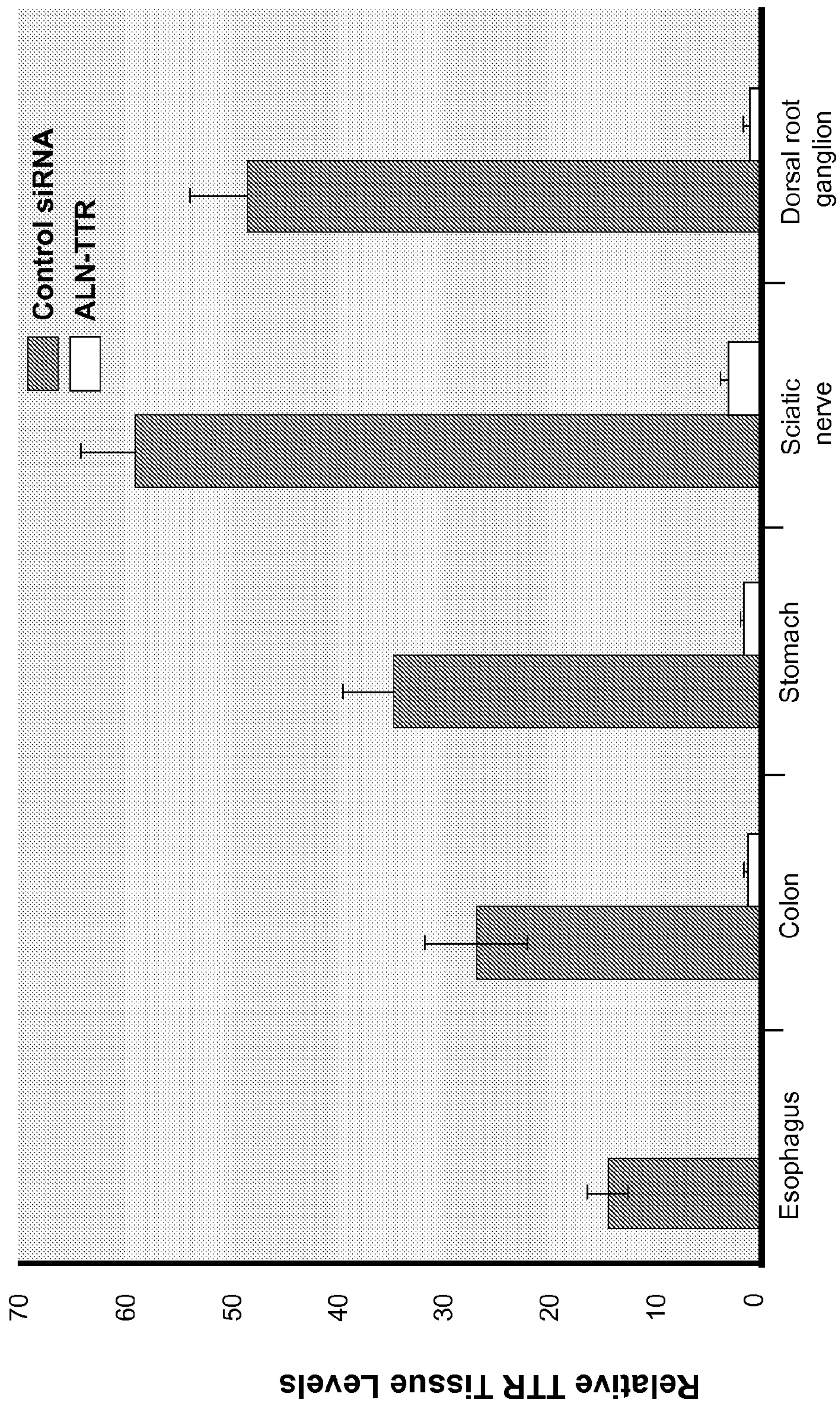
Relative TTR Tissue Levels
0
10
20
30
40
50
60
70
Control siRNA
ALN-TTR
Esophagus
Colon
Stomach
Sciatic nerve
Dorsal root ganglion

FIG. 21

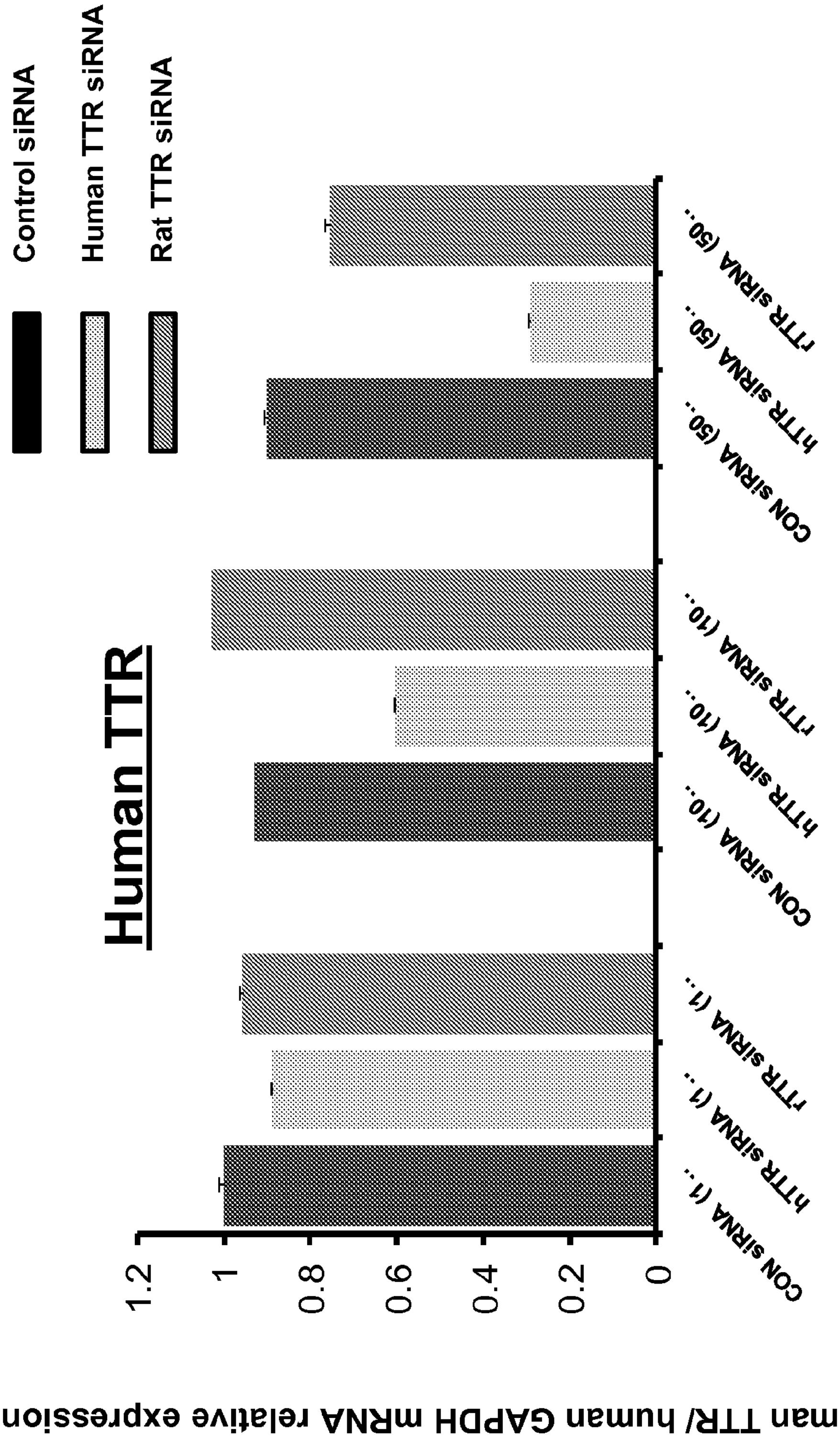
Human TTR
Control siRNA
Human TTR siRNA
Rat TTR siRNA
human TTR/ human GAPDH mRNA relative expression
1.2
1
0.8
0.6
0.4
0.2
0
CON siRNA (1...
hTTR siRNA (1...
rTTR siRNA (1...
CON siRNA (10...
hTTR siRNA (10...
rTTR siRNA (10...
CON siRNA (50...
hTTR siRNA (50...
rTTR siRNA (50...

FIG. 22

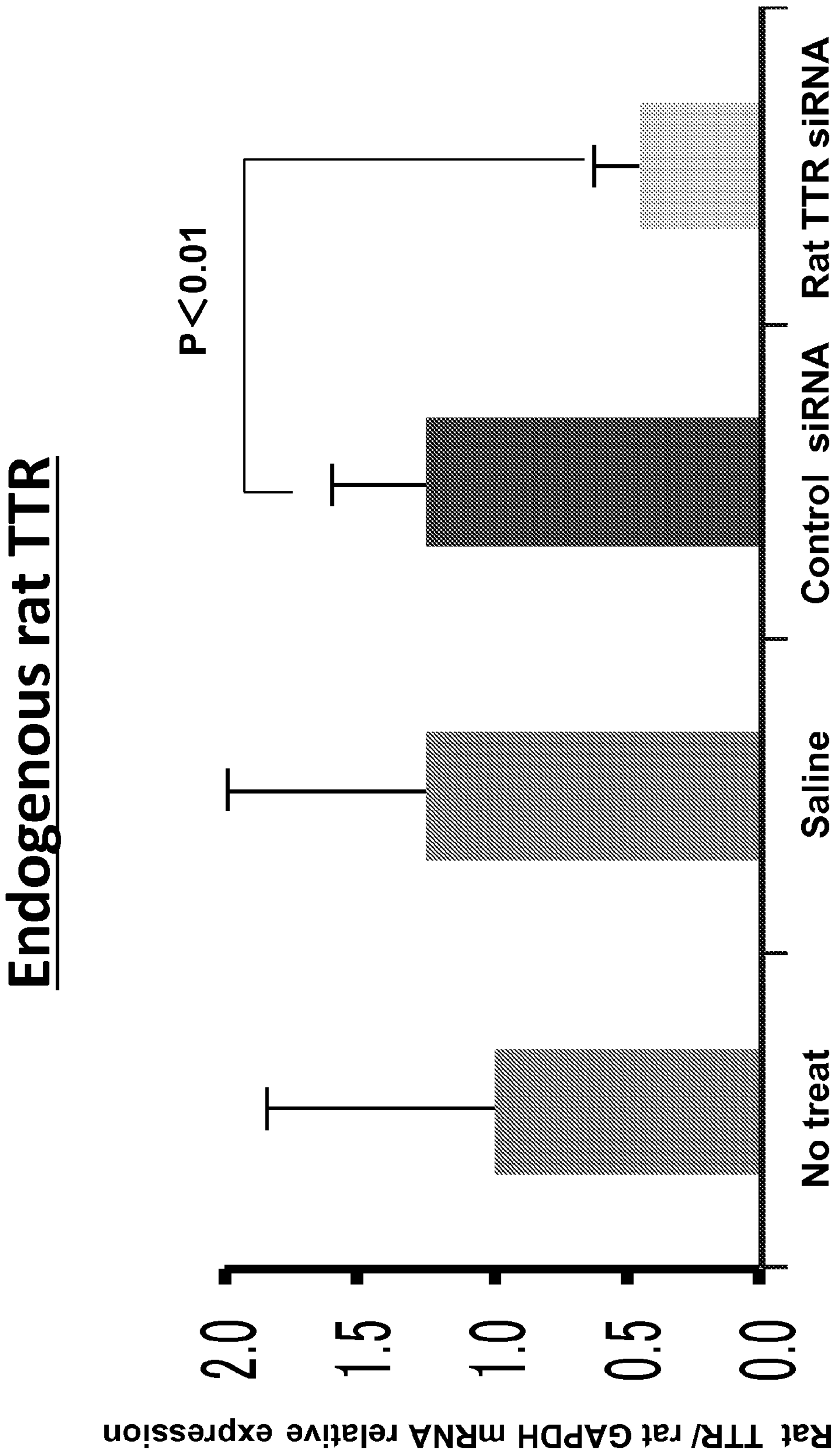
Endogenous rat TTR
Rat TTR/ rat GAPDH mRNA relative expression
2.0
1.5
1.0
0.5
0.0
P<0.01
No treat
Saline
Control siRNA
Rat TTR siRNA

FIG. 23

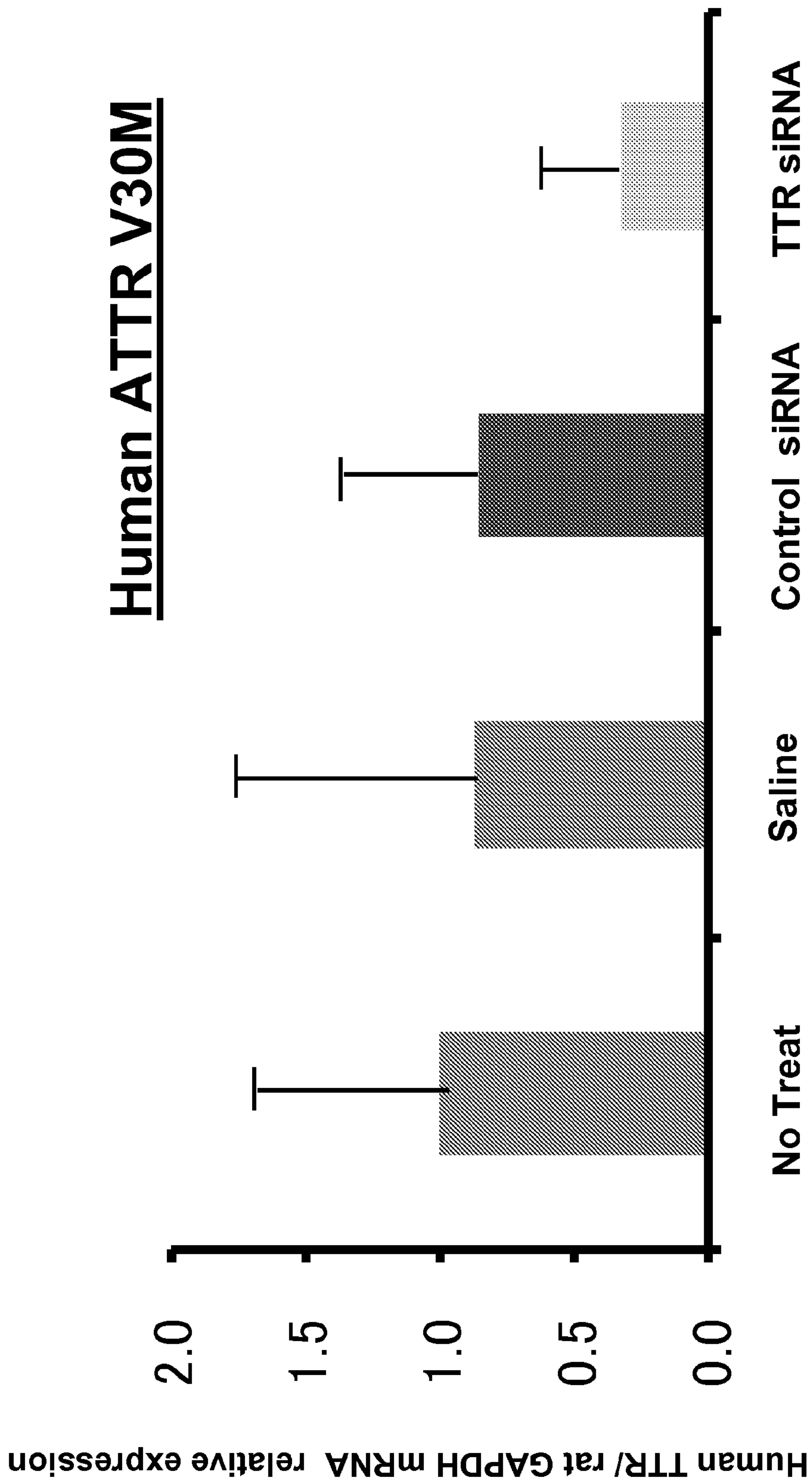
Human ATTR V30M
Human TTR/ rat GAPDH mRNA relative expression
2.0
1.5
1.0
0.5
0.0
No Treat
Saline
Control siRNA
TTR siRNA

FIG. 24

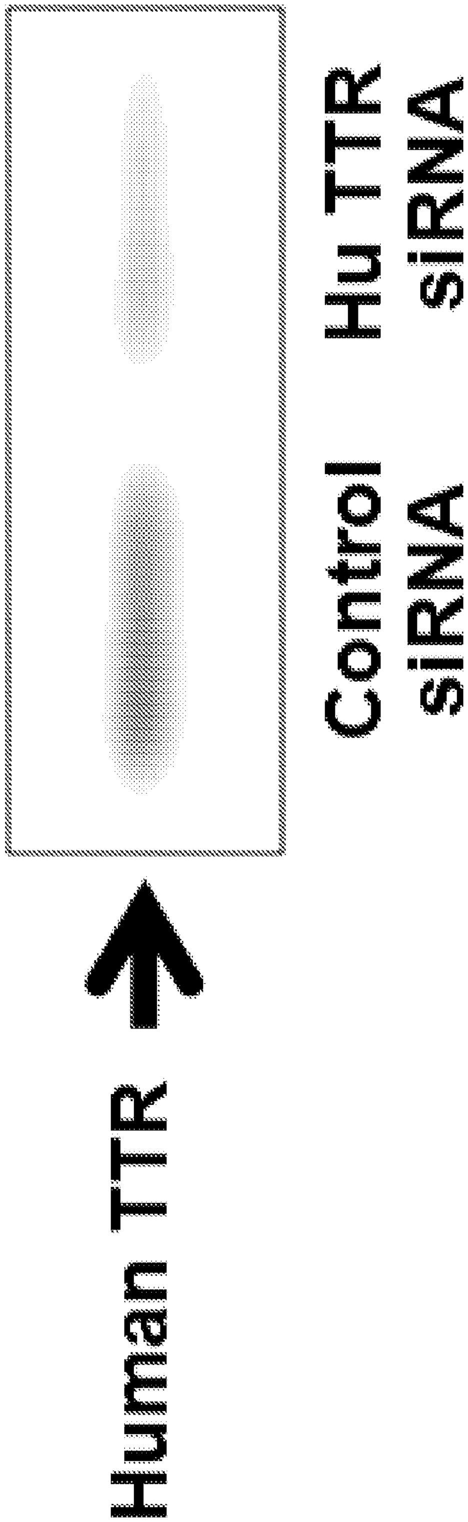


FIG. 25

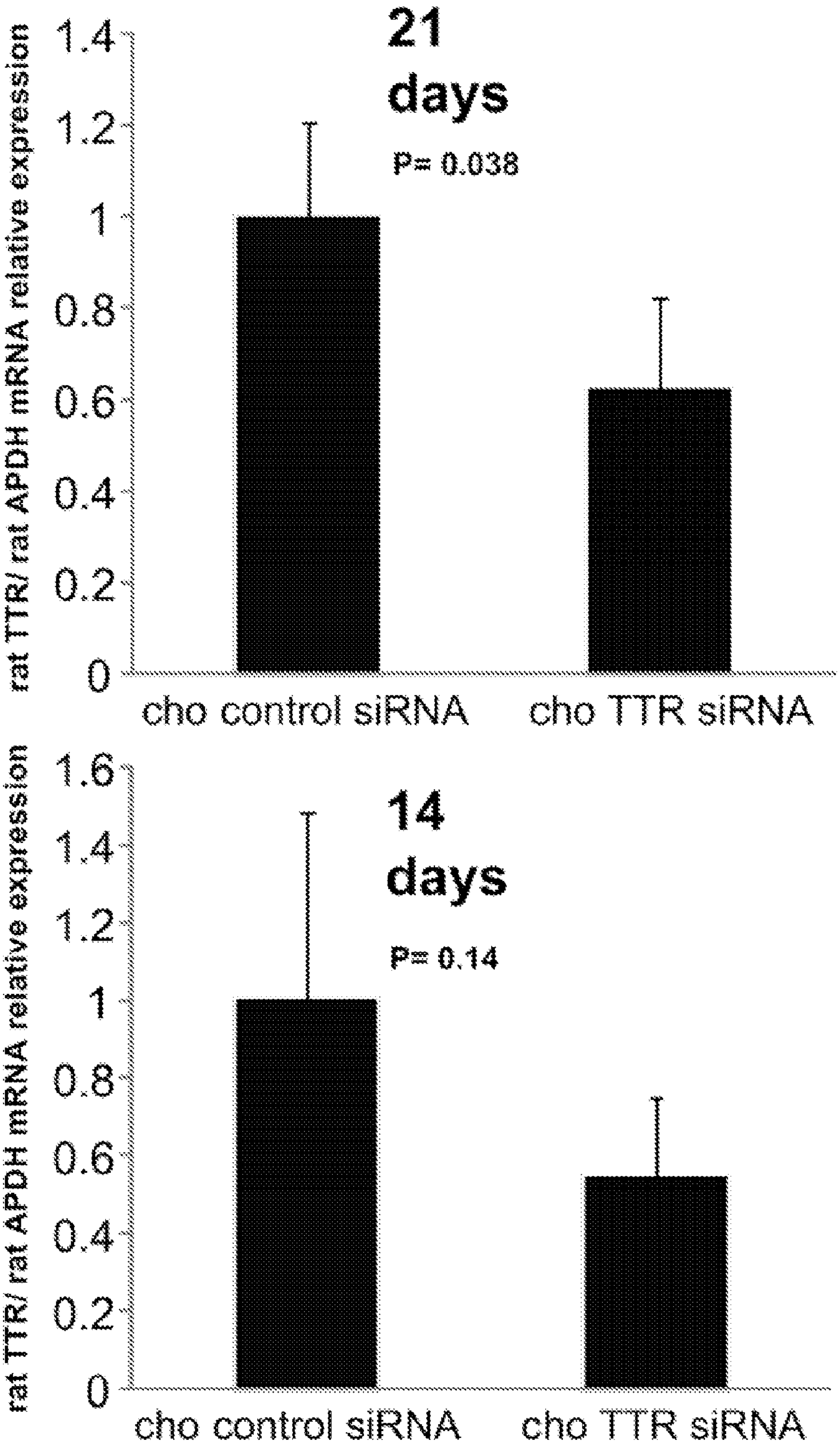
21
days
P= 0.038
rat TTR/ rat APDH mRNA relative expression
1.4
1.2
1
0.8
0.6
0.4
0.2
0
cho control siRNA
cho TTR siRNA
14
days
P= 0.14
rat TTR/ rat APDH mRNA relative expression
1.6
1.4
1.2
1
0.8
0.6
0.4
0.2
0
cho control siRNA
cho TTR siRNA

FIG. 26

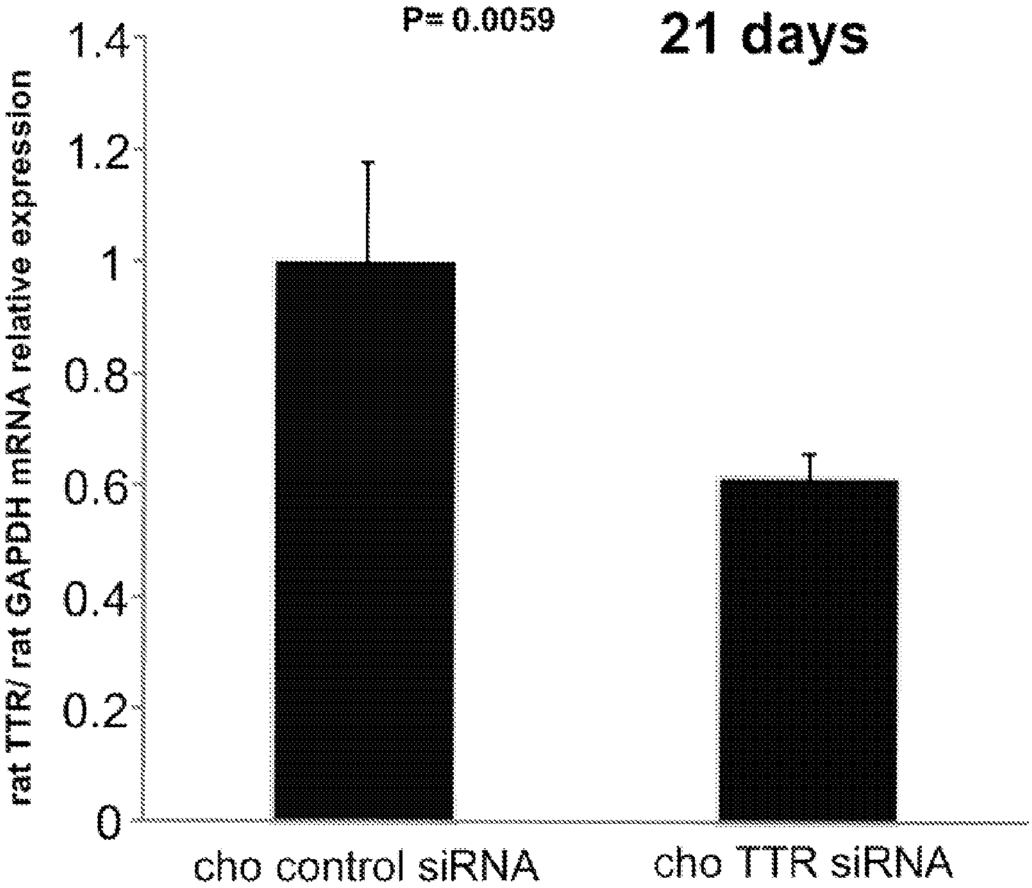
P= 0.0059
21 days
rat TTR/ rat GAPDH mRNA relative expression
1.4
1.2
1
0.8
0.6
0.4
0.2
0
cho control siRNA
cho TTR siRNA

FIG. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9932619 A, Fire
- WO 9953050 A, Waterhouse
- WO 9961631 A, Heifetz
- WO 0044895 A, Limmer
- DE 10100586, Kreutzer
- US 20070207974 A
- US 20090082300 A
- US 7250496 B
- US 20060240093 A
- US 20070135372 A
- US 61045228 B
- US 3687808 A
- US 4469863 A
- US 4476301 A
- US 5023243 A
- US 5177195 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541316 A
- US 5550111 A
- US 5563253 A
- US 5571799 A
- US 5587361 A
- US 5625050 A
- US 5034506 A
- US 5166315 A
- US 5185444 A
- US 5214134 A
- US 5216141 A
- US 5235033 A
- US 564562 A
- US 5264564 A
- US 5405938 A
- US 5434257 A
- US 5470967 A
- US 5489677 A
- US 5541307 A
- US 5561225 A
- US 5596086 A
- US 5602240 A
- US 5608046 A
- US 5610289 A
- US 5618704 A
- US 5623070 A
- US 5663312 A
- US 5633360 A
- US 5677437 A
- US 5677439 A
- US 5539082 A
- US 5714331 A
- US 5719262 A
- US 4981957 A
- US 5118800 A
- US 5319080 A
- US 5359044 A
- US 5393878 A
- US 5446137 A
- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5700920 A
- US 4845205 A
- US 513030 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A

- US 5614617 A
- US 5681941 A
- US 5750692 A
- US 4828979 A
- US 4948882 A
- US 5218105 A
- US 5525465 A
- US 5541313 A
- US 5545730 A
- US 5552538 A
- US 5578717 A
- US 5580731 A
- US 5591584 A
- US 5109124 A
- US 5118802 A
- US 5138045 A
- US 5414077 A
- US 5486603 A
- US 5512439 A
- US 5578718 A
- US 4587044 A
- US 4605735 A
- US 4667025 A
- US 4762779 A
- US 4789737 A
- US 4824941 A
- US 4835263 A
- US 4876335 A
- US 4904582 A
- US 4958013 A
- US 5082830 A
- US 5112963 A
- US 5214136 A
- US 5245022 A
- US 5254469 A
- US 5258506 A
- US 5262536 A
- US 5272250 A
- US 5292873 A
- US 5317098 A
- US 5371241 A
- US 5391723 A
- US 5416203 A
- US 5451463 A
- US 5510475 A
- US 5512667 A
- US 5565552 A
- US 5567810 A
- US 5574142 A
- US 5585481 A
- US 5587371 A
- US 5595726 A
- US 5597696 A
- US 5599923 A
- US 5599928 A
- US 5688941 A
- WO 0022113 A, Skillern, A.
- WO 0022114 A, Conrad
- US 6054299 A
- US 4868116 A
- US 4980286 A
- WO 8907136 A
- WO 8902468 A
- WO 8905345 A
- WO 9207573 A
- US 5252479 A
- US 5139941 A
- WO 9413788 A
- WO 9324641 A
- US 5328470 A
- US 6093180 A
- US 5814014 A
- US 6747014 B
- US 20050107325 A
- US 20050164235 A
- US 20050256069 A
- US 20080108801 A
- US 4837028 A
- WO 8804924 A
- US 5543152 A, Webb
- WO 9713499 A, Lim
- US 4426330 A
- US 4534899 A
- EP 0445131 B1
- WO 9004384 A, Fisher
- US 5013556 A, Woodle
- US 5356633 A
- US 5213804 A, Martin
- EP 0496813 B1
- WO 9105545 A
- US 5225212 A, Martin
- WO 9420073 A, Zalipsky
- WO 9610391 A, Choi
- US 5540935 A, Miyazaki
- US 5556948 A, Tagawa
- WO 9640062 A, Thierry
- US 5264221 A, Tagawa
- US 5665710 A, Rahman
- WO 9704787 A, Love
- WO 0003683 A
- US 5976567 A
- US 5981501 A
- US 6534484 B
- US 6586410 B
- US 6815432 B
- WO 9640964 A
- US 61107998 B
- WO 2008042973 A
- US 61239686 B
- US 61244834 B
- US 61175770 B
- US 6887906 B
- US 20030027780 A
- WO 2004065601 A
- EP 11763336 A
- US 61318704 B

- US 61318702 B

**Non-patent literature cited in the description**


- **YANG, D. et al.** *Curr. Biol.,* 2000, vol. 10, 1191-1200
- **ELBASHIR et al.** *EMBO,* 2001, vol. 20, 6877-6888
- **SOUTSCHEK et al.** *Nature,* 2004, vol. 432, 173-178
- Current protocols in nucleic acid chemistry. John Wiley & Sons, Inc,
- **NIELSEN et al.** *Science,* 1991, vol. 254, 1497-1500
- **MARTIN et al.** *Helv. Chim. Acta,* 1995, vol. 78, 486-504
- The Concise Encyclopedia Of Polymer Science And Engineering. John Wiley & Sons, 1990, 858-859
- **ENGLISCH et al.** *Angewandte Chemie, International Edition,* 1991, vol. 30, 613
- **SANGHVI, Y S.** DsRNA Research and Applications. CRC Press, 1993, 289-302
- DsRNA Research and Applications. CRC Press, 1993, 276-278
- **LETSINGER et al.** *Proc. Natl. Acid. Sci. USA,* 1989, vol. 86, 6553-6556
- **MANOHARAN et al.** *Biorg. Med. Chem. Let.,* 1994, vol. 4, 1053-1060
- **MANOHARAN et al.** *Ann. N.Y. Acad. Sci.,* 1992, vol. 660, 306-309
- **MANOHARAN et al.** *Biorg. Med. Chem. Let.,* 1993, vol. 3, 2765-2770
- **OBERHAUSER et al.** *Nucl. Acids Res.,* 1992, vol. 20, 533-538
- **SAISON-BEHMOARAS et al.** *EMBO J,* 1991, vol. 10, 1111-1118
- **KABANOV et al.** *FEBS Lett.,* 1990, vol. 259, 327-330
- **SVINARCHUK et al.** *Biochimie,* 1993, vol. 75, 49-54
- **MANOHARAN et al.** *Tetrahedron Lett.,* 1995, vol. 36, 3651-3654
- **SHEA et al.** *Nucl. Acids Res.,* 1990, vol. 18, 3777-3783
- **MANOHARAN et al.** *Nucleosides & Nucleotides,* 1995, vol. 14, 969-973
- **MISHRA et al.** *Biochim. Biophys. Acta,* 1995, vol. 1264, 229-237
- **CROOKE et al.** *J. Pharmacol. Exp. Ther.,* 1996, vol. 277, 923-937
- **LETSINGER et al.** *Proc. Natl. Acad. Sci. USA,* 1989, vol. 86, 6553
- **MANOHARAN et al.** *Bioorg. Med. Chem. Lett.,* 1994, vol. 4, 1053
- **MANOHARAN et al.** *Ann. N.Y. Acad. Sci.,* 1992, vol. 660, 306
- **MANOHARAN et al.** *Bioorg. Med. Chem. Let.,* 1993, vol. 3, 2765
- **OBERHAUSER et al.** *Nucl. Acids Res.,* 1992, vol. 20, 533
- **SAISON-BEHMOARAS et al.** *EMBO J.,* 1991, vol. 10, 111
- **KABANOV et al.** *FEBS Lett.,* 1990, vol. 259, 327
- **SVINARCHUK et al.** *Biochimie,* 1993, vol. 75, 49
- **MANOHARAN et al.** *Tetrahedron Lett.,* 1995, vol. 36, 3651
- **SHEA et al.** *Nucl. Acids Res.,* 1990, vol. 18, 3777
- **MANOHARAN et al.** *Nucleosides & Nucleotides,* 1995, vol. 14, 969
- **MISHRA et al.** *Biochim. Biophys. Acta,* 1995, vol. 1264, 229
- **CROOKE et al.** *J. Pharmacol. Exp. Ther.,* 1996, vol. 277, 923
- **COUTURE, A et al.** *TIG.,* 1996, vol. 12, 5-10
- **GASSMANN et al.** *Proc. Natl. Acad. Sci. USA,* 1995, vol. 92, 1292
- **MUZYCZKA et al.** *Curr. Topics Micro. Immunol.,* 1992, vol. 158, 97-129
- **BERKNER et al.** *BioTechniques,* 1998, vol. 6, 616
- **ROSENFELD et al.** *Science,* 1991, vol. 252, 431-434
- **ROSENFELD et al.** *Cell,* 1992, vol. 68, 143-155
- **EGLITIS et al.** *Science,* 1985, vol. 230, 1395-1398
- **DANOS ; MULLIGAN.** *Proc. Natl. Acad. Sci. USA,* 1998, vol. 85, 6460-6464
- **WILSON et al.** *Proc. Natl. Acad. Sci. USA,* 1988, vol. 85, 3014-3018
- **ARMENTANO et al.** *Proc. Natl. Acad. Sci. USA,* 1990, vol. 87, 61416145
- **HUBER et al.** *Proc. Natl. Acad. Sci. USA,* 1991, vol. 88, 8039-8043
- **FERRY et al.** *Proc. Natl. Acad. Sci. USA,* 1991, vol. 88, 8377-8381
- **CHOWDHURY et al.** *Science,* 1991, vol. 254, 1802-1805
- **VAN BEUSECHEM et al.** *Proc. Natl. Acad. Sci. USA,* 1992, vol. 89, 7640-19
- **KAY et al.** *Human Gene Therapy,* 1992, vol. 3, 641-647
- **DAI et al.** *Proc. Natl. Acad. Sci. USA,* 1992, vol. 89, 10892-10895
- **HWU et al.** *J. Immunol.,* 1993, vol. 150, 4104-4115

- **COMETTE et al.** *Human Gene Therapy,* 1991, vol. 2, 5-10
- **CONE et al.** *Proc. Natl. Acad. Sci. USA,* 1984, vol. 81, 6349
- **HSU et al.** *J. Infectious Disease,* 1992, vol. 166, 769
- **RABINOWITZ J E et al.** *J Virol,* 2002, vol. 76, 791-801
- **DORNBURG R.** *Gene Therap.,* 1995, vol. 2, 301-310
- **EGLITIS M A.** *Biotechniques,* 1988, vol. 6, 608-614
- **MILLER A D.** *Hum Gene Therap.,* 1990, vol. 1, 5-14
- **ANDERSON W F.** *Nature,* 1998, vol. 392, 25-30
- **RUBINSON D A et al.** *Nat. Genet.,* vol. 33, 401-406
- **XIA H et al.** *Nat. Biotech.,* 2002, vol. 20, 1006-1010
- **SAMULSKI R et al.** *J. Virol.,* 1987, vol. 61, 3096-3101
- **FISHER K J et al.** *J. Virol,* 1996, vol. 70, 520-532
- **SAMULSKI R et al.** *J. Virol.,* 1989, vol. 63, 3822-3826
- **BUCCHINI et al.** *Proc. Natl. Acad. Sci. USA,* 1986, vol. 83, 2511-2515
- **DOCHERTY et al.** *FASEB J.,* 1994, vol. 8, 20-24
- **CHEN et al.** *Proc. Natl. Acad. Sci. USA,* 1994, vol. 91, 3054-3057
- **ROSOFF.** Pharmaceutical Dosage Forms. Marcel Dekker, Inc, 1988, vol. 1, 245
- **WANG et al.** *Biochem. Biophys. Res. Commun.,* 1987, vol. 147, 980-985
- **ZHOU et al.** *Journal of Controlled Release,* 1992, vol. 19, 269-274
- **WEINER et al.** *Journal of Drug Targeting,* 1992, vol. 2, 405-410
- **DU PLESSIS et al.** *Antiviral Research,* 1992, vol. 18, 259-265
- **HU et al.** *S.T.P.Pharma. Sci.,* 1994, vol. 4 (6), 466
- **ALLEN et al.** *FEBS Letters,* 1987, vol. 223, 42
- **WU et al.** *Cancer Research,* 1993, vol. 53, 3765
- **PAPAHADJOPOULOS et al.** *Ann. N.Y. Acad. Sci.,* 1987, vol. 507, 64
- **GABIZON et al.** *Proc. Natl. Acad. Sci. U.S.A.,* 1988, vol. 85, 6949
- **SUNAMOTO et al.** *Bull. Chem. Soc. Jpn.,* 1980, vol. 53, 2778
- **ILLUM et al.** *FEBS Lett.,* 1984, vol. 167, 79
- **KLIBANOV et al.** *FEBS Lett.,* 1990, vol. 268, 235
- **BLUME et al.** *Biochimica et Biophysica Acta,* 1990, vol. 1029, 91
- **RIEGER.** Pharmaceutical Dosage Forms. Marcel Dekker, Inc, 1988, 285
- **IDSON.** Pharmaceutical Dosage Forms. Marcel Dekker, Inc, 1988, vol. 1, 199
- **BLOCK.** Pharmaceutical Dosage Forms. Marcel Dekker, Inc, 1988, vol. 2, 335
- **HIGUCHI et al.** Remington's Pharmaceutical Sciences. Mack Publishing Co, 1985, 301
- **IDSON.** Pharmaceutical Dosage Forms, Lieberman. Marcel Dekker, Inc, 1988, vol. 1, 199
- **RIEGER.** Pharmaceutical Dosage Forms. Marcel Dekker, Inc, 1988, vol. 1, 285
- **BLOCK.** Pharmaceutical Dosage Forms. Marcel Dekker, Inc, 1988, vol. 1, 335
- **LEUNG ; SHAH.** Controlled Release of Drugs: Polymers and Aggregate Systems. VCH Publishers, 1989, 185-215
- **SCHOTT.** Remington's Pharmaceutical Sciences. Mack Publishing Co, 1985, 271
- **CONSTANTINIDES et al.** Pharmaceutical Research. 1994, vol. 11, 1385-1390
- **RITSCHEL.** Meth. Find. Exp. Clin. Pharmacol. 1993, vol. 13, 205
- **CONSTANTINIDES et al.** *Pharmaceutical Research,* 1994, vol. 11, 1385
- **HO et al.** *J. Pharm. Sci.,* 1996, vol. 85, 138-143
- **LEE et al.** *Critical Reviews in Therapeutic Drug Carrier Systems,* 1991, 92
- **TAKAHASHI et al.** *J. Pharm. Pharmacol.,* 1988, vol. 40, 252
- **MURANISHI.** *Critical Reviews in Therapeutic Drug Carrier Systems,* 1990, vol. 7, 1-33
- **EL HARIRI et al.** *J. Pharm. Pharmacol.,* 1992, vol. 44, 651-654
- **BRUNTON et al.** Goodman & Gilman's The Pharmacological Basis of Therapeutics. McGraw-Hill, 1996, 934-935
- **SWINYARD.** Remington's Pharmaceutical Sciences. Mack Publishing Co, 1990, 782-783
- **YAMAMOTO et al.** *J. Pharm. Exp. Ther.,* 1992, vol. 263, 25
- **YAMASHITA et al.** J. Pharm. Sci. 1990, vol. 79, 579-583
- **JARRETT.** *J. Chromatogr.,* 1993, vol. 618, 315-339
- **MURANISHI.** Critical Reviews in Therapeutic Drug Carrier Systems. 1990, vol. 7, 1-33
- **BUUR et al.** J. Control Rel. 1990, vol. 14, 43-51
- **YAMASHITA et al.** *J. Pharm. Pharmacol.,* 1987, vol. 39, 621-626
- **MIYAO et al.** *DsRNA Res. Dev.,* 1995, vol. 5, 115-121

- **TAKAKURA et al.** *DsRNA & Nucl. Acid Drug Dev.,* 1996, vol. 6, 177-183
- **KAWAJI, T. et al.** *Ophthalmology,* 2010, vol. 117, 552-555
- **MAEDA, S.** Use of genetically altered mice to study the role of serum amyloid P component in amyloid deposition. *Amyloid Suppl.,* 2003, vol. 1, 17-20
- **MAEDA, S.** Use of genetically altered mice to study the role of serum amyloid P component in amyloid deposition. *Amyloid,* 2003, 17-20
- **JEFFS LB et al.** *A Scalable, Extrusion-Free Method for Efficient Liposomal Encapsulation of Plasmid DNA. Pharm Res,* 2004, vol. 22, 362-372

SEQUENCE LISTING

<110> ALNYLAM PHARMACEUTICALS, INC. KUMAMOTO UNIVERSITY

<120> SIRNA THERAPY FOR TRANSTHYRETIN (TTR) RELATED OCULAR AMYLOIDOSIS

<130> U2557 EP

<140> EP 11 76 3336.2
<141> 2011-03-29

<150> US 61/318,704
<151> 2010-03-29

<150> US 61/318,702
<151> 2010-03-29

<160> 1424

<170> PatentIn version 3.5

<210> 1
<211> 19
<212> RNA
<213> Artificial Sequence

<220>
<223> Description of Artificial Sequence: Synthetic oligonucleotide

<400> 1
ccggugaauc caagugucc 19

<210> 2
<211> 19
<212> RNA
<213> Artificial Sequence

<220>
<223> Description of Artificial Sequence: Synthetic oligonucleotide

<400> 2
ggacacuugg auucaccgg 19

<210> 3
<211> 19
<212> RNA
<213> Artificial Sequence

<220>
<223> Description of Artificial Sequence: Synthetic oligonucleotide

<400> 3
acucauucuu ggcaggaug 19

<210> 4
<211> 19
<212> RNA
<213> Artificial Sequence

<220>
<223> Description of Artificial Sequence: Synthetic oligonucleotide

<400> 4
cauccugcca agaaugagu 19

<210> 5
<211> 19
<212> RNA
<213> Artificial Sequence

<220>
<223> Description of Artificial Sequence: Synthetic oligonucleotide

<400> 5
aaguguccuc ugaugguca 19

<210> 6
<211> 19
<212> RNA
<213> Artificial Sequence

<220>
<223> Description of Artificial Sequence: Synthetic oligonucleotide

<400> 6
ugaccaucag aggacacuu 19

<210> 7
<211> 19
<212> RNA
<213> Artificial Sequence

<220>
<223> Description of Artificial Sequence: Synthetic oligonucleotide

<400> 7
ucauucuugg caggauggc 19

<210> 8
<211> 19
<212> RNA
<213> Artificial Sequence

<220>
<223> Description of Artificial Sequence: Synthetic oligonucleotide

<400> 8
gccauccugc caagaauga 19

<210> 9
<211> 19
<212> RNA
<213> Artificial Sequence

<220>
<223> Description of Artificial Sequence: Synthetic oligonucleotide

<400> 9
aaguucuaga ugcuguccg 19

<210> 10
<211> 19
<212> RNA
<213> Artificial Sequence

<220>
<223> Description of Artificial Sequence: Synthetic oligonucleotide

<400> 10
cggacagcau cuagaacuu 19

<210> 11
<211> 19
<212> RNA
<213> Artificial Sequence

<220>
<223> Description of Artificial Sequence: Synthetic oligonucleotide

<400> 11
guucuagaug cuguccgag 19

<210> 12
<211> 19
<212> RNA
<213> Artificial Sequence

<220>
<223> Description of Artificial Sequence: Synthetic oligonucleotide

<400> 12
cucggacagc aucuagaac 19

<210> 13
<211> 19
<212> RNA
<213> Artificial Sequence

<220>
<223> Description of Artificial Sequence: Synthetic oligonucleotide

<400> 13
cuagaugcug uccgaggca 19

<210> 14
<211> 19
<212> RNA
<213> Artificial Sequence

<220>
<223> Description of Artificial Sequence: Synthetic oligonucleotide

<400> 14
ugccucggac agcaucuag 19

<210> 15
<211> 19
<212> RNA
<213> Artificial Sequence

<220>
<223> Description of Artificial Sequence: Synthetic oligonucleotide

<400> 15
gaugcugucc gaggcaguc 19

<210> 16
<211> 19
<212> RNA
<213> Artificial Sequence

<220>
<223> Description of Artificial Sequence: Synthetic oligonucleotide

<400> 16
gacugccucg gacagcauc 19

<210> 17
<211> 19
<212> RNA

<213> Artificial Sequence

<220>
<223> Description of Artificial Sequence: Synthetic oligonucleotide

<400> 17
cauucuuggc aggauggcu 19

<210> 18
<211> 19
<212> RNA
<213> Artificial Sequence

<220>
<223> Description of Artificial Sequence: Synthetic oligonucleotide

<400> 18
agccauccug ccaagaaug 19

<210> 19
<211> 19
<212> RNA
<213> Artificial Sequence

<220>
<223> Description of Artificial Sequence: Synthetic oligonucleotide

<400> 19
ugcuguccga ggcaguccu 19

<210> 20
<211> 19
<212> RNA
<213> Artificial Sequence

<220>
<223> Description of Artificial Sequence: Synthetic oligonucleotide

<400> 20
aggacugccu cggacagca 19

<210> 21
<211> 19
<212> RNA
<213> Artificial Sequence

<220>
<223> Description of Artificial Sequence: Synthetic oligonucleotide

<400> 21
ccgaggcagu ccugccauc 19

<210> 22
<211> 19
<212> RNA
<213> Artificial Sequence

<220>

<223> Description of Artificial Sequence: Synthetic oligonucleotide

<400> 22
gauggcagga cugccucgg 19

<210> 23
<211> 19
<212> RNA
<213> Artificial Sequence

<220>
<223> Description of Artificial Sequence: Synthetic oligonucleotide

<400> 23
caguccugcc aucaaugug 19

<210> 24
<211> 19
<212> RNA
<213> Artificial Sequence

<220>
<223> Description of Artificial Sequence: Synthetic oligonucleotide

<400> 24
cacauugaug gcaggacug 19

<210> 25
<211> 19
<212> RNA
<213> Artificial Sequence

<220>
<223> Description of Artificial Sequence: Synthetic oligonucleotide

<400> 25
caauguggcc gugcaugug 19

<210> 26
<211> 19
<212> RNA
<213> Artificial Sequence

<220>
<223> Description of Artificial Sequence: Synthetic oligonucleotide

<400> 26
cacaugcacg gccacauug 19

<210> 27
<211> 19
<212> RNA
<213> Artificial Sequence

<220>
<223> Description of Artificial Sequence: Synthetic oligonucleotide

<400> 27

auguguucag aaaggcugc 19

<210> 28
<211> 19
<212> RNA
<213> Artificial Sequence

<220>
<223> Description of Artificial Sequence: Synthetic oligonucleotide

<400> 28
gcagccuuuc ugaacacau 19

<210> 29
<211> 19
<212> RNA
<213> Artificial Sequence

<220>
<223> Description of Artificial Sequence: Synthetic oligonucleotide

<400> 29
cagaagucca cucauucuu 19

<210> 30
<211> 19
<212> RNA
<213> Artificial Sequence

<220>
<223> Description of Artificial Sequence: Synthetic oligonucleotide

<400> 30
aagaaugagu ggacuucug 19

<210> 31
<211> 19
<212> RNA
<213> Artificial Sequence

<220>
<223> Description of Artificial Sequence: Synthetic oligonucleotide

<400> 31
ggcaggaugg cuucucauc 19

<210> 32
<211> 19
<212> RNA
<213> Artificial Sequence

<220>
<223> Description of Artificial Sequence: Synthetic oligonucleotide

<400> 32
gaugagaagc cauccugcc 19

<210> 33

<211> 19
<212> RNA
<213> Artificial Sequence

<220>
<223> Description of Artificial Sequence: Synthetic oligonucleotide

<400> 33
gagccauuug ccucuggga 19

<210> 34
<211> 19
<212> RNA
<213> Artificial Sequence

<220>
<223> Description of Artificial Sequence: Synthetic oligonucleotide

<400> 34
ucccagaggc aaauggcuc 19

<210> 35
<211> 19
<212> RNA
<213> Artificial Sequence

<220>
<223> Description of Artificial Sequence: Synthetic oligonucleotide

<400> 35
caggauggcu ucucaucgu 19

<210> 36
<211> 19
<212> RNA
<213> Artificial Sequence

<220>
<223> Description of Artificial Sequence: Synthetic oligonucleotide

<400> 36
acgaugagaa gccauccug 19

<210> 37
<211> 19
<212> RNA
<213> Artificial Sequence

<220>
<223> Description of Artificial Sequence: Synthetic oligonucleotide

<400> 37
aggauggcuu cucaucguc 19

<210> 38
<211> 19
<212> RNA
<213> Artificial Sequence

<220>
<223> Description of Artificial Sequence: Synthetic oligonucleotide

<400> 38
gacgaugaga agccauccu 19

<210> 39
<211> 19
<212> RNA
<213> Artificial Sequence

<220>
<223> Description of Artificial Sequence: Synthetic oligonucleotide

<400> 39
agagcugcau gggcucaca 19

<210> 40
<211> 19
<212> RNA
<213> Artificial Sequence

<220>
<223> Description of Artificial Sequence: Synthetic oligonucleotide

<400> 40
ugugagccca ugcagcucu 19

<210> 41
<211> 19
<212> RNA
<213> Artificial Sequence

<220>
<223> Description of Artificial Sequence: Synthetic oligonucleotide

<400> 41
gcugcauggg cucacaacu 19

<210> 42
<211> 19
<212> RNA
<213> Artificial Sequence

<220>
<223> Description of Artificial Sequence: Synthetic oligonucleotide

<400> 42
aguugugagc ccaugcagc 19

<210> 43
<211> 19
<212> RNA
<213> Artificial Sequence

<220>
<223> Description of Artificial Sequence: Synthetic oligonucleotide

<400> 43
ggauggcuuc ucaucgucu 19

<210> 44
<211> 19
<212> RNA
<213> Artificial Sequence

<220>
<223> Description of Artificial Sequence: Synthetic oligonucleotide

<400> 44
agacgaugag aagccaucc 19

<210> 45
<211> 19
<212> RNA
<213> Artificial Sequence

<220>
<223> Description of Artificial Sequence: Synthetic oligonucleotide

<400> 45
gcaugggcuc acaacugag 19

<210> 46
<211> 19
<212> RNA
<213> Artificial Sequence

<220>
<223> Description of Artificial Sequence: Synthetic oligonucleotide

<400> 46
cucaguugug agcccaugc 19

<210> 47
<211> 19
<212> RNA
<213> Artificial Sequence

<220>
<223> Description of Artificial Sequence: Synthetic oligonucleotide

<400> 47
augggcucac aacugagga 19

<210> 48
<211> 19
<212> RNA
<213> Artificial Sequence

<220>
<223> Description of Artificial Sequence: Synthetic oligonucleotide

<400> 48
uccucaguug ugagcccau 19

<210> 49
<211> 19
<212> RNA
<213> Artificial Sequence

<220>
<223> Description of Artificial Sequence: Synthetic oligonucleotide

<400> 49
ugggcucaca acugaggag 19

<210> 50
<211> 19
<212> RNA
<213> Artificial Sequence

<220>
<223> Description of Artificial Sequence: Synthetic oligonucleotide

<400> 50
cuccucaguu gugagccca 19

<210> 51
<211> 19
<212> RNA
<213> Artificial Sequence

<220>
<223> Description of Artificial Sequence: Synthetic oligonucleotide

<400> 51
gaggaauuug uagaaggga 19

<210> 52
<211> 19
<212> RNA
<213> Artificial Sequence

<220>
<223> Description of Artificial Sequence: Synthetic oligonucleotide

<400> 52
ucccuucuac aaauuccuc 19

<210> 53
<211> 19
<212> RNA
<213> Artificial Sequence

<220>
<223> Description of Artificial Sequence: Synthetic oligonucleotide

<400> 53
uuuguagaag ggauauaca 19

<210> 54
<211> 19
<212> RNA

<213> Artificial Sequence

<220>
<223> Description of Artificial Sequence: Synthetic oligonucleotide

<400> 54
uguauauccc uucuacaaa 19

<210> 55
<211> 19
<212> RNA
<213> Artificial Sequence

<220>
<223> Description of Artificial Sequence: Synthetic oligonucleotide

<400> 55
uuguagaagg gauauacaa 19

<210> 56
<211> 19
<212> RNA
<213> Artificial Sequence

<220>
<223> Description of Artificial Sequence: Synthetic oligonucleotide

<400> 56
uuguauaucc cuucuacaa 19

<210> 57
<211> 19
<212> RNA
<213> Artificial Sequence

<220>
<223> Description of Artificial Sequence: Synthetic oligonucleotide

<400> 57
uguagaaggg auauacaaa 19

<210> 58
<211> 19
<212> RNA
<213> Artificial Sequence

<220>
<223> Description of Artificial Sequence: Synthetic oligonucleotide

<400> 58
uuuguauauc ccuucuaca 19

<210> 59
<211> 19
<212> RNA
<213> Artificial Sequence

<220>

<223> Description of Artificial Sequence: Synthetic oligonucleotide

<400> 59
agaagggaua uacaaagug 19

<210> 60
<211> 19
<212> RNA
<213> Artificial Sequence

<220>
<223> Description of Artificial Sequence: Synthetic oligonucleotide

<400> 60
cacuuuguau aucccuucu 19

<210> 61
<211> 19
<212> RNA
<213> Artificial Sequence

<220>
<223> Description of Artificial Sequence: Synthetic oligonucleotide

<400> 61
aaguggaaau agacaccaa 19

<210> 62
<211> 19
<212> RNA
<213> Artificial Sequence

<220>
<223> Description of Artificial Sequence: Synthetic oligonucleotide

<400> 62
uuggugucua uuuccacuu 19

<210> 63
<211> 19
<212> RNA
<213> Artificial Sequence

<220>
<223> Description of Artificial Sequence: Synthetic oligonucleotide

<400> 63
ggaaauagac accaaaucu 19

<210> 64
<211> 19
<212> RNA
<213> Artificial Sequence

<220>
<223> Description of Artificial Sequence: Synthetic oligonucleotide

<400> 64

agauuuggug ucuauuucc 19

<210> 65
<211> 19
<212> RNA
<213> Artificial Sequence

<220>
<223> Description of Artificial Sequence: Synthetic oligonucleotide

<400> 65
gaaauagaca ccaaaucuu 19

<210> 66
<211> 19
<212> RNA
<213> Artificial Sequence

<220>
<223> Description of Artificial Sequence: Synthetic oligonucleotide

<400> 66
aagauuuggu gucuauuuc 19

<210> 67
<211> 19
<212> RNA
<213> Artificial Sequence

<220>
<223> Description of Artificial Sequence: Synthetic oligonucleotide

<400> 67
auagacacca aaucuuacu 19

<210> 68
<211> 19
<212> RNA
<213> Artificial Sequence

<220>
<223> Description of Artificial Sequence: Synthetic oligonucleotide

<400> 68
aguaagauuu ggugucuau 19

<210> 69
<211> 19
<212> RNA
<213> Artificial Sequence

<220>
<223> Description of Artificial Sequence: Synthetic oligonucleotide

<400> 69
uagacaccaa aucuuacug 19

<210> 70

<211> 19
<212> RNA
<213> Artificial Sequence

<220>
<223> Description of Artificial Sequence: Synthetic oligonucleotide

<400> 70
caguaagauu uggugucua 19

<210> 71
<211> 19
<212> RNA
<213> Artificial Sequence

<220>
<223> Description of Artificial Sequence: Synthetic oligonucleotide

<400> 71
agacaccaaa ucuuacugg 19

<210> 72
<211> 19
<212> RNA
<213> Artificial Sequence

<220>
<223> Description of Artificial Sequence: Synthetic oligonucleotide

<400> 72
ccaguaagau uuggugucu 19

<210> 73
<211> 19
<212> RNA
<213> Artificial Sequence

<220>
<223> Description of Artificial Sequence: Synthetic oligonucleotide

<400> 73
uuacuggaag gcacuuggc 19

<210> 74
<211> 19
<212> RNA
<213> Artificial Sequence

<220>
<223> Description of Artificial Sequence: Synthetic oligonucleotide

<400> 74
gccaagugcc uuccaguaa 19

<210> 75
<211> 19
<212> RNA
<213> Artificial Sequence

<220>
<223> Description of Artificial Sequence: Synthetic oligonucleotide

<400> 75
uucucaucgu cugcuccuc 19

<210> 76
<211> 19
<212> RNA
<213> Artificial Sequence

<220>
<223> Description of Artificial Sequence: Synthetic oligonucleotide

<400> 76
gaggagcaga cgaugagaa 19

<210> 77
<211> 19
<212> RNA
<213> Artificial Sequence

<220>
<223> Description of Artificial Sequence: Synthetic oligonucleotide

<400> 77
ggaaggcacu uggcaucuc 19

<210> 78
<211> 19
<212> RNA
<213> Artificial Sequence

<220>
<223> Description of Artificial Sequence: Synthetic oligonucleotide

<400> 78
gagaugccaa gugccuucc 19

<210> 79
<211> 19
<212> RNA
<213> Artificial Sequence

<220>
<223> Description of Artificial Sequence: Synthetic oligonucleotide

<400> 79
ggcacuuggc aucucccca 19

<210> 80
<211> 19
<212> RNA
<213> Artificial Sequence

<220>
<223> Description of Artificial Sequence: Synthetic oligonucleotide

<400> 80
uggggagaug ccaagugcc 19

<210> 81
<211> 19
<212> RNA
<213> Artificial Sequence

<220>
<223> Description of Artificial Sequence: Synthetic oligonucleotide

<400> 81
ggcaucuccc cauuccaug 19

<210> 82
<211> 20
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 82
auggaauggg gagaugcctt 20

<210> 83
<211> 19
<212> RNA
<213> Artificial Sequence

<220>
<223> Description of Artificial Sequence: Synthetic oligonucleotide

<400> 83
gcaucucccc auuccauga 19

<210> 84
<211> 19
<212> RNA
<213> Artificial Sequence

<220>
<223> Description of Artificial Sequence: Synthetic oligonucleotide

<400> 84
ucauggaaug gggagaugc 19

<210> 85
<211> 19
<212> RNA
<213> Artificial Sequence

<220>
<223> Description of Artificial Sequence: Synthetic oligonucleotide

<400> 85
caucucccca uuccaugag 19

<210> 86
<211> 19
<212> RNA
<213> Artificial Sequence

<220>
<223> Description of Artificial Sequence: Synthetic oligonucleotide

<400> 86
cucauggaau ggggagaug 19

<210> 87
<211> 19
<212> RNA
<213> Artificial Sequence

<220>
<223> Description of Artificial Sequence: Synthetic oligonucleotide

<400> 87
aucuccccau uccaugagc 19

<210> 88
<211> 19
<212> RNA
<213> Artificial Sequence

<220>
<223> Description of Artificial Sequence: Synthetic oligonucleotide

<400> 88
gcucauggaa uggggagau 19

<210> 89
<211> 19
<212> RNA
<213> Artificial Sequence

<220>
<223> Description of Artificial Sequence: Synthetic oligonucleotide

<400> 89
cuccccauuc caugagcau 19

<210> 90
<211> 19
<212> RNA
<213> Artificial Sequence

<220>
<223> Description of Artificial Sequence: Synthetic oligonucleotide

<400> 90
augcucaugg aauggggag 19

<210> 91
<211> 19
<212> RNA
<213> Artificial Sequence

<220>
<223> Description of Artificial Sequence: Synthetic oligonucleotide

<400> 91
cccauuccau gagcaugca 19

<210> 92
<211> 19
<212> RNA
<213> Artificial Sequence

<220>
<223> Description of Artificial Sequence: Synthetic oligonucleotide

<400> 92
ugcaugcuca uggaauggg 19

<210> 93
<211> 19
<212> RNA
<213> Artificial Sequence

<220>
<223> Description of Artificial Sequence: Synthetic oligonucleotide

<400> 93
ccaugagcau gcagaggug 19

<210> 94
<211> 19
<212> RNA
<213> Artificial Sequence

<220>
<223> Description of Artificial Sequence: Synthetic oligonucleotide

<400> 94
caccucugca ugcucaugg 19

<210> 95
<211> 19
<212> RNA
<213> Artificial Sequence

<220>
<223> Description of Artificial Sequence: Synthetic oligonucleotide

<400> 95
agcaugcaga ggugguauu 19

<210> 96
<211> 19
<212> RNA

<213> Artificial Sequence

<220>
<223> Description of Artificial Sequence: Synthetic oligonucleotide

<400> 96
aauaccaccu cugcaugcu 19

<210> 97
<211> 19
<212> RNA
<213> Artificial Sequence

<220>
<223> Description of Artificial Sequence: Synthetic oligonucleotide

<400> 97
caugcagagg ugguauuca 19

<210> 98
<211> 19
<212> RNA
<213> Artificial Sequence

<220>
<223> Description of Artificial Sequence: Synthetic oligonucleotide

<400> 98
ugaauaccac cucugcaug 19

<210> 99
<211> 19
<212> RNA
<213> Artificial Sequence

<220>
<223> Description of Artificial Sequence: Synthetic oligonucleotide

<400> 99
augcagaggu gguauucac 19

<210> 100
<211> 19
<212> RNA
<213> Artificial Sequence

<220>
<223> Description of Artificial Sequence: Synthetic oligonucleotide

<400> 100
gugaauacca ccucugcau 19

<210> 101
<211> 19
<212> RNA
<213> Artificial Sequence

<220>

<223> Description of Artificial Sequence: Synthetic oligonucleotide

<400> 101
ggugguauuc acagccaac 19

<210> 102
<211> 19
<212> RNA
<213> Artificial Sequence

<220>
<223> Description of Artificial Sequence: Synthetic oligonucleotide

<400> 102
guuggcugug aauaccacc 19

<210> 103
<211> 19
<212> RNA
<213> Artificial Sequence

<220>
<223> Description of Artificial Sequence: Synthetic oligonucleotide

<400> 103
gugguauuca cagccaacg 19

<210> 104
<211> 19
<212> RNA
<213> Artificial Sequence

<220>
<223> Description of Artificial Sequence: Synthetic oligonucleotide

<400> 104
cguuggcugu gaauaccac 19

<210> 105
<211> 19
<212> RNA
<213> Artificial Sequence

<220>
<223> Description of Artificial Sequence: Synthetic oligonucleotide

<400> 105
ugguauucac agccaacga 19

<210> 106
<211> 19
<212> RNA
<213> Artificial Sequence

<220>
<223> Description of Artificial Sequence: Synthetic oligonucleotide

<400> 106

ucguuggcug ugaauacca 19

<210> 107
<211> 19
<212> RNA
<213> Artificial Sequence

<220>
<223> Description of Artificial Sequence: Synthetic oligonucleotide

<400> 107
gguauucaca gccaacgac 19

<210> 108
<211> 19
<212> RNA
<213> Artificial Sequence

<220>
<223> Description of Artificial Sequence: Synthetic oligonucleotide

<400> 108
gucguuggcu gugaauacc 19

<210> 109
<211> 19
<212> RNA
<213> Artificial Sequence

<220>
<223> Description of Artificial Sequence: Synthetic oligonucleotide

<400> 109
guauucacag ccaacgacu 19

<210> 110
<211> 19
<212> RNA
<213> Artificial Sequence

<220>
<223> Description of Artificial Sequence: Synthetic oligonucleotide

<400> 110
agucguuggc ugugaauac 19

<210> 111
<211> 19
<212> RNA
<213> Artificial Sequence

<220>
<223> Description of Artificial Sequence: Synthetic oligonucleotide

<400> 111
uauucacagc caacgacuc 19

<210> 112

<211> 19
<212> RNA
<213> Artificial Sequence

<220>
<223> Description of Artificial Sequence: Synthetic oligonucleotide

<400> 112
gagucguugg cugugaaua 19

<210> 113
<211> 19
<212> RNA
<213> Artificial Sequence

<220>
<223> Description of Artificial Sequence: Synthetic oligonucleotide

<400> 113
ucacagccaa cgacuccgg 19

<210> 114
<211> 19
<212> RNA
<213> Artificial Sequence

<220>
<223> Description of Artificial Sequence: Synthetic oligonucleotide

<400> 114
ccggagucgu uggcuguga 19

<210> 115
<211> 19
<212> RNA
<213> Artificial Sequence

<220>
<223> Description of Artificial Sequence: Synthetic oligonucleotide

<400> 115
ccccgccgcu acaccauug 19

<210> 116
<211> 19
<212> RNA
<213> Artificial Sequence

<220>
<223> Description of Artificial Sequence: Synthetic oligonucleotide

<400> 116
caauggugua gcggcgggg 19

<210> 117
<211> 19
<212> RNA
<213> Artificial Sequence

<220>
<223> Description of Artificial Sequence: Synthetic oligonucleotide

<400> 117
gaaguccacu cauucuugg 19

<210> 118
<211> 19
<212> RNA
<213> Artificial Sequence

<220>
<223> Description of Artificial Sequence: Synthetic oligonucleotide

<400> 118
ccaagaauga guggacuuc 19

<210> 119
<211> 19
<212> RNA
<213> Artificial Sequence

<220>
<223> Description of Artificial Sequence: Synthetic oligonucleotide

<400> 119
cccugcugag ccccuacuc 19

<210> 120
<211> 19
<212> RNA
<213> Artificial Sequence

<220>
<223> Description of Artificial Sequence: Synthetic oligonucleotide

<400> 120
gaguaggggc ucagcaggg 19

<210> 121
<211> 19
<212> RNA
<213> Artificial Sequence

<220>
<223> Description of Artificial Sequence: Synthetic oligonucleotide

<400> 121
cugagccccu acuccuauu 19

<210> 122
<211> 19
<212> RNA
<213> Artificial Sequence

<220>
<223> Description of Artificial Sequence: Synthetic oligonucleotide

<400> 122
aauaggagua ggggcucag 19

<210> 123
<211> 19
<212> RNA
<213> Artificial Sequence

<220>
<223> Description of Artificial Sequence: Synthetic oligonucleotide

<400> 123
ugagccccua cuccuauuc 19

<210> 124
<211> 19
<212> RNA
<213> Artificial Sequence

<220>
<223> Description of Artificial Sequence: Synthetic oligonucleotide

<400> 124
gaauaggagu aggggcuca 19

<210> 125
<211> 19
<212> RNA
<213> Artificial Sequence

<220>
<223> Description of Artificial Sequence: Synthetic oligonucleotide

<400> 125
ccccuacucc uauuccacc 19

<210> 126
<211> 19
<212> RNA
<213> Artificial Sequence

<220>
<223> Description of Artificial Sequence: Synthetic oligonucleotide

<400> 126
gguggaauag gaguagggg 19

<210> 127
<211> 19
<212> RNA
<213> Artificial Sequence

<220>
<223> Description of Artificial Sequence: Synthetic oligonucleotide

<400> 127
cuacuccuau uccaccacg 19

<210> 128
<211> 19
<212> RNA
<213> Artificial Sequence

<220>
<223> Description of Artificial Sequence: Synthetic oligonucleotide

<400> 128
cgugguggaa uaggaguag 19

<210> 129
<211> 19
<212> RNA
<213> Artificial Sequence

<220>
<223> Description of Artificial Sequence: Synthetic oligonucleotide

<400> 129
uacuccuauu ccaccacgg 19

<210> 130
<211> 19
<212> RNA
<213> Artificial Sequence

<220>
<223> Description of Artificial Sequence: Synthetic oligonucleotide

<400> 130
ccguggugga auaggagua 19

<210> 131
<211> 19
<212> RNA
<213> Artificial Sequence

<220>
<223> Description of Artificial Sequence: Synthetic oligonucleotide

<400> 131
acuccuauuc caccacggc 19

<210> 132
<211> 19
<212> RNA
<213> Artificial Sequence

<220>
<223> Description of Artificial Sequence: Synthetic oligonucleotide

<400> 132
gccguggugg aauaggagu 19

<210> 133
<211> 19
<212> RNA

<213> Artificial Sequence

<220>
<223> Description of Artificial Sequence: Synthetic oligonucleotide

<400> 133
uccuauucca ccacggcug 19

<210> 134
<211> 19
<212> RNA
<213> Artificial Sequence

<220>
<223> Description of Artificial Sequence: Synthetic oligonucleotide

<400> 134
cagccguggu ggaauagga 19

<210> 135
<211> 19
<212> RNA
<213> Artificial Sequence

<220>
<223> Description of Artificial Sequence: Synthetic oligonucleotide

<400> 135
uauuccacca cggcugucg 19

<210> 136
<211> 19
<212> RNA
<213> Artificial Sequence

<220>
<223> Description of Artificial Sequence: Synthetic oligonucleotide

<400> 136
cgacagccgu gguggaaua 19

<210> 137
<211> 19
<212> RNA
<213> Artificial Sequence

<220>
<223> Description of Artificial Sequence: Synthetic oligonucleotide

<400> 137
auuccaccac ggcugucgu 19

<210> 138
<211> 19
<212> RNA
<213> Artificial Sequence

<220>

<223> Description of Artificial Sequence: Synthetic oligonucleotide

<400> 138
acgacagccg ugguggaau 19

<210> 139
<211> 19
<212> RNA
<213> Artificial Sequence

<220>
<223> Description of Artificial Sequence: Synthetic oligonucleotide

<400> 139
caccacggcu gucgucacc 19

<210> 140
<211> 19
<212> RNA
<213> Artificial Sequence

<220>
<223> Description of Artificial Sequence: Synthetic oligonucleotide

<400> 140
ggugacgaca gccguggug 19

<210> 141
<211> 19
<212> RNA
<213> Artificial Sequence

<220>
<223> Description of Artificial Sequence: Synthetic oligonucleotide

<400> 141
accacggcug ucgucacca 19

<210> 142
<211> 19
<212> RNA
<213> Artificial Sequence

<220>
<223> Description of Artificial Sequence: Synthetic oligonucleotide

<400> 142
uggugacgac agccguggu 19

<210> 143
<211> 19
<212> RNA
<213> Artificial Sequence

<220>
<223> Description of Artificial Sequence: Synthetic oligonucleotide

<400> 143

ccacggcugu cgucaccaa 19

<210> 144
<211> 19
<212> RNA
<213> Artificial Sequence

<220>
<223> Description of Artificial Sequence: Synthetic oligonucleotide

<400> 144
uuggugacga cagccgugg 19

<210> 145
<211> 19
<212> RNA
<213> Artificial Sequence

<220>
<223> Description of Artificial Sequence: Synthetic oligonucleotide

<400> 145
acggcugucg ucaccaauc 19

<210> 146
<211> 19
<212> RNA
<213> Artificial Sequence

<220>
<223> Description of Artificial Sequence: Synthetic oligonucleotide

<400> 146
gauuggugac gacagccgu 19

<210> 147
<211> 19
<212> RNA
<213> Artificial Sequence

<220>
<223> Description of Artificial Sequence: Synthetic oligonucleotide

<400> 147
cggcugucgu caccaaucc 19

<210> 148
<211> 19
<212> RNA
<213> Artificial Sequence

<220>
<223> Description of Artificial Sequence: Synthetic oligonucleotide

<400> 148
ggauugguga cgacagccg 19

<210> 149

<211> 19
<212> RNA
<213> Artificial Sequence

<220>
<223> Description of Artificial Sequence: Synthetic oligonucleotide

<400> 149
cgucaccaau cccaaggaa 19

<210> 150
<211> 19
<212> RNA
<213> Artificial Sequence

<220>
<223> Description of Artificial Sequence: Synthetic oligonucleotide

<400> 150
uuccuuggga uuggugacg 19

<210> 151
<211> 19
<212> RNA
<213> Artificial Sequence

<220>
<223> Description of Artificial Sequence: Synthetic oligonucleotide

<400> 151
caaucccaag gaaugaggg 19

<210> 152
<211> 19
<212> RNA
<213> Artificial Sequence

<220>
<223> Description of Artificial Sequence: Synthetic oligonucleotide

<400> 152
cccucauucc uugggauug 19

<210> 153
<211> 19
<212> RNA
<213> Artificial Sequence

<220>
<223> Description of Artificial Sequence: Synthetic oligonucleotide

<400> 153
ccugaaggac gagggaugg 19

<210> 154
<211> 19
<212> RNA
<213> Artificial Sequence

<220>
<223> Description of Artificial Sequence: Synthetic oligonucleotide

<400> 154
ccaucccucg uccuucagg 19

<210> 155
<211> 19
<212> RNA
<213> Artificial Sequence

<220>
<223> Description of Artificial Sequence: Synthetic oligonucleotide

<400> 155
ggacgaggga ugggauuuc 19

<210> 156
<211> 19
<212> RNA
<213> Artificial Sequence

<220>
<223> Description of Artificial Sequence: Synthetic oligonucleotide

<400> 156
gaaaucccau cccucgucc 19

<210> 157
<211> 19
<212> RNA
<213> Artificial Sequence

<220>
<223> Description of Artificial Sequence: Synthetic oligonucleotide

<400> 157
aaguccacuc auucuuggc 19

<210> 158
<211> 19
<212> RNA
<213> Artificial Sequence

<220>
<223> Description of Artificial Sequence: Synthetic oligonucleotide

<400> 158
gccaagaaug aguggacuu 19

<210> 159
<211> 19
<212> RNA
<213> Artificial Sequence

<220>
<223> Description of Artificial Sequence: Synthetic oligonucleotide

<400> 159
gggauuucau guaaccaag 19

<210> 160
<211> 19
<212> RNA
<213> Artificial Sequence

<220>
<223> Description of Artificial Sequence: Synthetic oligonucleotide

<400> 160
cuugguuaca ugaaauccc 19

<210> 161
<211> 19
<212> RNA
<213> Artificial Sequence

<220>
<223> Description of Artificial Sequence: Synthetic oligonucleotide

<400> 161
ggauuucaug uaaccaaga 19

<210> 162
<211> 19
<212> RNA
<213> Artificial Sequence

<220>
<223> Description of Artificial Sequence: Synthetic oligonucleotide

<400> 162
ucuugguuac augaaaucc 19

<210> 163
<211> 19
<212> RNA
<213> Artificial Sequence

<220>
<223> Description of Artificial Sequence: Synthetic oligonucleotide

<400> 163
ucauguaacc aagaguauu 19

<210> 164
<211> 19
<212> RNA
<213> Artificial Sequence

<220>
<223> Description of Artificial Sequence: Synthetic oligonucleotide

<400> 164
aauacucuug guuacauga 19

<210> 165
<211> 19
<212> RNA
<213> Artificial Sequence

<220>
<223> Description of Artificial Sequence: Synthetic oligonucleotide

<400> 165
auguaaccaa gaguauucc 19

<210> 166
<211> 19
<212> RNA
<213> Artificial Sequence

<220>
<223> Description of Artificial Sequence: Synthetic oligonucleotide

<400> 166
ggaauacucu ugguuacau 19

<210> 167
<211> 19
<212> RNA
<213> Artificial Sequence

<220>
<223> Description of Artificial Sequence: Synthetic oligonucleotide

<400> 167
uguaaccaag aguauucca 19

<210> 168
<211> 19
<212> RNA
<213> Artificial Sequence

<220>
<223> Description of Artificial Sequence: Synthetic oligonucleotide

<400> 168
uggaauacuc uugguuaca 19

<210> 169
<211> 19
<212> RNA
<213> Artificial Sequence

<220>
<223> Description of Artificial Sequence: Synthetic oligonucleotide

<400> 169
guaaccaaga guauuccau 19

<210> 170
<211> 19
<212> RNA

<213> Artificial Sequence

<220>
<223> Description of Artificial Sequence: Synthetic oligonucleotide

<400> 170
auggaauacu cuugguuac 19

<210> 171
<211> 19
<212> RNA
<213> Artificial Sequence

<220>
<223> Description of Artificial Sequence: Synthetic oligonucleotide

<400> 171
ugccuugcug gacugguau 19

<210> 172
<211> 19
<212> RNA
<213> Artificial Sequence

<220>
<223> Description of Artificial Sequence: Synthetic oligonucleotide

<400> 172
auaccagucc agcaaggca 19

<210> 173
<211> 19
<212> RNA
<213> Artificial Sequence

<220>
<223> Description of Artificial Sequence: Synthetic oligonucleotide

<400> 173
uaaagcagug uuuucaccu 19

<210> 174
<211> 19
<212> RNA
<213> Artificial Sequence

<220>
<223> Description of Artificial Sequence: Synthetic oligonucleotide

<400> 174
aggugaaaac acugcuuua 19

<210> 175
<211> 19
<212> RNA
<213> Artificial Sequence

<220>

<223> Description of Artificial Sequence: Synthetic oligonucleotide

<400> 175
gccuugcugg acugguauu 19

<210> 176
<211> 19
<212> RNA
<213> Artificial Sequence

<220>
<223> Description of Artificial Sequence: Synthetic oligonucleotide

<400> 176
aauaccaguc cagcaaggc 19

<210> 177
<211> 19
<212> RNA
<213> Artificial Sequence

<220>
<223> Description of Artificial Sequence: Synthetic oligonucleotide

<400> 177
uguuuucacc ucauaugcu 19

<210> 178
<211> 19
<212> RNA
<213> Artificial Sequence

<220>
<223> Description of Artificial Sequence: Synthetic oligonucleotide

<400> 178
agcauaugag gugaaaaca 19

<210> 179
<211> 19
<212> RNA
<213> Artificial Sequence

<220>
<223> Description of Artificial Sequence: Synthetic oligonucleotide

<400> 179
guuuucaccu cauaugcua 19

<210> 180
<211> 19
<212> RNA
<213> Artificial Sequence

<220>
<223> Description of Artificial Sequence: Synthetic oligonucleotide

<400> 180

uagcauauga ggugaaaac 19

<210> 181
<211> 19
<212> RNA
<213> Artificial Sequence

<220>
<223> Description of Artificial Sequence: Synthetic oligonucleotide

<400> 181
uuuucaccuc auaugcuau 19

<210> 182
<211> 19
<212> RNA
<213> Artificial Sequence

<220>
<223> Description of Artificial Sequence: Synthetic oligonucleotide

<400> 182
auagcauaug aggugaaaa 19

<210> 183
<211> 19
<212> RNA
<213> Artificial Sequence

<220>
<223> Description of Artificial Sequence: Synthetic oligonucleotide

<400> 183
uucaccucau augcuaugu 19

<210> 184
<211> 19
<212> RNA
<213> Artificial Sequence

<220>
<223> Description of Artificial Sequence: Synthetic oligonucleotide

<400> 184
acauagcaua ugaggugaa 19

<210> 185
<211> 19
<212> RNA
<213> Artificial Sequence

<220>
<223> Description of Artificial Sequence: Synthetic oligonucleotide

<400> 185
caccucauau gcuauguua 19

<210> 186

<211> 19
<212> RNA
<213> Artificial Sequence

<220>
<223> Description of Artificial Sequence: Synthetic oligonucleotide

<400> 186
uaacauagca uaugaggug 19

<210> 187
<211> 19
<212> RNA
<213> Artificial Sequence

<220>
<223> Description of Artificial Sequence: Synthetic oligonucleotide

<400> 187
ccuugcugga cugguauuu 19

<210> 188
<211> 19
<212> RNA
<213> Artificial Sequence

<220>
<223> Description of Artificial Sequence: Synthetic oligonucleotide

<400> 188
aaauaccagu ccagcaagg 19

<210> 189
<211> 19
<212> RNA
<213> Artificial Sequence

<220>
<223> Description of Artificial Sequence: Synthetic oligonucleotide

<400> 189
auaugcuaug uuagaaguc 19

<210> 190
<211> 19
<212> RNA
<213> Artificial Sequence

<220>
<223> Description of Artificial Sequence: Synthetic oligonucleotide

<400> 190
gacuucuaac auagcauau 19

<210> 191
<211> 19
<212> RNA
<213> Artificial Sequence

<220>
<223> Description of Artificial Sequence: Synthetic oligonucleotide

<400> 191
uaugcuaugu uagaagucc 19

<210> 192
<211> 19
<212> RNA
<213> Artificial Sequence

<220>
<223> Description of Artificial Sequence: Synthetic oligonucleotide

<400> 192
ggacuucuaa cauagcaua 19

<210> 193
<211> 19
<212> RNA
<213> Artificial Sequence

<220>
<223> Description of Artificial Sequence: Synthetic oligonucleotide

<400> 193
ugcuauguua gaaguccag 19

<210> 194
<211> 19
<212> RNA
<213> Artificial Sequence

<220>
<223> Description of Artificial Sequence: Synthetic oligonucleotide

<400> 194
cuggacuucu aacauagca 19

<210> 195
<211> 19
<212> RNA
<213> Artificial Sequence

<220>
<223> Description of Artificial Sequence: Synthetic oligonucleotide

<400> 195
cuugcuggac ugguauuug 19

<210> 196
<211> 19
<212> RNA
<213> Artificial Sequence

<220>
<223> Description of Artificial Sequence: Synthetic oligonucleotide

<400> 196
caaauaccag uccagcaag 19

<210> 197
<211> 19
<212> RNA
<213> Artificial Sequence

<220>
<223> Description of Artificial Sequence: Synthetic oligonucleotide

<400> 197
aguccaggca gagacaaua 19

<210> 198
<211> 20
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 198
auugucucug ccuggacutt 20

<210> 199
<211> 19
<212> RNA
<213> Artificial Sequence

<220>
<223> Description of Artificial Sequence: Synthetic oligonucleotide

<400> 199
uccaggcaga gacaauaaa 19

<210> 200
<211> 19
<212> RNA
<213> Artificial Sequence

<220>
<223> Description of Artificial Sequence: Synthetic oligonucleotide

<400> 200
uuuauugucu cugccugga 19

<210> 201
<211> 19
<212> RNA
<213> Artificial Sequence

<220>
<223> Description of Artificial Sequence: Synthetic oligonucleotide

<400> 201
gugaaaggca cuuuucauu 19

<210> 202
<211> 19
<212> RNA
<213> Artificial Sequence

<220>
<223> Description of Artificial Sequence: Synthetic oligonucleotide

<400> 202
aaugaaaagu gccuuucac 19

<210> 203
<211> 19
<212> RNA
<213> Artificial Sequence

<220>
<223> Description of Artificial Sequence: Synthetic oligonucleotide

<400> 203
uggacuggua uuugugucu 19

<210> 204
<211> 19
<212> RNA
<213> Artificial Sequence

<220>
<223> Description of Artificial Sequence: Synthetic oligonucleotide

<400> 204
agacacaaau accagucca 19

<210> 205
<211> 19
<212> RNA
<213> Artificial Sequence

<220>
<223> Description of Artificial Sequence: Synthetic oligonucleotide

<400> 205
gucugaggcu ggcccuacg 19

<210> 206
<211> 19
<212> RNA
<213> Artificial Sequence

<220>
<223> Description of Artificial Sequence: Synthetic oligonucleotide

<400> 206
cguagggcca gccucagac 19

<210> 207
<211> 19
<212> RNA
<213> Artificial Sequence

<220>
<223> Description of Artificial Sequence: Synthetic oligonucleotide

<400> 207
cugaggcugg cccuacggg 19

<210> 208
<211> 19
<212> RNA
<213> Artificial Sequence

<220>
<223> Description of Artificial Sequence: Synthetic oligonucleotide

<400> 208
cccguagggc cagccucag 19

<210> 209
<211> 19
<212> RNA
<213> Artificial Sequence

<220>
<223> Description of Artificial Sequence: Synthetic oligonucleotide

<400> 209
gaggcuggcc cuacgggca 19

<210> 210
<211> 19
<212> RNA
<213> Artificial Sequence

<220>
<223> Description of Artificial Sequence: Synthetic oligonucleotide

<400> 210
ugcccguagg gccagccuc 19

<210> 211
<211> 19
<212> RNA
<213> Artificial Sequence

<220>
<223> Description of Artificial Sequence: Synthetic oligonucleotide

<400> 211
aggcuggccc uacgggcac 19

<210> 212
<211> 19
<212> RNA

<213> Artificial Sequence

<220>
<223> Description of Artificial Sequence: Synthetic oligonucleotide

<400> 212
gugcccguag ggccagccu 19

<210> 213
<211> 19
<212> RNA
<213> Artificial Sequence

<220>
<223> Description of Artificial Sequence: Synthetic oligonucleotide

<400> 213
gcuggcccua cgggcaccg 19

<210> 214
<211> 19
<212> RNA
<213> Artificial Sequence

<220>
<223> Description of Artificial Sequence: Synthetic oligonucleotide

<400> 214
cggugcccgu agggccagc 19

<210> 215
<211> 19
<212> RNA
<213> Artificial Sequence

<220>
<223> Description of Artificial Sequence: Synthetic oligonucleotide

<400> 215
cuggcccuac gggcaccgg 19

<210> 216
<211> 19
<212> RNA
<213> Artificial Sequence

<220>
<223> Description of Artificial Sequence: Synthetic oligonucleotide

<400> 216
ccggugcccg uagggccag 19

<210> 217
<211> 19
<212> RNA
<213> Artificial Sequence

<220>

<223> Description of Artificial Sequence: Synthetic oligonucleotide

<400> 217
ggcccuacgg gcaccggug 19

<210> 218
<211> 19
<212> RNA
<213> Artificial Sequence

<220>
<223> Description of Artificial Sequence: Synthetic oligonucleotide

<400> 218
caccggugcc cguagggcc 19

<210> 219
<211> 19
<212> RNA
<213> Artificial Sequence

<220>
<223> Description of Artificial Sequence: Synthetic oligonucleotide

<400> 219
ccacucauuc uuggcagga 19

<210> 220
<211> 19
<212> RNA
<213> Artificial Sequence

<220>
<223> Description of Artificial Sequence: Synthetic oligonucleotide

<400> 220
uccugccaag aaugagugg 19

<210> 221
<211> 19
<212> RNA
<213> Artificial Sequence

<220>
<223> Description of Artificial Sequence: Synthetic oligonucleotide

<400> 221
ccuacgggca ccggugaau 19

<210> 222
<211> 19
<212> RNA
<213> Artificial Sequence

<220>
<223> Description of Artificial Sequence: Synthetic oligonucleotide

<400> 222

auucaccggu gcccguagg 19

<210> 223
<211> 19
<212> RNA
<213> Artificial Sequence

<220>
<223> Description of Artificial Sequence: Synthetic oligonucleotide

<400> 223
cuacgggcac cggugaauc 19

<210> 224
<211> 19
<212> RNA
<213> Artificial Sequence

<220>
<223> Description of Artificial Sequence: Synthetic oligonucleotide

<400> 224
gauucaccgg ugcccguag 19

<210> 225
<211> 19
<212> RNA
<213> Artificial Sequence

<220>
<223> Description of Artificial Sequence: Synthetic oligonucleotide

<400> 225
uacgggcacc ggugaaucc 19

<210> 226
<211> 19
<212> RNA
<213> Artificial Sequence

<220>
<223> Description of Artificial Sequence: Synthetic oligonucleotide

<400> 226
ggauucaccg gugcccgua 19

<210> 227
<211> 19
<212> RNA
<213> Artificial Sequence

<220>
<223> Description of Artificial Sequence: Synthetic oligonucleotide

<400> 227
acgggcaccg gugaaucca 19

<210> 228

<211> 19
<212> RNA
<213> Artificial Sequence

<220>
<223> Description of Artificial Sequence: Synthetic oligonucleotide

<400> 228
uggauucacc ggugcccgu 19

<210> 229
<211> 19
<212> RNA
<213> Artificial Sequence

<220>
<223> Description of Artificial Sequence: Synthetic oligonucleotide

<400> 229
gcaccgguga auccaagug 19

<210> 230
<211> 19
<212> RNA
<213> Artificial Sequence

<220>
<223> Description of Artificial Sequence: Synthetic oligonucleotide

<400> 230
cacuuggauu caccggugc 19

<210> 231
<211> 19
<212> RNA
<213> Artificial Sequence

<220>
<223> Description of Artificial Sequence: Synthetic oligonucleotide

<400> 231
caccggugaa uccaagugu 19

<210> 232
<211> 19
<212> RNA
<213> Artificial Sequence

<220>
<223> Description of Artificial Sequence: Synthetic oligonucleotide

<400> 232
acacuuggau ucaccggug 19

<210> 233
<211> 19
<212> RNA
<213> Artificial Sequence

<220>
<223> Description of Artificial Sequence: Synthetic oligonucleotide

<400> 233
uguggccaug cauguguuc 19

<210> 234
<211> 19
<212> RNA
<213> Artificial Sequence

<220>
<223> Description of Artificial Sequence: Synthetic oligonucleotide

<400> 234
gaacacaugc auggccaca 19

<210> 235
<211> 19
<212> RNA
<213> Artificial Sequence

<220>
<223> Description of Artificial Sequence: Synthetic oligonucleotide

<400> 235
guggccaugc auguguuca 19

<210> 236
<211> 19
<212> RNA
<213> Artificial Sequence

<220>
<223> Description of Artificial Sequence: Synthetic oligonucleotide

<400> 236
ugaacacaug cauggccac 19

<210> 237
<211> 19
<212> RNA
<213> Artificial Sequence

<220>
<223> Description of Artificial Sequence: Synthetic oligonucleotide

<400> 237
gccaugcaug uguucagaa 19

<210> 238
<211> 19
<212> RNA
<213> Artificial Sequence

<220>
<223> Description of Artificial Sequence: Synthetic oligonucleotide

<400> 238
uucugaacac augcauggc 19

<210> 239
<211> 19
<212> RNA
<213> Artificial Sequence

<220>
<223> Description of Artificial Sequence: Synthetic oligonucleotide

<400> 239
uauuccacca cggcuguca 19

<210> 240
<211> 19
<212> RNA
<213> Artificial Sequence

<220>
<223> Description of Artificial Sequence: Synthetic oligonucleotide

<400> 240
ugacagccgu gguggaaua 19

<210> 241
<211> 19
<212> RNA
<213> Artificial Sequence

<220>
<223> Description of Artificial Sequence: Synthetic oligonucleotide

<400> 241
gucaucacca aucccaagg 19

<210> 242
<211> 19
<212> RNA
<213> Artificial Sequence

<220>
<223> Description of Artificial Sequence: Synthetic oligonucleotide

<400> 242
ccuugggauu ggugaugac 19

<210> 243
<211> 19
<212> RNA
<213> Artificial Sequence

<220>
<223> Description of Artificial Sequence: Synthetic oligonucleotide

<400> 243
guccucugau ggucaaagu 19

<210> 244
<211> 19
<212> RNA
<213> Artificial Sequence

<220>
<223> Description of Artificial Sequence: Synthetic oligonucleotide

<400> 244
acuuugacca ucagaggac 19

<210> 245
<211> 19
<212> RNA
<213> Artificial Sequence

<220>
<223> Description of Artificial Sequence: Synthetic oligonucleotide

<400> 245
gauggucaaa guucuagau 19

<210> 246
<211> 19
<212> RNA
<213> Artificial Sequence

<220>
<223> Description of Artificial Sequence: Synthetic oligonucleotide

<400> 246
aucuagaacu uugaccauc 19

<210> 247
<211> 19
<212> RNA
<213> Artificial Sequence

<220>
<223> Description of Artificial Sequence: Synthetic oligonucleotide

<400> 247
augcuguccg aggcagucc 19

<210> 248
<211> 19
<212> RNA
<213> Artificial Sequence

<220>
<223> Description of Artificial Sequence: Synthetic oligonucleotide

<400> 248
ggacugccuc ggacagcau 19

<210> 249
<211> 19
<212> RNA

<213> Artificial Sequence

<220>
<223> Description of Artificial Sequence: Synthetic oligonucleotide

<400> 249
ccgugcaugu guucagaaa 19

<210> 250
<211> 19
<212> RNA
<213> Artificial Sequence

<220>
<223> Description of Artificial Sequence: Synthetic oligonucleotide

<400> 250
uuucugaaca caugcacgg 19

<210> 251
<211> 19
<212> RNA
<213> Artificial Sequence

<220>
<223> Description of Artificial Sequence: Synthetic oligonucleotide

<400> 251
agucuggaga gcugcaugg 19

<210> 252
<211> 19
<212> RNA
<213> Artificial Sequence

<220>
<223> Description of Artificial Sequence: Synthetic oligonucleotide

<400> 252
ccaugcagcu cuccagacu 19

<210> 253
<211> 19
<212> RNA
<213> Artificial Sequence

<220>
<223> Description of Artificial Sequence: Synthetic oligonucleotide

<400> 253
caugggcuca caacugagg 19

<210> 254
<211> 19
<212> RNA
<213> Artificial Sequence

<220>

<223> Description of Artificial Sequence: Synthetic oligonucleotide

<400> 254
ccucaguugu gagcccaug 19

<210> 255
<211> 19
<212> RNA
<213> Artificial Sequence

<220>
<223> Description of Artificial Sequence: Synthetic oligonucleotide

<400> 255
ucucaucguc ugcuccucc 19

<210> 256
<211> 19
<212> RNA
<213> Artificial Sequence

<220>
<223> Description of Artificial Sequence: Synthetic oligonucleotide

<400> 256
ggaggagcag acgaugaga 19

<210> 257
<211> 19
<212> RNA
<213> Artificial Sequence

<220>
<223> Description of Artificial Sequence: Synthetic oligonucleotide

<400> 257
ccccauucca ugagcaugc 19

<210> 258
<211> 19
<212> RNA
<213> Artificial Sequence

<220>
<223> Description of Artificial Sequence: Synthetic oligonucleotide

<400> 258
gcaugcucau ggaaugggg 19

<210> 259
<211> 19
<212> RNA
<213> Artificial Sequence

<220>
<223> Description of Artificial Sequence: Synthetic oligonucleotide

<400> 259

gccccuacuc cuauuccac 19

<210> 260
<211> 19
<212> RNA
<213> Artificial Sequence

<220>
<223> Description of Artificial Sequence: Synthetic oligonucleotide

<400> 260
guggaauagg aguaggggc 19

<210> 261
<211> 19
<212> RNA
<213> Artificial Sequence

<220>
<223> Description of Artificial Sequence: Synthetic oligonucleotide

<400> 261
cuauuccacc acggcuguc 19

<210> 262
<211> 19
<212> RNA
<213> Artificial Sequence

<220>
<223> Description of Artificial Sequence: Synthetic oligonucleotide

<400> 262
gacagccgug guggaauag 19

<210> 263
<211> 19
<212> RNA
<213> Artificial Sequence

<220>
<223> Description of Artificial Sequence: Synthetic oligonucleotide

<400> 263
cacggcuguc gucaccaau 19

<210> 264
<211> 19
<212> RNA
<213> Artificial Sequence

<220>
<223> Description of Artificial Sequence: Synthetic oligonucleotide

<400> 264
auuggugacg acagccgug 19

<210> 265

<211> 19
<212> RNA
<213> Artificial Sequence

<220>
<223> Description of Artificial Sequence: Synthetic oligonucleotide

<400> 265
aggacgaggg augggauuu 19

<210> 266
<211> 19
<212> RNA
<213> Artificial Sequence

<220>
<223> Description of Artificial Sequence: Synthetic oligonucleotide

<400> 266
aaaucccauc ccucguccu 19

<210> 267
<211> 19
<212> RNA
<213> Artificial Sequence

<220>
<223> Description of Artificial Sequence: Synthetic oligonucleotide

<400> 267
ucaccucaua ugcuauguu 19

<210> 268
<211> 19
<212> RNA
<213> Artificial Sequence

<220>
<223> Description of Artificial Sequence: Synthetic oligonucleotide

<400> 268
aacauagcau augagguga 19

<210> 269
<211> 19
<212> RNA
<213> Artificial Sequence

<220>
<223> Description of Artificial Sequence: Synthetic oligonucleotide

<400> 269
ccucauaugc uauguuaga 19

<210> 270
<211> 19
<212> RNA
<213> Artificial Sequence

<220>
<223> Description of Artificial Sequence: Synthetic oligonucleotide

<400> 270
ucuaacauag cauaugagg 19

<210> 271
<211> 19
<212> RNA
<213> Artificial Sequence

<220>
<223> Description of Artificial Sequence: Synthetic oligonucleotide

<400> 271
auguuagaag uccaggcag 19

<210> 272
<211> 19
<212> RNA
<213> Artificial Sequence

<220>
<223> Description of Artificial Sequence: Synthetic oligonucleotide

<400> 272
cugccuggac uucuaacau 19

<210> 273
<211> 19
<212> RNA
<213> Artificial Sequence

<220>
<223> Description of Artificial Sequence: Synthetic oligonucleotide

<400> 273
ucugaggcug gcccuacgg 19

<210> 274
<211> 19
<212> RNA
<213> Artificial Sequence

<220>
<223> Description of Artificial Sequence: Synthetic oligonucleotide

<400> 274
ccguagggcc agccucaga 19

<210> 275
<211> 19
<212> RNA
<213> Artificial Sequence

<220>
<223> Description of Artificial Sequence: Synthetic oligonucleotide

<400> 275
ggcccuacgg gcaccggug 19

<210> 276
<211> 19
<212> RNA
<213> Artificial Sequence

<220>
<223> Description of Artificial Sequence: Synthetic oligonucleotide

<400> 276
caccggugcc cguagggcc 19

<210> 277
<211> 19
<212> RNA
<213> Artificial Sequence

<220>
<223> Description of Artificial Sequence: Synthetic oligonucleotide

<400> 277
gggcaccggu gaauccaag 19

<210> 278
<211> 19
<212> RNA
<213> Artificial Sequence

<220>
<223> Description of Artificial Sequence: Synthetic oligonucleotide

<400> 278
cuuggauuca ccggugccc 19

<210> 279
<211> 19
<212> RNA
<213> Artificial Sequence

<220>
<223> Description of Artificial Sequence: Synthetic oligonucleotide

<400> 279
ccaugcaugu guucagaaa 19

<210> 280
<211> 19
<212> RNA
<213> Artificial Sequence

<220>
<223> Description of Artificial Sequence: Synthetic oligonucleotide

<400> 280
uuucugaaca caugcaugg 19

<210> 281
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 281
ccggugaauc caaguguccn n 21

<210> 282
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 282
ggacacuugg auucaccggn n 21

<210> 283
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 283
acucauucuu ggcaggaugn n 21

<210> 284

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 284
cauccugcca agaaugagun n 21

<210> 285
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 285
aaguguccuc ugauggucan n 21

<210> 286
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 286
ugaccaucag aggacacuun n 21

<210> 287
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 287
ucauucuugg caggauggcn n 21

<210> 288
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 288
gccauccugc caagaaugan n 21

<210> 289
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 289
aaguucuaga ugcuguccgn n 21

<210> 290
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 290
cggacagcau cuagaacuun n 21

<210> 291
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 291
guucuagaug cuguccgagn n 21

<210> 292
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 292
cucggacagc aucuagaacn n 21

<210> 293
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 293
cuagaugcug uccgaggcan n 21

<210> 294
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 294
ugccucggac agcaucuagn n 21

<210> 295
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 295
gaugcugucc gaggcagucn n 21

<210> 296
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 296
gacugccucg gacagcaucn n 21

<210> 297
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 297
cauucuuggc aggauggcun n 21

<210> 298
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 298
agccauccug ccaagaaugn n 21

<210> 299
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 299
ugcuguccga ggcaguccun n 21

<210> 300
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 300
aggacugccu cggacagcan n 21

<210> 301
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 301
ccgaggcagu ccugccaucn n 21

<210> 302
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 302
gauggcagga cugccucggn n 21

<210> 303
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 303
caguccugcc aucaaugugn n 21

<210> 304
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 304
cacauugaug gcaggacugn n 21

<210> 305
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 305
caauguggcc gugcaugugn n 21

<210> 306
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 306
cacaugcacg gccacauugn n 21

<210> 307
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 307
auguguucag aaaggcugcn n 21

<210> 308
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>

<221> modified_base
<222> (20)..(21)
<223> a, c, t, g, unknown or other

<400> 308
gcagccuuuc ugaacacaun n 21

<210> 309
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 309
cagaagucca cucauucuun n 21

<210> 310
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 310
aagaaugagu ggacuucugn n 21

<210> 311
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base

<222> (20)..(21)
<223> a, c, t, g, unknown or other

<400> 311
ggcaggaugg cuucucaucn n 21

<210> 312
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 312
gaugagaagc cauccugccn n 21

<210> 313
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 313
gagccauuug ccucugggan n 21

<210> 314
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)

<223> a, c, t, g, unknown or other

<400> 314
ucccagaggc aaauggcucn n 21

<210> 315
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 315
caggauggcu ucucaucgun n 21

<210> 316
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 316
acgaugagaa gccauccugn n 21

<210> 317
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 317
aggauggcuu cucaucgucn n 21

<210> 318
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 318
gacgaugaga agccauccun n 21

<210> 319
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 319
agagcugcau gggcucacan n 21

<210> 320
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 320

ugugagccca ugcagcucun n 21

<210> 321
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 321
gcugcauggg cucacaacun n 21

<210> 322
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 322
aguugugagc ccaugcagcn n 21

<210> 323
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 323
ggauggcuuc ucaucgucun n 21

<210> 324
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 324
agacgaugag aagccauccn n 21

<210> 325
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 325
gcaugggcuc acaacugagn n 21

<210> 326
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 326
cucaguugug agcccaugcn n 21

<210> 327

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 327
augggcucac aacugaggan n 21

<210> 328
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 328
uccucaguug ugagcccaun n 21

<210> 329
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 329
ugggcucaca acugaggagn n 21

<210> 330
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 330
cuccucaguu gugagcccan n 21

<210> 331
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 331
gaggaauuug uagaagggan n 21

<210> 332
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 332
ucccuucuac aaauuccucn n 21

<210> 333
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 333
uuuguagaag ggauauacan n 21

<210> 334
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 334
uguauauccc uucuacaaan n 21

<210> 335
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 335
uuguagaagg gauauacaan n 21

<210> 336
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 336
uuguauaucc cuucuacaan n 21

<210> 337
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 337
uguagaaggg auauacaaan n 21

<210> 338
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 338
uuuguauauc ccuucuacan n 21

<210> 339
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 339
agaagggaua uacaaagugn n 21

<210> 340
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 340
cacuuuguau aucccuucun n 21

<210> 341
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 341
aaguggaaau agacaccaan n 21

<210> 342
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 342
uuggugucua uuuccacuun n 21

<210> 343
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 343
ggaaauagac accaaaucun n 21

<210> 344
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 344
agauuuggug ucuauuuccn n 21

<210> 345
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 345
gaaauagaca ccaaaucuun n 21

<210> 346
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 346
aagauuuggu gucuauuucn n 21

<210> 347
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 347
auagacacca aaucuuacun n 21

<210> 348
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 348
aguaagauuu ggugucuaun n 21

<210> 349
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 349
uagacaccaa aucuuacugn n 21

<210> 350
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 350
caguaagauu uggugucuan n 21

<210> 351
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>

<221> modified_base
<222> (20)..(21)
<223> a, c, t, g, unknown or other

<400> 351
agacaccaaa ucuuacuggn n 21

<210> 352
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 352
ccaguaagau uuggugucun n 21

<210> 353
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 353
uuacuggaag gcacuuggcn n 21

<210> 354
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base

<222> (20)..(21)
<223> a, c, t, g, unknown or other

<400> 354
gccaagugcc uuccaguaan n 21

<210> 355
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 355
uucucaucgu cugcuccucn n 21

<210> 356
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 356
gaggagcaga cgaugagaan n 21

<210> 357
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)

<223> a, c, t, g, unknown or other

<400> 357
ggaaggcacu uggcaucucn n 21

<210> 358
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 358
gagaugccaa gugccuuccn n 21

<210> 359
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 359
ggcacuuggc aucuccccan n 21

<210> 360
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 360
uggggagaug ccaagugccn n 21

<210> 361
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 361
ggcaucuccc cauuccaugn n 21

<210> 362
<211> 22
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (21)..(22)
<223> a, c, t, g, unknown or other

<400> 362
auggaauggg gagaugcctt nn 22

<210> 363
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 363

gcaucucccc auuccaugan n 21

<210> 364
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 364
ucauggaaug gggagaugcn n 21

<210> 365
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 365
caucucccca uuccaugagn n 21

<210> 366
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 366
cucauggaau ggggagaugn n 21

<210> 367
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 367
aucuccccau uccaugagcn n 21

<210> 368
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 368
gcucauggaa uggggagaun n 21

<210> 369
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 369
cuccccauuc caugagcaun n 21

<210> 370

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 370
augcucaugg aauggggagn n 21

<210> 371
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 371
cccauuccau gagcaugcan n 21

<210> 372
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 372
ugcaugcuca uggaaugggn n 21

<210> 373
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 373
ccaugagcau gcagaggugn n 21

<210> 374
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 374
caccucugca ugcucauggn n 21

<210> 375
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 375
agcaugcaga ggugguauun n 21

<210> 376
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 376
aauaccaccu cugcaugcun n 21

<210> 377
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide
v<220>

<221> modified_base
<222> (20)..(21)
<223> a, c, t, g, unknown or other

<400> 377
caugcagagg ugguauucan n 21

<210> 378
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 378
ugaauaccac cucugcaugn n 21

<210> 379
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 379
augcagaggu gguauucacn n 21

<210> 380
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 380
gugaauacca ccucugcaun n 21

<210> 381
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 381
ggugguauuc acagccaacn n 21

<210> 382
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 382
guuggcugug aauaccaccn n 21

<210> 383
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 383
gugguauuca cagccaacgn n 21

<210> 384
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 384
cguuggcugu gaauaccacn n 21

<210> 385
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 385
ugguauucac agccaacgan n 21

<210> 386
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 386
ucguuggcug ugaauaccan n 21

<210> 387
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 387
gguauucaca gccaacgacn n 21

<210> 388
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 388
gucguuggcu gugaauaccn n 21

<210> 389
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 389
guauucacag ccaacgacun n 21

<210> 390
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 390
agucguuggc ugugaauacn n 21

<210> 391
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 391
uauucacagc caacgacucn n 21

<210> 392
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 392
gagucguugg cugugaauan n 21

<210> 393
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 393
ucacagccaa cgacuccggn n 21

<210> 394
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>

<221> modified_base
<222> (20)..(21)
<223> a, c, t, g, unknown or other

<400> 394
ccggagucgu uggcugugan n 21

<210> 395
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 395
ccccgccgcu acaccauugn n 21

<210> 396
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 396
caauggugua gcggcggggn n 21

<210> 397
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base

<222> (20)..(21)
<223> a, c, t, g, unknown or other

<400> 397
gaaguccacu cauucuuggn n 21

<210> 398
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 398
ccaagaauga guggacuucn n 21

<210> 399
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 399
cccugcugag ccccuacucn n 21

<210> 400
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)

<223> a, c, t, g, unknown or other

<400> 400
gaguaggggc ucagcagggn n 21

<210> 401
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 401
cugagccccu acuccuauun n 21

<210> 402
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 402
aauaggagua ggggcucagn n 21

<210> 403
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 403
ugagccccua cuccuauucn n 21

<210> 404
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 404
gaauaggagu aggggcucan n 21

<210> 405
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 405
ccccuacucc uauuccaccn n 21

<210> 406
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 406

gguggaauag gaguaggggn n 21

<210> 407
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 407
cuacuccuau uccaccacgn n 21

<210> 408
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 408
cgugguggaa uaggaguagn n 21

<210> 409
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 409
uacuccuauu ccaccacggn n 21

<210> 410
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 410
ccguggugga auaggaguan n 21

<210> 411
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 411
acuccuauuc caccacggcn n 21

<210> 412
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 412
gccguggugg aauaggagun n 21

<210> 413

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 413
uccuauucca ccacggcugn n 21

<210> 414
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 414
cagccguggu ggaauaggan n 21

<210> 415
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 415
uauuccacca cggcugucgn n 21

<210> 416
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 416
cgacagccgu gguggaauan n 21

<210> 417
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 417
auuccaccac ggcugucgun n 21

<210> 418
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 418
acgacagccg ugguggaaun n 21

<210> 419
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 419
caccacggcu gucgucaccn n 21

<210> 420
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 420
ggugacgaca gccguggugn n 21

<210> 421
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 421
accacggcug ucgucaccan n 21

<210> 422
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 422
uggugacgac agccguggun n 21

<210> 423
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 423
ccacggcugu cgucaccaan n 21

<210> 424
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 424
uuggugacga cagccguggn n 21

<210> 425
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 425
acggcugucg ucaccaaucn n 21

<210> 426
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 426
gauuggugac gacagccgun n 21

<210> 427
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 427
cggcugucgu caccaauccn n 21

<210> 428
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 428
ggauugguga cgacagccgn n 21

<210> 429
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 429
cgucaccaau cccaaggaan n 21

<210> 430
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 430
uuccuuggga uuggugacgn n 21

<210> 431
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 431
caaucccaag gaaugagggn n 21

<210> 432
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 432
cccucauucc uugggauugn n 21

<210> 433
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 433
ccugaaggac gagggauggn n 21

<210> 434
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 434
ccaucccucg uccuucaggn n 21

<210> 435
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 435
ggacgaggga ugggauuucn n 21

<210> 436
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 436
gaaaucccau cccucguccn n 21

<210> 437
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>

<221> modified_base
<222> (20)..(21)
<223> a, c, t, g, unknown or other

<400> 437
aaguccacuc auucuuggcn n 21

<210> 438
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 438
gccaagaaug aguggacuun n 21

<210> 439
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 439
gggauuucau guaaccaagn n 21

<210> 440
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base

<222> (20)..(21)
<223> a, c, t, g, unknown or other

<400> 440
cuugguuaca ugaaaucccn n 21

<210> 441
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 441
ggauuucaug uaaccaagan n 21

<210> 442
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 442
ucuugguuac augaaauccn n 21

<210> 443
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)

<223> a, c, t, g, unknown or other

<400> 443
ucauguaacc aagaguauun n 21

<210> 444
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 444
aauacucuug guuacaugan n 21

<210> 445
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 445
auguaaccaa gaguauuccn n 21

<210> 446
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 446
ggaauacucu ugguuacaun n 21

<210> 447
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 447
uguaaccaag aguauuccan n 21

<210> 448
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 448
uggaauacuc uugguuacan n 21

<210> 449
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 449

guaaccaaga guauuccaun n 21

<210> 450
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 450
auggaauacu cuugguuacn n 21

<210> 451
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 451
ugccuugcug gacugguaun n 21

<210> 452
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 452
auaccagucc agcaaggcan n 21

<210> 453
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 453
uaaagcagug uuuucaccun n 21

<210> 454
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 454
aggugaaaac acugcuuuan n 21

<210> 455
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 455
gccuugcugg acugguauun n 21

<210> 456

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 456
aauaccaguc cagcaaggcn n 21

<210> 457
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 457
uguuuucacc ucauaugcun n 21

<210> 458
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 458
agcauaugag gugaaaacan n 21

<210> 459
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 459
guuuucaccu cauaugcuan n 21

<210> 460
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 460
uagcauauga ggugaaaacn n 21

<210> 461
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 461
uuuucaccuc auaugcuaun n 21

<210> 462
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 462
auagcauaug aggugaaaan n 21

<210> 463
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 463
uucaccucau augcuaugun n 21

<210> 464
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 464
acauagcaua ugaggugaan n 21

<210> 465
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 465
caccucauau gcuauguuan n 21

<210> 466
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 466
uaacauagca uaugaggugn n 21

<210> 467
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 467
ccuugcugga cugguauuun n 21

<210> 468
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 468
aaauaccagu ccagcaaggn n 21

<210> 469
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 469
auaugcuaug uuagaagucn n 21

<210> 470
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 470
gacuucuaac auagcauaun n 21

<210> 471
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 471
uaugcuaugu uagaaguccn n 21

<210> 472
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 472
ggacuucuaa cauagcauan n 21

<210> 473
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 473
ugcuauguua gaaguccagn n 21

<210> 474
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 474
cuggacuucu aacauagcan n 21

<210> 475
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 475
cuugcuggac ugguauuugn n 21

<210> 476
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 476
caaauaccag uccagcaagn n 21

<210> 477
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 477
aguccaggca gagacaauan n 21

<210> 478
<211> 22
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (21)..(22)
<223> a, c, t, g, unknown or other

<400> 478
auugucucug ccuggacutt nn 22

<210> 479
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 479
uccaggcaga gacaauaaan n 21

<210> 480
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>

<221> modified_base
<222> (20)..(21)
<223> a, c, t, g, unknown or other

<400> 480
uuuauugucu cugccuggan n 21

<210> 481
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 481
gugaaaggca cuuuucauun n 21

<210> 482
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 482
aaugaaaagu gccuuucacn n 21

<210> 483
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base

<222> (20)..(21)
<223> a, c, t, g, unknown or other

<400> 483
uggacuggua uuugugucun n 21

<210> 484
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 484
agacacaaau accaguccan n 21

<210> 485
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 485
gucugaggcu ggcccuacgn n 21

<210> 486
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)

<223> a, c, t, g, unknown or other

<400> 486
cguagggcca gccucagacn n 21

<210> 487
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 487
cugaggcugg cccuacgggn n 21

<210> 488
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 488
cccguagggc cagccucagn n 21

<210> 489
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 489
gaggcuggcc cuacgggcan n 21

<210> 490
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 490
ugcccguagg gccagccucn n 21

<210> 491
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 491
aggcuggccc uacgggcacn n 21

<210> 492
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 492

gugcccguag ggccagccun n 21

<210> 493
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 493
gcuggcccua cgggcaccgn n 21

<210> 494
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 494
cggugcccgu agggccagcn n 21

<210> 495
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 495
cuggcccuac gggcaccggn n 21

<210> 496
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 496
ccggugcccg uagggccagn n 21

<210> 497
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 497
ggcccuacgg gcaccggugn n 21

<210> 498
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 498
caccggugcc cguagggccn n 21

<210> 499

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 499
ccacucauuc uuggcaggan n 21

<210> 500
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 500
uccugccaag aaugaguggn n 21

<210> 501
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 501
ccuacgggca ccggugaaun n 21

<210> 502
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 502
auucaccggu gcccguaggn n 21

<210> 503
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 503
cuacgggcac cggugaaucn n 21

<210> 504
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 504
gauucaccgg ugcccguagn n 21

<210> 505
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 505
uacgggcacc ggugaauccn n 21

<210> 506
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 506
ggauucaccg gugcccguan n 21

<210> 507
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 507
acgggcaccg gugaauccan n 21

<210> 508
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 508
uggauucacc ggugcccgun n 21

<210> 509
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 509
gcaccgguga auccaagugn n 21

<210> 510
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 510
cacuuggauu caccggugcn n 21

<210> 511
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 511
caccggugaa uccaagugun n 21

<210> 512
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 512
acacuuggau ucaccggugn n 21

<210> 513
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 513
uguggccaug cauguguucn n 21

<210> 514
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 514
gaacacaugc auggccacan n 21

<210> 515
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 515
guggccaugc auguguucan n 21

<210> 516
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 516
ugaacacaug cauggccacn n 21

<210> 517
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 517
gccaugcaug uguucagaan n 21

<210> 518
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 518
uucugaacac augcauggcn n 21

<210> 519
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 519
uauuccacca cggcugucan n 21

<210> 520
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 520
ugacagccgu gguggaauan n 21

<210> 521
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 521
gucaucacca aucccaaggn n 21

<210> 522
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 522
ccuugggauu ggugaugacn n 21

<210> 523
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>

<221> modified_base
<222> (20)..(21)
<223> a, c, t, g, unknown or other

<400> 523
guccucugau ggucaaagun n 21

<210> 524
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 524
acuuugacca ucagaggacn n 21

<210> 525
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 525
gauggucaaa guucuagaun n 21

<210> 526
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base

<222> (20)..(21)
<223> a, c, t, g, unknown or other

<400> 526
aucuagaacu uugaccaucn n 21

<210> 527
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 527
augcuguccg aggcaguccn n 21

<210> 528
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 528
ggacugccuc ggacagcaun n 21

<210> 529
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)

<223> a, c, t, g, unknown or other

<400> 529
ccgugcaugu guucagaaan n 21

<210> 530
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 530
uuucugaaca caugcacggn n 21

<210> 531
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 531
agucuggaga gcugcauggn n 21

<210> 532
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 532
ccaugcagcu cuccagacun n 21

<210> 533
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 533
caugggcuca caacugaggn n 21

<210> 534
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 534
ccucaguugu gagcccaugn n 21

<210> 535
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 535

ucucaucguc ugcuccuccn n 21

<210> 536
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 536
ggaggagcag acgaugagan n 21

<210> 537
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 537
ccccauucca ugagcaugcn n 21

<210> 538
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 538
gcaugcucau ggaauggggn n 21

<210> 539
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 539
gccccuacuc cuauuccacn n 21

<210> 540
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 540
guggaauagg aguaggggcn n 21

<210> 541
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 541
cuauuccacc acggcugucn n 21

<210> 542

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 542
gacagccgug guggaauagn n 21

<210> 543
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 543
cacggcuguc gucaccaaun n 21

<210> 544
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 544
auuggugacg acagccgugn n 21

<210> 545
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 545
aggacgaggg augggauuun n 21

<210> 546
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 546
aaaucccauc ccucguccun n 21

<210> 547
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 547
ucaccucaua ugcuauguun n 21

<210> 548
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 548
aacauagcau augaggugan n 21

<210> 549
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 549
ccucauaugc uauguuagan n 21

<210> 550
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 550
ucuaacauag cauaugaggn n 21

<210> 551
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 551
auguuagaag uccaggcagn n 21

<210> 552
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 552
cugccuggac uucuaacaun n 21

<210> 553
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 553
ucugaggcug gcccuacggn n 21

<210> 554
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 554
ccguagggcc agccucagan n 21

<210> 555
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 555
ggcccuacgg gcaccggugn n 21

<210> 556
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 556
caccggugcc cguagggccn n 21

<210> 557
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 557
gggcaccggu gaauccaagn n 21

<210> 558
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 558
cuuggauuca ccggugcccn n 21

<210> 559
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 559
ccaugcaugu guucagaaan n 21

<210> 560
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 560
uuucugaaca caugcauggn n 21

<210> 561
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 561
ccggugaauc caagugucct t 21

<210> 562
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 562
ggacacuugg auucaccggt t 21

<210> 563
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 563
acucauucuu ggcaggaugt t 21

<210> 564
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<400> 564
cauccugcca agaaugagut t 21

<210> 565
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 565
aaguguccuc ugauggucat t 21

<210> 566
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 566
ugaccaucag aggacacuut t 21

<210> 567
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 567
ucauucuugg caggauggct t 21

<210> 568
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 568
gccauccugc caagaaugat t 21

<210> 569
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 569
aaguucuaga ugcuguccgt t 21

<210> 570
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 570
cggacagcau cuagaacuut t 21

<210> 571
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 571
guucuagaug cuguccgagt t 21

<210> 572
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 572
cucggacagc aucuagaact t 21

<210> 573
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 573
cuagaugcug uccgaggcat t 21

<210> 574
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 574
ugccucggac agcaucuagt t 21

<210> 575
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 575
gaugcugucc gaggcaguct t 21

<210> 576
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 576
gacugccucg gacagcauct t 21

<210> 577
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 577
cauucuuggc aggauggcut t 21

<210> 578
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 578
agccauccug ccaagaaugt t 21

<210> 579
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 579
ugcuguccga ggcaguccut t 21

<210> 580
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 580
aggacugccu cggacagcat t 21

<210> 581
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 581
ccgaggcagu ccugccauct t 21

<210> 582
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 582
gauggcagga cugccucggt t 21

<210> 583
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 583
caguccugcc aucaaugugt t 21

<210> 584
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 584
cacauugaug gcaggacugt t 21

<210> 585
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 585
caauguggcc gugcaugugt t 21

<210> 586
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 586
cacaugcacg gccacauugt t 21

<210> 587
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 587
auguguucag aaaggcugct t 21

<210> 588
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 588
gcagccuuuc ugaacacaut t 21

<210> 589
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 589
cagaagucca cucauucuut t 21

<210> 590
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 590
aagaaugagu ggacuucugt t 21

<210> 591
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 591
ggcaggaugg cuucucauct t 21

<210> 592
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 592
gaugagaagc cauccugcct t 21

<210> 593
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 593
gagccauuug ccucugggat t 21

<210> 594
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 594
ucccagaggc aaauggcuct t 21

<210> 595
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 595
caggauggcu ucucaucgut t 21

<210> 596
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 596
acgaugagaa gccauccugt t 21

<210> 597
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 597
aggauggcuu cucaucguct t 21

<210> 598
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 598
gacgaugaga agccauccut t 21

<210> 599
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 599
agagcugcau gggcucacat t 21

<210> 600
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 600
ugugagccca ugcagcucut t 21

<210> 601
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 601
gcugcauggg cucacaacut t 21

<210> 602
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 602
aguugugagc ccaugcagct t 21

<210> 603
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 603
ggauggcuuc ucaucgucut t 21

<210> 604
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 604
agacgaugag aagccaucct t 21

<210> 605
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 605
gcaugggcuc acaacugagt t 21

<210> 606
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 606
cucaguugug agcccaugct t 21

<210> 607
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 607
augggcucac aacugaggat t 21

<210> 608
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 608
uccucaguug ugagcccaut t 21

<210> 609
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 609
ugggcucaca acugaggagt t 21

<210> 610
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 610
cuccucaguu gugagcccat t 21

<210> 611
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 611
gaggaauuug uagaagggat t 21

<210> 612
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 612
ucccuucuac aaauuccuct t 21

<210> 613
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 613
uuuguagaag ggauauacat t 21

<210> 614
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 614
uguauauccc uucuacaaat t 21

<210> 615
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 615
uuguagaagg gauauacaat t 21

<210> 616
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 616
uuguauaucc cuucuacaat t 21

<210> 617
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 617
uguagaaggg auauacaaat t 21

<210> 618
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 618
uuuguauauc ccuucuacat t 21

<210> 619
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 619
agaagggaua uacaaagugt t 21

<210> 620
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 620
cacuuuguau aucccuucut t 21

<210> 621
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 621
aaguggaaau agacaccaat t 21

<210> 622
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 622
uuggugucua uuuccacuut t 21

<210> 623
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 623
ggaaauagac accaaaucut t 21

<210> 624
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 624
agauuuggug ucuauuucct t 21

<210> 625
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 625
gaaauagaca ccaaaucuut t 21

<210> 626
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 626
aagauuuggu gucuauuuct t 21

<210> 627
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 627
auagacacca aaucuuacut t 21

<210> 628
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 628
aguaagauuu ggugucuaut t 21

<210> 629
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 629
uagacaccaa aucuuacugt t 21

<210> 630
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 630
caguaagauu uggugucuat t 21

<210> 631
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 631
agacaccaaa ucuuacuggt t 21

<210> 632
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 632
ccaguaagau uuggugucut t 21

<210> 633
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 633
uuacuggaag gcacuuggct t 21

<210> 634
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 634
gccaagugcc uuccaguaat t 21

<210> 635
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 635
uucucaucgu cugcuccuct t 21

<210> 636
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 636
gaggagcaga cgaugagaat t 21

<210> 637
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 637
ggaaggcacu uggcaucuct t 21

<210> 638
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 638
gagaugccaa gugccuucct t 21

<210> 639
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 639
ggcacuuggc aucuccccat t 21

<210> 640
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 640
uggggagaug ccaagugcct t 21

<210> 641
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 641
ggcaucuccc cauuccaugt t 21

<210> 642
<211> 22
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 642
auggaauggg gagaugcctt tt 22

<210> 643
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 643
gcaucucccc auuccaugat t 21

<210> 644
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 644
ucauggaaug gggagaugct t 21

<210> 645
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 645
caucucccca uuccaugagt t 21

<210> 646
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 646
cucauggaau ggggagaugt t 21

<210> 647
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 647
aucuccccau uccaugagct t 21

<210> 648
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 648
gcucauggaa uggggagaut t 21

<210> 649
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 649
cuccccauuc caugagcaut t 21

<210> 650
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 650
augcucaugg aauggggagt t 21

<210> 651
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 651
cccauuccau gagcaugcat t 21

<210> 652
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 652
ugcaugcuca uggaaugggt t 21

<210> 653
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 653
ccaugagcau gcagaggugt t 21

<210> 654
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 654
caccucugca ugcucauggt t 21

<210> 655
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 655
agcaugcaga ggugguauut t 21

<210> 656
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 656
aauaccaccu cugcaugcut t 21

<210> 657
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 657
caugcagagg ugguauucat t 21

<210> 658
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 658
ugaauaccac cucugcaugt t 21

<210> 659
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 659
augcagaggu gguauucact t 21

<210> 660
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 660
gugaauacca ccucugcaut t 21

<210> 661
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 661
ggugguauuc acagccaact t 21

<210> 662
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 662
guuggcugug aauaccacct t 21

<210> 663
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 663
gugguauuca cagccaacgt t 21

<210> 664
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 664
cguuggcugu gaauaccact t 21

<210> 665
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 665
ugguauucac agccaacgat t 21

<210> 666
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 666
ucguuggcug ugaauaccat t 21

<210> 667
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 667
gguauucaca gccaacgact t 21

<210> 668
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 668
gucguuggcu gugaauacct t 21

<210> 669
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 669
guauucacag ccaacgacut t 21

<210> 670
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 670
agucguuggc ugugaauact t 21

<210> 671
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 671
uauucacagc caacgacuct t 21

<210> 672
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 672
gagucguugg cugugaauat t 21

<210> 673
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 673
ucacagccaa cgacuccggt t 21

<210> 674
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 674
ccggagucgu uggcugugat t 21

<210> 675
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 675
ccccgccgcu acaccauugt t 21

<210> 676
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 676
caauggugua gcggcggggt t 21

<210> 677
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 677
gaaguccacu cauucuuggt t 21

<210> 678
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 678
ccaagaauga guggacuuct t 21

<210> 679
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 679
cccugcugag ccccuacuct t 21

<210> 680
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 680
gaguaggggc ucagcagggt t 21

<210> 681
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 681
cugagccccu acuccuauut t 21

<210> 682
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 682
aauaggagua ggggcucagt t 21

<210> 683
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 683
ugagccccua cuccuauuct t 21

<210> 684
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 684
gaauaggagu aggggcucat t 21

<210> 685
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 685
ccccuacucc uauuccacct t 21

<210> 686
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 686
gguggaauag gaguaggggt t 21

<210> 687
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 687
cuacuccuau uccaccacgt t 21

<210> 688
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 688
cguggugga a uaggaguagt t 21

<210> 689
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 689
uacuccuauu ccaccacggt t 21

<210> 690
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 690
ccguggugga auaggaguat t 21

<210> 691
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 691
acuccuauuc caccacggct t 21

<210> 692
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 692
gccguggugg aauaggagut t 21

<210> 693
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 693
uccuauucca ccacggcugt t 21

<210> 694
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 694
cagccguggu ggaauaggat t 21

<210> 695
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 695
uauuccacca cggcugucgt t 21

<210> 696
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 696
cgacagccgu gguggaauat t 21

<210> 697
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 697
auuccaccac ggcugucgut t 21

<210> 698
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 698
acgacagccg ugguggaaut t 21

<210> 699
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 699
caccacggcu gucgucacct t 21

<210> 700
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 700
ggugacgaca gccguggugt t 21

<210> 701
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 701
accacggcug ucgucaccat t 21

<210> 702
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 702
uggugacgac agccguggut t 21

<210> 703
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 703
ccacggcugu cgucaccaat t 21

<210> 704
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 704
uuggugacga cagccguggt t 21

<210> 705
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 705
acggcugucg ucaccaauct t 21

<210> 706
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 706
gauuggugac gacagccgut t 21

<210> 707
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 707
cggcugucgu caccaaucct t 21

<210> 708
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 708
ggauugguga cgacagccgt t 21

<210> 709
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 709
cgucaccaau cccaaggaat t 21

<210> 710
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 710
uuccuuggga uuggugacgt t 21

<210> 711
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 711
caaucccaag gaaugagggt t 21

<210> 712
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 712
cccucauucc uugggauugt t 21

<210> 713
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 713
ccugaaggac gagggauggt t 21

<210> 714
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 714
ccaucccucg uccuucaggt t 21

<210> 715
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 715
ggacgaggga ugggauuuct t 21

<210> 716
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 716
gaaaucccau cccucgucct t 21

<210> 717
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 717
aaguccacuc auucuuggct t 21

<210> 718
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 718
gccaagaaug aguggacuut t 21

<210> 719
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 719
gggauuucau guaaccaagt t 21

<210> 720
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 720
cuugguuaca ugaaauccct t 21

<210> 721
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 721
ggauuucaug uaaccaagat t 21

<210> 722
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 722
ucuugguuac augaaaucct t 21

<210> 723
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 723
ucauguaacc aagaguauut t 21

<210> 724
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 724
aauacucuug guuacaugat t 21

<210> 725
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 725
auguaaccaa gaguauucct t 21

<210> 726
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 726
ggaauacucu ugguuacaut t 21

<210> 727
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 727
uguaaccaag aguauuccat t 21

<210> 728
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 728
uggaauacuc uugguuacat t 21

<210> 729
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 729
guaaccaaga guauuccaut t 21

<210> 730
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 730
auggaauacu cuugguuact t 21

<210> 731
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 731
ugccuugcug gacugguaut t 21

<210> 732
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 732
auaccagucc agcaaggcat t 21

<210> 733
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 733
uaaagcagug uuuucaccut t 21

<210> 734
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 734
aggugaaaac acugcuuuat t 21

<210> 735
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 735
gccuugcugg acugguauut t 21

<210> 736
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 736
aauaccaguc cagcaaggct t 21

<210> 737
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 737
uguuuucacc ucauaugcut t 21

<210> 738
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 738
agcauaugag gugaaaacat t 21

<210> 739
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 739
guuuucaccu cauaugcuat t 21

<210> 740
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 740
uagcauauga ggugaaaact t 21

<210> 741
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 741
uuuucaccuc auaugcuaut t 21

<210> 742
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 742
auagcauaug aggugaaaat t 21

<210> 743
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 743
uucaccucau augcuaugut t 21

<210> 744
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 744
acauagcaua ugaggugaat t 21

<210> 745
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 745
caccucauau gcuauguuat t 21

<210> 746
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 746
uaacauagca uaugaggugt t 21

<210> 747
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 747
ccuugcugga cugguauuut t 21

<210> 748
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 748
aaauaccagu ccagcaaggt t 21

<210> 749
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 749
auaugcuaug uuagaaguct t 21

<210> 750
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 750
gacuucuaac auagcauaut t 21

<210> 751
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 751
uaugcuaugu uagaagucct t 21

<210> 752
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 752
ggacuucuaa cauagcauat t 21

<210> 753
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 753
ugcuauguua gaaguccagt t 21

<210> 754
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 754
cuggacuucu aacauagcat t 21

<210> 755
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 755
cuugcuggac ugguauuugt t 21

<210> 756
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 756
caaauaccag uccagcaagt t 21

<210> 757
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 757
aguccaggca gagacaauat t 21

<210> 758
<211> 22
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 758
auugucucug ccuggacutt tt 22

<210> 759
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 759
uccaggcaga gacaauaaat t 21

<210> 760
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 760
uuuauugucu cugccuggat t 21

<210> 761
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 761
gugaaaggca cuuuucauut t 21

<210> 762
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 762
aaugaaaagu gccuuucact t 21

<210> 763
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 763
uggacuggua uuugugucut t 21

<210> 764
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 764
agacacaaau accaguccat t 21

<210> 765
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 765
gucugaggcu ggcccuacgt t 21

<210> 766
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 766
cguagggcca gccucagact t 21

<210> 767
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 767
cugaggcugg cccuacgggt t 21

<210> 768
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 768
cccguagggc cagccucagt t 21

<210> 769
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 769
gaggcuggcc cuacgggcat t 21

<210> 770
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 770
ugcccguagg gccagccuct t 21

<210> 771
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 771
aggcuggccc uacgggcact t 21

<210> 772
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 772
gugcccguag ggccagccut t 21

<210> 773
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 773
gcuggcccua cgggcaccgt t 21

<210> 774
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 774
cggugcccgu agggccagct t 21

<210> 775
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 775
cuggcccuac gggcaccggt t 21

<210> 776
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 776
ccggugcccg uagggccagt t 21

<210> 777
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 777
ggcccuacgg gcaccggugt t 21

<210> 778
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 778
caccggugcc cguagggcct t 21

<210> 779
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 779
ccacucauuc uuggcaggat t 21

<210> 780
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 780
uccugccaag aaugaguggt t 21

<210> 781
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 781
ccuacgggca ccggugaaut t 21

<210> 782
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 782
auucaccggu gcccguaggt t 21

<210> 783
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 783
cuacgggcac cggugaauct t 21

<210> 784
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 784
gauucaccgg ugcccguagt t 21

<210> 785
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 785
uacgggcacc ggugaaucct t 21

<210> 786
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 786
ggauucaccg gugcccguat t 21

<210> 787
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 787
acgggcaccg gugaauccat t 21

<210> 788
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 788
uggauucacc ggugcccgut t 21

<210> 789
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 789
gcaccgguga auccaagugt t 21

<210> 790
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 790
cacuuggauu caccggugct t 21

<210> 791
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 791
caccggugaa uccaagugut t 21

<210> 792
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 792
acacuuggau ucaccggugt t 21

<210> 793
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 793
uguggccaug cauguguuct t 21

<210> 794
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 794
gaacacaugc auggccacat t 21

<210> 795
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 795
guggccaugc auguguucat t 21

<210> 796
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 796
ugaacacaug cauggccact t 21

<210> 797
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 797
gccaugcaug uguucagaat t 21

<210> 798
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 798
uucugaacac augcauggct t 21

<210> 799
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 799
uauuccacca cggcugucat t 21

<210> 800
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 800
ugacagccgu gguggaauat t 21

<210> 801
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 801
gucaucacca aucccaaggt t 21

<210> 802
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 802
ccuugggauu ggugaugact t 21

<210> 803
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 803
guccucugau ggucaaagut t 21

<210> 804
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 804
acuuugacca ucagaggact t 21

<210> 805
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 805
gauggucaaa guucuagaut t 21

<210> 806
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 806
aucuagaacu uugaccauct t 21

<210> 807
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 807
augcuguccg aggcagucct t 21

<210> 808
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 808
ggacugccuc ggacagcaut t 21

<210> 809
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 809
ccgugcaugu guucagaaat t 21

<210> 810
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 810
uuucugaaca caugcacggt t 21

<210> 811
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 811
agucuggaga gcugcauggt t 21

<210> 812
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 812
ccaugcagcu cuccagacut t 21

<210> 813
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 813
caugggcuca caacugaggt t 21

<210> 814
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 814
ccucaguugu gagcccaugt t 21

<210> 815
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 815
ucucaucguc ugcuccucct t 21

<210> 816
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 816
ggaggagcag acgaugagat t 21

<210> 817
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 817
ccccauucca ugagcaugct t 21

<210> 818
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 818
gcaugcucau ggaauggggt t 21

<210> 819
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 819
gccccuacuc cuauuccact t 21

<210> 820
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 820
guggaauagg aguaggggct t 21

<210> 821
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 821
cuauuccacc acggcuguct t 21

<210> 822
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 822
gacagccgug guggaauagt t 21

<210> 823
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 823
cacggcuguc gucaccaaut t 21

<210> 824
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 824
auuggugacg acagccgugt t 21

<210> 825
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 825
aggacgaggg augggauuut t 21

<210> 826
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 826
aaaucccauc ccucguccut t 21

<210> 827
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 827
ucaccucaua ugcuauguut t 21

<210> 828
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 828
aacauagcau augaggugat t 21

<210> 829
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 829
ccucauaugc uauguuagat t 21

<210> 830
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 830
ucuaacauag cauaugaggt t 21

<210> 831
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 831
auguuagaag uccaggcagt t 21

<210> 832
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 832
cugccuggac uucuaacaut t 21

<210> 833
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 833
ucugaggcug gcccuacggt t 21

<210> 834
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 834
ccguagggcc agccucagat t 21

<210> 835
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 835
ggcccuacgg gcaccggugt t 21

<210> 836
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 836
caccggugcc cguagggcct t 21

<210> 837
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 837
gggcaccggu gaauccaagt t 21

<210> 838
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 838
cuuggauuca ccggugccct t 21

<210> 839
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 839
ccaugcaugu guucagaaat t 21

<210> 840
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 840
uuucugaaca caugcauggt t 21

<210> 841
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 841
ccggugaauc caagugucct t 21

<210> 842
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 842
ggacacuugg auucaccggt t 21

<210> 843
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 843
acucauucuu ggcaggaugt t 21

<210> 844
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 844
cauccugcca agaaugagut t 21

<210> 845
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 845
aaguguccuc ugauggucat t 21

<210> 846
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 846
ugaccaucag aggacacuut t 21

<210> 847
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 847
ucauucuugg caggauggct t 21

<210> 848
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 848
gccauccugc caagaaugat t 21

<210> 849
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 849
aaguucuaga ugcuguccgt t 21

<210> 850
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 850
cggacagcau cuagaacuut t 21

<210> 851
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 851
guucuagaug cuguccgagt t 21

<210> 852
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 852
cucggacagc aucuagaact t 21

<210> 853
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 853
cuagaugcug uccgaggcat t 21

<210> 854
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 854
ugccucggac agcaucuagt t 21

<210> 855
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 855
gaugcugucc gaggcaguct t 21

<210> 856
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 856
gacugccucg gacagcauct t 21

<210> 857
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 857
cauucuuggc aggauggcut t 21

<210> 858
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 858
agccauccug ccaagaaugt t 21

<210> 859
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 859
ugcuguccga ggcaguccut t 21

<210> 860
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 860
aggacugccu cggacagcat t 21

<210> 861
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 861
ccgaggcagu ccugccauct t 21

<210> 862
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 862
gauggcagga cugccucggt t 21

<210> 863
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 863
caguccugcc aucaaugugt t 21

<210> 864
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 864
cacauugaug gcaggacugt t 21

<210> 865
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 865
caauguggcc gugcaugugt t 21

<210> 866
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 866
cacaugcacg gccacauugt t 21

<210> 867
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 867
auguguucag aaaggcugct t 21

<210> 868
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 868
gcagccuuuc ugaacacaut t 21

<210> 869
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 869
cagaagucca cucauucuut t 21

<210> 870
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 870
aagaaugagu ggacuucugt t 21

<210> 871
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 871
ggcaggaugg cuucucauct t 21

<210> 872
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 872
gaugagaagc cauccugcct t 21

<210> 873
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 873
gagccauuug ccucugggat t 21

<210> 874
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 874
ucccagaggc aaauggcuct t 21

<210> 875
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 875
caggauggcu ucucaucgut t 21

<210> 876
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 876
acgaugagaa gccauccugt t 21

<210> 877
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 877
aggauggcuu cucaucguct t 21

<210> 878
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 878
gacgaugaga agccauccut t 21

<210> 879
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 879
agagcugcau gggcucacat t 21

<210> 880
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 880
ugugagccca ugcagcucut t 21

<210> 881
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 881
gcugcauggg cucacaacut t 21

<210> 882
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 882
aguugugagc ccaugcagct t 21

<210> 883
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 883
ggauggcuuc ucaucgucut t 21

<210> 884
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 884
agacgaugag aagccaucct t 21

<210> 885
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 885
gcaugggcuc acaacugagt t 21

<210> 886
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 886
cucaguugug agcccaugct t 21

<210> 887
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 887
augggcucac aacugaggat t 21

<210> 888
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 888
uccucaguug ugagcccaut t 21

<210> 889
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 889
ugggcucaca acugaggagt t 21

<210> 890
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 890
cuccucaguu gugagcccat t 21

<210> 891
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 891
gaggaauuug uagaagggat t 21

<210> 892
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 892
ucccuucuac aaauuccuct t 21

<210> 893
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 893
uuuguagaag ggauauacat t 21

<210> 894
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 894
uguauauccc uucuacaaat t 21

<210> 895
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 895
uuguagaagg gauauacaat t 21

<210> 896
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 896
uuguauaucc cuucuacaat t 21

<210> 897
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 897
uguagaaggg auauacaaat t 21

<210> 898
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 898
uuuguauauc ccuucuacat t 21

<210> 899
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 899
agaagggaua uacaaagugt t 21

<210> 900
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 900
cacuuuguau aucccuucut t 21

<210> 901
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 901
aaguggaaau agacaccaat t 21

<210> 902
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 902
uuggugucua uuuccacuut t 21

<210> 903
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 903
ggaaauagac accaaaucut t 21

<210> 904
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 904
agauuuggug ucuauuucct t 21

<210> 905
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 905
gaaauagaca ccaaaucuut t 21

<210> 906
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 906
aagauuuggu gucuauuuct t 21

<210> 907
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 907
auagacacca aaucuuacut t 21

<210> 908
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 908
aguaagauuu ggugucuaut t 21

<210> 909
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 909
uagacaccaa aucuuacugt t 21

<210> 910
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 910
caguaagauu uggugucuat t 21

<210> 911
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 911
agacaccaaa ucuuacuggt t 21

<210> 912
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 912
ccaguaagau uuggugucut t 21

<210> 913
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 913
uuacuggaag gcacuuggct t 21

<210> 914
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 914
gccaagugcc uuccaguaat t 21

<210> 915
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 915
uucucaucgu cugcuccuct t 21

<210> 916
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 916
gaggagcaga cgaugagaat t 21

<210> 917
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 917
ggaaggcacu uggcaucuct t 21

<210> 918
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 918
gagaugccaa gugccuucct t 21

<210> 919
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 919
ggcacuuggc aucuccccat t 21

<210> 920
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 920
uggggagaug ccaagugcct t 21

<210> 921
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 921
ggcaucuccc cauuccaugt t 21

<210> 922
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 922
cauggaaugg ggagaugcct t 21

<210> 923
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 923
gcaucucccc auuccaugat t 21

<210> 924
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 924
ucauggaaug gggagaugct t 21

<210> 925
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 925
caucucccca uuccaugagt t 21

<210> 926
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 926
cucauggaau ggggagaugt t 21

<210> 927
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 927
aucuccccau uccaugagct t 21

<210> 928
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 928
gcucauggaa uggggagaut t 21

<210> 929
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 929
cuccccauuc caugagcaut t 21

<210> 930
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 930
augcucaugg aauggggagt t 21

<210> 931
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 931
cccauuccau gagcaugcat t 21

<210> 932
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 932
ugcaugcuca uggaaugggt t 21

<210> 933
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 933
ccaugagcau gcagaggugt t 21

<210> 934
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 934
caccucugca ugcucauggt t 21

<210> 935
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 935
agcaugcaga ggugguauut t 21

<210> 936
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 936
aauaccaccu cugcaugcut t 21

<210> 937
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 937
caugcagagg ugguauucat t 21

<210> 938
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 938
ugaauaccac cucugcaugt t 21

<210> 939
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 939
augcagaggu gguauucact t 21

<210> 940
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 940
gugaauacca ccucugcaut t 21

<210> 941
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 941
ggugguauuc acagccaact t 21

<210> 942
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 942
guuggcugug aauaccacct t 21

<210> 943
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 943
gugguauuca cagccaacgt t 21

<210> 944
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 944
cguuggcugu gaauaccact t 21

<210> 945
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 945
ugguauucac agccaacgat t 21

<210> 946
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 946
ucguuggcug ugaauaccat t 21

<210> 947
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 947
gguauucaca gccaacgact t 21

<210> 948
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 948
gucguuggcu gugaauacct t 21

<210> 949
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 949
guauucacag ccaacgacut t 21

<210> 950
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 950
agucguuggc ugugaauact t 21

<210> 951
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 951
uauucacagc caacgacuct t 21

<210> 952
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 952
gagucguugg cugugaauat t 21

<210> 953
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 953
ucacagccaa cgacuccggt t 21

<210> 954
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 954
ccggagucgu uggcugugat t 21

<210> 955
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 955
ccccgccgcu acaccauugt t 21

<210> 956
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 956
caauggugua gcggcggggt t 21

<210> 957
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 957
gaaguccacu cauucuuggt t 21

<210> 958
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 958
ccaagaauga guggacuuct t 21

<210> 959
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 959
cccugcugag ccccuacuct t 21

<210> 960
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 960
gaguaggggc ucagcagggt t 21

<210> 961
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 961
cugagccccu acuccuauut t 21

<210> 962
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 962
aauaggagua ggggcucagt t 21

<210> 963
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 963
ugagccccua cuccuauuct t 21

<210> 964
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 964
gaauaggagu aggggcucat t 21

<210> 965
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 965
ccccuacucc uauuccacct t 21

<210> 966
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 966
gguggaauag gaguaggggt t 21

<210> 967
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 967
cuacuccuau uccaccacgt t 21

<210> 968
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 968
cgugguggaa uaggaguagt t 21

<210> 969
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 969
uacuccuauu ccaccacggt t 21

<210> 970
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 970
ccguggugga auaggaguat t 21

<210> 971
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 971
acuccuauuc caccacggct t 21

<210> 972
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 972
gccguggugg aauaggagut t 21

<210> 973
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 973
uccuauucca ccacggcugt t 21

<210> 974
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 974
cagccguggu ggaauaggat t 21

<210> 975
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 975
uauuccacca cggcugucgt t 21

<210> 976
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 976
cgacagccgu gguggaauat t 21

<210> 977
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 977
auuccaccac ggcugucgut t 21

<210> 978
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 978
acgacagccg ugguggaaut t 21

<210> 979
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 979
caccacggcu gucgucacct t 21

<210> 980
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 980
ggugacgaca gccguggugt t 21

<210> 981
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 981
accacggcug ucgucaccat t 21

<210> 982
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 982
uggugacgac agccguggut t 21

<210> 983
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 983
ccacggcugu cgucaccaat t 21

<210> 984
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 984
uuggugacga cagccguggt t 21

<210> 985
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 985
acggcugucg ucaccaauct t 21

<210> 986
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 986
gauuggugac gacagccgut t 21

<210> 987
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 987
cggcugucgu caccaaucct t 21

<210> 988
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 988
ggauugguga cgacagccgt t 21

<210> 989
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 989
cgucaccaau cccaaggaat t 21

<210> 990
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 990
uuccuuggga uuggugacgt t 21

<210> 991
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 991
caaucccaag gaaugagggt t 21

<210> 992
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 992
cccucauucc uugggauugt t 21

<210> 993
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 993
ccugaaggac gagggauggt t 21

<210> 994
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 994
ccaucccucg uccuucaggt t 21

<210> 995
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 995
ggacgaggga ugggauuuct t 21

<210> 996
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 996
gaaaucccau cccucgucct t 21

<210> 997
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 997
aaguccacuc auucuuggct t 21

<210> 998
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 998
gccaagaaug aguggacuut t 21

<210> 999
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 999
gggauuucau guaaccaagt t 21

<210> 1000
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1000
cuugguuaca ugaaauccct t 21

<210> 1001
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1001
ggauuucaug uaaccaagat t 21

<210> 1002
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1002
ucuugguuac augaaaucct t 21

<210> 1003
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1003
ucauguaacc aagaguauut t 21

<210> 1004
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1004
aauacucuug guuacaugat t 21

<210> 1005
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1005
auguaaccaa gaguauucct t 21

<210> 1006
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1006
ggaauacucu ugguuacaut t 21

<210> 1007
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1007
uguaaccaag aguauuccat t 21

<210> 1008
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1008
uggaauacuc uugguuacat t 21

<210> 1009
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1009
guaaccaaga guauuccaut t 21

<210> 1010
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1010
auggaauacu cuugguuact t 21

<210> 1011
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1011
ugccuugcug gacugguaut t 21

<210> 1012
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1012
auaccagucc agcaaggcat t 21

<210> 1013
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1013
uaaagcagug uuuucaccut t 21

<210> 1014
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1014
aggugaaaac acugcuuuat t 21

<210> 1015
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1015
gccuugcugg acugguauut t 21

<210> 1016
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1016
aauaccaguc cagcaaggct t 21

<210> 1017
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1017
uguuuucacc ucauaugcut t 21

<210> 1018
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1018
agcauaugag gugaaaacat t 21

<210> 1019
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1019
guuuucaccu cauaugcuat t 21

<210> 1020
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1020
uagcauauga ggugaaaact t 21

<210> 1021
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1021
uuuucaccuc auaugcuaut t 21

<210> 1022
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1022
auagcauaug aggugaaaat t 21

<210> 1023
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1023
uucaccucau augcuaugut t 21

<210> 1024
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1024
acauagcaua ugaggugaat t 21

<210> 1025
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1025
caccucauau gcuauguuat t 21

<210> 1026
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1026
uaacauagca uaugaggugt t 21

<210> 1027
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1027
ccuugcugga cugguauuut t 21

<210> 1028
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1028
aaauaccagu ccagcaaggt t 21

<210> 1029
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1029
auaugcuaug uuagaaguct t 21

<210> 1030
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1030
gacuucuaac auagcauaut t 21

<210> 1031
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1031
uaugcuaugu uagaagucct t 21

<210> 1032
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1032
ggacuucuaa cauagcauat t 21

<210> 1033
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1033
ugcuauguua gaaguccagt t 21

<210> 1034
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1034
cuggacuucu aacauagcat t 21

<210> 1035
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1035
cuugcuggac ugguauuugt t 21

<210> 1036
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1036
caaauaccag uccagcaagt t 21

<210> 1037
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1037
aguccaggca gagacaauat t 21

<210> 1038
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1038
uauugucucu gccuggacut t 21

<210> 1039
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1039
uccaggcaga gacaauaaat t 21

<210> 1040
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1040
uuuauugucu cugccuggat t 21

<210> 1041
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1041
gugaaaggca cuuuucauut t 21

<210> 1042
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1042
aaugaaaagu gccuuucact t 21

<210> 1043
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1043
uggacuggua uuugugucut t 21

<210> 1044
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1044
agacacaaau accaguccat t 21

<210> 1045
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1045
gucugaggcu ggcccuacgt t 21

<210> 1046
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1046
cguagggcca gccucagact t 21

<210> 1047
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1047
cugaggcugg cccuacgggt t 21

<210> 1048
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1048
cccguagggc cagccucagt t 21

<210> 1049
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1049
gaggcuggcc cuacgggcat t 21

<210> 1050
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1050
ugcccguagg gccagccuct t 21

<210> 1051
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1051
aggcuggccc uacgggcact t 21

<210> 1052
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1052
gugcccguag ggccagccut t 21

<210> 1053
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1053
gcuggcccua cgggcaccgt t 21

<210> 1054
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1054
cggugcccgu agggccagct t 21

<210> 1055
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1055
cuggcccuac gggcaccggt t 21

<210> 1056
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1056
ccggugcccg uagggccagt t 21

<210> 1057
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1057
ggcccuacgg gcaccggugt t 21

<210> 1058
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1058
caccggugcc cguagggcct t 21

<210> 1059
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1059
ccacucauuc uuggcaggat t 21

<210> 1060
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1060
uccugccaag aaugaguggt t 21

<210> 1061
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1061
ccuacgggca ccggugaaut t 21

<210> 1062
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1062
auucaccggu gcccguaggt t 21

<210> 1063
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1063
cuacgggcac cggugaauct t 21

<210> 1064
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1064
gauucaccgg ugcccguagt t 21

<210> 1065
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1065
uacgggcacc ggugaaucct t 21

<210> 1066
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1066
ggauucaccg gugcccguat t 21

<210> 1067
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1067
acgggcaccg gugaauccat t 21

<210> 1068
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1068
uggauucacc ggugcccgut t 21

<210> 1069
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1069
gcaccgguga auccaagugt t 21

<210> 1070
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1070
cacuuggauu caccggugct t 21

<210> 1071
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1071
caccggugaa uccaagugut t 21

<210> 1072
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1072
acacuuggau ucaccggugt t 21

<210> 1073
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1073
uguggccaug cauguguuct t 21

<210> 1074
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1074
gaacacaugc auggccacat t 21

<210> 1075
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1075
guggccaugc auguguucat t 21

<210> 1076
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1076
ugaacacaug cauggccact t 21

<210> 1077
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1077
gccaugcaug uguucagaat t 21

<210> 1078
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1078
uucugaacac augcauggct t 21

<210> 1079
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1079
uauuccacca cggcugucat t 21

<210> 1080
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1080
ugacagccgu gguggaauat t 21

<210> 1081
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1081
gucaucacca aucccaaggt t 21

<210> 1082
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1082
ccuugggauu ggugaugact t 21

<210> 1083
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1083
guccucugau ggucaaagut t 21

<210> 1084
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1084
acuuugacca ucagaggact t 21

<210> 1085
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1085
gauggucaaa guucuagaut t 21

<210> 1086
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1086
aucuagaacu uugaccauct t 21

<210> 1087
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1087
augcuguccg aggcagucct t 21

<210> 1088
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1088
ggacugccuc ggacagcaut t 21

<210> 1089
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1089
ccgugcaugu guucagaaat t 21

<210> 1090
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1090
uuucugaaca caugcacggt t 21

<210> 1091
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1091
agucuggaga gcugcauggt t 21

<210> 1092
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1092
ccaugcagcu cuccagacut t 21

<210> 1093
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1093
caugggcuca caacugaggt t 21

<210> 1094
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1094
ccucaguugu gagcccaugt t 21

<210> 1095
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1095
ucucaucguc ugcuccucct t 21

<210> 1096
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1096
ggaggagcag acgaugagat t 21

<210> 1097
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1097
ccccauucca ugagcaugct t 21

<210> 1098
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1098
gcaugcucau ggaauggggt t 21

<210> 1099
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1099
gccccuacuc cuauuccact t 21

<210> 1100
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1100
guggaauagg aguaggggct t 21

<210> 1101
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1101
cuauuccacc acggcuguct t 21

<210> 1102
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1102
gacagccgug guggaauagt t 21

<210> 1103
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1103
cacggcuguc gucaccaaut t 21

<210> 1104
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1104
auuggugacg acagccgugt t 21

<210> 1105
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1105
aggacgaggg augggauuut t 21

<210> 1106
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1106
aaaucccauc ccucguccut t 21

<210> 1107
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1107
ucaccucaua ugcuauguut t 21

<210> 1108
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1108
aacauagcau augaggugat t 21

<210> 1109
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1109
ccucauaugc uauguuagat t 21

<210> 1110
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1110
ucuaacauag cauaugaggt t 21

<210> 1111
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1111
auguuagaag uccaggcagt t 21

<210> 1112
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1112
cugccuggac uucuaacaut t 21

<210> 1113
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1113
ucugaggcug gcccuacggt t 21

<210> 1114
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1114
ccguagggcc agccucagat t 21

<210> 1115
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1115
ggcccuacgg gcaccggugt t 21

<210> 1116
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1116
caccggugcc cguagggcct t 21

<210> 1117
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1117
gggcaccggu gaauccaagt t 21

<210> 1118
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1118
cuuggauuca ccggugccct t 21

<210> 1119
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1119
ccaugcaugu guucagaaat t 21

<210> 1120
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1120
uuucugaaca caugcauggt t 21

<210> 1121
<211> 19
<212> RNA
<213> Artificial Sequence

<220>
<223> Description of Artificial Sequence: Synthetic oligonucleotide

<400> 1121
guccucugau ggucaaagu 19

<210> 1122
<211> 19
<212> RNA
<213> Artificial Sequence

<220>
<223> Description of Artificial Sequence: Synthetic oligonucleotide

<400> 1122
acuuugacca ucagaggac 19

<210> 1123
<211> 19
<212> RNA
<213> Artificial Sequence

<220>
<223> Description of Artificial Sequence: Synthetic oligonucleotide

<400> 1123
uucuugcucu auaaaccgu 19

<210> 1124
<211> 19
<212> RNA
<213> Artificial Sequence

<220>
<223> Description of Artificial Sequence: Synthetic oligonucleotide

<400> 1124
acgguuuaua gagcaagaa 19

<210> 1125
<211> 19
<212> RNA
<213> Artificial Sequence

<220>
<223> Description of Artificial Sequence: Synthetic oligonucleotide

<400> 1125
cucuauaaac cguguuagc 19

<210> 1126
<211> 19
<212> RNA
<213> Artificial Sequence

<220>
<223> Description of Artificial Sequence: Synthetic oligonucleotide

<400> 1126
gcuaacacgg uuuauagag 19

<210> 1127
<211> 19
<212> RNA
<213> Artificial Sequence

<220>
<223> Description of Artificial Sequence: Synthetic oligonucleotide

<400> 1127
ucgccacuac accaucgca 19

<210> 1128
<211> 19
<212> RNA
<213> Artificial Sequence

<220>
<223> Description of Artificial Sequence: Synthetic oligonucleotide

<400> 1128
ugcgauggug uaguggcga 19

<210> 1129
<211> 19
<212> RNA
<213> Artificial Sequence

<220>
<223> Description of Artificial Sequence: Synthetic oligonucleotide

<400> 1129
ucuugcucua uaaaccgug 19

<210> 1130
<211> 19
<212> RNA
<213> Artificial Sequence

<220>
<223> Description of Artificial Sequence: Synthetic oligonucleotide

<400> 1130
cacgguuuau agagcaaga 19

<210> 1131
<211> 19
<212> RNA
<213> Artificial Sequence

<220>
<223> Description of Artificial Sequence: Synthetic oligonucleotide

<400> 1131
ugcucuauaa accguguua 19

<210> 1132
<211> 19
<212> RNA
<213> Artificial Sequence

<220>
<223> Description of Artificial Sequence: Synthetic oligonucleotide

<400> 1132
uaacacgguu uauagagca 19

<210> 1133
<211> 19
<212> RNA
<213> Artificial Sequence

<220>
<223> Description of Artificial Sequence: Synthetic oligonucleotide

<400> 1133
caguguucuu gcucuauaa 19

<210> 1134
<211> 19
<212> RNA
<213> Artificial Sequence

<220>
<223> Description of Artificial Sequence: Synthetic oligonucleotide

<400> 1134
uuauagagca agaacacug 19

<210> 1135
<211> 19
<212> RNA
<213> Artificial Sequence

<220>
<223> Description of Artificial Sequence: Synthetic oligonucleotide

<400> 1135
gcucuauaaa ccguguuag 19

<210> 1136
<211> 19
<212> RNA
<213> Artificial Sequence

<220>
<223> Description of Artificial Sequence: Synthetic oligonucleotide

<400> 1136
cuaacacggu uuauagagc 19

<210> 1137
<211> 19
<212> RNA
<213> Artificial Sequence

<220>
<223> Description of Artificial Sequence: Synthetic oligonucleotide

<400> 1137
ccuggaugcu guccgaggc 19

<210> 1138
<211> 19
<212> RNA
<213> Artificial Sequence

<220>
<223> Description of Artificial Sequence: Synthetic oligonucleotide

<400> 1138
gccucggaca gcauccagg 19

<210> 1139
<211> 19
<212> RNA

<213> Artificial Sequence

<220>
<223> Description of Artificial Sequence: Synthetic oligonucleotide

<400> 1139
ucugaugguc aaaguccug 19

<210> 1140
<211> 19
<212> RNA
<213> Artificial Sequence

<220>
<223> Description of Artificial Sequence: Synthetic oligonucleotide

<400> 1140
caggacuuug accaucaga 19

<210> 1141
<211> 19
<212> RNA
<213> Artificial Sequence

<220>
<223> Description of Artificial Sequence: Synthetic oligonucleotide

<400> 1141
cuggagagcu gcacgggcu 19

<210> 1142
<211> 19
<212> RNA
<213> Artificial Sequence

<220>
<223> Description of Artificial Sequence: Synthetic oligonucleotide

<400> 1142
agcccgugca gcucuccag 19

<210> 1143
<211> 19
<212> RNA
<213> Artificial Sequence

<220>
<223> Description of Artificial Sequence: Synthetic oligonucleotide

<400> 1143
ucuauaaacc guguuagca 19

<210> 1144
<211> 19
<212> RNA
<213> Artificial Sequence

<220>

<223> Description of Artificial Sequence: Synthetic oligonucleotide

<400> 1144
ugcuaacacg guuuauaga 19

<210> 1145
<211> 19
<212> RNA
<213> Artificial Sequence

<220>
<223> Description of Artificial Sequence: Synthetic oligonucleotide

<400> 1145
aacaguguuc uugcucuau 19

<210> 1146
<211> 19
<212> RNA
<213> Artificial Sequence

<220>
<223> Description of Artificial Sequence: Synthetic oligonucleotide

<400> 1146
auagagcaag aacacuguu 19

<210> 1147
<211> 19
<212> RNA
<213> Artificial Sequence

<220>
<223> Description of Artificial Sequence: Synthetic oligonucleotide

<400> 1147
cucugauggu caaaguccu 19

<210> 1148
<211> 19
<212> RNA
<213> Artificial Sequence

<220>
<223> Description of Artificial Sequence: Synthetic oligonucleotide

<400> 1148
aggacuuuga ccaucagag 19

<210> 1149
<211> 19
<212> RNA
<213> Artificial Sequence

<220>
<223> Description of Artificial Sequence: Synthetic oligonucleotide

<400> 1149

ugcuguccga ggcagcccu 19

<210> 1150
<211> 19
<212> RNA
<213> Artificial Sequence

<220>
<223> Description of Artificial Sequence: Synthetic oligonucleotide

<400> 1150
agggcugccu cggacagca 19

<210> 1151
<211> 19
<212> RNA
<213> Artificial Sequence

<220>
<223> Description of Artificial Sequence: Synthetic oligonucleotide

<400> 1151
gucuggagag cugcacggg 19

<210> 1152
<211> 19
<212> RNA
<213> Artificial Sequence

<220>
<223> Description of Artificial Sequence: Synthetic oligonucleotide

<400> 1152
cccgugcagc ucuccagac 19

<210> 1153
<211> 19
<212> RNA
<213> Artificial Sequence

<220>
<223> Description of Artificial Sequence: Synthetic oligonucleotide

<400> 1153
acaguguucu ugcucuaua 19

<210> 1154
<211> 19
<212> RNA
<213> Artificial Sequence

<220>
<223> Description of Artificial Sequence: Synthetic oligonucleotide

<400> 1154
uauagagcaa gaacacugu 19

<210> 1155

<211> 19
<212> RNA
<213> Artificial Sequence

<220>
<223> Description of Artificial Sequence: Synthetic oligonucleotide

<400> 1155
ccucugaugg ucaaagucc 19

<210> 1156
<211> 19
<212> RNA
<213> Artificial Sequence

<220>
<223> Description of Artificial Sequence: Synthetic oligonucleotide

<400> 1156
ggacuuugac caucagagg 19

<210> 1157
<211> 19
<212> RNA
<213> Artificial Sequence

<220>
<223> Description of Artificial Sequence: Synthetic oligonucleotide

<400> 1157
aguccuggau gcuguccga 19

<210> 1158
<211> 19
<212> RNA
<213> Artificial Sequence

<220>
<223> Description of Artificial Sequence: Synthetic oligonucleotide

<400> 1158
ucggacagca uccaggacu 19

<210> 1159
<211> 19
<212> RNA
<213> Artificial Sequence

<220>
<223> Description of Artificial Sequence: Synthetic oligonucleotide

<400> 1159
uugccucugg gaagaccgc 19

<210> 1160
<211> 19
<212> RNA
<213> Artificial Sequence

<220>
<223> Description of Artificial Sequence: Synthetic oligonucleotide

<400> 1160
gcggucuucc cagaggcaa 19

<210> 1161
<211> 19
<212> RNA
<213> Artificial Sequence

<220>
<223> Description of Artificial Sequence: Synthetic oligonucleotide

<400> 1161
uggagagcug cacgggcuc 19

<210> 1162
<211> 19
<212> RNA
<213> Artificial Sequence

<220>
<223> Description of Artificial Sequence: Synthetic oligonucleotide

<400> 1162
gagcccgugc agcucucca 19

<210> 1163
<211> 19
<212> RNA
<213> Artificial Sequence

<220>
<223> Description of Artificial Sequence: Synthetic oligonucleotide

<400> 1163
gagagcugca cgggcucac 19

<210> 1164
<211> 19
<212> RNA
<213> Artificial Sequence

<220>
<223> Description of Artificial Sequence: Synthetic oligonucleotide

<400> 1164
gugagcccgu gcagcucuc 19

<210> 1165
<211> 19
<212> RNA
<213> Artificial Sequence

<220>
<223> Description of Artificial Sequence: Synthetic oligonucleotide

<400> 1165
gagcugcacg ggcucacca 19

<210> 1166
<211> 19
<212> RNA
<213> Artificial Sequence

<220>
<223> Description of Artificial Sequence: Synthetic oligonucleotide

<400> 1166
uggugagccc gugcagcuc 19

<210> 1167
<211> 19
<212> RNA
<213> Artificial Sequence

<220>
<223> Description of Artificial Sequence: Synthetic oligonucleotide

<400> 1167
uacaccaucg cagcccugc 19

<210> 1168
<211> 19
<212> RNA
<213> Artificial Sequence

<220>
<223> Description of Artificial Sequence: Synthetic oligonucleotide

<400> 1168
gcagggcugc gauggugua 19

<210> 1169
<211> 19
<212> RNA
<213> Artificial Sequence

<220>
<223> Description of Artificial Sequence: Synthetic oligonucleotide

<400> 1169
guccuggaug cuguccgag 19

<210> 1170
<211> 19
<212> RNA
<213> Artificial Sequence

<220>
<223> Description of Artificial Sequence: Synthetic oligonucleotide

<400> 1170
cucggacagc auccaggac 19

<210> 1171
<211> 19
<212> RNA
<213> Artificial Sequence

<220>
<223> Description of Artificial Sequence: Synthetic oligonucleotide

<400> 1171
agagcugcac gggcucacc 19

<210> 1172
<211> 19
<212> RNA
<213> Artificial Sequence

<220>
<223> Description of Artificial Sequence: Synthetic oligonucleotide

<400> 1172
ggugagcccg ugcagcucu 19

<210> 1173
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1173
guccucugau ggucaaagun n 21

<210> 1174
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1174

acuuugacca ucagaggacn n 21

<210> 1175
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1175
uucuugcucu auaaaccgun n 21

<210> 1176
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1176
acgguuuaua gagcaagaan n 21

<210> 1177
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1177
cucuauaaac cguguuagcn n 21

<210> 1178
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1178
gcuaacacgg uuuauagagn n 21

<210> 1179
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1179
ucgccacuac accaucgcan n 21

<210> 1180
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1180
ugcgauggug uaguggcgan n 21

<210> 1181

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1181
ucuugcucua uaaaccgugn n 21

<210> 1182
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1182
cacgguuuau agagcaagan n 21

<210> 1183
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1183
ugcucuauaa accguguuan n 21

<210> 1184
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1184
uaacacgguu uauagagcan n 21

<210> 1185
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1185
caguguucuu gcucuauaan n 21

<210> 1186
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1186
uuauagagca agaacacugn n 21

<210> 1187
<211> 21
<212> DNA

<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1187
gcucuauaaa ccguguuagn n 21

<210> 1188
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1188
cuaacacggu uuauagagcn n 21

<210> 1189
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1189
ccuggaugcu guccgaggcn n 21

<210> 1190
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1190
gccucggaca gcauccaggn n 21

<210> 1191
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1191
ucugaugguc aaaguccugn n 21

<210> 1192
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1192
caggacuuug accaucagan n 21

<210> 1193
<211> 21
<212> DNA
<213> Artificial Sequence

<220>

<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1193
cuggagagcu gcacgggcun n 21

<210> 1194
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1194
agcccgugca gcucuccagn n 21

<210> 1195
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1195
ucuauaaacc guguuagcan n 21

<210> 1196
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1196
ugcuaacacg guuuauagan n 21

<210> 1197
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1197
aacaguguuc uugcucuaun n 21

<210> 1198
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1198
auagagcaag aacacuguun n 21

<210> 1199
<211> 21
<212> DNA
<213> Artificial Sequence

<220>
<223> Description of Artificial Sequence: Synthetic oligonucleotide

<220>

<223> Description of Combined DNA/RNA Molecule: Synthetic oligonucleotide

<220>
<221> modified_base
<222> (20)..(21)
<223> a, c, t, g, unknown or other

<400> 1199
cucugauggu caaaguccun n 21

<210> 1200
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1200
aggacuuuga ccaucagagn n 21

<210> 1201
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1201
ugcuguccga ggcagcccun n 21

<210> 1202
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1202
agggcugccu cggacagcan n 21

<210> 1203
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1203
gucuggagag cugcacgggn n 21

<210> 1204
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1204
cccgugcagc ucuccagacn n 21

<210> 1205
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>

<221> modified_base
<222> (20)..(21)
<223> a, c, t, g, unknown or other

<400> 1205
acaguguucu ugcucuauan n 21

<210> 1206
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1206
uauagagcaa gaacacugun n 21

<210> 1207
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1207
ccucugaugg ucaaaguccn n 21

<210> 1208
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base

<222> (20)..(21)
<223> a, c, t, g, unknown or other

<400> 1208
ggacuuugac caucagaggn n 21

<210> 1209
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1209
aguccuggau gcuguccgan n 21

<210> 1210
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1210
ucggacagca uccaggacun n 21

<210> 1211
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)

<223> a, c, t, g, unknown or other

<400> 1211
uugccucugg gaagaccgcn n 21

<210> 1212
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1212
gcggucuucc cagaggcaan n 21

<210> 1213
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1213
uggagagcug cacgggcucn n 21

<210> 1214
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1214
gagcccgugc agcucuccan n 21

<210> 1215
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1215
gagagcugca cgggcucacn n 21

<210> 1216
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1216
gugagcccgu gcagcucucn n 21

<210> 1217
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1217

gagcugcacg ggcucaccan n 21

<210> 1218
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1218
uggugagccc gugcagcucn n 21

<210> 1219
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1219
uacaccaucg cagcccugcn n 21

<210> 1220
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1220
gcagggcugc gaugguguan n 21

<210> 1221
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1221
guccuggaug cuguccgagn n 21

<210> 1222
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1222
cucggacagc auccaggacn n 21

<210> 1223
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1223
agagcugcac gggcucaccn n 21

<210> 1224

<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<220>
<221> modified_base
<222> (20)..(21)
<223> a, c, t, g, unknown or other

<400> 1224
ggugagcccg ugcagcucun n 21

<210> 1225
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1225
guccucugau ggucaaagut t 21

<210> 1226
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1226
acuuugacca ucagaggact t 21

<210> 1227
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1227

uucuugcucu auaaaccgut t 21

<210> 1228
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1228
acgguuuaua gagcaagaat t 21

<210> 1229
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1229
cucuauaaac cguguuagct t 21

<210> 1230
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1230
gcuaacacgg uuuauagagt t 21

<210> 1231
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1231
ucgccacuac accaucgcat t 21

<210> 1232
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1232
ugcgauggug uaguggcgat t 21

<210> 1233
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1233
ucuugcucua uaaaccgugt t 21

<210> 1234
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1234
cacgguuuau agagcaagat t 21

<210> 1235
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1235
ugcucuauaa accguguuat t 21

<210> 1236
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1236
uaacacgguu uauagagcat t 21

<210> 1237
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1237
caguguucuu gcucuauaat t 21

<210> 1238
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1238
uuauagagca agaacacugt t 21

<210> 1239
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1239
gcucuauaaa ccguguuagt t 21

<210> 1240
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1240
cuaacacggu uuauagagct t 21

<210> 1241
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1241
ccuggaugcu guccgaggct t 21

<210> 1242
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1242
gccucggaca gcauccaggt t 21

<210> 1243
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1243
ucugaugguc aaaguccugt t 21

<210> 1244
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1244
caggacuuug accaucagat t 21

<210> 1245
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1245
cuggagagcu gcacgggcut t 21

<210> 1246
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1246
agcccgugca gcucuccagt t 21

<210> 1247
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1247
ucuauaaacc guguuagcat t 21

<210> 1248
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1248
ugcuaacacg guuuauagat t 21

<210> 1249
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1249
aacaguguuc uugcucuaut t 21

<210> 1250
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1250
auagagcaag aacacuguut t 21

<210> 1251
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1251
cucugauggu caaaguccut t 21

<210> 1252
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1252
aggacuuuga ccaucagagt t 21

<210> 1253
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1253
ugcuguccga ggcagcccut t 21

<210> 1254
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1254
agggcugccu cggacagcat t 21

<210> 1255
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1255
gucuggagag cugcacgggt t 21

<210> 1256
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1256
cccgugcagc ucuccagact t 21

<210> 1257
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1257
acaguguucu ugcucuauat t 21

<210> 1258
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1258
uauagagcaa gaacacugut t 21

<210> 1259
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1259
ccucugaugg ucaaagucct t 21

<210> 1260
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1260
ggacuuugac caucagaggt t 21

<210> 1261
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1261
aguccuggau gcuguccgat t 21

<210> 1262
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1262
ucggacagca uccaggacut t 21

<210> 1263
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1263
uugccucugg gaagaccgct t 21

<210> 1264
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1264
gcggucuucc cagaggcaat t 21

<210> 1265
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1265
uggagagcug cacgggcuct t 21

<210> 1266
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1266
gagcccgugc agcucuccat t 21

<210> 1267
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1267
gagagcugca cgggcucact t 21

<210> 1268
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1268
gugagcccgu gcagcucuct t 21

<210> 1269
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1269
gagcugcacg ggcucaccat t 21

<210> 1270
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1270
uggugagccc gugcagcuct t 21

<210> 1271
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1271
uacaccaucg cagcccugct t 21

<210> 1272
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1272
gcagggcugc gaugguguat t 21

<210> 1273
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1273
guccuggaug cuguccgagt t 21

<210> 1274
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1274
cucggacagc auccaggact t 21

<210> 1275
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1275
agagcugcac gggcucacct t 21

<210> 1276
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1276
ggugagcccg ugcagcucut t 21

<210> 1277
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1277
guccucugau ggucaaagut t 21

<210> 1278
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1278
acuuugacca ucagaggact t 21

<210> 1279
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1279
uucuugcucu auaaaccgut t 21

<210> 1280
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1280
acgguuuaua gagcaagaat t 21

<210> 1281
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1281
cucuauaaac cguguuagct t 21

<210> 1282
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1282
gcuaacacgg uuuauagagt t 21

<210> 1283
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1283
ucgccacuac accaucgcat t 21

<210> 1284
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1284
ugcgauggug uaguggcgat t 21

<210> 1285
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1285
ucuugcucua uaaaccgugt t 21

<210> 1286
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1286
cacgguuuau agagcaagat t 21

<210> 1287
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1287
ugcucuauaa accguguuat t 21

<210> 1288
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1288
uaacacgguu uauagagcat t 21

<210> 1289
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1289
caguguucuu gcucuauaat t 21

<210> 1290
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1290
uuauagagca agaacacugt t 21

<210> 1291
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1291
gcucuauaaa ccguguuagt t 21

<210> 1292
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1292
cuaacacggu uuauagagct t 21

<210> 1293
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1293
ccuggaugcu guccgaggct t 21

<210> 1294
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1294
gccucggaca gcauccaggt t 21

<210> 1295
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1295
ucugaugguc aaaguccugt t 21

<210> 1296
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1296
caggacuuug accaucagat t 21

<210> 1297
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1297
cuggagagcu gcacgggcut t 21

<210> 1298
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1298
agcccgugca gcucuccagt t 21

<210> 1299
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1299
ucuauaaacc guguuagcat t 21

<210> 1300
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1300
ugcuaacacg guuuauagat t 21

<210> 1301
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1301
aacaguguuc uugcucuaut t 21

<210> 1302
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1302
auagagcaag aacacuguut t 21

<210> 1303
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1303
cucugauggu caaaguccut t 21

<210> 1304
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1304
aggacuuuga ccaucagagt t 21

<210> 1305
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1305
ugcuguccga ggcagcccut t 21

<210> 1306
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1306
agggcugccu cggacagcat t 21

<210> 1307
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1307
gucuggagag cugcacgggt t 21

<210> 1308
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1308
cccgugcagc ucuccagact t 21

<210> 1309
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1309
acaguguucu ugcucuauat t 21

<210> 1310
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1310
uauagagcaa gaacacugut t 21

<210> 1311
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1311
ccucugaugg ucaaagucct t 21

<210> 1312
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1312
ggacuuugac caucagaggt t 21

<210> 1313
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1313
aguccuggau gcuguccgat t 21

<210> 1314
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1314
ucggacagca uccaggacut t 21

<210> 1315
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1315
uugccucugg gaagaccgct t 21

<210> 1316
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1316
gcggucuucc cagaggcaat t 21

<210> 1317
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1317
uggagagcug cacgggcuct t 21

<210> 1318
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1318
gagcccgugc agcucuccat t 21

<210> 1319
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1319
gagagcugca cgggcucact t 21

<210> 1320
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1320
gugagcccgu gcagcucuct t 21

<210> 1321
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1321
gagcugcacg ggcucaccat t 21

<210> 1322
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1322
uggugagccc gugcagcuct t 21

<210> 1323
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1323
uacaccaucg cagcccugct t 21

<210> 1324
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1324
gcagggcugc gaugguguat t 21

<210> 1325
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1325
guccuggaug cuguccgagt t 21

<210> 1326
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1326
cucggacagc auccaggact t 21

<210> 1327
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1327
agagcugcac gggcucacct t 21

<210> 1328
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1328
ggugagcccg ugcagcucut t 21

<210> 1329
<211> 650
<212> DNA
<213> Artificial Sequence

<220>
<223> Description of Artificial Sequence: Synthetic polynucleotide

<220>
<223> Description of Combined DNA/RNA Molecule: Synthetic polynucleotide

<400> 1329

**acagaagtcc actcattctt ggcaggatgg cttctcatcg tctgctcctc ctctgccttg 60**

**ctggactggt atttgtgtct gaggctggcc ctacgggcac cggtgaatcc aagtgtcctc 120**

**tgatggtcaa agttctagat gctgtccgag gcagtcctgc catcaatgtg gccgtgcatg 180**

**tgttcagaaa ggctgctgat gacacctggg agccatttgc ctctgggaaa accagtgagt 240**

**ctggagagct gcatgggctc acaactgagg aggaatttgt agaagggata tacaaagtgg 300**

**aaatagacac caaatcttac tggaaggcac ttggcatctc cccattccat gagcatgcag 360**

**aggtggtatt cacagccaac gactccggcc cccgccgcta caccattgcc gccctgctga 420**

**gcccctactc ctattccacc acggctgtcg tcaccaatcc caaggaatga gggacttctc 480**

**ctccagtgga cctgaaggac gagggatggg atttcatgta accaagagta ttccattttt 540**

**actaaagcag tgttttcacc tcatatgcta tgttagaagt ccaggcagag acaataaaac 600**

**attcctgtga aaggcacttt tcattccaaa aaaaaaaaaa aaaaaaaaaa 650**

<210> 1330
<211> 595
<212> DNA
<213> Artificial Sequence

<220>
<223> Description of Artificial Sequence: Synthetic polynucleotide

<400> 1330

```
cctgacagga tggcttccct tcgcctgttc ctcctctgcc tcgctggact gatatttgcg        60
tctgaagctg gccctggggg tgctggagaa tccaagtgtc ctctgatggt caaagtcctg       120
gatgctgtcc gaggcagccc tgctgtcgat gtggccgtga aagtgttcaa aaggactgca       180
gacggaagct gggagccgtt tgcctctggg aagaccgccg agtctggaga gctgcacggg       240
ctcaccacag atgagaagtt cacggaaggg gtgtacaggg tagaactgga caccaaatca       300
tactggaagg ctcttggcat ttccccattc catgaatacg cagaggtggt tttcacagcc       360
aatgactctg gtcatcgcca ctacaccatc gcagccctgc tcagcccgta ctcctacagc       420
accactgctg tcgtcagtaa cccccagaac tgagggaccc agcccacgag gaccaagatc       480
ttgccaaagc agtagctccc atttgtactg aaacagtgtt cttgctctat aaaccgtgtt       540

agcaactcgg gaagatgccg tgaaacgttc ttattaaacc acctttattt cattc            595
```

<210> 1331
<211> 938
<212> DNA
<213> Artificial Sequence

<220>
<223> Description of Artificial Sequence: Synthetic polynucleotide

<400> 1331

```
gttgactaag tcaataatca gaatcagcag gtttgcagtc agattggcag ggataagcag      60
cctagctcag gagaagtgag tataaaagcc ccaggctggg agcagccatc acagaagtcc     120
actcattctt ggcaggatgg cttctcatcg tctgctcctc ctctgccttg ctggactggt     180
atttgtgtct gaggctggcc ctacgggcac cggtgaatcc aagtgtcctc tgatggtcaa     240
agttctagat gctgtccgag gcagtcctgc catcaatgtg gccgtgcatg tgttcagaaa     300
ggctgctgat gacacctggg agccatttgc ctctgggaaa accagtgagt ctggagagct     360
gcatgggctc acaactgagg aggaatttgt agaagggata tacaaagtgg aaatagacac     420
caaatcttac tggaaggcac ttggcatctc cccattccat gagcatgcag aggtggtatt     480
cacagccaac gactccggcc cccgccgcta caccattgcc gccctgctga gcccctactc     540
ctattccacc acggctgtcg tcaccaatcc caaggaatga gggacttctc ctccagtgga     600
cctgaaggac gagggatggg atttcatgta accaagagta ttccattttt actaaagcag     660
tgttttcacc tcatatgcta tgttagaagt ccaggcagag acaataaaac attcctgtga     720
aaggcacttt tcattccact ttaacttgat tttttaaatt cccttattgt cccttccaaa     780
aaaaagagaa tcaaaatttt acaaagaatc aaaggaattc tagaaagtat ctgggcagaa     840
cgctaggaga gatccaaatt tccattgtct tgcaagcaaa gcacgtatta aatatgatct     900
gcagccatta aaaagacaca ttctgtaaaa aaaaaaaa                             938
```

<210> 1332
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1332
gggauuucau guaaccaagt t          21

<210> 1333
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1333
cuugguuaca ugaaauccct t          21

<210> 1334
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1334
ggauuucaug uaaccaagat t 21

<210> 1335
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1335
ucuugguuac augaaaucct t 21

<210> 1336
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1336
gauuucaugu aaccaagagt t 21

<210> 1337
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1337
cucuugguua caugaaauct t 21

<210> 1338
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1338
auuucaugua accaagagut t 21

<210> 1339
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1339
acucuugguu acaugaaaut t 21

<210> 1340
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1340
uuucauguaa ccaagaguat t 21

<210> 1341
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1341
uacucuuggu uacaugaaat t 21

<210> 1342
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1342
uucauguaac caagaguaut t 21

<210> 1343
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1343
auacucuugg uuacaugaat t 21

<210> 1344
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1344
ucauguaacc aagaguauut t 21

<210> 1345
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1345
aauacucuug guuacaugat t 21

<210> 1346
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1346
cauguaacca agaguauuct t 21

<210> 1347
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1347
gaauacucuu gguuacaugt t 21

<210> 1348
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1348
auguaaccaa gaguauucct t 21

<210> 1349
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1349
ggaauacucu ugguuacaut t 21

<210> 1350
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1350
uguaaccaag aguauuccat t 21

<210> 1351
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1351
uggaauacuc uugguuacat t 21

<210> 1352
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1352
uaaccaagag uauuccauut t 21

<210> 1353
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1353
aauggaauac ucuugguuat t 21

<210> 1354
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1354
aaccaagagu auuccauuut t 21

<210> 1355
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1355
aaauggaaua cucuugguut t 21

<210> 1356
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1356
accaagagua uuccauuuut t 21

<210> 1357
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1357
aaaauggaau acucuuggut t 21

<210> 1358
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1358
ccaagaguau uccauuuuut t 21

<210> 1359
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1359
aaaaauggaa uacucuuggt t 21

<210> 1360
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1360
caagaguauu ccauuuuuat t 21

<210> 1361
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1361
uaaaaaugga auacucuugt t 21

<210> 1362
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1362
aagaguauuc cauuuuuact t 21

<210> 1363
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1363
guaaaaaugg aauacucuut t 21

<210> 1364
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1364
agaguauucc auuuuuacut t 21

<210> 1365
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1365
aguaaaaaug gaauacucut t 21

<210> 1366
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1366
gaguauucca uuuuuacuat t 21

<210> 1367
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1367
uaguaaaaau ggaauacuct t 21

<210> 1368
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1368
aguauuccau uuuuacuaat t 21

<210> 1369
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1369
uuaguaaaaa uggaauacut t 21

<210> 1370
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1370
guauuccauu uuuacuaaat t 21

<210> 1371
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1371
uuuaguaaaa auggaauact t 21

<210> 1372
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1372
uauuccauuu uuacuaaagt t 21

<210> 1373
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1373
cuuuaguaaa aauggaauat t 21

<210> 1374
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1374
auuccauuuu uacuaaagct t 21

<210> 1375
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1375
gcuuuaguaa aaauggaaut t 21

<210> 1376
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1376
uuccauuuuu acuaaagcat t 21

<210> 1377
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1377
ugcuuuagua aaaauggaat t 21

<210> 1378
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1378
uccauuuuua cuaaagcagt t 21

<210> 1379
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1379
cugcuuuagu aaaaauggat t 21

<210> 1380
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1380
ccauuuuuac uaaagcagut t 21

<210> 1381
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1381
acugcuuuag uaaaaauggt t 21

<210> 1382
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1382
cauuuuuacu aaagcagugt t 21

<210> 1383
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1383
cacugcuuua guaaaaaugt t 21

<210> 1384
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1384
auuuuuacua aagcagugut t 21

<210> 1385
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1385
acacugcuuu aguaaaaaut t 21

<210> 1386
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1386
uuuuuacuaa agcaguguut t 21

<210> 1387
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1387
aacacugcuu uaguaaaaat t 21

<210> 1388
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1388
uuuuacuaaa gcaguguuut t 21

<210> 1389
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1389
aaacacugcu uuaguaaaat t 21

<210> 1390
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1390
uuuacuaaag caguguuuut t 21

<210> 1391
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1391
aaaacacugc uuuaguaaat t 21

<210> 1392
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1392
uuacuaaagc aguguuuuct t 21

<210> 1393
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1393
gaaaacacug cuuuaguaat t 21

<210> 1394
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1394
uacuaaagca guguuuucat t 21

<210> 1395
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1395
ugaaaacacu gcuuuaguat t 21

<210> 1396
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1396
acuaaagcag uguuuucact t 21

<210> 1397
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1397
gugaaaacac ugcuuuagut t 21

<210> 1398
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1398
cuaaagcagu guuuucacct t 21

<210> 1399
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1399
ggugaaaaca cugcuuuagt t 21

<210> 1400
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1400
uaaagcagug uuuucaccut t 21

<210> 1401
<211> 21

<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1401
aggugaaaac acugcuuuat t 21

<210> 1402
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1402
aaagcagugu uuucaccuct t 21

<210> 1403
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1403
gaggugaaaa cacugcuuut t 21

<210> 1404
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1404
aagcaguguu uucaccucat t 21

<210> 1405
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1405
ugaggugaaa acacugcuut t 21

<210> 1406
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1406
agcaguguuu ucaccucaut t 21

<210> 1407
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1407
augaggugaa aacacugcut t 21

<210> 1408
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1408
guaaccaaga guauuccaut t 21

<210> 1409
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1409
auggaauacu cuugguuact t 21

<210> 1410
<211> 19
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1410
gtaaccaa gagtattccat 19

<210> 1411
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1411
caguguucuu gcucuauaat t 21

<210> 1412
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1412
uuauagagca agaacacugt t 21

<210> 1413
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1413
ggauuucaug uaaccaagat t 21

<210> 1414
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1414
ucuugguuac augaaaucct t 21

<210> 1415
<211> 21
<212> DNA
<213> Artificial Sequence

<220>
<223> Description of Artificial Sequence: Synthetic primer

<400> 1415
cattcttggc aggatggctt c 21

<210> 1416
<211> 20
<212> DNA
<213> Artificial Sequence

<220>
<223> Description of Artificial Sequence: Synthetic primer

<400> 1416
ctcccaggtg tcatcagcag 20

<210> 1417
<211> 22
<212> DNA
<213> Artificial Sequence

<220>
<223> Description of Artificial Sequence: Synthetic primer

<400> 1417
tgcctcgctg gactgatatt tg 22

<210> 1418
<211> 21
<212> DNA
<213> Artificial Sequence

<220>
<223> Description of Artificial Sequence: Synthetic primer

<400> 1418
ttgaacactt tcacggccac a 21

<210> 1419
<211> 18
<212> DNA
<213> Artificial Sequence

<220>
<223> Description of Artificial Sequence: Synthetic primer

<400> 1419
gccgtgcatg tgttcaga 18

<210> 1420
<211> 22
<212> DNA
<213> Artificial Sequence

<220>
<223> Description of Artificial Sequence: Synthetic primer

<400> 1420
gctctccaga ctcactggtt tt 22

<210> 1421
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1421
caguguucuu gcucuauaat t 21

<210> 1422
<211> 21
<212> DNA
<213> Artificial Sequence

<220>
<223> Description of Artificial Sequence: Synthetic oligonucleotide

<220>
<223> Description of Combined DNA/RNA Molecule: Synthetic oligonucleotide

<400> 1422
uuauagagca agaacacugt t 21

<210> 1423
<211> 22
<212> DNA
<213> Artificial Sequence

<220>
<223> Description of Artificial Sequence: Synthetic primer

<400> 1423
tgcctcgctg gactgatatt tg 22

<210> 1424
<211> 21
<212> DNA
<213> Artificial Sequence

<220>
<223> Description of Artificial Sequence: Synthetic primer

<400> 1424
ttgaacactt tcacggccac a 21